# EUROPEAN PATENT APPLICATION

(11) **EP 3 882 239 A1**
(43) Date of publication of application: **22.09.2021**
(21) Application number: 19883405.3
(22) Date of filing: 14.11.2019
(51) Int. Cl.: C07D 271/113, A61K 31/4245, A61K 31/454, A61K 31/496, A61K 31/538, A61K 31/55, A61K 31/553, A61P 37/00, A61P 43/00, C07D 413/04, C07D 413/10, C07D 413/12, C07D 413/14, C07D 487/10

(54) **1,3,4-OXADIAZOLONE COMPOUND AND MEDICINE**

(30) Priority: 15.11.2018 JP 2018214950
(71) Applicant: Nippon Shinyaku Co., Ltd., Kyoto-shi Kyoto 601-8550 (JP)
(72) Inventor: KAMITANI Hirotaka, Kamakura-shi, Kanagawa 247-0056 (JP); ZAIMOKU Hisaaki, Kyoto-shi, Kyoto 601-8550 (JP); HARUTA Yoshinari, Kyoto-shi, Kyoto 601-8550 (JP); KIKUCHI Takeo, Kyoto-shi, Kyoto 601-8550 (JP)
(74) Representative: Vossius & Partner Patentanwälte Rechtsanwälte mbB
(86) International application number: PCT/JP2019/044615
(87) International publication number: WO 2020/100959

(57) **Abstract**

The purpose of the present invention is to provide a compound having PIM inhibitory activity.

Examples of the present invention include 1,3,4-oxadiazolone compounds represented by the following formula [1], and pharmaceutically acceptable salts, and solvates thereof. 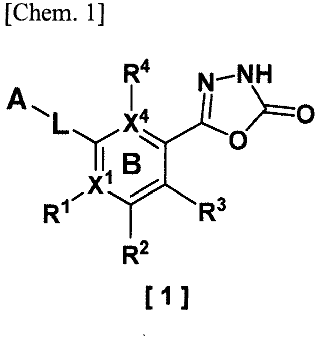 The compounds of the present invention have PIM inhibitory activity. In addition, since the compounds of the present invention have PIM inhibitory activity, the compounds of the present invention are useful as therapeutic agents for systemic lupus erythematosus, lupus nephritis, etc.

## Description

### TECHNICAL FIELD

The present invention relates to a 1,3,4-oxadiazolone compound and a pharmaceutical.

### BACKGROUND ART

Protein kinases are enzymes that phosphorylate proteins and control various biological functions such as cell proliferation, survival, differentiation, and organogenesis. The PIM kinase family includes protein kinases that phosphorylate a serine group and a threonine group, and consists of three types, PIM1, PIM2, and PIM3. Although the substrate proteins and functions recognized by PIM1, PIM2, and PIM3 overlap, differences in expression tissues therebetween are recognized. The functions of PIM kinases are known to be involved in transcription and translation and to control cell proliferation and survival (see, for example, NON-PATENT DOCUMENT 1). Also, unlike other kinases that require phosphorylation for activation, PIM kinases are characterized by being constitutively activated. It is known that the expression of PIM kinases is induced by cytokines and growth factors, and the induction by cytokines is mediated by the JAK/STAT pathway. In addition, it is also known to share substrates such as BAD and 4EBP1 with the PI3K/AKT pathway involved in cell survival (see, for example, NON-PATENT DOCUMENT 2). Since PIM kinases act downstream of the JAK/STAT pathway and share substrates with the PI3K/AKT pathway as described above, PIM inhibitors are considered to have a drug efficacy similar to that of inhibitors for the above two pathways.

Studies on gene-deficient mice have reported that PIM1, PIM2, and PIM3 triple gene-deficient mice have reduced individual sizes but are viable (see, for example, NON-PATENT DOCUMENT 3). Therefore, PIM inhibitors are presumed to have a good safety profile. In addition, PIM kinases are known to be involved in immune response and inflammatory reaction, and are expected to be effective for immune disorders and inflammatory diseases in consideration of the safety profile of PIM inhibitors. Specifically, PIM kinases are considered to be effective for diseases such as multiple sclerosis (see, for example, PATENT DOCUMENT 1), rheumatoid arthritis (see, for example, NON PATENT DOCUMENT 4), food allergy (see, for example, NON PATENT DOCUMENT 5), asthma (see, for example, NON PATENT DOCUMENT 6), systemic lupus erythematosus (see, for example, PATENT DOCUMENT 1, NON PATENT DOCUMENT 4), lupus nephritis (see, for example, PATENT DOCUMENT 1, NON PATENT DOCUMENT 4), inflammatory bowel disease (see, for example, NON PATENT DOCUMENT 7), ulcerative colitis (see, for example, NON PATENT DOCUMENT 8), atopic dermatitis (see, for example, NON PATENT DOCUMENT 9), autoimmune lymphoproliferative syndrome (see, for example, PATENT DOCUMENT 1), chronic obstructive pulmonary disease (see, for example, NON PATENT DOCUMENT 10), allergic airway disease (see, for example, NON PATENT DOCUMENT 11), eosinophilic polyangiitis granulomatosis (see, for example, NON PATENT DOCUMENT 9), hypereosinophilic syndrome (see, for example, NON PATENT DOCUMENT 9), chorioamnionitis (see, for example, NON PATENT DOCUMENT 12), ankylosing spondylitis (see, for example, NON PATENT DOCUMENT 4), myasthenia gravis (see, for example, NON PATENT DOCUMENT 13), psoriasis (see, for example, PATENT DOCUMENT 14).

PIM kinases have been reported to be highly expressed in a wide range of hematological cancers and solid cancers and are involved in pathogenesis. For example, prostate cancer (see, for example, NON PATENT DOCUMENT 15), colon cancer (see, for example, NON PATENT DOCUMENT 16, NON PATENT DOCUMENT 17), esophageal cancer (see, for example, NON PATENT DOCUMENT 18, NON PATENT DOCUMENT 19), ovarian cancer (see, for example, NON PATENT DOCUMENT 20), uterine cancer (see, for example, NON PATENT DOCUMENT 21, NON PATENT DOCUMENT 22, NON PATENT DOCUMENT 23), renal cancer (see, for example, NON PATENT DOCUMENT 24), liver cancer (see, for example, NON PATENT DOCUMENT 25), pancreatic cancer (see, for example, NON PATENT DOCUMENT 26), gastric cancer (see, for example, NON PATENT DOCUMENT 27), breast cancer (see, for example, NON PATENT DOCUMENT 28), lung cancer (see, for example, NON PATENT DOCUMENT 29, NON PATENT DOCUMENT 30), head and neck cancer (see, for example, NON PATENT DOCUMENT 31), glioma (see, for example, NON PATENT DOCUMENT 32, NON PATENT DOCUMENT 33), osteosarcoma (see, for example, NON PATENT DOCUMENT 34, NON PATENT DOCUMENT 35, NON PATENT DOCUMENT 36), bladder cancer (see, for example, NON PATENT DOCUMENT 37), acute lymphocytic leukemia (see, for example, NON PATENT DOCUMENT 38), acute myeloid leukemia (see, for example, NON PATENT DOCUMENT 39), chronic lymphocytic leukemia (see, for example, NON PATENT DOCUMENT 40), chronic myeloid leukemia (see, for example, NON PATENT DOCUMENT 41), B-cell lymphoma (see, for example, NON PATENT DOCUMENT 42, NON PATENT DOCUMENT 43, NON PATENT DOCUMENT 44), multiple myeloma (see, for example, NON PATENT DOCUMENT 45, NON PATENT DOCUMENT 46), T-cell lymphoma (see, for example, NON PATENT DOCUMENT 47), skin cancer (see, for example, NON PATENT DOCUMENT 48), Kaposi's sarcoma (see, for example, NON PATENT DOCUMENT 49), Hodgkin's lymphoma (see, for example, NON PATENT DOCUMENT 50), myeloproliferative tumor (see, for example, NON PATENT DOCUMENT 51), adenoid cystic carcinoma (see, for example, NON PATENT DOCUMENT 52), Ewing's sarcoma (see, for example, NON PATENT DOCUMENT 53), adult T-cell leukemia (see, for example, NON PATENT DOCUMENT 54), mesothelioma (see, for example, NON PATENT DOCUMENT 55), acute promyelocytic leukemia (see, for example, NON PATENT DOCUMENT 56), choriocarcinoma (see, for example, NON PATENT DOCUMENT 57), liposarcoma (see, for example, NON PATENT DOCUMENT 58), neuroblastoma (see, for example, NON PATENT DOCUMENT 59), seminoma (see, for example, NON PATENT DOCUMENT 60), lymphoblastic lymphoma (see, for example, NON PATENT DOCUMENT 46) etc., are known. Due to the above, PIM inhibitors are useful for the treatment of these cancers.

Moreover, PIM kinases are located downstream of the JAK/STAT pathway, and thus can be expected to be effective for diseases in which an abnormality is found in the JAK/STAT pathway. Examples of such diseases include Crohn's disease, irritable bowel syndrome, pancreatitis, diverticulosis, Basedow's disease, juvenile rheumatoid arthritis, osteoarthritis, psoriatic arthritis, vasculitis, autoimmune thyroiditis, dermatitis, scleroderma, leukoplakia, graft-versus-host disease, Sjogren's syndrome, and glomerulonephritis.

PIM kinases are also known to be involved in infectious diseases. For example, for Epstein-Barr virus infection and hemophagocytic syndrome in which Epstein-Barr virus is known to be involved (see, for example, NON PATENT DOCUMENT 61), influenza (see, for example, NON PATENT DOCUMENT 62), hepatitis C (see, for example, NON PATENT DOCUMENT 63), salmonellosis (see, for example, NON PATENT DOCUMENT 64), herpesvirus infection (see, for example, NON PATENT DOCUMENT 65), vaginal trichomonas infection (see, for example, NON PATENT DOCUMENT 66), human granulocytic ehrlichiosis (see, for example, NON PATENT DOCUMENT 67). In addition, PIM kinases have also been reported to contribute to the pathological conditions of aplastic anemia (see, for example, NON PATENT DOCUMENT 68), atherosclerosis (see, for example, NON PATENT DOCUMENT 69, NON PATENT DOCUMENT 70), pulmonary hypertension (see, for example, NON PATENT DOCUMENT 71), diabetes (see, for example, NON PATENT DOCUMENT 69, NON PATENT DOCUMENT 70), enlarged prostate (see, for example, NON PATENT DOCUMENT 72), Alzheimer's disease (see, for example, NON PATENT DOCUMENT 73) suggesting the usefulness of PIM inhibitors.

PIM kinases have been reported to have an autoantibody production inhibitory effect (see, for example, PATENT DOCUMENT 1). Therefore, PIM kinases can be expected to be effective for nephrosis syndrome, polymyositis, dermatomyositis, mixed connective tissue disease, dilated cardiomyopathy, idiopathic thrombocytopenic purpura, thrombotic thrombocytopenic purpura, pemphigus, pemphigoid, and neuromyelitis optica, in all of which autoantibodies are involved.

### CITATION LIST

### [PATENT DOCUMENT]

[PATENT DOCUMENT 1] WO2010/022076 A1

### [NON-PATENT DOCUMENT]

[NON-PATENT DOCUMENT 1] Nawijn et al., Nat. Rev. Cancer, 2011, 11, 23-34.
[NON-PATENT DOCUMENT 2] Mondello et al., J. Hematol. Oncol., 2014, 7, 95.
[NON-PATENT DOCUMENT 3] Mikkers et al., Mol. Cell. Biol., 2004, 24, 6104-6115.
[NON-PATENT DOCUMENT 4] Lin et al., Int. Immunopharmacol., 2015, 28, 859-865.
[NON-PATENT DOCUMENT 5] Wang et al., J. Allergy Clin. Immunol., 2012, 130, 932-944.
[NON-PATENT DOCUMENT 6] Vries et al., Eur. Respir. J., 2016, 47, 783-791.
[NON-PATENT DOCUMENT 7] Jackson et al., Cell Immunol., 2012, 272, 200-213.
[NON-PATENT DOCUMENT 8] Shen et al., Dig. Dis. Sci., 2012, 57, 1822-1831.
[NON-PATENT DOCUMENT 9] Andina et al., J. Allergy. Clin. Immunol., 2009, 123, 603-611.
[NON-PATENT DOCUMENT 10] Yang et al., Pathol. Res. Pract., 2017, 213, 322-326.
[NON-PATENT DOCUMENT 11] Shin et al., Am. J. Respir. Cell Mol. Biol., 2012, 46, 488-497.
[NON-PATENT DOCUMENT 12] Lim et al., Mol. Hum. Reprod., 2017, 23, 428-440.
[NON-PATENT DOCUMENT 13] Egli et al., PLoS One, 2015, 10, e0142741.
[NON-PATENT DOCUMENT 14] Perera et al., Sci. Transl. Med., 2014, 6, 223ra22.
[NON-PATENT DOCUMENT 15] Kirschner et al., J. Natl. Cancer. Inst., 2014, 107, dju407.
[NON-PATENT DOCUMENT 16] Weirauch et al., Neoplasia, 2013, 15, 783-794.
[NON-PATENT DOCUMENT 17] Peng et al., PLoS One, 2013, 8, e76693.
[NON-PATENT DOCUMENT 18] Li et al., Oncol. Rep., 2010, 24, 997-1004.
[NON-PATENT DOCUMENT 19] Liu et al., J. Surg. Oncol., 2010, 102, 683-688.
[NON-PATENT DOCUMENT 20] Xie et al., Zhong Nan Da Xue Bao Yi Xue Ban, 2014, 39, 649-657.
[NON-PATENT DOCUMENT 21] Rimon et al., Int. J. Oncol., 2004, 24, 1325-1338.
[NON-PATENT DOCUMENT 22] Jimenez-Garcia et al., Oncotarget, 2017, 8, 58872-58886.
[NON-PATENT DOCUMENT 23] Liu et al., Oncotarget, 2015, 6, 8019-8035.
[NON-PATENT DOCUMENT 24] Mahalingam et al., Br. J. Cancer, 2011, 105, 1563-1573.
[NON-PATENT DOCUMENT 25] Fujii et al., Int. J. Cancer, 2005, 114, 209-218.
[NON-PATENT DOCUMENT 26] Li et al., Cancer Res., 2006, 66, 6741-6747.
[NON-PATENT DOCUMENT 27] Warnecke-Eberz et al., Anticancer Res., 2009, 29, 4451-4455.
[NON-PATENT DOCUMENT 28] Braso-Maristany et al., Nat. Med., 2016, 22, 1303-1313.
[NON-PATENT DOCUMENT 29] Kim et al., Pharmacol. Res., 2013, 70, 90-101.
[NON-PATENT DOCUMENT 30] Jin et al., PLoS One, 2012, 7, e48575.
[NON-PATENT DOCUMENT 31] Peltola et al., Neoplasia, 2009, 11, 629-636.
[NON-PATENT DOCUMENT 32] Herzog et al., Neuro-Oncol., 2015, 17, 223-242.
[NON-PATENT DOCUMENT 33] Iqbal et al., Oncotarget, 2016, 7, 33192-33201.
[NON-PATENT DOCUMENT 34] Mou et al., Int. J. Oncol., 2016, 49, 2116-2126.
[NON-PATENT DOCUMENT 35] Liao et al., J. Orthop. Res., 2016, 34, 1185-1194.
[NON-PATENT DOCUMENT 36] Narlik-Grassow et al., Carcinogenesis., 2012, 33, 1479-1486.
[NON-PATENT DOCUMENT 37] Foulks et al., Neoplasia, 2014, 16, 403-412.
[NON-PATENT DOCUMENT 38] Padi et al., Oncotarget, 2017, 8, 30199-30216.
[NON-PATENT DOCUMENT 39] Burger et al., J. Med. Chem., 2015, 58, 8373-8386.
[NON-PATENT DOCUMENT 40] Chen et al., Blood, 2009, 114, 4150-4157.
[NON-PATENT DOCUMENT 41] Fan et al., Mol. Med. Rep., 2017, 16, 4603-4612.
[NON-PATENT DOCUMENT 42] Kuo et al., Am. J. Cancer Res., 2016, 6, 2489-2501.
[NON-PATENT DOCUMENT 43] Yang et al., Blood, 2012, 120, 3491-3500.
[NON-PATENT DOCUMENT 44] Hsi et al., Leuk. Lymphoma, 2008, 49, 2081 - 2090.
[NON-PATENT DOCUMENT 45] Keane et al., Blood Cancer J., 2015, 5, e325.
[NON-PATENT DOCUMENT 46] Paino et al., Clin. Cancer Res., 2017, 23, 225-238.
[NON-PATENT DOCUMENT 47] Martin-Sanchez et al., PLoS One, 2014, 9, e112148.
[NON-PATENT DOCUMENT 48] Shannan et al., Oncotarget, 2016, 7, 54897-54912.
[NON-PATENT DOCUMENT 49] Bajaj et al., Virology, 2006, 351, 18-28.
[NON-PATENT DOCUMENT 50] Szydlowski et al., Blood, 2017, 130, 1418-1429.
[NON-PATENT DOCUMENT 51] Mazzacurati et al., Oncotarget, 2015, 6, 40141-40157.
[NON-PATENT DOCUMENT 52] Xu et al., Cancer Cell Int., 2018, 18, 22.
[NON-PATENT DOCUMENT 53] Mukaida et al., Cancer Sci., 2011, 102, 1437-1442.
[NON-PATENT DOCUMENT 54] Bellon et al., Blood, 2016, 127, 2439-2450.
[NON-PATENT DOCUMENT 55] Mawas et al., Int. J. Oncol., 2017, 50, 1029-1034.
[NON-PATENT DOCUMENT 56] Zhang et al., Pharmacol. Rep., 2017, 69, 1270-1281.
[NON-PATENT DOCUMENT 57] Mary Photini et al., Exp. Cell Res., 2017, 359, 275-283.
[NON-PATENT DOCUMENT 58] Nga et al., Int. J. Exp. Pathol., 2010, 91, 34-43.
[NON-PATENT DOCUMENT 59] Brunen et al., Mol. Cancer Ther., 2018, 17, 849-857.
[NON-PATENT DOCUMENT 60] Jimenez-Garcia et al., Sci. Rep., 2016, 6, 38079.
[NON-PATENT DOCUMENT 61] Rainio et al., Virology, 2005, 333, 201-206.
[NON-PATENT DOCUMENT 62] de Vries et al., Eur. Respir. J., 2015, 45, 1745-1748.
[NON-PATENT DOCUMENT 63] Park et al., J. Virol., 2015, 89, 10073-10086.
[NON-PATENT DOCUMENT 64] Rogers et al., Sci. Signal., 2011, 4, rs9.
[NON-PATENT DOCUMENT 65] Cheng et al., PLoS Pathog., 2009, 5, e1000324.
[NON-PATENT DOCUMENT 66] Sutcliffe et al., PLoS Pathog., 2012, 8, e1002801.
[NON-PATENT DOCUMENT 67] Lee et al., Genomics, 2006, 88, 496-503.
[NON-PATENT DOCUMENT 68] Chiocchetti et al., Haematologica, 2005, 90, 1453-1462.
[NON-PATENT DOCUMENT 69] Wang et al., Oncotarget, 2017, 8, 88320-88331.
[NON-PATENT DOCUMENT 70] Kaneto et al., Mediators Inflamm., 2010, 2010, 453892.
[NON-PATENT DOCUMENT 71] Paulin et al., Circulation, 2011, 123, 1205-1215.
[NON-PATENT DOCUMENT 72] He et al., Med. Oncol., 2009, 26, 303-308.
[NON-PATENT DOCUMENT 73] Velazquez et al., Mol. Neurodegener., 2016, 11,52.

### SUMMARY OF THE INVENTION

### PROBLEMS TO BE SOLVED BY THE INVENTION

An object of the present invention is to provide a compound having PIM kinase inhibitory activity.

As a result of intensive studies, the inventors discovered that a 1,3,4-oxadiazolone compound represented by the following general formula [1] or a pharmaceutically acceptable salt thereof, or a solvate thereof, which may be herein referred to as a "compound of the present invention", has PIM kinase inhibitory activity, and achieved the prevent invention.

That is, disclosed herein are the following (item 1) to (item 14).
(Item 1) A 1,3,4-oxadiazolone compound of the formula [1]: 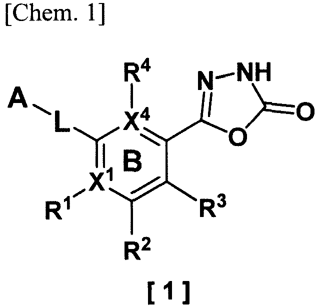 wherein
   X¹ is a carbon atom or a nitrogen atom,
   when X¹ is a carbon atom, R¹ is a hydrogen atom, a halogen atom, alkyl, alkenyl, a non-aromatic carbocyclic group, dihaloalkyl, trihaloalkyl, alkoxy, dihaloalkoxy, trihaloalkoxy, alkylsulfonyl, cyano, an aromatic carbocyclic group, or an aromatic heterocyclic group,
   when X¹ is a nitrogen atom, R¹ does not exist,
   R² is a hydrogen atom, a halogen atom, alkyl, a non-aromatic carbocyclic group, trihaloalkyl, pentafluorosulfanyl (SF₅), cyano, amino, or nitro,
   R¹ and R² optionally combine with adjacent atoms to form an indazole ring,
   R³ is a hydrogen atom, a halogen atom, or alkyl,
   X⁴ is a carbon atom or a nitrogen atom,
   when X⁴ is a carbon atom, R⁴ is a hydrogen atom, a halogen atom, or alkyl,
   when X⁴ is a nitrogen atom, R⁴ does not exist,
   both X¹ and X⁴ are not nitrogen atoms at the same time,
   L is a bond, an alkylene, an alkenylene, an alkynylene, or a group represented by L-1, L-2, L-3, or L-4: 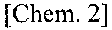 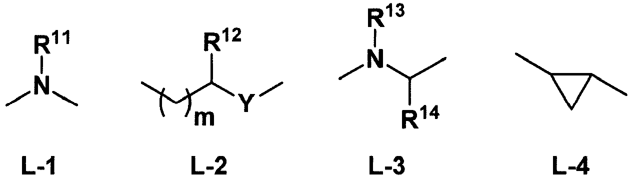 wherein the bond on the left side of each group is attached to A in the formula [1], the bond on the right side of each group is attached to a ring B in the formula [1], R¹¹, R¹³, and R¹⁴ are each a hydrogen atom or alkyl, R¹² is a hydrogen atom, alkyl, monohaloalkyl, dihaloalkyl, or trihaloalkyl, R¹³ is a hydrogen atom or alkyl, Y is O, S, or -NR¹⁵- (R¹⁵ is a hydrogen atom or alkyl), and m is 0, 1, or 2,
   R¹ and R¹⁵ (if L is L-2 and Y is -NR¹⁵-) combine with adjacent atoms to form a group represented by z-1, z-2, or z-3: 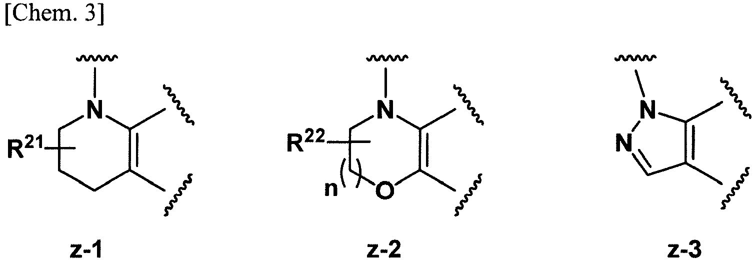 wherein R²¹ is a hydrogen atom, oxo (=O), or an alkoxyimino (=N-O-R²³), n is 1 or 2, and R²² is a hydrogen atom or alkyl,
      A represents aminoalkylamino, a non-aromatic heterocyclic group, a non-aromatic carbocyclic group, an aromatic carbocyclic group, an aromatic heterocyclic group, or 1,3-dioxa-2-yl,
      the non-aromatic heterocyclic group for A is optionally substituted with one or two groups selected from the group consisting of the following (1) to (7):
         (1) amino (-NH₂),
         (2) alkyl,
         (3) aminoalkyl,
         (4) alkyl substituted with amino and hydroxy,
         (5) halogen,
         (6) alkylcarbonyl, and
         (7) alkoxycarbonyl,
      the non-aromatic carbocyclic group for A is optionally substituted with 1 to 3 groups selected from the group consisting of the following (1) to (15):
         (1) amino,
         (2) alkyl,
         (3) alkylamino substituted with a non-aromatic carbocyclic group,
         (4) trihaloalkylamino,
         (5) hydroxyalkyl,
         (6) aminoalkyl,
         (7) hydroxy,
         (8) monoalkylamino,
         (9) hydroxyalkylamino,
         (10) alkoxycarbonyl,
         (11) carboxyl,
         (12) carbamoyl,
         (13) acetamide (Me-C(=O)-NH-),
         (14) piperazinyl, and
         (15) alkylamino,
      the aromatic carbocyclic group for A is optionally substituted with one group selected from the group consisting of the following (1) to (4):
         (1) aminoalkyl,
         (2) aminoalkoxy,
         (3) alkoxy substituted with piperidinyl, and
         (4) alkoxycarbonylaminoalkyl,
      the aromatic heterocyclic group for A is optionally substituted with a piperazinyl group, and
      A and L are selected from any of the following cases (a) to (h):
         (a) when L is a bond,
            A is aminoalkylamino, a non-aromatic heterocyclic group, an aromatic carbocyclic group, or an aromatic heterocyclic group,
         (b) when L is an alkylene,
            A is a non-aromatic heterocyclic group or a non-aromatic carbocyclic group,
         (c) when L is an alkenylene,
            A is a non-aromatic heterocyclic group,
         (d) when L is an alkynylene,
            A is a non-aromatic heterocyclic group,
         (e) when L is L-1,
            A is a non-aromatic heterocyclic group, a non-aromatic carbocyclic group, or an aromatic carbocyclic group,
         (f) when L is L-2,
            A is a non-aromatic heterocyclic group or a non-aromatic carbocyclic group,
         (g) when L is L-3,
            A is a non-aromatic heterocyclic group, and
         (h) when L is L-4,
            A is a non-aromatic heterocyclic group,
   or a pharmaceutically acceptable salt thereof, or a solvate thereof.
(Item 2) The 1,3,4-oxadiazolone compound according to Item 1, or a pharmaceutically acceptable salt thereof, or a solvate thereof, wherein X¹ is a carbon atom, and X² is a carbon atom.
(Item 3) The 1,3,4-oxadiazolone compound according to Item 1 or 2, or a pharmaceutically acceptable salt thereof, or a solvate thereof, wherein L is a bond, an alkylene, an alkenylene, an alkynylene, L-1, or L-2.
(Item 4) The 1,3,4-oxadiazolone compound according to any one of Items 1 to 3, or a pharmaceutically acceptable salt thereof, or a solvate thereof, wherein A is aminoalkylamino, a non-aromatic heterocyclic group, a non-aromatic carbocyclic group, an aromatic carbocyclic group, or an aromatic heterocyclic group.
(Item 5) The 1,3,4-oxadiazolone compound according to any one of Items 1 to 4, or a pharmaceutically acceptable salt thereof, or a solvate thereof, wherein L is a bond, L-1, or L-2.
(Item 6) The 1,3,4-oxadiazolone compound according to any one of Items 1 to 5, or a pharmaceutically acceptable salt thereof, or a solvate thereof, wherein A and L are any of groups of the following (aa), (ee), and (ff):
   (aa) when L is a bond, A is aminoalkylamino, a non-aromatic heterocyclic group, an aromatic carbocyclic group, or an aromatic heterocyclic group,
   (ee) when L is L-1, A is a non-aromatic heterocyclic group or a non-aromatic carbocyclic group, or
   (ff) when L is L-2, A is a non-aromatic heterocyclic group or a non-aromatic carbocyclic group.
(Item 7) The 1,3,4-oxadiazolone compound according to any one of Items 1 to 5, or a pharmaceutically acceptable salt thereof, or a solvate thereof, wherein L is L-2, and A is a non-aromatic heterocyclic group or a non-aromatic carbocyclic group.
(Item 8) The 1,3,4-oxadiazolone compound according to Item 7, or a pharmaceutically acceptable salt thereof, or a solvate thereof, wherein L is L-2, m is 0, Y is -NR¹⁵-, and A is a non-aromatic heterocyclic group or a non-aromatic carbocyclic group.
(Item 9) The 1,3,4-oxadiazolone compound according to any one of Items 1 to 8, or a pharmaceutically acceptable salt thereof, or a solvate thereof, wherein
   the non-aromatic heterocyclic group for A is piperidinyl, piperazinyl, pyrrolidinyl, azepanyl, azocanyl, 1,3-dioxanyl, tetrahydrofuranyl, 6-azaspiro[2.5]octanyl, 3,9-diazaspiro[5.5]undecanyl, 2,7-diazaspiro[3.5]nonan-7-yl, 7-azaspiro[3.5]nonanyl, 3-azabicyclo[3.2.1]octanyl, or 2-azaspiro[3.3]heptan-6-yl,
   the non-aromatic carbocyclic group for A is cyclohexanyl, cyclopentyl, cyclobutenyl, bicyclo[2.2.1]heptanyl, bicyclo[1.1.1]pentanyl, cuban-1-yl, or 2-azaspiro[3.3]heptanyl,
   the aromatic carbocyclic group for A is phenyl, and
   the aromatic heterocyclic group for A is pyridyl.
(Item 10) The 1,3,4-oxadiazolone compound according to Item 1, or a pharmaceutically acceptable salt thereof, or a solvate thereof, wherein
   X¹ is a carbon atom,
   R¹ is a halogen atom, dihaloalkyl, trihaloalkyl, dihaloalkyl, or trihaloalkoxy,
   R² is a halogen atom or trihaloalkyl,
   R³ is a hydrogen atom,
   X⁴ is a carbon atom,
   R⁴ is a hydrogen atom,
   L is L-2,
   m is 0,
   Y is NR¹⁵,
   R¹⁵ is a hydrogen atom,
   R¹² is a hydrogen atom or alkyl, and
   A is piperidinyl, piperazinyl, pyrrolidinyl, azepanyl, azocanyl, 1,3-dioxanyl, tetrahydrofuranyl, 6-azaspiro[2.5]octanyl, 3,9-diazaspiro[5.5]undecanyl, 2,7-diazaspiro[3.5]nonan-7-yl, 7-azaspiro[3.5]nonanyl, 3-azabicyclo[3.2.1]octanyl, or 2-azaspiro[3.3]heptan-6-yl.
(Item 11) The 1,3,4-oxadiazolone compound according to any one of Items 1 to 9, or a pharmaceutically acceptable salt thereof, or a solvate thereof, wherein the 1,3,4-oxadiazolone compound is any one of the following compounds (1) to (254):
   (1) 5- {3-[(4-aminobutyl)amino]-4-(trifluoromethyl)phenyl}-1,3,4-oxadiazol-2(3H)-one,
   (2) 5-{3-[(3 -aminopropyl)amino]-4-(trifluoromethyl)phenyl)-1,3,4-oxadiazol-2(3H)-one,
   (3) 5-{3-[(5-aminopentyl)amino]-4-(trifluoromethyl)phenyl}-1,3,4-oxadiazol-2(3H)-one,
   (4) 5-{3-[(6-aminohexyl)amino]-4-(trifluoromethyl)phenyl}-1,3,4-oxadiazol-2(3H)-one,
   (5) 5-{3-[(6-aminohexan-2-yl)amino]-4-(trifluoromethyl)phenyl}-1 ,3,4-oxadiazol-2(3H)-one,
   (6) 5-{3-[4-(aminomethyl)piperidin-1-yl]-4-chlorophenyl}-1,3,4-oxadiazol-2(3H)-one,
   (7) tert-butyl 4-[2-chloro-5-(5-oxo-4,5-dihydro-1,3,4-oxadiazol-2-yl)phenyl]piperazine-1-carboxylate,
   (8) 5-[4-chloro-3-(piperazin-1-yl)phenyl]-1,3,4-oxadiazol-2(3H)-one,
   (9) 5-[3-(4-aminopiperidin-1-yl)-4-(trifluoromethyl)phenyl]-1,3,4-oxadiazol-2(3H)-one,
   (10) 5- {3-[4-(2-aminoethyl)piperidin-1-yl]-4-(trifluoromethyl)phenyl}-1,3,4-oxadiazol-2(3H)-one,
   (11) 5-{3-[3-(2-aminoethyl)piperidin-1-yl]-4-(trifluoromethyl)phenyl}-1,3,4-oxadiazol-2(3H)-one,
   (12) 5-{4-[4-(2-aminoethyl)piperidin-1-yl]-1H-indazol-6-yl} -1,3,4-oxadiazol-2(3H)-one,
   (13) 5-{3-[4-(1-amino-2-methylpropan-2-yl)piperidin-1-yl]-4-(trifluoromethyl)phenyl}-1,3,4-oxadiazol-2(3H)-one,
   (14) 5-13-[4-(2-amino-1-hydroxyethyl)piperidin-1-yl]-4-(trifluoromethyl)phenyl} - 1,3,4-oxadiazol-2(3H)-one,
   (15) 5-[3-(3,9-diazaspiro[5.5]undecan-3-yl)-4-(trifluoromethyl)phenyl]-1,3,4-oxadiazol-2(3H)-one,
   (16) 5-[3-(2,7-diazaspiro[3.5]nonan-7-yl)-4-(trifluoromethyl)phenyl]-1,3,4-oxadiazol-2(3H)-one,
   (17) tert-butul{[2'-chloro-5'-(5-oxo-4,5-dihydro-1,3,4-oxadiazol-2-yl)[1,1'-biphenyl]-3-yl]methyl} carbamate,
   (18) 5-[3'-(aminomethyl)-6-chloro[1,1'-biphenyl]-3-yl]-1,3,4-oxadiazol-2(3H)-one,
   (19) tert-butyl {[5'-(5-oxo-4,5-dihydro-1,3,4-oxadiazol-2-yl)-2'-(trifluoromethyl)[1,1'-biphenyl]-4-yl]methyl}carbamate,
   (20) 5-[4'-(aminomethyl)-6-(trifluoromethyl)[1,1'-biphenyl]-3-yl]-1,3,4-oxadiazol-2(3H)-one,
   (21) 5-[3'-(aminomethyl)-6-(trifluoromethyl)[1,1'-biphenyl]-3-yl]-1,3,4-oxadiazol-2(3H)-one,
   (22) 5-[4'-(2-aminoethyl)-6-(trifluoromethyl)[1,1'-biphenyl]-3-yl]-1,3,4-oxadiazol-2(3H)-one,
   (23) 5-{4'-[(piperidin-4-yl)methoxy]-6-(trifluoromethyl)[1,1'-biphenyl]-3-yl}-1,3,4-oxadiazol-2(3H)-one,
   (24) 5-[4'-{[(2S)-1-aminopropan-2-yl]oxy} -6-(trifluoromethyl) [1,1'-biphenyl]-3-yl] - 1,3,4-oxadiazol-2(3H)-one,
   (25) 5-{3-[5-(piperazin-1-yl)pyridin-3-yl]-4-(trifluoromethyl)phenyl)-1,3,4-oxadiazol-2(3H)-one,
   (26) 5-{3-[2-(piperidin-4-yl)ethyl]-4-(trifluoromethyl)phenyl}-1,3,4-oxadiazol-2(3H)-one,
   (27) 5-[3-{2-[(1r,4s)-4-aminocyclohexyl]ethyl}-4-(trifluoromethyl)phenyl]-1,3,4-oxadiazol-2(3H)-one,
   (28) 5-[3-{2-[(2r,5r)-5-amino-l,3-dioxan-2-yl]ethyl}-4-(trifluoromethyl)phenyl]-1,3,4-oxadiazol-2(3 H)-one,
   (29) 5-{3-[(piperidin-4-yl)ethynyl]-4-(trifluoromethyl)phenyl}-1,3,4-oxadiazol-2(3H)-one,
   (30) 5-{3-[(E)-2-(piperidin-4-yl)ethenyl]-4-(trifluoromethyl)phenyl}-1,3,4-oxadiazol-2(3 H)-one,
   (31) 5-{4-chloro-3-[(piperidin-4-yl)amino]phenyl}-1,3,4-oxadiazol-2(3H)-one,
   (32) 5-(3-{[(1r,4r)-4-aminocyclohexyl]amino}-4-chlorophenyl)-1,3,4-oxadiazol-2(3H)-one,
   (33) 5-(3-{[(1s,4s)-4-aminocyclohexyl]amino}-4-chlorophenyl)-1,3,4-oxadiazol-2(3H)-one,
   (34) 5-[3-{[(1r,4r)-4-aminocyclohexyl]amino}-4-(trifluoromethyl)phenyl]-1,3,4-oxadiazol-2(3H)-one,
   (35) 5-(3-{[(1r,4r)-4-aminocyclohexyl]amino}-4-bromophenyl)-1,3,4-oxadiazol-2(3H)-one,
   (36) 5-[3- {[(1r,4r)-4-aminocyclohexyl]amino} -5-fluoro-4-(trifluoromethyl)phenyl]-1,3,4-oxadiazol-2(3H)-one ,
   (37) 5-[3- {[(1r,4r)-4-(aminomethyl)cyclohexyl] amino} -4-(trifluoromethyl)phenyl]-1,3,4-oxadiazol-2(3H)-one,
   (38) 5-[3-{[(1r,4r)-4-aminocyclohexyl]amino}-4-(trifluoromethoxy)phenyl]-1,3,4-oxadiazol-2(3H)-one,
   (39) 5-[3-{[(1r,4r)-4-(1-aminoethyl)cyclohexyl]amino}-4-(trifluoromethyl)phenyl]-1,3,4-oxadiazol-2(3H)-one,
   (40) 5-(3-{ [(1r,4r)-4-aminocyclohexyl]amino}-4-chloro-5-fluorophenyl)-1 ,3,4-oxadiazol-2(3H)-one,
   (41) 5-{3-[4-(aminomethyl)anilino]-4-(trifluoromethyl)phenyl)-1,3,4-oxadiazol-2(3H)-one,
   (42) 5-{3-[(6-azaspiro[2.5] octan-1-yl)amino]-4-(trifluoromethyl)phenyl}-1,3,4-oxadiazol-2(3H)-one,
   (43) 5-{3-[(6-aminospiro[3.3]heptan-2-yl)amino]-4-(trifluoromethyl)phenyl}-1,3,4-oxadiazol-2(3H)-one,
   (44) 5-[3-{[(1r,4r)-4-(2-aminoethyl)cyclohexyl]amino}-4-(trifluoromethyl)phenyl]-1,3,4-oxadiazol-2(3H)-one,
   (45) 5-[3-{ [(1S)-7-azaspiro[3.5]nonan-1-yl]amino}-4-(trifluoromethyl)phenyl]-1,3,4-oxadiazol-2(3H)-one,
   (46) 5-[3-{[(1R)-7-azaspiro [3.5] nonan-1-yl]amino}-4-(trifluoromethyl)phenyl]-1,3,4-oxadiazol-2(3H)-one,
   (47) 5-(4-chloro-3-{[(piperidin-4-yl)methyl]amino}phenyl)-1,3,4-oxadiazol-2(3H)-one,
   (48) 5-[3-{[(piperidin-4-yl)methyl]amino}-4-(trifluoromethyl)phenyl]-1,3,4-oxadiazol-2(3H)-one,
   (49) 5-[4-chloro-3-({[(3R)-pyrrolidin-3-yl]methyl}amino)phenyl]-1,3,4-oxadiazol-2(3H)-one,
   (50) 5-(4-bromo-3-{[(piperidin-4-yl)methyl]amino}phenyl)-1,3,4-oxadiazol-2(3H)-one,
   (51) 5-[3-{methyl[(piperidin-4-yl)methyl]amino}-4-(trifluoromethyl)phenyl]-1,3,4-oxadiazol-2(3H)-one,
   (52) 5-[3-{[1-(piperidin-4-yl)ethyl]amino}-4-(trifluoromethyl)phenyl]-1,3,4-oxadiazol-2(3H)-one,
   (53) 5-[3-({[(3S)-piperidin-3-yl]methyl}amino)-4-(trifluoromethyl)phenyl]-1,3,4-oxadiazol-2(3H)-one,
   (54) 5-[3-({[(3R)-piperidin-3-yl]methyl}amino)-4-(trifluoromethyl)phenyl]-1,3,4-oxadiazol-2(3H)-one,
   (55) 5-[3-({[(1r,4r)-4-aminocyclohexyl]methyl}amino)-4-(trifluoromethyl)phenyl]-1,3,4-oxadiazol-2(3H)-one,
   (56) 5-[3-{[1-(piperidin-4-yl)propyl]amino}-4-(trifluoromethyl)phenyl]-1,3,4-oxadiazol-2(3H)-one,
   (57) 5-[3-{[(4-methylpiperidin-4-yl)methyl]amino}-4-(trifluoromethyl)phenyl]-1,3,4-oxadiazol-2(3H)-one,
   (58) 5-[3-{[(1S)-1-(piperidin-4-yl)ethyl]amino}-4-(trifluoromethyl)phenyl]-1,3,4-oxadiazol-2(3H)-one,
   (59) 5-[3-{ [(1R)-1-(piperidin-4-yl)ethyl]amino}-4-(trifluoromethyl)phenyl]-1,3,4-oxadiazol-2(3H)-one,
   (60) 5-[3-{[2-(piperidin-3-yl)ethyl]amino}-4-(trifluoromethyl)phenyl]-1,3,4-oxadiazol-2(3H)-one,
   (61) 5-[3-({[(1r,4r)-4-aminocyclohexyl]methyl}amino)-4-chlorophenyl]-1,3,4-oxadiazol-2(3H)-one,
   (62) 5-[3-({[(1r,4r)-4-aminocyclohexyl]methyl}amino)phenyl]-1,3,4-oxadiazol-2(3H)-one,
   (63) 5-[3-({1-[(1r,4r)-4-aminocyclohexyl]ethyl}amino)-4-(trifluoromethyl)phenyl]-1,3,4-oxadiazol-2(3H)-one,
   (64) 5-[3-{[2-(piperidin-4-yl)ethyl]amino}-4-(trifluoromethyl)phenyl]-1,3,4-oxadiazol-2(3 H)-one,
   (65) 5-[3-{[2-(piperazin-1-yl)ethyl]amino}-4-(tritluoromethyl)phenyl]-1,3,4-oxadiazol-2(3H)-one,
   (66) 5-[3-({[(1r,4r)-4-aminocyclohexyl]methyl}amino)-4-(trifluoromethoxy)phenyl] - 1,3,4-oxadiazol-2(3H)-one,
   (67) 5-[3-({[(1r,4r)-4-aminocyclohexyl]methyl} amino)-4-methylphenyl]-1 ,3,4-oxadiazol-2(3H)-one,
   (68) 5-[3-({[(1r,4r)-4-aminocyclohexyl]methyl}amino)-4-bromophenyl]-1,3,4-oxadiazol-2(3H)-one,
   (69) 5-[3-({[(1r,4r)-4-aminocyclohexyl]methyl}amino)-5-fluoro-4-(trifluoromethyl)phenyl]-1,3,4-oxadiazol-2(3H)-one,
   (70) 5-[3-{[2-(4-methylpiperidin-4-yl)ethyl]amino}-4-(trifluoromethyl)phenyl]-1,3,4-oxadiazol-2(3H)-one,
   (71) 5-[2-({[(1r,4r)-4-aminocyclohexyl]methyl}amino)[1,1'-biphenyl]-4-yl]-1,3,4-oxadiazol-2(3H)-one,
   (72) 5-[3-({2-[(3R)-3-aminopiperidin-1-yl]ethyl}amino)-4-(trifluoromethyl)phenyl]-1,3,4-oxadiazol-2(3H)-one,
   (73) 5-[3-({2-[(3S)-3-aminopiperidin-1-yl]ethyl}amino)-4-(trifluoromethyl)phenyl]-1,3,4-oxadiazol-2(3H)-one,
   (74) 5-[3-({[(1s,4s)-4-aminocyclohexyl]methyl} amino)-4-(trifluoromethyl)phenyl]-1,3,4-oxadiazol-2(3H)-one,
   (75) 5-[3-fluoro-5-{[(1S)-1-(piperidin-4-yl)ethyl]amino}-4-(trifluoromethyl)phenyl]-1,3,4-oxadiazol-2(3H)-one,
   (76) 5-(4-chloro-3-fluoro-5-{[(1S)-1-(piperidin-4-yl)ethyl]amino}phenyl)-1,3,4-oxadiazol-2(3H)-one,
   (77) 5-(4-chloro-3-{[(1S)-1-(piperidin-4-yl)ethyl]aminophenyl)-1,3,4-oxadiazol-2(3H)-one,
   (78) 5-(4-bromo-3-{[(1S)-1-(piperidin-4-yl)ethyl]amino}phenyl)-1,3,4-oxadiazol-2(3H)-one,
   (79) 5-(3,4-dichloro-5-{ [(1S)-1-(piperidin-4-yl)ethyl]amino}phenyl)-1,3,4-oxadiazol-2(3H)-one,
   (80) 5-(4-fluoro-3-{[(1S)-1-(piperidin-4-yl)ethyl]amino}phenyl)-1,3,4-oxadiazol-2(3H)-one,
   (81) 5-[3-{[1-(piperidin-4-yl)propan-2-yl]amino}-4-(trifluoromethyl)phenyl]-1,3,4-oxadiazol-2(3H)-one,
   (82) 5-(4-methoxy-3-{[(1S)-1-(piperidin-4-yl)ethyl]amino}phenyl)-1,3,4-oxadiazol-2(3H)-one,
   (83) 5-(4-bromo-3-fluoro-5-1[(1S)-1-(piperidin-4-yl)ethyl]amino}phenyl)-1,3,4-oxadiazol-2(3H)-one,
   (84) 5-(4-chloro-3-methyl-5-{[(1S)-1-(piperidin-4-yl)ethyl]amino}phenyl)-1,3,4-oxadiazol-2(3H)-one,
   (85) 4-(5-oxo-4,5-dihydro-1,3,4-oxadiazol-2-yl)-2-{[(1S)-1-(piperidin-4-yl)ethyl]amino}benzonitrile,
   (86) 5-[3-({[(1r,4r)-4-aminocyclohexyl]methyl}amino)-4-chloro-5-fluorophenyl]-1,3,4-oxadiazol-2(3H)-one,
   (87) 5-(4,5-dichloro-2-fluoro-3-{[(1S)-1-(piperidin-4-yl)ethyl]amino}phenyl)-1,3,4-oxadiazol-2(3H)-one,
   (88) 5-(4-chloro-2,5-difluoro-3-{[(1S)-1-(piperidin-4-yl)ethyl]amino}phenyl)-1,3,4-oxadiazol-2(3H)-one,
   (89) 5-(3,4-difluoro-5-{ [(1S)-1-(piperidin-4-yl)ethyl]amino}phenyl)-1,3,4-oxadiazol-2(3H)-one,
   (90) 5-[4-(difluoromethyl)-3-{[(1S)-1-(piperidin-4-yl)ethyl]amino}phenyl]-1,3,4-oxadiazol-2(3 H)-one,
   (91) 5-(4-chloro-3 -nitro-5-{[(1S)-1-(piperidin-4-yl)ethyl]amino}phenyl)-1,3,4-oxadiazol-2(3H)-one,
   (92) 5-(3-amino-4-chloro-5-{[(1S)-1-(piperidin-4-yl)ethyl]amino}phenyl)-1,3,4-oxadiazol-2(3H)-one,
   (93) 5-(4,5-dichloro-2-methyl-3-{[(1S)-1-(piperidin-4-yl)ethyl]amino}phenyl)-1,3,4-oxadiazol-2(3H)-one,
   (94) 5-(4-chloro-2-methyl-5-{[(1S)-1-(piperidin-4-yl)ethyl]amino}phenyl)-1,3,4-oxadiazol-2(3H)-one,
   (95) 5-(4-chloro-2-fluoro-5-{[(1S)-1-(piperidin-4-yl)ethyl] amino}phenyl)-1,3,4-oxadiazol-2(3H)-one,
   (96) 5-(2,4-dichloro-5-{[(1S)-1-(piperidin-4-yl)ethyl]amino}phenyl)-1,3,4-oxadiazol-2(3H)-one,
   (97) 5-(3-bromo-4-chloro-5-{[(1S)-1-(piperidin-4-yl)ethyl]amino}phenyl)-1,3,4-oxadiazol-2(3H)-one,
   (98) 5-(3-chloro-4-methyl-5-{[(1S)-1-(piperidin-4-yl)ethyl]amino}phenyl)-1,3,4-oxadiazol-2(3H)-one,
   (99) 5-(3-fluoro-4-methyl-5-{[(1S)-1-(piperidin-4-yl)ethyl]amino}phenyl)-1,3,4-oxadiazol-2(3 H)-one,
   (100) 5-(4-{[(1S)-1-(piperidin-4-yl)ethyl]amino}-1H-indazol-6-yl)-1,3,4-oxadiazol-2(3H)-one,
   (101) 5-[4-chloro-3-{[(1S)-1-(piperidin-4-yl)ethyl]amino}-5 -(trifluoromethyl)phenyl]-1,3,4-oxadiazol-2(3H)-one,
   (102) 5-(4-chloro-3-cyclopropyl-5-{[(1 S)-1-(piperidin-4-yl)ethyl]amino}phenyl)-1,3,4-oxadiazol-2(3 H)-one,
   (103) 5-(3-chloro-5-{[(1S)-1-(piperidin-4-yl)ethyl]amino}-4-[(propan-2-yl)oxy]phenyl)-1,3,4-oxadiazol-2(3H)-one,
   (104) 5-(3-chloro-4-methoxy-5-{[(1S)-1-(piperidin-4-yl)ethyl]amino}phenyl)-1,3,4-oxadiazol-2(3H)-one,
   (105) 5-(3-chloro-5-{[(1S)-1-(piperidin-4-yl)ethyl]amino}phenyl)-1,3,4-oxadiazol-2(3H)-one,
   (106) 5-[3-({(1S)-1-[(1r,4S)-4-aminocyclohexyl]ethyl}amino)-4-(trifluoromethyl)phenyl]-1,3 ,4-oxadiazol-2(3H)-one,
   (107) 5-[3-(pentafluoro-λ6-sulfanyl)-5-{[(1S)-1-(piperidin-4-yl)ethyl]amino}phenyl]-1,3,4-oxadiazol-2(3H)-one,
   (108) 5-[3-{[(4-fluoropiperidin-4-yl)methyl]amino}-4-(trifluoromethyl)phenyl]-1,3,4-oxadiazol-2(3H)-one,
   (109) 5-[3-{[(3-fluoropiperidin-3-yl)methyl]amino}-4-(trifluoromethyl)phenyl]-1,3,4-oxadiazol-2(3H)-one,
   (110) 2-chloro-5-(5-oxo-4,5-dihydro-1,3,4-oxadiazol-2-yl)-3-{[(1S)-1-(piperidin-4-yl)ethyl]amino}benzonitrile,
   (111) 5-[3-({(1R)-1-[(1r,4R)-4-aminocyclohexyl]ethyl}amino)-4-(trifluoromethyl)phenyl]-1,3 ,4-oxadiazol-2(3H)-one,
   (112) 5-(2-{[(1 S)-1 -(piperidin-4-yl)ethyl]amino}pyridin-4-yl)-1 ,3,4-oxadiazol-2(3H)-one,
   (113) 5-[3-{[(1R)-2,2-difluoro-1-(piperidin-4-yl)ethyl]amino}-4-(trifluoromethyl)phenyl]-1,3,4-oxadiazol-2(3H)-one,
   (114) 5-[6-{[(1S)-1-(piperidin-4-yl)ethyl]amino}-5-(trifluoromethyl)pyridin-2-yl]-1,3,4-oxadiazol-2(3H)-one,
   (115) 5-[3-{[1-(4-methylpiperidin-4-yl)ethyl]amino}-4-(trifluoromethyl)phenyl]-1,3,4-oxadiazol-2(3H)-one,
   (116) 5-(4-{[(4-fluoropiperidin-4-yl)methyl]amino}-1H-indazol-6-yl)-1,3,4-oxadiazol-2(3H)-one,
   (117) 5-[3-({2-[(1r,4r)-4-aminocyclohexyl]ethyl}amino)-4-(trifluoromethyl)phenyl]-1,3,4-oxadiazol-2(3H)-one,
   (118) tert-butyl(1r,4r)-4-{[5-(5-oxo-4,5-dihydro-1,3,4-oxadiazol-2-yl)-2-(trifluoromethyl)anilino]methyl}cyclohexane-1-carboxylate,
   (119) (1r,4r)-4-{[5-(5-oxo-4,5-dihydro-1,3,4-oxadiazol-2-yl)-2-(trifluoromethyl)anilino]methyl}cyclohexane-1-carboxylicacid,
   (120) 5-[3-({[(1r,4r)-4-(hydroxymethyl)cyclohexyl]methyl}amino)-4-(trifluoromethyl)phenyl]-1,3,4-oxadiazol-2(3H)-one,
   (121) (1r,4r)-4-{[5-(5-oxo-4,5-dihydro-1,3,4-oxadiazol-2-yl)-2-(trifluoromethyl)anilino]methyl} cyclohexane-1 -carboxamide,
   (122) 5-[3-{[(4-ethylpiperidin-4-yl)methyl]amino}-4-(trifluoromethyl)phenyl]-1,3,4-oxadiazol-2(3H)-one,
   (123) 5-(4-chloro-3-fluoro-5-{ [(4-fluoropiperidin-4-yl)methyl]amino}phenyl)-1,3,4-oxadiazol-2(3H)-one,
   (124) 5- {4-bromo-1-[(piperidin-4-yl)methyl]-1H-indazol-6-yl}-1,3,4-oxadiazol-2(3H)-one,
   (125) 5-[3-{ [(1S)-1-(4-fluoropiperidin-4-yl)ethyl]amino}-4-(trifluoromethyl)phenyl]-1,3,4-oxadiazol-2(3H)-one,
   (126) 5-[3-({[(3 S,4R)-3-fluoropiperidin-4-yl]methyl} amino)-4-(trifluoromethyl)phenyl]-1,3,4-oxadiazol-2(3H)-one,
   (127) 5-[3-({[(3S,4S)-3-fluoropiperidin-4-yl]methyl}amino)-4-(trifluoromethyl)phenyl]-1,3,4-oxadiazol-2(3H)-one,
   (128) 5-[4-(methanesulfonyl)-3-{[(1S)-1-(piperidin-4-yl)ethyl]amino}phenyl]-1,3,4-oxadiazol-2(3H)-one,
   (129) 5-[3-fluoro-5-{[(4-fluoropiperidin-4-yl)methyl]amino}-4-(trifluoromethyl)phenyl]-1,3,4-oxadiazol-2(3H)-one,
   (130) 5-{3-[{[(1r,4r)-4-aminocyclohexyl]methyl}(methyl)amino]-4-(trifluoromethyl)phenyl}-1,3,4-oxadiazol-2(3H)-one,
   (131) 5-[3-({[(1r,4r)-4-(methylamino)cyclohexyl]methyl}amino)-4-(trifluoromethyl)phenyl]-1,3,4-oxadiazol-2(3H)-one,
   (132) 5-[3-(methyl{[(1r,4r)-4-(methylamino)cyclohexyl]methyl}amino)-4-(trifluoromethyl)phenyl]-1,3,4-oxadiazol-2(3H)-one,
   (133) 5-[3-{[(3 -azabicyclo[3.2.1]octan-8-yl)methyl]amino}-4-(trifluoromethyl)phenyl]-1,3,4-oxadiazol-2(3H)-one,
   (134) 5-[3-({1-[(3S)-pyrrolidin-3-yl]propan-2-yl}amino)-4-(trifluoromethyl)phenyl]-1,3,4-oxadiazol-2(3H)-one,
   (135)5-[3-({[(1R,3s,5S)-8-azabicyclo[3.2.1]octan-3-yl]methyl}amino)-4-(trifluoromethyl)phenyl]-1,3,4-oxadiazol-2(3H)-one,
   (136) 5-[3-({1-[(3R)-pyrrolidin-3-yl]propan-2-yl}amino)-4-(trifluoromethyl)phenyl]-1,3,4-oxadiazol-2(3H)-one,
   (137) 5-[3-({(1S)-1-[(1R,3S)-3-amino-2,2-dimethylcyclobutyl]ethyl}amino)-4-(trifluoromethyl)phenyl]-1,3,4-oxadiazol-2(3H)-one,
   (138) 5-[3-{[(1S)-1-(piperidin-4-yl)propyl]amino}-4-(trifluoromethyl)phenyl]-1,3,4-oxadiazol-2(3 H)-one,
   (139) 5-(4-chloro-3-fluoro-5-{[(1S)-1-(piperidin-4-yl)propyl]amino}phenyl)-1 ,3,4-oxadiazol-2(3H)-one,
   (140) 5-[3-{[(1R)-2,2-difluoro-1-(4-fluoropiperidin-4-yl)ethyl]amino}-4-(trifluoromethyl)phenyl]-1,3,4-oxadiazol-2(3H)-one,
   (141) 5-[3-({[(3R,4R)-3-methylpiperidin-4-yl]methyl}amino)-4-(trifluoromethyl)phenyl]-1,3,4-oxadiazol-2(3H)-one,
   (142) 5-[3-{[(1S)-1-(7-azaspiro[3.5]nonan-2-yl)ethyl]amino}-4-(trifluoromethyl)phenyl]-1,3,4-oxadiazol-2(3H)-one,
   (143) 5-[3-{[(2-methylpiperidin-4-yl)methyl]amino}-4-(trifluoromethyl)phenyl]-1,3,4-oxadiazol-2(3H)-one,
   (144) 5-[3-{[(1S)-1-(4-methylpiperidin-4-yl)ethyl]amino}-4-(trifluoromethyl)phenyl]-1,3,4-oxadiazol-2(3H)-one,
   (145) 5-[3-({(1S)-1-[(1s,3R)-3-aminocyclobutyl]ethyl}amino)-4-(trifluoromethyl)phenyl]-1,3,4-oxadiazol-2(3H)-one,
   (146) 5-[4-(trifluoromethyl)-3-{[(1R)-2,2,2-trifluoro-1-(piperidin-4-yl)ethyl]amino}phenyl]-1,3,4-oxadiazol-2(3H)-one,
   (147) 5-[4-(trifluoromethyl)-3-{[(1S)-2,2,2-trifluoro-1-(piperidin-4-yl)ethyl]amino}phenyl]-1,3,4-oxadiazol-2(3H)-one,
   (148) 5-[3-({(1S)-1-[(1r,4S)-4-aminocyclohexyl]ethyl}amino)-4-chlorophenyl]-1,3,4-oxadiazol-2(3H)-one,
   (149) 5-[3-{[(1S)-1-(2-azaspiro[3.3]heptan-6-yl)ethyl]amino}-4-(trifluoromethyl)phenyl]-1,3,4-oxadiazol-2(3H)-one,
   (150) 5-[3-({(1S)-1-[(1R,3S)-3-amino-2,2-dimethylcyclobutyl]ethyl}amino)-5-fluoro-4-(trifluoromethyl)phenyl]-1,3,4-oxadiazol-2(3H)-one,
   (151) 5-[3-({(1S)-1-[(1r,4S)-4-aminocyclohexyl]ethyl}amino)-5-fluoro-4-(trifluoromethyl)phenyl]-1,3,4-oxadiazol-2(3H)-one,
   (152) 5-[3-{[(1R)-2-fluoro-1-(piperidin-4-yl)ethyl]amino}-4-(trifluoromethyl)phenyl]-1,3,4-oxadiazol-2(3H)-one,
   (153) 5-[3-({(1S)-1-[(1S,3R)-3-amino-2,2-dimethylcyclobutyl]ethyl}amino)-4-(trifluoromethyl)phenyl]-1,3,4-oxadiazol-2(3H)-one,
   (154) 5-[3-({(1S)-1-[(1r,4S)-4-aminocyclohexyl]ethyl}amino)-4-bromophenyl]-1,3,4-oxadiazol-2(3H)-one,
   (155) 5-[3-({(1S)-1-[(1R,5S,8r)-3-azabicyclo[3.2.1]octan-8-yl]ethyl}amino)-4-(trifluoromethyl)phenyl]-1,3,4-oxadiazol-2(3H)-one,
   (156) 5-[3-({(1S)-1-[(1R,5S,8s)-3-azabicyclo[3.2.1]octan-8-yl]ethyl}amino)-4-(trifluoromethyl)phenyl]-1,3,4-oxadiazol-2(3H)-one,
   (157) 5-[3-{[(1S)-1-(azepan-4-yl)ethyl]amino}-4-(trifluoromethyl)phenyl]-1,3,4-oxadiazol-2(3H)-one,
   (158) 5-[3-({(1S)-1-[(1R,3S)-3-amino-2,2-dimethylcyclobutyl]ethyl}amino)-4-chlorophenyl]-1,3,4-oxadiazol-2(3H)-one,
   (159) 5-[3-fluoro-5-{[(1S)-1-(4-fluoropiperidin-4-yl)ethyl]amino}-4-(trifluoromethyl)phenyl]-1,3,4-oxadiazol-2(3H)-one,
   (160) 5-[3-fluoro-5- {[(1R)-2-fluoro-1-(piperidin-4-yl)ethyl]amino}-4-(trifluoromethyl)phenyl]-1,3,4-oxadiazol-2(3H)-one,
   (161) 5-[3-{2-[(2r,5r)-5-amino-1,3-dioxan-2-yl]cyclopropyl}-4-(trifluoromethyl)phenyl]-1,3,4-oxadiazol-2(3H)-one,
   (162) 5-[3-{[(1S)-1-(azocan-5-yl)ethyl]amino}-4-(trifluoromethyl)phenyl]-1,3,4-oxadiazol-2(3H)-one,
   (163) 5-[3-{[(1R)-2,2-difluoro-1-(4-fluoropiperidin-4-yl)ethyl]amino}-5-fluoro-4-(trifluoromethyl)phenyl]-1,3,4-oxadiazol-2(3H)-one,
   (164) 5-[3-({(1S)-1-[(1S,3R)-3-amino-2,2-dimethylcyclobutyl]ethyl}amino)-5-fluoro-4-(trifluoromethyl)phenyl]-1,3,4-oxadiazol-2(3H)-one,
   (165) 5-[3-({(1S)-1-[(1s,3R)-3-aminocyclobutyl]ethyl}amino)-5-fluoro-4-(trifluoromethyl)phenyl]-1,3,4-oxadiazol-2(3H)-one,
   (166) 5-[3-({[(1R,3S)-3-amino-2,2-dimethylcyclobutyl]methyl}amino)-4-(trifluoromethyl)phenyl]-1,3,4-oxadiazol-2(3H)-one,
   (167) 5-[3-({(1S)-1-[(1r,4S)-4-aminocyclohexyl]ethyl}amino)-5-fluoro-4-methoxyphenyl]-1,3,4-oxadiazol-2(3H)-one,
   (168) 5-[3-({(1S)-1-[(1S,3R)-3-amino-2,2-dimethylcyclobutyl]ethyl}amino)-4-chlorophenyl]-1,3,4-oxadiazol-2(3H)-one,
   (169) 5-[3-({(1S)-1-[(1r,4S)-4-aminocyclohexyl]ethyl}amino)-4-chloro-2,5-difluorophenyl]-1,3,4-oxadiazol-2(3H)-one,
   (170) 5-[3-({(1S)-1-[(1r,4S)-4-aminocyclohexyl]ethyl}amino)-5-fluoro-4-(trifluoromethoxy)phenyl]-1,3,4-oxadiazol-2(3H)-one,
   (171) 5-[3-({(1S)-1-[(1r,4S)-4-aminocyclohexyl]ethyl}amino)-5-bromo-4-chlorophenyl]-1,3,4-oxadiazol-2(3H)-one,
   (172) 5-[3-({(1S)-1-[(1r,4S)-4-aminocyclohexyl]ethyl}amino)-5-fluoro-4-methylphenyl]-1,3,4-oxadiazol-2(3H)-one,
   (173) 5-[3-({(1S)-1-[(1r,4S)-4-aminocyclohexyl]ethyl}amino)-4-ethoxy-5-fluorophenyl]-1,3,4-oxadiazol-2(3H)-one,
   (174) 5-[3-({(1S)-1-[(1r,4S)-4-aminocyclohexyl]ethyl}amino)-4,5-dichlorophenyl]-1,3,4-oxadiazol-2(3H)-one,
   (175) 5-{3-({(1S)-1-[(1r,4S)-4-aminocyclohexyl]ethyl}amino)-5-fluoro-4-[(propan-2-yl)oxy]phenyl}-1,3,4-oxadiazol-2(3H)-one,
   (176) 5-[3-({[(1S,3R)-3-amino-2,2-dimethylcyclobutyl]methyl}amino)-4-(trifluoromethyl)phenyl]-1,3,4-oxadiazol-2(3H)-one,
   (177) 5-[3-({(1S)-1-[(1r,4S)-4-aminocyclohexyl]ethyl}amino)-4-chloro-5-fluorophenyl]-1,3,4-oxadiazol-2(3H)-one,
   (178) 5-[3-({(1S)-1-[(1s,3R)-3-aminocyclobutyl]ethyl}amino)-4-chloro-5-fluorophenyl]-1,3,4-oxadiazol-2(3H)-one,
   (179) 5-[3-({(1S)-1-[(1s,3R)-3-aminocyclobutyl]ethyl}amino)-4,5-dichlorophenyl]-1,3,4-oxadiazol-2(3H)-one,
   (180) 5-[3-({(1S)-1-[(1s,3R)-3-aminocyclobutyl]ethyl}amino)-5-fluoro-4-(trifluoromethoxy)phenyl]-1,3,4-oxadiazol-2(3H)-one,
   (181) 5-[3-({(1S)-1-[(1r,4S)-4-aminocyclohexyl]propyl}amino)-4-(trifluoromethyl)phenyl]-1,3,4-oxadiazol-2(3H)-one,
   (182) 5-[3-({(1S)-1-[(1s,3R)-3-aminocyclobutyl]ethyl}amino)-5-bromo-4-chlorophenyl]-1,3,4-oxadiazol-2(3H)-one,
   (183) 5-[3-({(1S)-1-[(1s,3R)-3-aminocyclobutyl]ethyl}amino)-4-chloro-5-(trifluoromethyl)phenyl]-1,3,4-oxadiazol-2(3H)-one,
   (184) N-[(1S,3R)-2,2-dimethyl-3-{[5-(5-oxo-4,5-dihydro-1,3,4-oxadiazol-2-yl)-2-(trifluoromethyl)anilino]methyl}cyclobutyl]acetamide
   (185) 5-[3-{[(1S)-1-(3-aminobicyclo[1.1.1]pentan-1-yl)ethyl]amino}-4-(trifluoromethyl)phenyl]-1,3,4-oxadiazol-2(3H)-one,
   (186) 5-[3-({(1S)-1-[(2S,3R)-4-aminocuban-1-yl]ethyl}amino)-4-(trifluoromethyl)phenyl]-1,3,4-oxadiazol-2(3H)-one,
   (187) 5-[3-{[(1S)-1-(4-aminobicyclo[2.2.1]heptan-1-yl)ethyl]amino}-4-(trifluoromethyl)phenyl]-1,3,4-oxadiazol-2(3H)-one,
   (188) 5-[3-({(S)-1-[(2S,5R)-5-aminotetrahydro-2H-pyran-2-yl]ethyl}amino)-4-(trifluoromethyl)phenyl]-1,3,4-oxadiazol-2(3H)-one,
   (189) 5-[3-({(1S)-1-[(1r,4S)-4-(methylamino)cyclohexyl]ethyl}amino)-4-(trifluoromethyl)phenyl]-1,3,4-oxadiazol-2(3H)-one,
   (190) 5-[3-fluoro-5-({(1S)-1-[(1r,4S)-4-(methylamino)cyclohexyl]ethyl}amino)-4-(trifluoromethyl)phenyl]-1,3,4-oxadiazol-2(3H)-one,
   (191) 5-[3-({(1S)-1-[(1R,3S)-3-aminocyclohexyl]ethyl}amino)-4-(tritluoromethyl)phenyl]-1,3,4-oxadiazol-2(3H)-one,
   (192) 5-[3-({(1S)-1-[(1s,3R)-3-(methylamino)cyclobutyl]ethyl}amino)-4-(trifluoromethyl)phenyl]-1,3,4-oxadiazol-2(3H)-one,
   (193) 5-[3-({(1S)-1-[(1r,4S)-4-(ethylamino)cyclohexyl]ethyl}amino)-4-(trifluoromethyl)phenyl]-1,3,4-oxadiazol-2(3H)-one,
   (194) 5-[3-({(1S)-1-[(1S,3R)-3-aminocyclohexyl]ethyl}amino)-4-(trifluoromethyl)phenyl]-1,3,4-oxadiazol-2(3H)-one,
   (195) 5-[3-({(1S)-1-[(1s,3R)-3-(ethylamino)cyclobutyl]ethyl}amino)-4-(trifluoromethyl)phenyl]-1,3,4-oxadiazol-2(3H)-one,
   (196) 5-[3-{[(1S)-1-{(1s,3R)-3-[(cyclopropylmethyl)amino]cyclobutyl}ethyl]amino}-4-(trifluoromethyl)phenyl]-1,3 ,4-oxadiazol-2(3H)-one,
   (197) 5-[3-{[(1S)-1-{(1r,4S)-4-[(2,2,2-trifluoroethyl)amino]cyclohexyl}ethyl]amino}-4-(trifluoromethyl)phenyl]-1,3 ,4-oxadiazol-2(3H)-one,
   (198) 5-[3-({(1S)-1-[(1r,4S)-4-aminocyclohexyl]propyl}amino)-5-fluoro-4-(trifluoromethyl)phenyl]-1,3,4-oxadiazol-2(3H)-one,
   (199) 5-[3-({(1S)-1-[(1S,3R)-3-aminocyclopentyl]ethyl}amino)-4-(trifluoromethyl)phenyl]-1,3,4-oxadiazol-2(3H)-one,
   (200) 5-[3-({(1S)-1-[(1R,3S)-3-aminocyclopentyl]ethyl}amino)-4-(trifluoromethyl)phenyl]-1,3,4-oxadiazol-2(3H)-one,
   (201) 5-[3-{[(1S)-1-{(1r,4S)-4-[(2-hydroxyethyl)amino]cyclohexyl}ethyl]amino}-4-(trifluoromethyl)phenyl]-1,3,4-oxadiazol-2(3H)-one,
   (202) 5-[3-fluoro-5-{[(1S)-1-(piperidin-4-yl)ethyl]amino}-4-(trifluoromethoxy)phenyl]-1,3,4-oxadiazol-2(3H)-one,
   (203) 5-[3-fluoro-5-({(1S)-1-[(1s,3R)-3-(methylamino)cyclobutyl]ethyl}amino)-4-(trifluoromethyl)phenyl]-1,3,4-oxadiazol-2(3H)-one,
   (204) 5-[3-fluoro-5-({(1S)-1-[(1r,4S)-4-(methylamino)cyclohexyl]ethyl} amino)-4-(tritluoromethoxy)phenyl]-1,3,4-oxadiazol-2(3H)-one,
   (205) 5-(4-chloro-3-{[methyl(piperidin-4-yl)amino]methyl}phenyl)-1,3,4-oxadiazol-2(3H)-one,
   (206) 5-[3-fluoro-5-{[(1S)-1-(4-methylpiperidin-4-yl)ethyl]amino}-4-(trifluoromethyl)phenyl]-1,3,4-oxadiazol-2(3H)-one,
   (207) 5-[3-fluoro-5-{[(1S)-1-(4-methylpiperidin-4-yl)ethyl] amino}-4-(trifluoromethoxy)phenyl]-1,3,4-oxadiazol-2(3H)-one,
   (208) 5-[3-fluoro-5-({(1S)-1-[(ls,3R)-3-(methylamino)cyclobutyl]ethyl}amino)-4-(trifluoromethoxy)phenyl]-1,3,4-oxadiazol-2(3H)-one,
   (209) 5-[3-{[(1S)-2-fluoro-1-(4-fluoropiperidin-4-yl)ethyl]amino} -4-(trifluoromethyl)phenyl]-1,3 ,4-oxadiazol-2(3H)-one,
   (210) 5-[3-fluoro-5-({(1S)-1-[(3S,4S)-3-methylpiperidin-4-yl]ethyl}amino)-4-(trifluoromethyl)phenyl]-1,3,4-oxadiazol-2(3H)-one,
   (211) 5-[3-fluoro-5-({(1S)-1-[(3R,4R)-3-methylpiperidin-4-yl]ethyl}amino)-4-(trifluoromethyl)phenyl]-1,3,4-oxadiazol-2(3H)-one,
   (212) 5-[4-(difluoromethoxy)-3-fluoro-5-{[(1S)-1-(piperidin-4-yl)ethyl]amino}phenyl]-1,3,4-oxadiazol-2(3H)-one,
   (213) 5-[4-(difluoromethoxy)-3-fluoro-5-{[(1S)-1-(4-methylpiperidin-4-yl)ethyl]amino}phenyl]-1,3,4-oxadiazol-2(3H)-one,
   (214) 5-[4-(difluoromethoxy)-3-fluoro-5-({(1S)-1-[(3S,4S)-3-methylpiperidin-4-yl]ethyl}amino)phenyl]-1,3,4-oxadiazol-2(3H)-one,
   (215) 5-(3-{[(1-acetylpiperidin-4-yl)(methyl)amino]methyl}-4-chlorophenyl)-1,3,4-oxadiazol-2(3H)-one,
   (216) 5-[4-(difluoromethoxy)-3-fluoro-5-({(1S)-1-[(2R,4R)-2-methylpiperidin-4-yl]ethyl}amino)phenyl]-1,3,4-oxadiazol-2(3H)-one,
   (217) 5-[3-({(1S)-1-[(3S,4S)-3-methylpiperidin-4-yl]ethyl}amino)-4-(trifluoromethyl)phenyl]-1,3,4-oxadiazol-2(3H)-one,
   (218) 5-[3-({(1S)-1-[(3S,4S)-3-methylpiperidin-4-yl]ethyl}amino)-4-(trifluoromethoxy)phenyl]-1,3,4-oxadiazol-2(3H)-one,
   (219) 5-[3-fluoro-5-({(1S)-1-[(3S,4S)-3-methylpiperidin-4-yl]ethyl} amino)-4-(trifluoromethoxy)phenyl]-1,3,4-oxadiazol-2(3H)-one,
   (220) 5-[4-(difluoromethoxy)-3-({(1S)-1-[(3S,4S)-3-methylpiperidin-4-yl]ethyl}amino)phenyl]-1,3,4-oxadiazol-2(3H)-one,
   (221) 5-[4-chloro-3-({(1S)-1-[(3S,4S)-3-methylpiperidin-4-yl]ethyl}amino)phenyl]-1,3,4-oxadiazol-2(3H)-one,
   (222) 5-[4-chloro-3-fluoro-5-({(1S)-1-[(3S,4S)-3-methylpiperidin-4-yl]ethyl} amino)phenyl]-1,3,4-oxadiazol-2(3H)-one,
   (223) 5-[3-fluoro-5-({(1S)-1-[(3S,4R)-3-fluoropiperidin-4-yl]ethyl}amino)-4-(trifluoromethyl)phenyl]-1,3,4-oxadiazol-2(3H)-one,
   (224) 5-[3-fluoro-5-({(1 S)-1-[(3S,4R)-3-fluoropiperidin-4-yl)ethyl}amino)-4-(trifluoromethoxy)phenyl]-1,3 ,4-oxadiazol-2(3 H)-one,
   (225) 5-[3-({(1S)-1-[(3S,4S)-3-ethylpiperidin-4-yl]ethyl}amino)-5-fluoro-4-(trifluoromethyl)phenyl]-1,3,4-oxadiazol-2(3H)-one,
   (226) 5-[3-({(1S)-1-[(3R,4R)-3-ethylpiperidin-4-yl]ethyl}amino)-5-fluoro-4-(trifluoromethyl)phenyl]-1,3,4-oxadiazol-2(3H)-one,
   (227) 5-[3-fluoro-5-({(1S)-1-[(3 S,4R)-3-fluoropiperidin-4-yl]ethyl}amino)-4-methylphenyl]-1,3,4-oxadiazol-2(3H)-one,
   (228) 5-[3-fluoro-4-methyl-5-({(1S)-1-[(3S,4S)-3-methylpiperidin-4-yl]ethyl} amino)phenyl]-1,3,4-oxadiazol-2(3H)-one,
   (229) 5-[3-({(1S)-1-[(3S,4S)-3-ethylpiperidin-4-yl]ethyl}amino)-5-fluoro-4-methylphenyl]-1,3,4-oxadiazol-2(3H)-one,
   (230) 5-[3-({(1S)-1-[(1r,4S)-4-aminocyclohexyl]ethyl}amino)-4-cyclopropyl-5-fluorophenyl]-1,3,4-oxadiazol-2(3H)-one,
   (231) 5-[3-({(1S)-1-[(1r,4S)-4-aminocyclohexyl]ethyl} amino)-4-ethyl-5-fluorophenyl] - 1,3,4-oxadiazol-2(3H)-one,
   (232) 5-[3-({(1S)-1-[(1r,4S)-4-aminocyclohexyl]ethyl}amino)-5-fluoro-4-(prop-1-en-2-yl)phenyl]-1,3,4-oxadiazol-2(3H)-one,
   (233) 5-[3-({(1S)-1-[(1r,4S)-4-aminocyclohexyl]ethyl}amino)-5-fluoro-4-(propan-2-yl)phenyl]-1,3,4-oxadiazol-2(3H)-one,
   (234) 5-[3-({[(1r,4r)-4-aminocyclohexyl]methyl}sulfanyl)-4-(trifluoromethyl)phenyl]-1,3,4-oxadiazol-2(3H)-one,
   (235) 5-{4-bromo-3-[(piperidin-4-yl)methoxy]phenyl}-1,3,4-oxadiazol-2(3H)-one,
   (236) 5-{4-bromo-3-[1-(piperidin-4-yl)ethoxy]phenyl}-1,3,4-oxadiazol-2(3H)-one,
   (237) 5-(3-{1-[(1r,4r)-4-aminocyclohexyl]ethoxy}-4-bromophenyl)-1,3,4-oxadiazol-2(3H)-one,
   (238) 5-(3-{[(1r,4r)-4-aminocyclohexyl]methoxy}-4-bromophenyl)-1,3 ,4-oxadiazol-2(3H)-one,
   (239) 5-{3-[1-(piperidin-4-yl)ethoxy]-4-(trifluoromethyl)phenyl}-1,3,4-oxadiazol-2(3H)-one,
   (240) 5-[3-{[(1r,4r)-4-aminocyclohexyl]methoxy}-4-(trifluoromethyl)phenyl]-1,3,4-oxadiazol-2(3H)-one,
   (241) 5-(3-{[(1r,4r)-4-aminocyclohexyl]methoxy}-4-chlorophenyl)-1,3,4-oxadiazol-2(3H)-one,
   (242) 5-(4-chloro-3-{[(1s,3s)-3-(piperazin-1-yl)cyclobutyl]methoxy}phenyl)-1,3,4-oxadiazol-2(3H)-one,
   (243) 5-(3-{[(1s,4s)-4-aminocyclohexyl]methoxy}-4-chlorophenyl)-1,3,4-oxadiazol-2(3H)-one,
   (244) 5-(1-{[(1r,4r)-4-aminocyclohexyl]methyl}-1,2,3,4-tetrahydroquinolin-7-yl)-1,3,4-oxadiazol-2(3H)-one,
   (245) 5-fluoro-1-{(1S)-1-[(3S,4S)-3-methylpiperidin-4-yl]ethyl}-7-(5-oxo-4,5-dihydro-1,3,4-oxadiazol-2-yl)-2,3-dihydroquinolin-4(1H)-one,
   (246) 5-[(4E)-5-fluoro-4-(methoxyimino)-1-{(1S)-1-[(3S,4S)-3-methylpiperidin-4-yl]ethyl}-1,2,3,4-tetrahydroquinolin-7-yl]-1,3,4-oxadiazol-2(3H)-one,
   (247) 5-(4-{(1S)-1-[(1r,4S)-4-aminocyclohexyl]ethyl}-3,4-dihydro-2H-1,4-benzoxazin-6-yl)-1,3,4-oxadiazol-2(3H)-one,
   (248) 5-{8-fluoro-4-[(1S)-1-(piperidin-4-yl)ethyl]-3,4-dihydro-2H-1,4-benzoxazin-6-yl}-1,3,4-oxadiazol-2(3H)-one,
   (249) 5-(4-{(1S)-1-[(1r,4S)-4-aminocyclohexyl]ethyl}-8-fluoro-3,4-dihydro-2H-1,4-benzoxazin-6-yl)-1,3,4-oxadiazol-2(3H)-one,
   (250) 5-{(2R)-8-fluoro-2-methyl-4-[(1S)-1-(piperidin-4-yl)ethyl)-3,4-dihydro-2H-1,4-benzoxazin-6-yl}-1,3,4-oxadiazol-2(3H)-one,
   (251) 5-{(2S)-8-fluoro-2-methyl-4-[(1S)-1-(piperidin-4-yl)ethyl]-3,4-dihydro-2H-1,4-benzoxazin-6-yl}-1,3,4-oxadiazol-2(3H)-one,
   (252) 5-[(2S)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one,
   (253) 5-[(2R)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one,
   (254) 5-{9-fluoro-5-[(1S)-1-(piperidin-4-yl)ethyl]-2,3,4,5-tetrahydro-1,5-benzoxazepin-7-yl}-1,3,4-oxadiazol-2(3H)-one.
(Item 12) A pharmaceutical composition comprising the 1,3,4-oxadiazolone compound according to any one of Items 1 to 9, or a pharmaceutically acceptable salt thereof, or a solvate thereof, as an active ingredient.
(Item 13) A PIM kinase inhibitor comprising the 1,3,4-oxadiazolone compound according to any one of Items 1 to 9, or a pharmaceutically acceptable salt thereof, or a solvate thereof, as an active ingredient.
(Item 14) A therapeutic agent for multiple sclerosis, rheumatoid arthritis, food allergy, asthma, systemic lupus erythematosus, lupus nephritis, inflammatory bowel disease, ulcerative colitis, atopic dermatitis, autoimmune lymphoproliferative syndrome, chronic obstructive pulmonary disease, allergic airway disease, eosinophilic polyangiitis granulomatosis, hypereosinophilic syndrome, chorioamnionitis, ankylosing spondylitis, myasthenia gravis, psoriasis, prostate cancer, colon cancer, esophageal cancer, ovarian cancer, uterine cancer, renal cancer, liver cancer, pancreatic cancer, gastric cancer, breast cancer, lung cancer, head and neck cancer, glioma, osteosarcoma, bladder cancer, acute lymphocytic leukemia, acute myeloid leukemia, chronic lymphocytic leukemia, chronic myeloid leukemia, B-cell lymphoma, multiple myeloma, T-cell lymphoma, skin cancer, Kaposi's sarcoma, Hodgkin's lymphoma, myeloproliferative tumor, adenoid cystic carcinoma, Ewing's sarcoma, adult T-cell leukemia, mesothelioma, acute promyelocytic leukemia, choriocarcinoma, liposarcoma, neuroblastoma, seminoma, lymphoblastic lymphoma, Epstein-Barr virus infection, hemophagocytic syndrome in which Epstein-Barr virus is known to be involved, influenza, hepatitis C, salmonellosis, herpesvirus infection, vaginal trichomonas infection, human granulocytic ehrlichiosis, aplastic anemia, atherosclerosis, pulmonary hypertension, diabetes, enlarged prostate, or Alzheimer's disease in all of which PIM kinase is involved, the therapeutic agent comprising the 1,3,4-oxadiazolone compound according to any one of Items 1 to 12, or a pharmaceutically acceptable salt thereof, or a solvate thereof, as an active ingredient

### ADVANTAGEOUS EFFECTS OF THE INVENTION

The compound of the formula [1] or the pharmaceutically acceptable salt thereof, or the solvate thereof has a PIM kinase inhibitory effect, and thus is useful as a therapeutic agent for diseases in which PIM kinases are involved (for example, systemic lupus erythematosus, lupus nephritis, etc.)

### MODE FOR CARRYING OUT THE INVENTION

The meaning of each term as used herein is described below. Unless otherwise specified, each term is used in the same meaning when used alone or in combination with other terms.

"Halogen atom" refers to a fluorine atom, a chlorine atom, a bromine atom, and an iodine atom.

Examples of "alkyl" include linear or branched alkyl having 1 to 10 carbon atoms, preferably 1 to 8 carbon atoms, and more preferably 1 to 6 carbon atoms. Specific examples of "alkyl" include methyl, ethyl, n-propyl, isopropyl, n-butyl, isobutyl, sec-butyl, tert-butyl, n-pentyl, sec-pentyl, 1-ethylpropyl, 1,2-dimethylpropyl, tert-pentyl, 2-methylbutyl, isopentyl, neopentyl, n-hexyl, sec-hexyl, 1-ethylbutyl, isohexyl, neohexyl, 1,1-dimethylbutyl, texyl, 2-ethylbutyl, 1,2,2-trimethylpropyl, 2,2-dimethylbutyl, n-heptyl, isoheptyl, n-octyl, and isooctyl.

"Alkenyl" refers to a linear or branched hydrocarbon group having one or more double bonds at any positions and having 2 to 10 carbon atoms, preferably 2 to 8 carbon atoms, more preferably 2 to 6 carbon atoms, and further preferably 2 to 10 carbon atoms. Specific examples of "alkenyl" include vinyl, allyl, propenyl, isopropenyl, butenyl, isobutenyl, prenyl, butadienyl, pentenyl, isopentenyl, pentadienyl, hexenyl, isohexenyl, and hexadienyl.

Examples of the alkyl moieties of "monoalkylamino", "alkylsulfonyl", and "alkylcarbonyl" include the same "alkyl" as described above.

"Amino" refers to -NH₂.

"Monoalkylamino" refers to a group in which one hydrogen atom bound to the nitrogen atom of an amino group is replaced by the above "alkyl". Specific examples of "monoalkylamino" include methylamino, ethylamino, and isopropylamino.

"Hydroxyalkyl" refers to a group in which a hydrogen atom bound to a carbon atom of the above "alkyl" is replaced by a hydroxy group. Specific examples of "hydroxyalkyl" include hydroxymethyl, 1-hydroxyethyl, 2-hydroxyethyl, 1-hydroxypropyl, and 2-hydroxypropyl.

"Aminoalkyl" refers to a group in which a hydrogen atom bound to a carbon atom of the above "alkyl" is replaced by an amino group. Specific examples of "aminoalkyl" include aminomethyl, 1-aminoethyl, 2-aminoethyl, 1-aminopropyl, 2-aminopropyl, and 3-aminopropyl.

"Alkylamino substituted with a non-aromatic carbocyclic group" refers to a group in which a hydrogen atom bound to a carbon atom of the alkyl of alkylamino is replaced by a non-aromatic carbocyclic group described below. Examples of "alkylamino substituted with a non-aromatic carbocyclic group" include methylamino substituted with cyclopropyl.

"Hydroxyalkylamino" refers to a group in which a hydrogen atom bound to the nitrogen atom of an amino group is replaced by the above "hydroxyalkyl".

"Alkylcarbonyl" means a group in which the above "alkyl" is bound to a carbonyl group. Examples of "alkylcarbonyl" include methylcarbonyl, ethylcarbonyl, propylcarbonyl, isopropylcarbonyl, tert-butylcarbonyl, isobutylcarbonyl, sec-butylcarbonyl, pentylcarbonyl, isopentylcarbonyl, and hexylcarbonyl.

"Monohaloalkyl" refers to a group in which one hydrogen atom of the above "alkyl" is replaced by the above "halogen". Specific examples of "monohaloalkyl" include fluoromethyl, chloromethyl, and fluoroethyl.

"Dihaloalkyl" refers to a group in which two hydrogen atoms of the above "alkyl" are replaced by the above "halogens". Specific examples of "dihaloalkyl" include difluoromethyl, dichloromethyl, and difluoroethyl.

"Trihaloalkyl" refers to a group in which three hydrogen atoms of the above "alkyl" are replaced by the above "halogens". Specific examples of "trihaloalkyl" include trifluoromethyl, trichloromethyl, and trifluoroethyl.

"Trihaloalkylamino" refers to a group in which one hydrogen atom bound to the nitrogen atom of an amino group is replaced by the above "trihaloalkyl". Specific examples of "trihaloalkylamino" include trifluoromethylamino and trifluoroethylamino.

"Alkoxy" refers to a group in which the above "alkyl" is bound to an oxygen atom. Examples of "alkoxy" include linear or branched alkoxy having 1 to 8 carbon atoms and preferably 1 to 6 carbon atoms. Specific examples of "alkoxy" include methoxy, ethoxy, n-propoxy, isopropoxy, n-butoxy, isobutoxy, sec-butoxy, tert-butoxy, n-pentyloxy, n-hexyloxy, n-heptyloxy, and n-octyloxy.

"Aminoalkoxy" refers to a group in which a hydrogen atom bound to a carbon atom of the "alkoxy" is replaced by an amino group. Specific examples of "aminoalkoxy" include aminomethyl, 1-aminoethyl, aminomethoxy, 2-aminoethoxy, and 3-aminopropoxy.

"Alkoxycarbonyl" refers to a group in which the above "alkoxy" is bound to a carbonyl group. Examples of "alkoxycarbonyl" include methoxycarbonyl, ethoxycarbonyl, n-propoxycarbonyl, isopropoxycarbonyl, tert-butoxycarbonyl, isobutoxycarbonyl, secbutoxycarbonyl, pentoxycarbonyl, isopentoxycarbonyl, and hexyloxycarbonyl.

Examples of the alkoxy moieties of "alkoxycarbonyl", "alkoxycarbonylamino", "alkoxycarbonylaminoalkyl", and "alkoxyimino" include the same "alkoxy" as described above.

Examples of "alkylene" include an alkylene having a linear or branched divalent hydrocarbon group having 1 to 6 carbon atoms. Specific examples of "alkylene" include methylene, ethylene, and propylene.

Examples of "alkenylene" include an alkylene having a linear or branched divalent hydrocarbon group having 2 to 6 carbon atoms. Specific examples of "alkenylene" include vinylene, propenylene, butenylen, and pentenylene.

"Alkynylene" includes a linear divalent hydrocarbon group having one or more triple bonds at any positions and having 2 to 8 carbon atoms, preferably 2 to 6 carbon atoms, and more preferably 2 to 4 carbon atoms. These groups may further have a double bond at any position. Examples of "alkynylene" include ethynylene, propynylene, butynylene, pentynylene, and hexynylene.

"Oxo" refers to double-bond oxygen (=O).

"Imino" refers to a divalent atomic group (=NH) obtained by removing two hydrogen atoms from ammonia (NH3).

"Alkoxyimino" refers to a group in which a hydrogen atom of the above "imino" is replaced by the above "alkoxy". Specific examples of "alkoxyimino" include methoxyimino, 2-ethoxyimino, and 3-propoxyimino.

Examples of "carbocyclic group" include a saturated hydrocarbon group that is a monocyclic to tricyclic group and has 3 to 20 carbon atoms, and include aromatic carbocyclic groups and non-aromatic carbocyclic groups.

Examples of "aromatic carbocyclic group" include an aromatic hydrocarbon group that is a monocyclic to tricyclic group and has 6 to 14 carbon atoms. Specific examples of "aromatic carbocyclic group" include phenyl, 1-naphthyl, 2-naphthyl, 1-anthryl, 2-anthryl, 9-anthryl, 1-phenanthryl, 2-phenanthryl, 3-phenanthryl, 4-phenanthryl, and 10-phenanthryl. Among them, phenyl is preferred.

Examples of "non-aromatic carbocyclic group" include a cyclic non-aromatic hydrocarbon group that is a monocyclic to tricyclic group. Specific examples of "non-aromatic carbocyclic group" include cyclopropyl, cyclobutyl, cyclopentyl, cyclohexyl, cycloheptyl, and cyclooctyl.

The above "non-aromatic carbocyclic group" may be a bridged hydrocarbon group. Examples of the bridged hydrocarbon group include
bicyclo[2.2.1]heptanyl (for example, bicyclo[2.2.1]heptan-1-yl, bicyclo[2.2.1]heptan-2-yl,
bicyclo[2.2.1]heptan-7-yl,),
bicyclo[1.1.1]pentanyl (for example, bicyclo[1.1.1]pentan-1-yl, bicyclo[1.1.1]pentan-2-yl),
bicyclo[4.1.0]heptanyl (for example, bicyclo[4.1.0]heptan-1-yl, bicyclo[4.1.0]heptan-2-yl,
bicyclo[4.1.0]heptan-3-yl, bicyclo[4.1.0]heptan-7-yl),
bicyclo [2.2.2]octanyl (for example, bicyclo[2.2.2]octan-1-yl, bicyclo[2.2.2]octan-2-yl),
bicyclo[3.1.1]heptanyl (for example, bicyclo[3.1.1]heptan-1-yl, bicyclo[3.1.1]heptan-2-yl,
bicyclo[3.1.1]heptan-3-yl, bicyclo[3.1.1]heptan-6-yl), or
cuban-1-yl.

The above "non-aromatic carbocyclic group" may be a spirocyclic group. Examples of the spirocyclic group include
spiro[3.3]heptanyl (for example, spiro[3.3]heptan-1-yl, spiro[3.3]heptan-2-yl),
spiro[4.4]nonanyl (for example, spiro[4.4]nonan-1-yl, spiro[4.4]nonan-2-yl),
spiro[5.5]undecanyl (for example, spiro[5.5]undecan-1-yl, spiro[5.5]undecan-2-yl,
spiro[5.5]undecan-3-yl), or
spiro[2.5]octanyl (for example, spiro[2.5]octan-1-yl, spiro[2.5]octan-4-yl, spiro[2.5]octan-5-yl,
spiro[2.5]octan-6-yl).

Examples of "aromatic heterocyclic group" include an aromatic ring that is monocyclic to tricyclic, has 1 to 3 heteroatoms selected from the group consisting of nitrogen atom, oxygen atom, and sulfur atom as constituent atoms, and has 6 to 14 carbon atoms. Specific examples of "aromatic heterocyclic group" include
furyl (for example, 2-furyl, 3-furyl),
thienyl (for example, 2-thienyl, 3-thienyl),
pyrrolyl (for example, 1-pyrrolyl, 2-pyrrolyl, 3-pyrrolyl),
imidazolyl (for example, 1-imidazolyl, 2-imidazolyl, 4-imidazolyl),
pyrazolyl (for example, 1-pyrazolyl, 3-pyrazolyl, 4-pyrazolyl),
triazolyl (for example, 1,2,4-triazol-1-yl, 1,2,4-triazol-3-yl, 1,2,4-triazol-4-yl),
tetrazolyl (for example, 1-tetrazolyl, 2-tetrazolyl, 5-tetrazolyl),
oxazolyl (for example, 2-oxazolyl, 4-oxazolyl, 5-oxazolyl),
isoxazolyl (for example, 3-isoxazolyl, 4-isoxazolyl, 5-isoxazolyl),
oxadiazolyl (for example, 1,3,4-oxadiazol-2-yl),
thiazolyl (for example, 2-thiazolyl, 4-thiazolyl, 5-thiazolyl),
thiadiazolyl (for example, 1,3,4-thiadiazolyl, 1,2,4-thiadiazolyl, 1,2,3-thiadiazolyl),
isothiazolyl (for example, 3-isothiazolyl, 4-isothiazolyl, 5-isothiazolyl),
pyridyl (for example, 2-pyridyl, 3-pyridyl, 4-pyridyl),
pyridazinyl (for example, 3-pyridazinyl, 4-pyridazinyl),
pyrimidinyl (for example 2-pyrimidinyl, 4-pyrimidinyl, 5-pyrimidinyl),
pyrazinyl (for example, 2-pyrazinyl),
benzothiadiazolyl (for example, 1,2,3-benzothiadiazol-4-yl, 1,2,3-benzothiadiazol-5-yl, 2,1,3-benzothiadiazol-4-yl, 2,1,3-benzothiadiazol-5-yl),
benzothiazolyl (for example, benzothiazol-2-yl, benzothiazol-4-yl, benzothiazol-5-yl, benzothiazol-6-yl, benzothiazol-7-yl),
indolyl (for example, indol-3-yl, indol-4-yl, indol-5-yl, indol-6-yl, indol-7-yl),
benzothiophenyl (for example, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl),
1,1-dioxo-1-benzothiophenyl (for example, 1,1-dioxo-1-benzothiophen-2-yl, 1,1-dioxo-1-benzothiophen-3-yl, 1,1-dioxo-1-benzothiophen-4-yl, 1,1-dioxo-1-benzothiophen-5-yl, 1,1-dioxo-1-benzothiophen-6-yl, 1,1-dioxo-1-benzothiophen-7-yl),
quinolyl (quinolin-2-yl, quinolin-3-yl, quinolin-4-yl, quinolin-5-yl, quinolin-6-yl, quinolin-7-yl, quinolin-8-yl), or
1,3-benzoxazol-2-yl.

Examples of "non-aromatic heterocyclic group" include a monocyclic or polycyclic non-aromatic cyclic group having one or more identical or different heteroatoms selected from among nitrogen atom, oxygen atom, and sulfur atom within a ring thereof. Specific examples of "non-aromatic heterocyclic group" include
oxetanyl (for example, 2-oxetanyl, 3-oxetanyl),
azetidinyl (for example, 2-azetidinyl, 3-azetidinyl),
tetrahydropyranyl (for example, 2-tetrahydropyranyl, 3-tetrahydropyranyl, 4-tetrahydropyranyl), 1,4-dioxanyl (for example, 1,4-dioxan-2-yl),
1,3-dioxanyl (for example, 1,3-dioxan-2-yl, 1,3-dioxan-4-yl, 1,3-dioxan-5-yl),
pyrrolidinyl (for example, 1-pyrrolidinyl, 2-pyrrolidinyl, 3-pyrrolidinyl),
piperidinyl (for example, 1-piperidinyl, 2-piperidinyl, 3-piperidinyl, 4-piperidinyl),
piperazinyl (for example, 1-piperazinyl, 2-piperazinyl, 3-piperazinyl),
azepanyl (for example, 1-azepanyl, 2-azepanyl, 3-azepanyl, 4-azepanyl),
azocanyl (for example, 1-azocanyl, 2-azocanyl, 3-azocanyl, 4-azocanyl, 5-azocanyl),
homopiperidinyl (for example, 2-homopiperidinyl, 3-homopiperidinyl, 4-homopiperidinyl),
morpholinyl (for example, 2-morpholinyl, 3-morpholinyl, 4-morpholinyl),
thiomorpholinyl (for example, 2-thiomorpholinyl, 3-thiomorpholinyl, 4-thiomorpholinyl), or
tetrahydrofuryl (2-tetrahydrofuryl, 3-tetrahydrofuryl).

The above "non-aromatic heterocyclic group" may be a bridged cyclic group. Examples of the bridged cyclic group include
3-azabicyclo[3.2.1]octanyl (for example, 3-azabicyclo[3.2.1]octan-1-yl, 3-azabicyclo[3.2.1]octan-2-yl, 3-azabicyclo[3.2.1]octan-3-yl, 3-azabicyclo[3.2.1]octan-6-yl, 3-azabicyclo[3.2.1]octan-8-yl),
quinuclidinyl (for example, quinuclidin-2-yl, quinuclidin-3-yl, quinuclidin-4-yl), or
6-oxa-3-azabicyclo[3.1.1]heptanyl (for example, 6-oxa-3-azabicyclo[3.1.1]heptan-1-yl, 6-oxa-3-azabicyclo[3.1.1]heptan-2-yl, 6-oxa-3-azabicyclo[3.1.1]heptan-3-yl, 6-oxa-3-azabicyclo[3.1.1]heptan-7-yl).

The above "non-aromatic heterocyclic group" may be a spiro-cyclic group. Examples of the spiro-cyclic group include
6-azaspiro[2.5]octan-1-yl (for example, 6-azaspiro[2.5]octan-1-yl, 6-azaspiro[2.5]octan-4-yl, 6-azaspiro[2.5]octan-5-yl),
3,9-dazaspiro[5.5]undecan-1-yl (for example, 3,9-dazaspiro[5.5]undecan-1-yl, 3,9-dazaspiro[5.5]undecan-2-yl, 3,9-dazaspiro[5.5]undecan-3-yl),
2,7-diazaspiro[3.5]nonan-1-yl (for example, 2,7-diazaspiro[3.5]nonan-1-yl, 2,7-diazaspiro[3.5]nonan-2-yl, 2,7-diazaspiro[3.5]nonan-5-yl, 2,7-diazaspiro[3.5]nonan-6-yl, 2,7-diazaspiro[3.5]nonan-7-yl)
7-azaspiro[3.5]nonanyl (7-azaspiro[3.5]nonan-1-yl, 7-azaspiro[3.5]nonan-2-yl, 7-azaspiro[3.5]nonan-5-yl, 7-azaspiro[3.5]nonan-6-yl), or
2,5-diazabicyclo[2.2.1]heptanyl (2,5-diazabicyclo[2.2.1]heptan-1-yl, 2,5-diazabicyclo[2.2.1]heptan-2-yl, 2,5-diazabicyclo[2.2.1]heptan-3-yl, 2,5-diazabicyclo[2.2.1]heptan-7-yl).

Hereinafter, each symbol in the formula [1] is described.

X¹ in the formula [1] is a carbon atom or a nitrogen atom. A carbon atom is preferred.

When X¹ is a carbon atom, R¹ is a hydrogen atom, a halogen atom, alkyl, alkenyl, a non-aromatic carbocyclic group, dihaloalkyl, trihaloalkyl, alkoxy, dihaloalkoxy, trihaloalkoxy, alkylsulfonyl, cyano, an aromatic carbocyclic group, or an aromatic heterocyclic group. A halogen atom, alkyl, dihaloalkyl, trihaloalkyl, alkoxy, dihaloalkoxy, alkylsulfonyl, cyano, and trihaloalkoxy are preferred, a halogen atom, trihaloalkyl, dihaloalkoxy, and trihaloalkoxy are more preferred, and a halogen atom, trihaloalkyl, and trihaloalkoxy are further preferred.

As the "halogen atom" for R¹, a chlorine atom, a bromine atom, and a fluorine atom are preferred, and a chlorine atom and a fluorine atom are more preferred.

As the "alkyl" for R¹, alkyl having 1 to 6 carbon atoms is preferred.

As the "alkenyl" for R¹, alkenyl having 2 to 4 carbon atoms is preferred.

As the "non-aromatic carbocyclic group" for R¹, a monocyclic non-aromatic carbocyclic group having 3 to 8 carbon atoms is preferred.

As the "dihaloalkyl" for R¹, dihaloalkyl having 1 to 6 carbon atoms is preferred.

As the "trihaloalkyl" for R¹, trihaloalkyl having 1 to 6 carbon atoms is preferred.

As the "alkoxy" for R¹, alkoxy having 1 to 6 carbon atoms is preferred.

As the "dihaloalkoxy" for R¹, dihaloalkyl having 1 to 6 carbon atoms is preferred.

As the "trihaloalkoxy" for R¹, trihaloalkoxy having 1 to 6 carbon atoms is preferred.

As the "alkylsulfonyl" for R¹, alkylsulfonyl having 1 to 6 carbon atoms is preferred.

As the "aromatic carbocyclic group" for R¹, phenyl is preferred.

As the "aromatic heterocyclic group" for R¹, pyridyl is preferred.

R² is a hydrogen atom, a halogen atom, alkyl, a non-aromatic carbocyclic group, trihaloalkyl, trihaloalkoxy, pentafluorosulfanyl (SF₅), cyano, amino, or nitro. A hydrogen atom, a halogen atom, alkyl, trihaloalkyl, trihaloalkoxy, amino, and nitro are preferred, and a hydrogen atom, a halogen atom, and trihaloalkyl are more preferred.

As the "halogen atom" for R², a chlorine atom, a bromine atom, and a fluorine atom are preferred.

As the "alkyl" for R², alkyl having 1 to 6 carbon atoms is preferred.

As the "non-aromatic carbocyclic group" for R², a monocyclic non-aromatic carbocyclic group having 3 to 8 carbon atoms is preferred.

As the "trihaloalkyl" for R², trihaloalkyl having 1 to 6 carbon atoms is preferred.

As the "alkyl" for R³, alkyl having 1 to 6 carbon atoms is preferred.

X⁴ is a carbon atom or a nitrogen atom.

When X⁴ is a carbon atom, R⁴ is a hydrogen atom, a halogen atom, or alkyl.

As the "alkyl" for R⁴, alkyl having 1 to 6 carbon atoms is preferred.

L is a bond, an alkylene, an alkenylene, an alkynylene, or a group represented by L-1, L-2, L-3, or L-4: 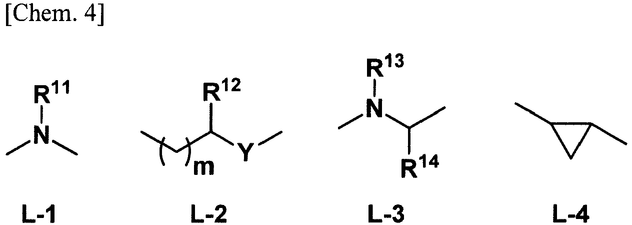 wherein the bond on the left side of each group is attached to A in the formula [1], the bond on the right side of each group is attached to a ring B in the formula [1], R¹¹, R¹³, and R¹⁴ are each a hydrogen atom or alkyl, R¹² is a hydrogen atom, alkyl, monohaloalkyl, dihaloalkyl, or trihaloalkyl, and Y is O, S, or -NR¹⁵- (R¹⁵ is a hydrogen atom or alkyl, and m is 0, 1, or 2).

As the "alkylene" for L, a linear or branched alkylene having 1 to 6 carbon atoms is preferred.

As the "alkenylene" for L, a linear or branched alkenylene having 2 to 6 carbon atoms is preferred.

As the "alkynylene" for L, a linear or branched alkynylene having 2 to 6 carbon atoms is preferred.

R¹¹ for L-1 is a hydrogen atom or alkyl.

As the "alkyl" for R¹¹, alkyl having 1 to 6 carbon atoms is preferred.

R¹² for L-2 is a hydrogen atom, alkyl, monohaloalkyl, dihaloalkyl, or trihaloalkyl.

As the "alkyl" for R¹², alkyl having 1 to 6 carbon atoms is preferred.

As the "monohaloalkyl" for R¹², monohaloalkyl having 1 to 6 carbon atoms is preferred.

As the "dihaloalkyl" for R¹², dihaloalkyl having 1 to 6 carbon atoms is preferred.

As the "trihaloalkyl" for R¹², trihaloalkyl having 1 to 6 carbon atoms is preferred.

m for L-2 is 0, 1, or 2. 0 and 1 are preferred.

Y for L-2 is O, S, or -NR¹⁵-, and R¹⁵ is a hydrogen atom or alkyl.

R¹³ for L-3 is a hydrogen atom or alkyl.

R¹³ is a hydrogen atom or alkyl.

As the "alkyl" for R¹³, alkyl having 1 to 6 carbon atoms is preferred.

As the "alkyl" for R¹⁴, alkyl having 1 to 6 carbon atoms is preferred.

A is aminoalkylamino, a non-aromatic heterocyclic group, a non-aromatic carbocyclic group, an aromatic carbocyclic group, an aromatic heterocyclic group, or 1,3-dioxa-2-yl.

As the aminoalkylamino for A, aminoalkylamino having 1 to 8 carbon atoms is preferred.

As the non-aromatic heterocyclic group for A, pyrrolidinyl, piperidinyl, piperazinyl, azepanyl, azocanyl, 1,3-dioxanyl, tetrahydrofuranyl, tetrahydropyranyl, 6-azaspiro[2.5]octanyl, 3,9-diazaspiro[5.5]undecanyl, 2,7-diazaspiro[3.5]nonanyl, 7-azaspiro[3.5]nonanyl, 3-azabicyclo[3.2.1]octanyl, and 2-azaspiro[3.3]heptanyl are preferred.

As the non-aromatic carbocyclic group for A, cyclobutyl, cyclopentyl, cyclohexyl, bicyclo[2.2.1]heptanyl, bicyclo[1.1.1]pentanyl, cuban-1-yl, and spiro[3.3]heptanyl are preferred.

As the aromatic carbocyclic group for A, phenyl is preferred.

As the aromatic heterocyclic group for A, pyridyl is preferred.

The non-aromatic heterocyclic group for A is optionally substituted with one or two groups selected from the group consisting of the following (1) to (8):
(1) amino,
(2) alkyl,
(3) aminoalkyl,
(4) alkyl substituted with amino and hydroxy,
(5) halogen,
(6) alkylcarbonyl, and
(7) alkoxycarbonyl.

As the substituent with which the non-aromatic heterocyclic group for A is optionally substituted, amino, alkyl having 1 to 6 carbon atoms, aminoalkyl having 1 to 6 carbon atoms, alkyl having 1 to 6 carbon atoms and substituted with amino and hydroxy, a halogen, and monoalkylamino having 1 to 6 carbon atoms are preferred.

As the "alkyl" with which the non-aromatic heterocyclic group for A is optionally substituted, alkyl having 1 to 6 carbon atoms is preferred.

As the alkyl moiety of the "aminoalkyl" with which the non-aromatic heterocyclic group for A is optionally substituted, alkyl having 1 to 6 carbon atoms is preferred.

As the alkyl moiety of the "alkyl substituted with amino and hydroxy" with which the non-aromatic heterocyclic group for A is optionally substituted, alkyl having 1 to 6 carbon atoms is preferred.

As the alkyl moiety of the "monoalkylamino" with which the non-aromatic heterocyclic group for A is optionally substituted, alkyl having 1 to 6 carbon atoms is preferred.

As the alkyl moiety of the "alkylcarbonyl" with which the non-aromatic heterocyclic group for A is optionally substituted, alkyl having 1 to 6 carbon atoms is preferred.

As the alkoxy moiety of the "alkoxycarbonyl" with which the non-aromatic heterocyclic group for A is optionally substituted, alkyl having 1 to 6 carbon atoms is preferred.

The non-aromatic carbocyclic group for A is optionally substituted with 1 to 3 groups selected from the group consisting of the following (1) to (15):
(1) amino,
(2) alkyl,
(3) alkylamino substituted with a non-aromatic carbocyclic group,
(4) trihaloalkylamino,
(5) alkyl substituted with hydroxy,
(6) aminoalkyl,
(7) hydroxy,
(8) monoalkylamino,
(9) hydroxyalkylamino,
(10) alkoxycarbonyl,
(11) carboxyl,
(12) carbamoyl,
(13) acetamide (Me-C(=O)-NH-),
(14) piperazinyl, and
(15) alkylamino.

As the substituent with which the non-aromatic carbocyclic group for A is optionally substituted, amino, alkyl having 1 to 6 carbon atoms, aminoalkyl having 1 to 6 carbon atoms, hydroxy, and monoalkylamino having 1 to 6 carbon atoms are preferred.

As the "alkyl" with which the non-aromatic carbocyclic group for A is optionally substituted, alkyl having 1 to 6 carbon atoms is preferred.

As the non-aromatic carbocyclic group of the "alkyl substituted with a non-aromatic carbocyclic group" with which the non-aromatic carbocyclic group for A is optionally substituted, a monocyclic non-aromatic carbocyclic group having 3 to 8 carbon atoms is preferred.

As the alkyl of the "trihaloalkylamino" with which the non-aromatic carbocyclic group for A is optionally substituted, alkyl having 1 to 6 carbon atoms is preferred.

As the alkyl of the "alkyl substituted with hydroxy" with which the non-aromatic carbocyclic group for A is optionally substituted, alkyl having 1 to 6 carbon atoms is preferred.

As the alkyl of the "aminoalkyl" with which the non-aromatic carbocyclic group for A is optionally substituted, alkyl having 1 to 6 carbon atoms is preferred.

As the alkyl of the "monoalkylamino" with which the non-aromatic carbocyclic group for A is optionally substituted, alkyl having 1 to 6 carbon atoms is preferred.

As the alkyl of the "hydroxyalkylamino" with which the non-aromatic carbocyclic group for A is optionally substituted, alkyl having 1 to 6 carbon atoms is preferred.

As the alkoxy of the "alkoxycarbonyl" with which the non-aromatic carbocyclic group for A is optionally substituted, alkyl having 1 to 6 carbon atoms is preferred.

The aromatic carbocyclic group for A is optionally substituted with one group selected from the group consisting of the following (1) to (4):
(1) aminoalkyl,
(2) aminoalkoxy,
(3) alkoxy substituted with piperidinyl, and
(4) alkoxycarbonylaminoalkyl.

As the alkyl of the "alkyl substituted with amino" with which the aromatic carbocyclic group for A is optionally substituted, alkyl having 1 to 6 carbon atoms is preferred.

As the alkoxy of the "alkoxy substituted with amino" with which the aromatic carbocyclic group for A is optionally substituted, alkoxy having 1 to 6 carbon atoms is preferred.

As the alkoxy of the "alkoxy substituted with piperidinyl" with which the aromatic carbocyclic group for A is optionally substituted, alkoxy having 1 to 6 carbon atoms is preferred.

As the alkoxy of the "alkoxycarbonylaminoalkyl" with which the aromatic carbocyclic group for A is optionally substituted, alkoxy having 1 to 6 carbon atoms is preferred.

As the alkyl of the "alkoxycarbonylaminoalkyl" with which the aromatic carbocyclic group for A is optionally substituted, alkyl having 1 to 6 carbon atoms is preferred.

A and L are selected from any of the following cases (a) to (h):
(a) when L is a bond,
   A is aminoalkylamino, a non-aromatic heterocyclic group, an aromatic carbocyclic group, or an aromatic heterocyclic group,
(b) when L is an alkylene,
   A is a non-aromatic heterocyclic group or a non-aromatic carbocyclic group,
(c) when L is an alkenylene,
   A is a non-aromatic heterocyclic group,
(d) when L is an alkynylene,
   A is a non-aromatic heterocyclic group,
(e) when L is L-1,
   A is a non-aromatic heterocyclic group, a non-aromatic carbocyclic group, or an aromatic carbocyclic group,
(f) when L is L-2,
   A is a non-aromatic heterocyclic group or a non-aromatic carbocyclic group,
(g) when L is L-3,
   A is a non-aromatic heterocyclic group, and
(h) when L is L-4,
   A is 1,3-dioxa-2-yl substituted with an amino group.

More specifically, the compounds of the present invention include compounds shown in Table 1 below.

**[Table 1]**

| | | |
|---|---|---|
| Preparation Process a-1 | Preparation Process a-2 | Preparation Process a-3 |
| 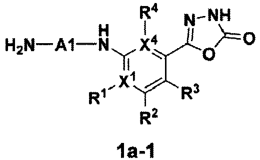 | 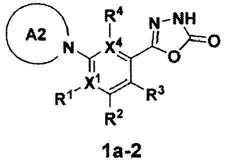 | 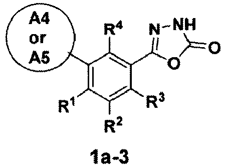 |
| Preparation Process b | Preparation Process c | Preparation Process d |
| 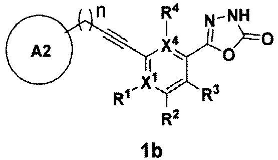 | 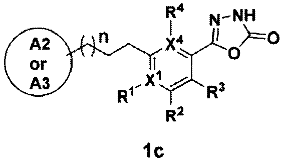 | 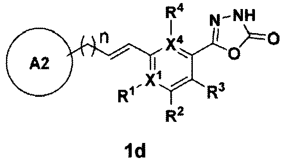 |
| Preparation Process e | Preparation Process f-1 | Preparation Process f-2 |
| 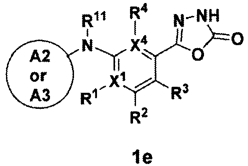 | 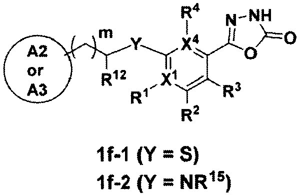 | 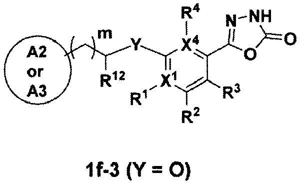 |
| Preparation Process g | Preparation Process h | Preparation Process i-1 |
| 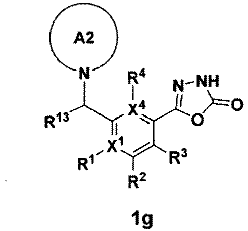 | 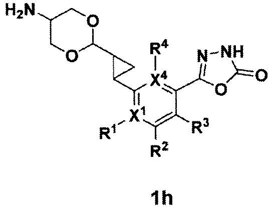 | 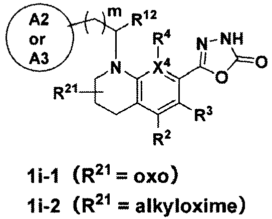 |
| Preparation Process i-2 | Preparation Process 1j | Preparation Process 1k |
| 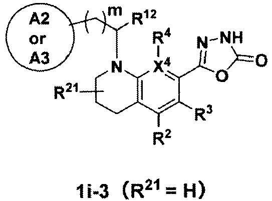 | 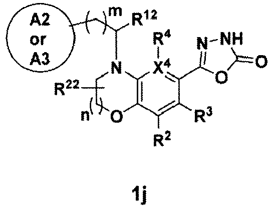 | 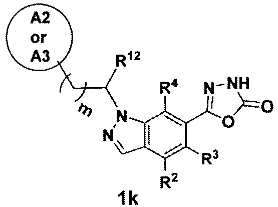 |

(in the table, X¹, X², R¹, R², R³, R⁴, R¹¹, R¹², R²², Y, m, and n are as defined above, A1 is alkyl, and is as defined for the alkyl of the "aminoalkylamino". A2 is a non-aromatic heterocyclic group, A3 is a non-aromatic carbocyclic group, A4 is an aromatic heterocyclic group, and A5 is an aromatic carbocyclic group. The "A2 or A3" represents a non-aromatic heterocyclic group or a non-aromatic carbocyclic group, the "A4 or A5" represents an aromatic heterocyclic group or an aromatic carbocyclic group, and the "A2 or A3 or A5" represents a non-aromatic heterocyclic group, a non-aromatic carbocyclic group, or an aromatic carbocyclic group.)

In a compound 1a-1, as the alkyl of aminoalkylamino for A1, alkyl having 1 to 6 carbon atoms is preferred, and aminopropylamino, aminobutylamino, aminopentylamino, aminohexylamino, and aminohexan-2-ylamino are more preferred. As X¹, a carbon atom is preferred, and as R¹, trihaloalkyl having 1 to 6 carbon atoms is preferred. As R², a halogen atom, trihaloalkyl having 1 to 6 carbon atoms, and trihaloalkoxy having 1 to 6 carbon atoms are preferred, and trihaloalkyl having 1 to 6 carbon atoms is more preferred. As R³, a hydrogen atom is preferred. As X⁴, a carbon atom is preferred, and as R⁴, a hydrogen atom is preferred.

In a compound 1a-2, as A2, non-aromatic heterocyclic groups are preferred, and among them, piperidinyl, piperazinyl, 3,9-dazaspiro[5.5]undecan-3-yl, and 2,7-diazaspiro[3.5]nonan-7-yl are preferred. As the group with which the non-aromatic carbocyclic group for A2 is optionally substituted, amino, aminoalkyl having 1 to 6 carbon atoms, hydroxyalkylamino having 1 to 6 carbon atoms, and alkoxycarbonyl having 1 to 6 carbon atoms are preferred. As X¹, a carbon atom is preferred, and as R¹, alkyl having 1 to 6 carbon atoms, a halogen, and trihaloalkyl having 1 to 6 carbon atoms are preferred. As R², a hydrogen atom is preferred. Furthermore, R¹ and R² may combine with adjacent atoms to form an indazole ring, and these embodiments are also preferred embodiments. As R³, a hydrogen atom is preferred. As X⁴, a carbon atom is preferred, and as R⁴, a hydrogen atom is preferred.

In a compound 1a-3, as the aromatic carbocyclic group for A5, phenyl is preferred, and as the aromatic heterocyclic group for A4, pyridyl is preferred. As the group with which the aromatic carbocyclic group for A4 is optionally substituted, aminoalkyl having 1 to 6 carbon atoms, aminoalkoxy having 1 to 6 carbon atoms, alkoxy having 1 to 6 carbon atoms and substituted with piperidinyl, alkyl having 1 to 6 carbon atoms, and alkoxycarbonylaminoalkyl that is alkoxy having 1 to 6 carbon atoms are preferred, and as the group with which the aromatic heterocyclic group for A5 is optionally substituted, piperazinyl is preferred. As X¹, a carbon atom is preferred, and as R¹, a halogen and trihaloalkyl having 1 to 6 carbon atoms are preferred. As R², a hydrogen atom is preferred. As R³, a hydrogen atom is preferred. As X⁴, a carbon atom is preferred, and as R⁴, a hydrogen atom is preferred.

In a compound 1b, as the non-aromatic heterocyclic group for A2, piperidinyl is preferred. As X¹, a carbon atom is preferred, and as R¹, trihaloalkyl having 1 to 6 carbon atoms is preferred. As R², a hydrogen atom is preferred. As R³, a hydrogen atom is preferred. As X⁴, a carbon atom is preferred, and as R⁴, a hydrogen atom is preferred.

In a compound 1c, as the non-aromatic heterocyclic group for A2, piperidinyl and 1,3-dioxanyl are preferred, and as the non-aromatic carbocyclic group for A3, a 3- to 8-membered monocyclic non-aromatic carbocyclic group is preferred, and cyclohexyl is more preferred. As X¹, a carbon atom is preferred, and as R¹, trihaloalkyl having 1 to 6 carbon atoms is preferred. As R², a hydrogen atom is preferred. As R³, a hydrogen atom is preferred. As X⁴, a carbon atom is preferred, and as R⁴, a hydrogen atom is preferred.

In a compound 1d, as the non-aromatic heterocyclic group for A2, piperidinyl is preferred. As X¹, a carbon atom is preferred, and as R¹, trihaloalkyl having 1 to 6 carbon atoms is preferred. As R², a hydrogen atom is preferred. As R³, a hydrogen atom is preferred. As X⁴, a carbon atom is preferred, and as R⁴, a hydrogen atom is preferred.

In a compound 1e, as the non-aromatic heterocyclic group for A2, piperidinyl, 6-azaspiro[2.5]octan-1-yl, and 7-azaspiro[3.5]nonan-1-yl are preferred, and as the non-aromatic carbocyclic group for A3, a 3- to 8-membered monocyclic non-aromatic carbocyclic group and spiro[3.3]heptanyl are preferred, and cyclohexyl and spiro[3.3]heptanyl are more preferred. As the aromatic carbocyclic group for A5, phenyl is preferred.

In a compound 1e, as the group with which the non-aromatic heterocyclic group for A2 is optionally substituted, 1 to 3 groups selected from the group consisting of amino, aminoalkyl having 1 to 6 carbon atoms, and hydroxy are preferred.

In a compound 1e, as the group with which the aromatic carbocyclic group for A5 is optionally substituted, aminoalkyl having 1 to 6 carbon atoms is preferred.

In the compound 1e, as X¹, a carbon atom is preferred, and as R¹, a halogen, trihaloalkyl having 1 to 6 carbon atoms, and trihaloalkoxy having 1 to 6 carbon atoms are preferred. As R², a hydrogen atom is preferred. As R³, a hydrogen atom is preferred. As X⁴, a carbon atom is preferred, and as R⁴, a hydrogen atom is preferred.

In a compound 1f-1, as A3, a 3- to 8-membered monocyclic non-aromatic carbocyclic group is preferred. As the group with which the 3- to 8-membered monocyclic non-aromatic carbocyclic group is optionally substituted, amino is preferred. As X¹, a carbon atom is preferred, and as R¹, trihaloalkyl having 1 to 6 carbon atoms is preferred. As R², a hydrogen atom is preferred. As R³, a hydrogen atom is preferred. As X⁴, a carbon atom is preferred, and as R⁴, a hydrogen atom is preferred.

In a compound 1f-2, as the non-aromatic heterocyclic group for A2, pyrrolidinyl, piperidinyl, 3-azabicyclo[3.2.1]octan-8-yl, 7-azaspiro[3.5]nonan-2-yl, 2-azaspiro[3.3]heptan-6-yl, azepanyl, azocanyl, and tetrahydropyranyl are preferred, 3-azabicyclo[3.2.1]octan-8-yl, 2-azaspiro[3.3]heptan-6-yl, pyrrolidinyl, and piperidinyl are more preferred, and piperidinyl is further preferred. As the non-aromatic carbocyclic group for A3, a 3- to 8-membered monocyclic non-aromatic carbocyclic group, bicyclo[1.1.1]pentan-1-yl, cuban-1-yl, and bicyclo[2.2.1]heptan-1-yl are preferred, and cyclobutyl and cyclohexyl are more preferred.

In the compound 1f-2, as the group with which the non-aromatic heterocyclic group for A2 is optionally substituted, a halogen atom and alkyl having 1 to 6 carbon atoms are preferred.

In the compound 1f-2, as the group with which the non-aromatic carbocyclic group for A3 is optionally substituted, 1 to 3 groups selected from the group consisting of amino, aminoalkyl having 1 to 6 carbon atoms, hydroxy, and alkyl are preferred.

In the compound 1f-2, as X¹, a carbon atom is preferred. As R¹, a halogen atom, alkyl having 1 to 6 carbon atoms, dihaloalkyl having 1 to 6 carbon atoms, trihaloalkyl having 1 to 6 carbon atoms, alkoxy having 1 to 6 carbon atoms, dihaloalkoxy having 1 to 6 carbon atoms, trihaloalkoxy having 1 to 6 carbon atoms, and alkylsulfonyl having 1 to 6 carbon atoms are preferred, a halogen atom, dihaloalkyl having 1 to 6 carbon atoms, trihaloalkyl having 1 to 6 carbon atoms, dihaloalkoxy having 1 to 6 carbon atoms, and trihaloalkoxy having 1 to 6 carbon atoms are more preferred, and a halogen atom, trihaloalkyl having 1 to 6 carbon atoms, and trihaloalkoxy having 1 to 6 carbon atoms are further preferred. As R², a hydrogen atom and a halogen atom are preferred. As R³, a hydrogen atom is preferred. As X⁴, a carbon atom is preferred, and as R⁴, a hydrogen atom is preferred. As m, 0 and 1 are preferred, and 0 is more preferred. As R¹², a hydrogen atom and alkyl having 1 to 6 carbon atoms are preferred, alkyl having 1 to 6 carbon atoms is more preferred, and alkyl having 1 to 3 carbon atoms is further preferred.

In a compound 1f-3, as A3, a 3- to 8-membered monocyclic non-aromatic carbocyclic group is preferred. As the group with which the 3- to 8-membered monocyclic non-aromatic carbocyclic group is optionally substituted, amino is preferred. As X¹, a carbon atom is preferred, and as R¹, trihaloalkyl is preferred. As R², a hydrogen atom is preferred. As R³, a hydrogen atom is preferred. As X⁴, a carbon atom is preferred, and as R⁴, a hydrogen atom is preferred.

In the compound 1f-3, as the non-aromatic heterocyclic group for A2, pyrrolidinyl, piperidinyl, and piperazinyl are preferred, and piperidinyl is more preferred. As the non-aromatic carbocyclic group for A3, a 3- to 8-membered monocyclic non-aromatic carbocyclic group is preferred. Cyclobutyl and cyclohexyl are more preferred, and cyclohexyl is further preferred.

In the compound 1f-3, as the group with which the non-aromatic carbocyclic group for A2 is optionally substituted, amino and piperazinyl are preferred.

In a compound 1g, as the non-aromatic heterocyclic group for A2, piperidinyl is preferred, and as the group with which the non-aromatic carbocyclic group for A2 is optionally substituted, acetyl is preferred. As R², a hydrogen atom is preferred. As R³, a halogen atom is preferred. As X⁴, a carbon atom is preferred, and as R⁴, a hydrogen atom is preferred.

In compounds 1i-1 and 1i-2, as the non-aromatic heterocyclic group for A2, piperidinyl is preferred, and as the group with which the non-aromatic carbocyclic group for A2 is optionally substituted, alkyl having 1 to 6 carbon atoms is preferred. As X¹, a carbon atom is preferred, and as R¹, trihaloalkyl is preferred. As R², a hydrogen atom is preferred. As R³, a hydrogen atom is preferred. As X⁴, a carbon atom is preferred, and as R⁴, a hydrogen atom is preferred.

In A of a compound 1i-3, a 3- to 8-membered monocyclic non-aromatic carbocyclic group is preferred. As the group with which the 3- to 8-membered monocyclic non-aromatic carbocyclic group is optionally substituted, amino is preferred. As X¹, a carbon atom is preferred, and as R¹, a halogen atom is preferred. As R², a hydrogen atom is preferred. As R³, a hydrogen atom is preferred. As X⁴, a carbon atom is preferred, and as R⁴, a hydrogen atom is preferred.

The compound of the present invention can be prepared from a known compound or an easily synthesizable intermediate, for example, according to the following method, Examples described below, or a known method. In the preparation of the compound of the present invention, in the case where a starting material has a substituent that affects the reaction, the reaction is generally carried out after protecting the starting material with a suitable protective group in advance by a known method. The protective group can be removed by a known method after the reaction.

The 1,3,4-oxadiazolone compound according to the present invention may be used as it is for pharmaceuticals, and can also be used in the form of a pharmaceutically acceptable salt or solvate, or solvate of the salt according to a known method. Examples of pharmaceutically acceptable salts include salts of inorganic acids such as hydrochloric acid, hydrobromic acid, sulfuric acid, phosphoric acid, and organic acids such as acetic acid, malic acid, lactic acid, citric acid, tartaric acid, maleic acid, succinic acid, fumaric acid, p-toluenesulfonic acid, benzenesulfonic acid, and methanesulfonic acid, salts with alkali metal such as lithium, potassium, and sodium, salts with alkaline earth metal such as magnesium and calcium, and salts with organic base such as ammonium salts. These salts can be formed by methods well known in the art.

For example, a hydrochloride salt of the 1,3,4-oxadiazolone compound of the present invention can be prepared by dissolving the 1,3,4-oxadiazolone compound according to the present invention in a solution of hydrogen chloride in alcohol, a solution of hydrogen chloride in ethyl acetate, a solution of hydrogen chloride in 1,4-dioxane, a solution of hydrogen chloride in cyclopentyl methyl ether, or a solution of hydrogen chloride in diethyl ether.

Some of the compounds of the present invention may have an asymmetric carbon, and the respective stereo isomers and mixtures thereof are all included in the present invention. The stereo isomers can be prepared, for example, by means of optical resolution from the racemate thereof according to a known method using an optically active acid (for example, tartaric acid, dibenzoyltartaric acid, mandelic acid, 10-camphor sulfonic acid, etc.), utilizing its basicity, or by using an optically active compound prepared in advance as a starting material. In addition, the stereo isomers may also be prepared by optical resolution using a chiral column or by asymmetric synthesis.

The formula [1] of the present invention is not limited to a specific isomer, but includes all possible isomers and racemates.

### (Preparation method for the compound of the present invention)

The Compound [1] of the present invention and a salt thereof can be prepared from a known compound per se or an intermediate that is easily preparable from the known compound, according to the following method, Examples described below, or a known method.

If the solvents, reagents and starting materials used in each step in the following preparation methods are commercially available, such commercially available products can be used as they are. Also, the compound obtained or the starting material used in each step in the following preparation method may form a salt and can be converted by a known method into another type of salt or a free form. Alternatively, when the compound obtained or the starting material used in each step in the following preparation method is a free form, it can be converted into a desired salt by a known method. Examples of such salts include those similar to the salts described above for the compound of the present invention.

In the preparation of the compound of the present invention, when the starting material has a substituent capable of affecting the reaction, a protective group may be introduced in these substituents by a known method in advance, and the target compound can be obtained by removing the protective group after the reaction if necessary. For such introduction of a protective group and removal of the protective group, for example, the conditions described in Wuts and Greene, "Greene's Protective Groups in Organic Synthesis", 4th edition, John Wiley & Sons Inc., 2006, or P.J. Kocienski, "Protecting Groups", 3rd edition, Thieme, 2005, may be selected and used as appropriate.

The compound obtained in each step of the following preparation methods can be isolated or purified according to a conventional method such as solvent extraction, concentration, distillation, sublimation, recrystallization, reprecipitation, chromatography, and the like. Alternatively, the compound may also be used in the next step as a reaction mixture or a crude product.

Unless otherwise specified, the reaction in each step in the following preparation methods is conducted according to known methods, for example, such as methods as described in "Comprehensive Organic Transformations: A Guide to Functional Group Preparations", 2nd Ed. by R. C. Larock, John Wiley & Sons, Inc., 1999; The Chemical Society of Japan, "Experimental Chemistry", 4th edition, Maruzen, 1992; L. Kuerti and B. Czako, "Strategic Applications of Named Reactions in Organic Synthesis", translated by Kiyoshi Tomioka, Kagaku-Dojin Publishing Company, Inc., 2006; and G. S. Zweifel and M.H. Nantz, "Modern Organic Synthesis: An Introduction", translated by Tamejiro Hiyama, Kagaku-Dojin Publishing Company, Inc., 2009, or methods in similar manners as described in the Examples, such that these methods are modified or combined as appropriate.

The outline of the Compound [1] of the present invention is as follows. 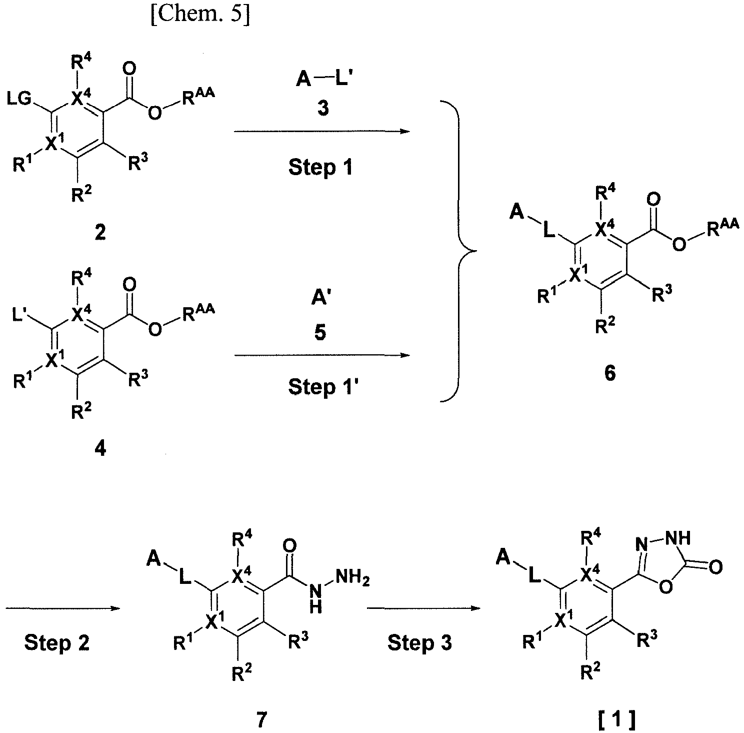 wherein X¹, X⁴, R¹, R², R³, R⁴, A, and L are as defined above, A' or L' represents a group that is converted to A or L, respectively, after the reaction, LG is a leaving group, examples of LG include halogens and trifluoromethanesulfonate, R^{AA} is alkyl, and examples of R^{AA} include methyl and ethyl.

That is, Compound 6, which is an intermediate of the compound of the present invention, is obtained by a reaction between Compound 2 and Compound 3 (Step 1) or Compound 4 and Compound 5 (Step 1'). Subsequently, the ester moiety of Compound 6 is converted to Hydrazide Product 7 (Step 2), and then the 1,3,4-Oxadiazolone Compound [1], which is the target, is obtained (Step 3).

Specifically, the Compound [1] of the present invention is configured with the following (a) to (h) depending on the combination of A and L in the formula. Furthermore, depending on the type of L, the condensed rings represented by z-1, z-2, and z-3 may be formed, and these compounds are also included.
(a) when L is a bond,
   A is aminoalkylamino, a non-aromatic heterocyclic group, an aromatic carbocyclic group, or an aromatic heterocyclic group.
(b) when L is an alkylene,
   A is a non-aromatic heterocyclic group or a non-aromatic carbocyclic group.
(c) when L is an alkenylene,
   A is a non-aromatic heterocyclic group.
(d) when L is an alkynylene,
   A is a non-aromatic heterocyclic group or a non-aromatic carbocyclic group.
(e) when L is L-1,
   A is a non-aromatic heterocyclic group, a non-aromatic carbocyclic group, or an aromatic carbocyclic group.
(f) when L is L-2,
   A is a non-aromatic heterocyclic group or a non-aromatic carbocyclic group.
(g) when L is L-3.
   A is a non-aromatic heterocyclic group.
(h) when L is L-4,
   A is 1,3-dioxa-2-yl substituted with an amino group.

More specifically, the compounds of the present invention include the compounds shown in Table 1 above.

The preparation methods for the respective compounds in the above (a) to (h) and z-1, z-2, and z-3 are described, but the preparation method for the compound of the present invention is not limited to the following examples.

The following preparation processes can be performed by methods described below.
Preparation Process a-1: a preparation process in the case where L is a bond and A is aminoalkylamino (Compound 1a-1).
Preparation Process a-2: a preparation process in the case where L is a bond and A is a non-aromatic heterocyclic group (Compound 1a-2).
Preparation Process a-3: a preparation process in the case where L is a bond and A is an aromatic carbocyclic group or an aromatic heterocyclic group (Compound 1a-3).
Preparation Process b: a preparation process in the case where L is an alkylene and A is a non-aromatic heterocyclic group or a non-aromatic carbocyclic group (Compound 1b).
Preparation Process c: a preparation process in the case where L is an alkenylene and A is a non-aromatic heterocyclic group (Compound 1c).
Preparation Process d: a preparation process in the case where L is an alkynylene and A is a non-aromatic heterocyclic group (Compound Id).
Preparation Process e: a preparation process in the case where L is L-1 and A is a non-aromatic heterocyclic group or a non-aromatic carbocyclic group (Compound 1e).
Preparation Process f-1: a preparation process in the case where L is L-2, A is a non-aromatic heterocyclic group or a non-aromatic carbocyclic group, and Y is S or -NR¹⁴- (Compound 1f-1 or 1f-2). 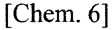 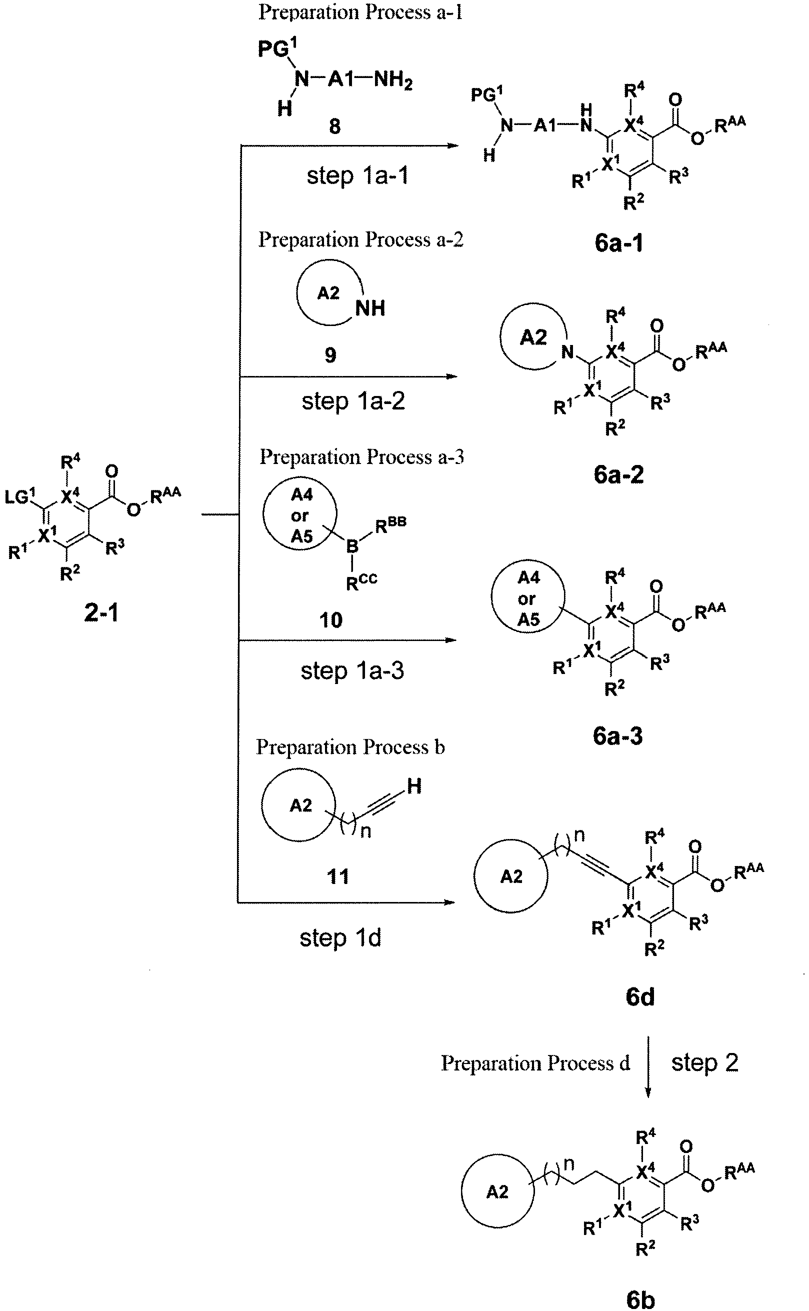
wherein X¹, X⁴, R¹, R², R³, R⁴, R¹¹, R¹², R¹⁴, A1, A2, A3, A4, A5, "A2 or A3", or "A4 or A5" is as defined above, LG¹ is a leaving group, examples of LG¹ include the same groups as those for the above LG, PG¹ is a protective group, examples of PG¹ include t-butoxycarbonyl and benzyloxycarbonyl, R^{AA} is alkyl, examples of R^{AA} include methyl and ethyl, and R^{BB} and R^{CC} both represent a hydroxy group, or R^{BB} and R^{CC} combine to form -O-C(CH₃)₂-C(CH₃)₂-O-, -O-(CH₂)₃-O-, or O-CH₂-C(CH₃)₂-CH₂-O-. 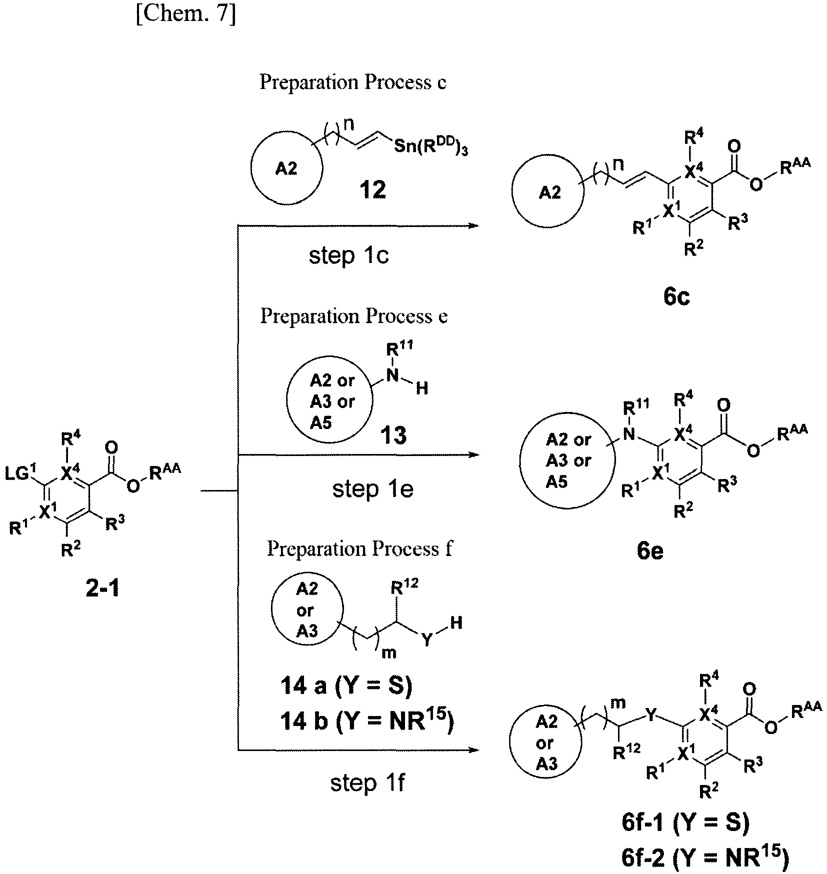 wherein X¹, X⁴, R¹, R², R³, R⁴, R¹¹, R¹², R¹⁴, A1, A2, A3, A4, A5, "A2 or A3", "A2 or A3 or A5", LG¹, and R^{AA} are as defined above, R^{DD} is alkyl, and examples of R^{DD} include methyl, ethyl, and n-butyl. 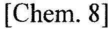 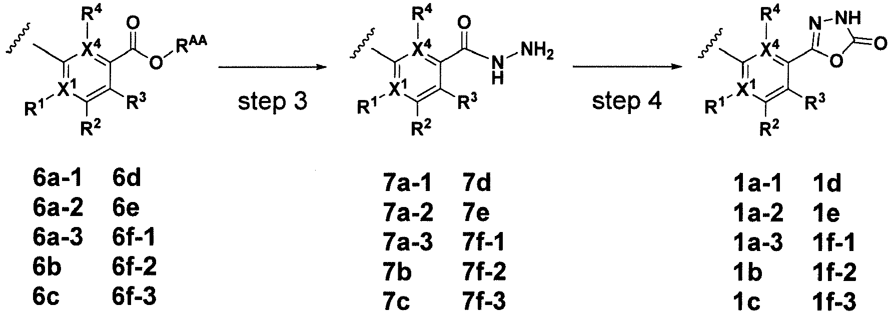 wherein X¹, X⁴, R¹, R², R³, R⁴, R¹¹, R¹², R¹⁴, and R^{AA} are as defined above.

Preparation Process a-1, Preparation Process a-2, Preparation Process a-3, Preparation Process b, Preparation Process c, Preparation Process e, and Preparation Process f-1

### Step 1

This step is a step of obtaining Compound 6a-1, 6a-2, 6a-3, 6c, 6d, 6e, 6f-1, or 6f-2 by causing a coupling reaction between Compound 2-1 and the following compound, that is, Compound 8 (Preparation Process a-1), Compound 9 (Preparation Process a-2), Compound 10 (Preparation Process a-3), Compound 11 (Preparation Process b), Compound 12 (Preparation Process c), Compound 13 (Preparation Process e), Compound 14a (Preparation Process f), or Compound 14b (Preparation Process f), respectively, in the presence of a transition metal such as palladium.

This reaction can be carried out under the conditions normally used in coupling reactions using transition metals. Examples of coupling reactions using transition metals include Suzuki-Miyaura coupling reaction, Stille reaction, Sonogashira coupling reaction, Heck reaction, and coupling reaction of Buckwald et al.

Examples of Suzuki-Miyaura coupling reaction include the reactions in documents such as Suzuki et al., Chem. Rev., 1995, 95, 2457-2483, and can be applied to Step la-3 of the above Preparation Process a-3.

Examples of Stille reaction include the reactions in documents such as Stille et al., Angew. Chem. Int. Ed. Engl., 1986, 25, 508-524, and Stille et al., Org, Synthesys, 1990, 68, 116, and can be applied to Step 1c of the above Preparation Process c.

Examples of Sonogashira coupling reaction include the reactions in documents such as Sonogashira et al., J. Organomet. Chem., 2002, 653, 46-49, and Negishi et al., Chem. Rev., 2003, 103, 1979-2017, and can be applied to Step 1d of the above Preparation Process b.

Examples of Heck reaction include the reactions in documents such as Heck et al., Org. Synth., 2005, 81, 63-76, Heck et al., J. Org. Chem., 1972, 37, 2320-2322, and Beletskaya et al, Chem. Rev., 2000, 100, 3009-3066.

Examples of coupling reaction of Buckwald et al., include the reactions in documents such as Buckwald et al., J. Am. Chem. Soc., 1994, 116, 7901-7902, Buckwald et al., Org. Synth., 2002, 78, 23-28, and Hartwig et al., Acc. Chem. Res., 2008, 41, 1534-1544, and can be applied to Step 1a-1 of the above Preparation Process a-1, Step 1a-2 of the above Preparation Process a-2, Step 1e of the above Preparation Process e, or Step If of the above Preparation Process f.

The amount of Compound 8 to Compound 13, Compound 14a, or Compound 14b to be used is preferably within the range of 0.5 to 3 molar equivalents to Compound 2-1.

The organometallic catalyst used in this reaction is not particularly limited. Preferred examples of the organometallic catalyst include metal catalysts such as tris(dibenzylideneacetone)bispalladium chloroform adduct (hereinafter, referred to as "Pd₂(dba)₃•CHCl₃"), tris(dibenzylideneacetone)bispalladium (hereinafter, referred to as "Pd2(dba)3"), tetrakistriphenylphosphine palladium (hereinafter, referred to as "Pd(PPh₃)₄"), [1,1''-bis(diphenylphosphino)ferrocene]-dichloropalladium(II)•dichloromethane adduct (hereinafter referred to as "Pd(dppf)Cl₂•CH₂Cl₂"), bis(triphenylphosphine)palladium(II) dichloride (hereinafter, referred to as "PdCl₂(PPh₃)₂"), [1,1'-bis(di-tert-butylphosphino)ferrocene]-dichloropalladium(II) (hereinafter, referred to as "Pd(dtbpf)Cl₂), bis(tricyclohexylphosphine)palladium(II) dichloride (hereinafter, referred to as "PdCl₂(PCy₃)₂"), palladium(II) acetate (hereinafter, referred to as "Pd(OAc)2"), and [1,3-bis(diphenylphosphino)propane]nickel(II), and mixtures of these metal catalysts.

The amount of the transition metal to be used is preferably within the range of, for example, 0.01 to 0.3 molar equivalents to Compound 2-1.

In this step, a base or a salt may be used as necessary. Examples of the base or the salt to be used include bases or salts such as potassium carbonate, cesium carbonate, sodium carbonate, sodium bicarbonate, sodium acetate, potassium acetate, trisodium phosphate, tripotassium phosphate, solutions thereof, triethylamine (hereinafter, referred to as "TEA"), N,N-diisopropylethylamine (hereinafter, referred to as "DIPEA"), lithium chloride, and copper(I) iodide.

The amount of the base to be used is preferably within the range of, for example, 1 to 4 molar equivalents to Compound 2-1.

In this step, a suitable ligand may be used as necessary. Examples of ligands that can be used include 1,1'-bis(diphenylphosphino)ferrocene (hereinafter, referred to as "dppf"), 4,5-bis(diphenylphosphino)-9,9-dimethylxanthene (hereinafter, referred to as "Xantphos"), 2-dicyclohexylphosphino-2',4',6'-triisopropylbiphenyl (hereinafter, referred to as "XPhos"), 2,2'-bis(diphenylphosphino)-1,1'-binaphthyl (hereinafter, referred to as "BINAP"), 2-dicyclohexylphosphino-2',6'-diisopropylbiphenyl (hereinafter, referred to as "RuPhos"), triphenylphosphine (hereinafter, referred to as "PPh₃"), and tricyclohexylphosphine (hereinafter referred to as "PCy₃").

The amount of the ligand to be used is preferably within the range of, for example, 1 to 5 molar equivalents to the transition metal to be used.

The solvent to be used in this step is not particularly limited as long as it is not involved in the reaction, and examples of the solvent include: hydrocarbons such as toluene and xylene; ethers such as 1,4-dioxane, tetrahydrofuran (hereinafter, referred to as "THF"), and dimethoxyethane (hereinafter, referred to as "DME"); amides such as N,N-dimethylformamide (hereinafter, referred to as "DMF"), N,N-dimethylacetamide (hereinafter, referred to as "DMA"), and N-methylpyrrolidone (hereinafter, referred to as "NMP"); alcohols such as ethanol, 2-propanol, and tert-butanol; water; and mixed solvents thereof.

The reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 0.1 to 24 hours.

### Step 2

This step is a reaction of reducing the triple bond of Compound 6d to obtain Compound 6b.

This reaction is carried out by reacting Compound 6d in the presence of a metal catalyst and a hydrogen source under a hydrogen pressure of 1 to 20 atm in an inert solvent.

The reaction is not limited as long as it is a metal catalyst that is usually used for the reduction of unsaturated carbon bonds, and examples of such metal catalysts include heterogeneous catalysts such as palladium-carbon, palladium black, palladium chloride, palladium hydroxide, rhodium-carbon, platinum oxide, platinum black, platinum-palladium, Raney nickel, and a palladium carbon ethylenediamine complex.

The amount of the metal catalyst to be used is usually preferably within the range of, for example, 0.001 to 1000 equivalents to Compound 6d.

Examples of the hydrogen source include hydrogen gas and ammonium formate.

In the case where ammonium formate is used as the hydrogen source, the amount of the ammonium formate to be used is usually preferably within the range of 2 to 100 equivalents to Compound 15d.

The inert solvent to be used in this step is not particularly limited as long as it is not involved in the reaction, and examples of the inert solvent include hydrocarbons such as toluene and xylene, ethers such as 1,4-dioxane, THF, and DME, amides such as DMF, DMA, and NMP, alcohols such as ethanol, 2-propanol, and tert-butanol, water, and mixed solvents thereof.

The reaction temperature can vary depending on the types of the starting material and the reagent to be used, and is usually preferably within the range of 20°C to 200°C.

The reaction time can vary depending on the type of the starting material to be used and the reaction temperature, and is usually preferably within the range of 0.1 to 24 hours. Step 3

This step is a step of converting an ester of Compound 6a-1, 6a-2, 6a-3, 6b, 6c, 6d, 6e, 6f-1, or 6f-2 into Hydrazide Compound 7a-1, 7a-2, 7a-3, 7b, 7c, 7d, 7e, 7f-1, 7f-2, or 7f-3 in the presence of hydrazine or a salt of hydrazine.

Examples of the hydrazine or salt of hydrazine to be used include hydrazine monohydrate, hydrazine hydrochloride, and hydrazine sulfate.

The amount of the hydrazine or the salt of hydrazine to be used is preferably within the range of 1 to 100 molar equivalents to Compound 6a-1, 6a-2, 6a-3, 6b, 6c, 6d, 6e, or 6f-1.

The solvent to be used in this step is not particularly limited as long as it is not involved in the reaction, and examples of the solvent include hydrocarbons such as toluene and xylene, ethers such as 1,4-dioxane, THF, and DME, amides such as DMF, DMA, and NMP, alcohols such as ethanol, 2-propanol, and tert-butanol, water, and mixed solvents thereof.

The reaction temperature can vary depending on the types of the starting material and the reagent to be used, and is usually preferably within the range of 20°C to 200°C.

The reaction time can vary depending on the type of the starting material to be used and the reaction temperature, and is usually preferably within the range of 0.1 to 24 hours.

### Step 3

This step is a step of converting Hydrazide Compound 7a-1, 7a-2, 7a-3, 7b, 7c, 7d, 7e, 7f-1, 7f-2, or 7f-3 into 1,3,4-Oxadiazolone Compound 1a-1, 1a-2, 1a-3, 1b, 1c, Id, 1e, 1f-1, 1f-2, or 1f-3, respectively, in the presence of a base and a carbonyl reagent.

Examples of the carbonylation reagent to be used include N,N'-carbonyldiimidazole, triphosgene, methyl chlorocarbonate, and ethyl chlorocarbonate.

The amount of the carbonylation reagent to be used is preferably within the range of 1 to 10 molar equivalents to the hydrazide compound which is the starting material.

Examples of the base to be used include potassium carbonate, cesium carbonate, sodium carbonate, sodium bicarbonate, sodium acetate, potassium acetate, trisodium phosphate, tripotassium phosphate, solutions thereof, TEA, DIPEA, pyridine, and 1,8-diazabicyclo[5.4.0]undec-7-ene.

The amount of the base to be used is preferably within the range of 1 to 10 molar equivalents to the hydrazide compound which is the starting material.

The solvent to be used in this step is not particularly limited as long as it is not involved in the reaction, and examples of the solvent include hydrocarbons such as toluene and xylene, ethers such as 1,4-dioxane, THF, and DME, amides such as DMF, DMA, and NMP, and mixed solvents thereof.

The reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 0.1 to 24 hours.

When a protective group is introduced as in Compound 1a'-1 below, the target compound can be obtained by removing the protective group if necessary as described above. The protective group can be removed with reference to Wuts and Greene, "Greene's Protective Groups in Organic Synthesis", 4th edition, John Wiley & Sons Inc., 2006, or P.J. Kocienski, "Protecting Groups", 3rd edition, Thieme, 2005. 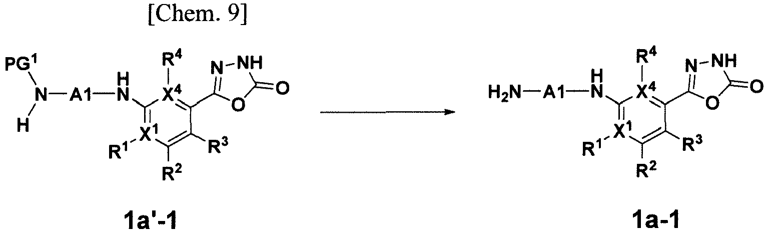 wherein X¹, X⁴, R¹, R², R³, R⁴, A1, and PG¹ are as defined above.

Preparation Process f-1: a preparation process (Part 2) in the case where L is L-2, A is a non-aromatic heterocyclic group or a non-aromatic carbocyclic group, and Y is -NR¹⁵-(Compound 1f-2).

Compound 7f-2 can also be prepared by the following method. 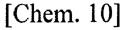 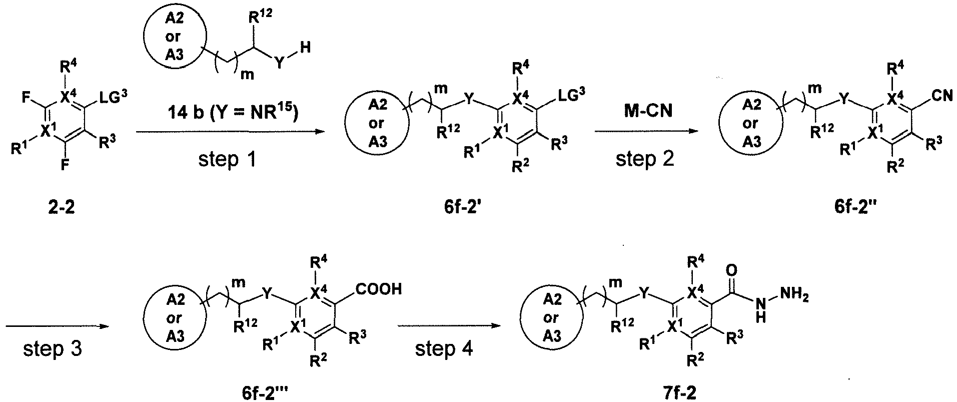 wherein X¹, X⁴, R¹, R⁴, R¹² , R¹⁴ , A2, A3, "A2 or A3", and m are as defined above, Y' is -NR¹⁵-, R¹⁴ is as defined above, LG³ is a leaving group, and examples of LG³ include a bromine atom, an iodine atom, and trifluoromethanesulfonate.

### Step 1

This step is a step of obtaining Compound 6f-2' by reacting Compound 2-2 and Compound 14b in the presence of a base in a suitable solvent.

Examples of the base to be used in this reaction include pyridine, TEA, DIPEA, potassium carbonate, and sodium bicarbonate.

The amount of the base to be used is preferably within the range of 1 to 10 molar equivalents to Compound 2-2.

The solvent to be used is not particularly limited as long as it is not involved in the reaction, and examples of the solvent include alcohols such as isopropanol, 1-butanol, and 2-methoxyethanol, ethers such as THF and 1,4-dioxane, amides such as DMF, DMA, and NMP, hydrocarbons such as benzene and toluene, dimethylsulfoxide (hereinafter, referred to as "DMSO"), acetonitrile, and mixed solvents thereof.

In this step, the reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 1 to 24 hours.

### Step 2

This step is a step of obtaining Cyano Compound 6f-2" by reacting Compound 6f-2' in the presence of a cyanide and a transition metal such as palladium in a suitable solvent.

Examples of the cyanide to be used include zinc cyanide, copper cyanide, sodium cyanide, and potassium cyanide.

The amount of the cyanide to be used is preferably within the range of 1 to 10 molar equivalents to Compound 6f-2'.

Examples of the transition metal to be used include the same transition metals as those for Steps 1 of Preparation Process a-1, Preparation Process a-2, Preparation Process a-3, Preparation Process b, Preparation Process c, Preparation Process e, and Preparation Process f-1.

The amount of the transition metal to be used is preferably within the range of, for example, 0.01 to 0.3 molar equivalents to Compound 6f-2'.

In this step, a base or a salt may be used as necessary. Examples of the base or the salt to be used include bases or salts such as potassium carbonate, cesium carbonate, sodium carbonate, sodium bicarbonate, sodium acetate, potassium acetate, trisodium phosphate, tripotassium phosphate, solutions thereof, TEA, DIPEA, lithium chloride, and copper(I) iodide.

The amount of the base to be used is preferably within the range of, for example, 1 to 4 molar equivalents to Compound 6f-2'.

The solvent to be used in this step is not particularly limited as long as it is not involved in the reaction, and examples of the solvent include hydrocarbons such as toluene and xylene, ethers such as 1,4-dioxane, THF, and DME, amides such as DMF, DMA, and NMP, alcohols such as ethanol, 2-propanol, and tert-butanol, water, and mixed solvents thereof.

The reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 0.1 to 24 hours.

### Step 3

This step is a step of obtaining Compound 6f-2'" by hydrolyzing the nitrile moiety of Compound 6f-2" in the presence of a suitable acid or base.

Examples of the acid to be used in this step include inorganic acids such as hydrochloric acid and sulfuric acid, and organic acids such as trifluoroacetic acid (hereinafter, referred to as "TFA"), methanesulfonic acid, and toluenesulfonic acid. Examples of the base include inorganic bases such as sodium hydroxide, potassium hydroxide, and lithium hydroxide.

The amount of the acid or the base to be used in this step is preferably within the range of 1 to 10 molar equivalents to Compound 6f-2". If necessary, an excess amount of the acid or the base with respect to Compound 6f-2" may be used.

The solvent to be used is not particularly limited as long as it is not involved in the reaction, and examples of the solvent include alcohols such as methanol, ethanol, and 2-propanol, ethers such as THF, diethyl ether, 1,4-dioxane, and DME, nitriles such as acetonitrile and propionitrile, ketones such as acetone, water, and mixed solvents thereof.

The reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 0.5 hours to 4 days.

Preparation Process f-2: a preparation process in the case where L is L-2, A is a non-aromatic heterocyclic group or a non-aromatic carbocyclic group, and Y is O. 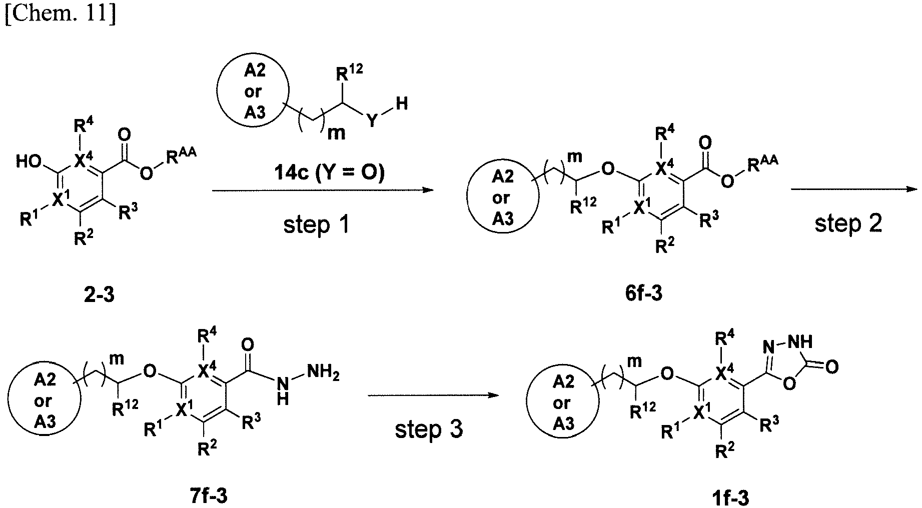 wherein X¹, X⁴, R¹, R², R³, R⁴, R¹², A2 or A3, m, and R^{AA} are as defined above.

### Step 1

This step is a step of obtaining Ether Compound 6f-3 by Mitsunobu reaction between Compound 2-3 and Compound 14c, and can be carried out according to a known method.

This step is usually carried out in the presence of an azodicarboxylic acid ester reagent and a phosphine reagent in a suitable solvent.

The amount of Compound 14c to be used is preferably within the range of 0.5 to 1.5 molar equivalents to Compound 2-3.

Examples of the azodicarboxylic acid ester reagent to be used include diethyl azodicarboxylate (hereinafter, referred to as "DEAD"), diisopropyl azodicarboxylate (hereinafter, referred to as "DIAD"), and bis (2-methoxyethyl)azodicarboxylate (hereinafter referred to as "DMEAD").

Examples of the phosphine reagent to be used include triphenylphosphine and tributylphosphine.

The amount of the azodicarboxylic acid ester reagent to be used is preferably within the range of 1 to 2 molar equivalents to Compound 2-3.

The amount of the phosphine reagent to be used is preferably within the range of 1 to 2 molar equivalents to Compound 2-3.

The solvent to be used is not particularly limited as long as it is not involved in the reaction, and examples of the solvent include hydrocarbons such as toluene and xylene, ethers such as 1,4-dioxane, THF, and DME, and mixed solvents thereof.

In this step, the reaction temperature can vary depending on the types of the starting material and the reagent to be used, and is usually preferably within the range of 0°C to 100°C.

The reaction time can vary depending on the type of the starting material to be used and the reaction temperature, and is usually preferably within the range of 0.5 hours to 24 hours.

### Step 2, Step 3

These steps are steps of converting the ester moiety of Compound 6f-3 into 1,3,4-Oxadiazolone Compound 1f-3 via Hydrazide Compound 7f-3, and 1,3,4-Oxadiazolone Compound 1f-3 can be prepared by the same method as Step 2 and Step 3 of the above Preparation Process 1a.

Preparation Process g: the case where L is L-3 and A is a non-aromatic heterocyclic group. 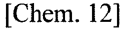 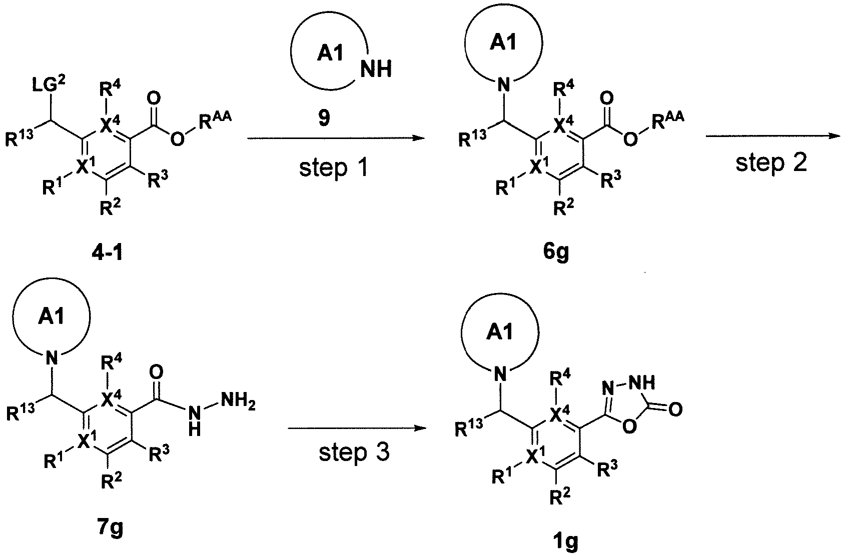 wherein X¹, X⁴, R¹, R², R³, R⁴, R^{AA}, and A1 are as defined above, LG² is a leaving group, and examples of LG² include a chlorine atom, a bromine atom, trifluoromethanesulfonate, methanesulfonate, and p-toluenesulfonate.

### Step 1

This step is a step of obtaining Compound 6g by reacting Compound 4-1 and Compound 9 in the presence of a base in a suitable solvent, and Compound 6g can be produced by the same method as Step 1 of Preparation Process f-1 (Part 2).

Examples of the base to be used in this reaction include pyridine, TEA, DIPEA, potassium carbonate, and sodium bicarbonate.

The amount of the base to be used is preferably within the range of 1 to 10 molar equivalents to Compound 4'.

The solvent to be used is not particularly limited as long as it is not involved in the reaction, and examples of the solvent include alcohols such as isopropanol, 1-butanol, and 2-methoxyethanol, ethers such as THF and 1,4-dioxane, amides such as DMF, DMA, and NMP, hydrocarbons such as benzene and toluene, DMSO, acetonitrile, halogenated hydrocarbons such as dichloromethane, and mixed solvents thereof.

In this step, the reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 1 to 24 hours.

### Step 2, Step 3

These steps are steps of converting the ester moiety of Compound 6g into 1,3,4-Oxadiazolone Compound 1g via Hydrazide Compound 7g, and 1,3,4-Oxadiazolone Compound 1g can be prepared by the same method as Step 2 and Step 3 of the above Preparation Process 1a.

Preparation Process h: the case where L is L-4 and A is 1,3-dioxa-2-yl substituted with an amino group. 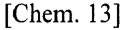 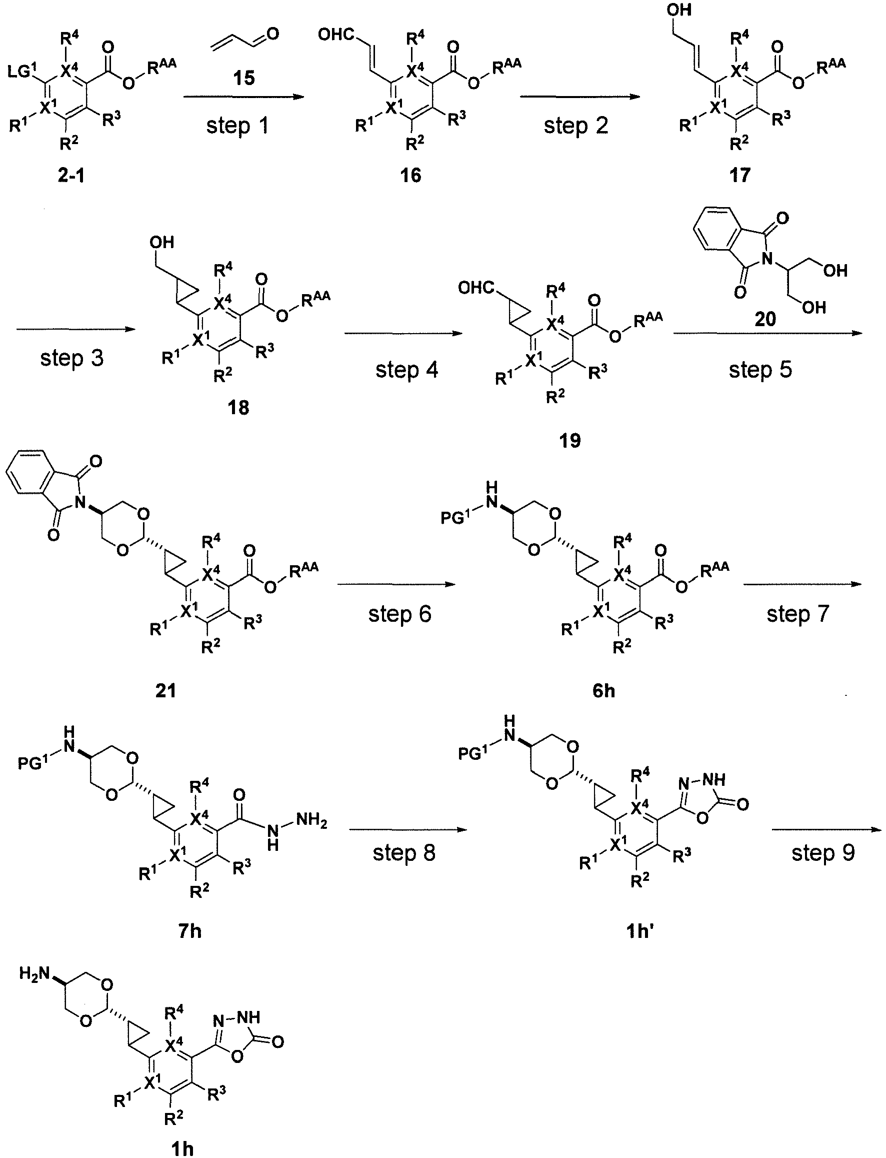 wherein X¹, X⁴, R¹, R², R³, R⁴, R^{AA}, LG¹, and PG¹ are as defined above.

### Step 1

This step is a step of obtaining Compound 16 by causing a coupling reaction between Compound 2-1 and Compound 15 in the presence of a transition metal such as palladium, and Compound 16 can be prepared by the same method as Steps 1 of the above Preparation Process a-1, Preparation Process a-2, Preparation Process a-3, Preparation Process b, Preparation Process c, Preparation Process e, and Preparation Process f-1.

### Step 2

This step is a step of reducing Compound 16 with a reducing agent to obtain Compound 17.

Examples of the reducing agent to be used include methods using metal hydrogen complex compounds such as lithium borohydride, metal hydrides such as diisobutylaluminum hydride, diborane, and substituted boranes.

The amount of the reducing agent to be used is preferably within the range of 1 to 5 molar equivalents to Compound 16.

If necessary, an organic acid such as hydrochloric acid or a Lewis acid such as lithium chloride or boron trifluoride diethyl ether complex may be used for this reaction.

In the case of using an organic acid or a Lewis acid, the amount of this acid to be used is preferably within the range of 1 to 5 molar equivalents to Compound 16, and the acid is usually used in the same molar amount as the reducing agent to be used.

The solvent to be used in this step is not particularly limited as long as it is not involved in the reaction, and examples of the solvent include alcohols such as methanol and ethanol, ethers such as THF, 1,4-dioxane, and DME, halogenated hydrocarbons such as dichloromethane, water, and mixed solvents thereof.

The reaction temperature can vary depending on the types of the starting material and the reagent to be used, and is usually preferably within the range of -10°C to 80°C.

The reaction time can vary depending on the type of the starting material to be used and the reaction temperature, and is usually preferably within the range of 0.1 to 24 hours.

### Step 3

This step is a step of cyclopropanating the double bond portion of Compound 17 to obtain Compound 18, and Compound 18 can be prepared with reference to, for example, Simmons et al., Org. Synth., 1961, 72-73, Simmons et al., J. Am. Chem. Soc., 1958, 80, 5323-5324, Simmons et al., J. Am. Chem. Soc., 1959, 81, 4256-4264., or Hoveyda et al., Chem. Rev., 1993, 93, 1307-1370.

This step is usually carried out in the presence of a zinc carbenoid prepared from dihalomethane and zinc, in a suitable solvent.

The zinc carbenoid to be used in this step is prepared from, for example, dihalomethane such as diiodomethane or dibromomethane and zinc.

In this step, zinc is usually used as an alloy with copper or silver.

Examples of zinc to be used include metallic zinc. Diethylzinc, samarium, triethylaluminum, etc., can also be used instead of zinc.

The amount of dihalomethane to be used is preferably within the range of 1 to 10 molar equivalents to Compound 17.

The amount of metallic zinc, diethylzinc, samarium, or triethylaluminum to be used is preferably within the range of 0.5 to 10 molar equivalents to Compound 17.

In this step, if necessary, an organic acid such as trifluoroacetic acid or dibutyl phosphate may be used.

The amount of the organic acid to be used is preferably within the range of, for example, 0.5 to 10 molar equivalents to Compound 17.

The solvent to be used is not particularly limited as long as it is not involved in the reaction, and examples of the solvent include hydrocarbons such as toluene and xylene, ethers such as 1,4-dioxane, THF, and DME, and mixed solvents thereof.

In this step, the reaction temperature can vary depending on the types of the starting material and the reagent to be used, and is usually preferably within the range of -20°C to 100°C.

The reaction time can vary depending on the type of the starting material to be used and the reaction temperature, and is usually preferably within the range of 0.5 hours to 24 hours.

### Step 4

This step is a step of oxidizing the hydroxyl group of Compound 18 to obtain Compound 19.

The reaction is not particularly limited as long as it can oxidize the hydroxyl group into aldehyde. Examples of the hydroxyl group oxidation reaction include the following reactions:
Dess-Martin reaction using a Dess-Martin periodinane reagent (Dess et al., J. Org. Chem., 1983, 48, 4155-4156, Dess et al., Org. Synth., 2000, 77, 141-147),
Swern oxidation using oxalyl chloride or DMSO (Swern et al., J. Org. Chem., 1978, 43, 2480-2482, Swern et al., Tetrahedron, 1978, 73, 1651-1660),
oxidation of Parik and Doering et al., using a sulfur trioxide-pyridine complex or DMSO (Parik et al., J. Am. Chem. Soc., 1967, 89, 5505-5507), and
chromate oxidation using pyridinium chlorochromate or pyridinium dichromate (Coley et al., Tetrahedron Lett., 1979, 399-402, Cheng et al., Synthesis, 1980, 223-224).
Compound 19 can be prepared with reference to these documents.

For example, in the case of obtaining Compound 19 by Dess-Martin reaction, the Dess-Martin reaction is carried out on Compound 18 in the presence of a base and a Dess-Martin periodinane reagent (hereinafter, referred to as "Dess-Martin reagent") in a suitable solvent.

The amount of the Dess-Martin reagent to be used is preferably within the range of, for example, 1 to 3 molar equivalents to Compound 18.

Examples of the base to be used include TEA, DIPEA, pyridine, and 2,6-lutidine.

The amount of the base to be used is preferably within the range of, for example, 1 to 3 molar equivalents to Compound 18.

The solvent to be used in this step is not particularly limited as long as it is not involved in the reaction, and examples of the solvent include hydrocarbons such as toluene and xylene, ethers such as 1,4-dioxane, tetrahydrofuran (hereinafter, referred to as "THF"), and dimethoxyethane (hereinafter, referred to as "DME"), halogenated hydrocarbons such as dichloromethane and dichloroethane, and mixed solvents thereof.

The reaction temperature can vary depending on the types of the starting material and the reagent to be used, and is usually preferably within the range of -20°C to 40°C.

The reaction time can vary depending on the type of the starting material to be used and the reaction temperature, and is usually preferably within the range of 0.1 to 24 hours.

### Step 5

This step is a step of reacting the aldehyde of Compound 19 and Diol Compound 20 to obtain Acetal Compound 21, and can be carried out with reference to, for example, Wuts and Greene, "Greene's Protective Groups in Organic Synthesis", 4th edition, John Wiley & Sons Inc., 2006, or P.J. Kocienski, "Protecting Groups", 3rd edition, Thieme, 2005.

### Step 6

This step is a step of cleaving the phthalimide moiety of Compound 21 to obtain an amine compound, and then introducing a protective group (PG¹) into the amine moiety of the amine compound, and can be carried out with reference to, for example, Wuts and Greene, "Greene's Protective Groups in Organic Synthesis", 4th edition, John Wiley & Sons Inc., 2006, or P.J. Kocienski, "Protecting Groups", 3rd edition, Thieme, 2005.

### Step 7, Step 8

These steps are steps of converting the ester moiety of Compound 6h into 1,3,4-Oxadiazolone Compound lh' via Hydrazide Compound 7h, and 1,3,4-Oxadiazolone Compound 1h' can be prepared by the same method as Step 2 and Step 3 of the above Preparation Process 1a.

### Step 9

This step is a step of deprotecting the protective group PG¹, and can be carried out with reference to, for example, Wuts and Greene, "Greene's Protective Groups in Organic Synthesis", 4th edition, John Wiley & Sons Inc., 2006, or P.J. Kocienski, "Protecting Groups", 3rd edition, Thieme, 2005.

Preparation Process i-1: a preparation method in the case where L is L-2, Y is - NR¹⁵-, and R¹ and R¹⁵ combine with adjacent atoms (in the case where, in z-1, R²¹ is oxo or alkyl oxime). 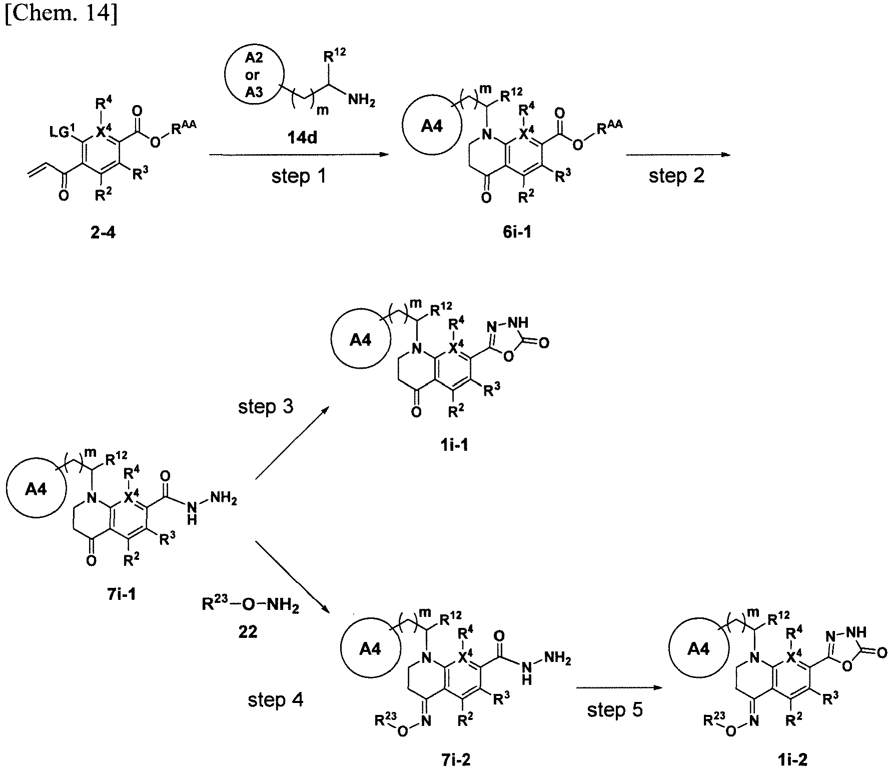 wherein X⁴, R², R³, R⁴, R¹², R²³, R^{AA}, A4, m, LG¹, and PG¹ are as defined above.

### Step 1

This step is a step of reacting Compound 2-4 and Amino Compound 14d to obtain Cyclic Compound 6i-1, and Cyclic Compound 6i-1 can be prepared by the same method as Step 1 of Preparation Process f-1 (Part 2).

### Step 2, Step 3

These steps are steps of converting the ester moiety of Compound 6i into 1,3,4-Oxadiazolone Compound 1i-1 via Hydrazide Compound 7i-1, and 1,3,4-Oxadiazolone Compound 1i-1 can be prepared by the same method as Step 2 and Step 3 of the above Preparation Process 1a.

### Step 4

This step is a step of converting Compound 7i-1 into Oxime Compound 7i-2 by reacting the ketone moiety of Compound 7i-1 with an O-alkylhydroxylamine in the presence of a base in a suitable solvent.

Examples of the O-alkylhydroxylamine to be used include O-methylhydroxylamine and O-ethylhydroxylamine.

The amount of the O-alkylhydroxylamine to be used is preferably within the range of 1 to 10 molar equivalents to Compound 7i-1.

Examples of the base to be used in this reaction include pyridine, TEA, DIPEA, potassium carbonate, and sodium bicarbonate.

The amount of the base to be used is preferably within the range of 1 to 10 molar equivalents to Compound 7i-1.

The solvent to be used is not particularly limited as long as it is not involved in the reaction, and examples of the solvent include alcohols such as isopropanol, 1-butanol, and 2-methoxyethanol, ethers such as THF and 1,4-dioxane, amides such as DMF, DMA, and NMP, hydrocarbons such as benzene and toluene, dimethylsulfoxide (hereinafter, referred to as "DMSO"), acetonitrile, and mixed solvents thereof.

In this step, the reaction temperature can vary depending on the types of the starting material and the reagent to be used, and is usually preferably within the range of 20°C to 200°C. Also, a microwave reaction apparatus may be used as necessary.

The reaction time can vary depending on the type of the starting material to be used and the reaction temperature, and is usually preferably within the range of 1 to 24 hours.

### Step 5

This step is a step of converting the hydrazide moiety of Compound 7i-2 into 1,3,4-Oxadiazolone 1i-2, and 1,3,4-Oxadiazolone 1i-2 can be prepared by the same method as Step 3 of the above Preparation Process 1a.

Preparation Process i-2: a preparation method in the case where L is L-2, Y is - NR¹⁵-, and R¹ and R¹⁵ combine with adjacent atoms (in the case where, in z-1, R²¹ is a hydrogen atom). 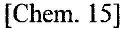 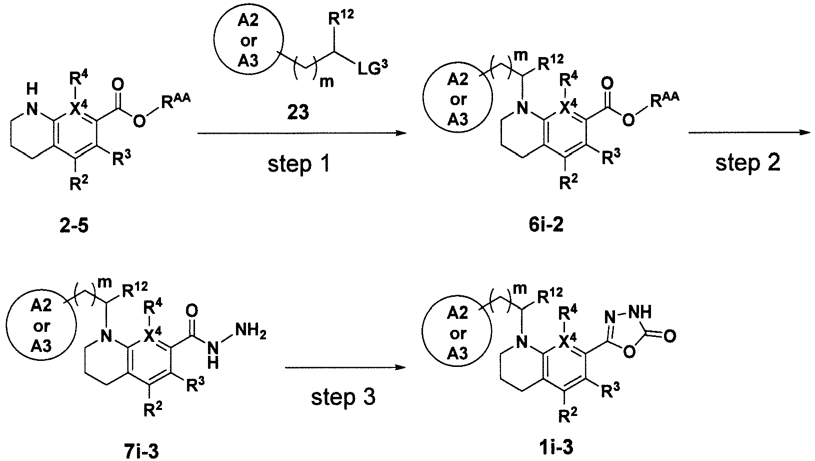 wherein X⁴, R², R³, R⁴, R¹², R^{AA}, A2 or A3, and m are as defined above, LG³ is a leaving group, and examples of LG³ include a chlorine atom, a bromine atom, trifluoromethanesulfonate, methanesulfonate, and p-toluenesulfonate.

### Step 1

This step is a step of obtaining Compound 6i-2 by reacting Compound 2-5 and Compound 23 in the presence of a base in a suitable solvent, and Compound 6i-2 can be produced by the same method as Step 1 of Preparation Process g.

### Step 2, Step 3

These steps are steps of converting the ester moiety of Compound 6i-2 into 1,3,4-Oxadiazolone Compound 1i-3 via Hydrazide Compound 7i-3, and 1,3,4-Oxadiazolone Compound 1i-3 can be prepared by the same method as Step 2 and Step 3 of the above Preparation Process 1a.

Preparation Process j-1: a preparation method (Part 1) in the case where L is L-2, Y is -NR¹⁵-, and R¹ and R¹⁵ combine with adjacent atoms (in the case of z-2). 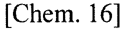 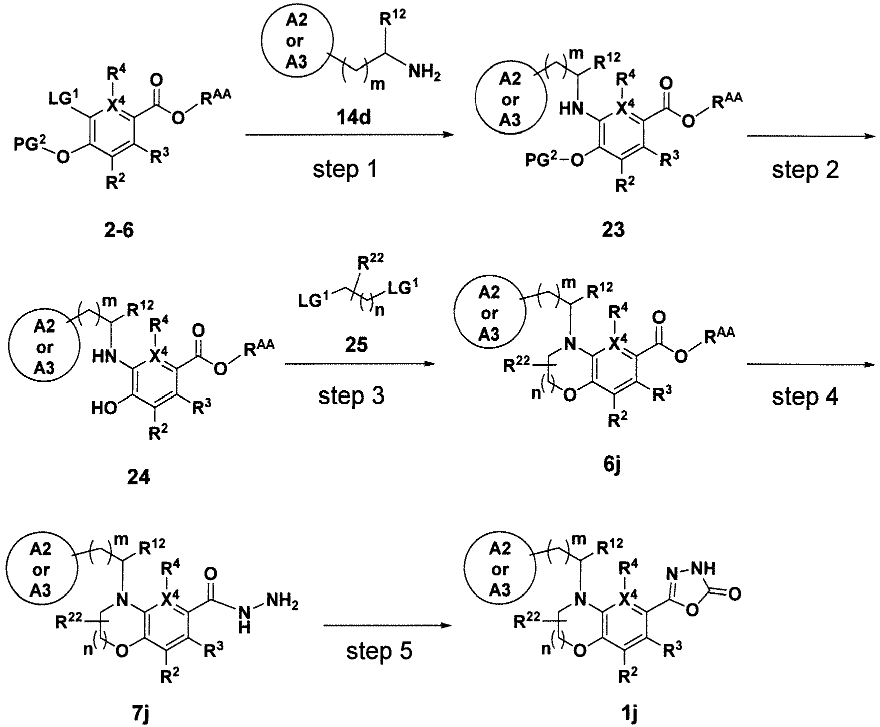 wherein X⁴, R², R³, R⁴, R¹², R²², R^{AA}, A2 or A3, m, n, and LG¹ are as defined above, PG² is a protective group, and examples of PG² include benzyl and p-methoxybenzyl.

### Step 1

This step is a step of obtaining Compound 24 by a coupling reaction between Compound 2-6 and Compound 14d in the presence of a transition metal such as palladium, and Compound 24 can be prepared by the same method as Step 1 of Preparation Process f.

### Step 2

This step is a step of deprotecting the protective group PG², and can be carried out with reference to, for example, Wuts and Greene, "Greene's Protective Groups in Organic Synthesis", 4th edition, John Wiley & Sons Inc., 2006, or P.J. Kocienski, "Protecting Groups", 3rd edition, Thieme, 2005.

### Step 3

This step is a step of obtaining Compound 6j by using Compound 26, which is an alkylating agent, on Compound 25 in the presence of a base, and can be carried out according to a known method per se.

Examples of the alkylating agent to be used include 1,2-dibromoethane and 1,3-dibromopropane.

The amount of the alkylating agent to be used is preferably within the range of 2 to 3 molar equivalents to Compound 25.

Examples of the base to be used include sodium hydride, potassium hydride, potassium carbonate, sodium carbonate, cesium carbonate, sodium bicarbonate, sodium methoxyde, sodium ethoxyde, sodium tert-butoxide, potassium tert-butoxide, and DBU.

The amount of the base to be used is preferably within the range of 2 to 5 molar equivalents to Compound 25.

The reaction solvent is not particularly limited as long as it is not involved in the reaction, and examples of the reaction solvent include amides such as DMF and DMA, ethers such as THF, nitriles such as acetonitrile, DMSO, and mixed solvents thereof.

The reaction temperature can vary depending on the types of the starting material and the reagent to be used, and is usually preferably within the range of 20°C to 150°C.

The reaction time can vary depending on the type of the starting material to be used and the reaction temperature, and is usually preferably within the range of 0.5 hours to 24 hours.

### Step 4, Step 5

These steps are steps of converting the ester moiety of Compound 6j into 1,3,4-Oxadiazolone Compound 1j via Hydrazide Compound 7j, and 1,3,4-Oxadiazolone Compound 1j can be prepared by the same method as Step 2 and Step 3 of the above Preparation Process 1a.

The above Compound 6j can also be prepared by the following method.

Preparation method for Compound 6j 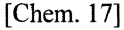 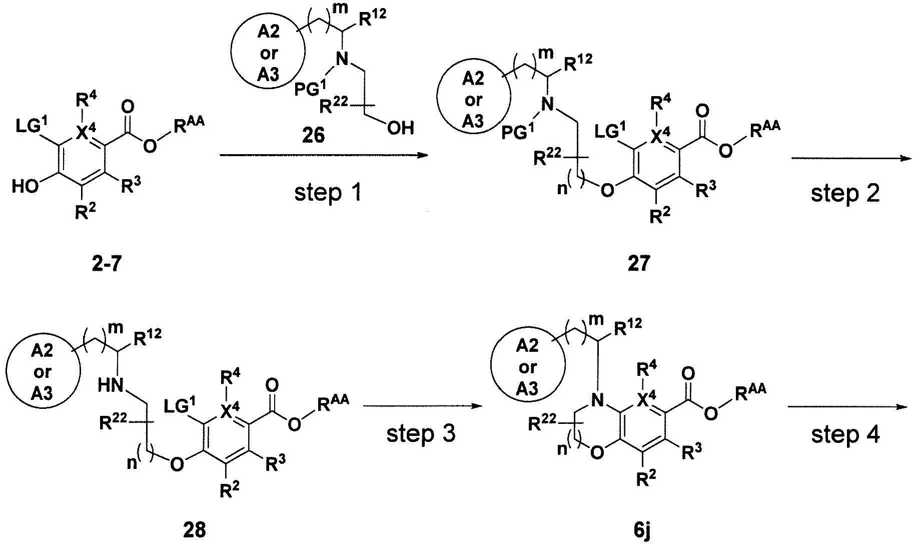 wherein X⁴, R², R³, R⁴, R¹², R²², R^{AA}, A2 or A3, m, n, PG¹, and LG¹ are as defined above.

### Step 1

This step is a step of obtaining Ether Compound 27 by Mitsunobu reaction between Compound 2-7 and Compound 26, and Ether Compound 27 can be prepared by the same method as Step 1 of Preparation Process f-2.

### Step 2

This step is a step of deprotecting the protective group PG², and can be carried out with reference to, for example, Wuts and Greene, "Greene's Protective Groups in Organic Synthesis", 4th edition, John Wiley & Sons Inc., 2006, or P.J. Kocienski, "Protecting Groups", 3rd edition, Thieme, 2005.

### Step 3

This step is a step of obtaining Compound 6j by an intramolecular coupling reaction of Compound 28 in the presence of a transition metal such as palladium, and Compound 6j can be prepared by the same method as Step 1 of Preparation Process f.

Preparation Process k: a preparation method in the case where L is L-2, Y is - NR¹⁵-, and R¹ and R¹⁵ combine with adjacent atoms (in the case of z-3). 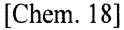 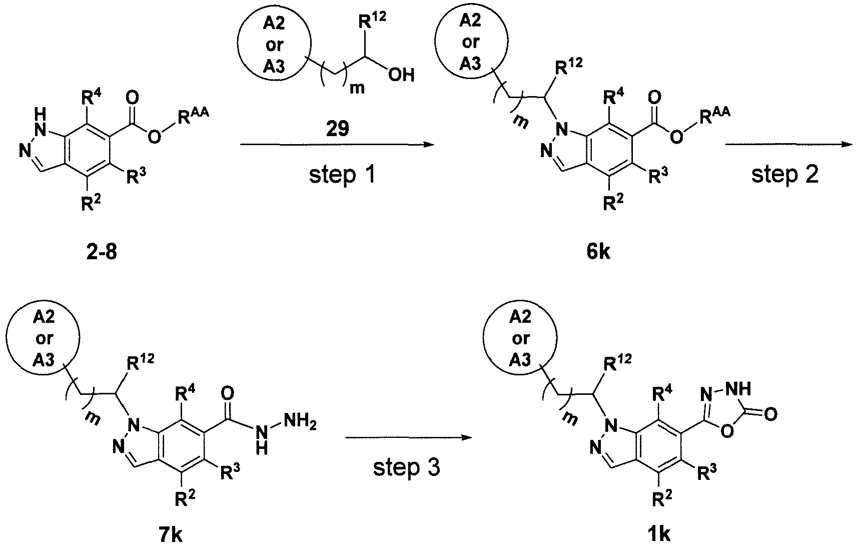 wherein R², R³, R⁴, R¹², R^{AA}, A2 or A3, and m are as defined above.

### Step 1

This step is a step of obtaining Ether Compound 6k by Mitsunobu reaction between Compound 2-8 and Compound 29, and Ether Compound 6k can be prepared by the same method as Step 1 of Preparation Process f-2.

### Step 2, Step 3

These steps are steps of converting the ester moiety of Compound 6k into 1,3,4-Oxadiazolone Compound 1k via Hydrazide Compound 7k, and 1,3,4-Oxadiazolone Compound 1k can be prepared by the same method as Step 2 and Step 3 of the above Preparation Process 1a.

The compound of the present invention has PIM kinase inhibitory activity as shown in test examples described below. Moreover, since the compound of the present invention has PIM kinase inhibitory activity, the compound of the present invention has an antiimmune disorder effect, an anti-inflammatory effect, and an anti-cancer effect.

Therefore, the compound of the present invention or a pharmaceutically acceptable salt thereof can be used as a preventive agent or a therapeutic agent for diseases in which PIM kinases are involved.

Examples of the diseases to which the compound of the present invention or a pharmaceutically acceptable salt thereof can be applied include multiple sclerosis (see, for example, PATENT DOCUMENT 1), rheumatoid arthritis (see, for example, NON PATENT DOCUMENT 4), food allergy (see, for example, NON PATENT DOCUMENT 5), asthma (see, for example, NON PATENT DOCUMENT 6), systemic lupus erythematosus (see, for example, PATENT DOCUMENT 1, NON PATENT DOCUMENT 4), lupus nephritis (see, for example, PATENT DOCUMENT 1, NON PATENT DOCUMENT 4), inflammatory bowel disease (see, for example, NON PATENT DOCUMENT 7), ulcerative colitis (see, for example, NON PATENT DOCUMENT 8), atopic dermatitis (see, for example, NON PATENT DOCUMENT 9), autoimmune lymphoproliferative syndrome (see, for example, PATENT DOCUMENT 1), chronic obstructive pulmonary disease (see, for example, NON PATENT DOCUMENT 10), allergic airway disease (see, for example, NON PATENT DOCUMENT 11), eosinophilic polyangiitis granulomatosis (see, for example, NON PATENT DOCUMENT 9), hypereosinophilic syndrome (see, for example, NON PATENT DOCUMENT 9), chorioamnionitis (see, for example, NON PATENT DOCUMENT 12), ankylosing spondylitis (see, for example, NON PATENT DOCUMENT 4), myasthenia gravis (see, for example, NON PATENT DOCUMENT 13), psoriasis (see, for example, PATENT DOCUMENT 14), prostate cancer (see, for example, NON PATENT DOCUMENT 15), colon cancer (see, for example, NON PATENT DOCUMENT 16, NON PATENT DOCUMENT 17), esophageal cancer (see, for example, NON PATENT DOCUMENT 18, NON PATENT DOCUMENT 19), ovarian cancer (see, for example, NON PATENT DOCUMENT 20), uterine cancer (see, for example, NON PATENT DOCUMENT 21, NON PATENT DOCUMENT 22, NON PATENT DOCUMENT 23), renal cancer (see, for example, NON PATENT DOCUMENT 24), liver cancer (see, for example, NON PATENT DOCUMENT 25), pancreatic cancer (see, for example, NON PATENT DOCUMENT 26), gastric cancer (see, for example, NON PATENT DOCUMENT 27), breast cancer (see, for example, NON PATENT DOCUMENT 28), lung cancer (see, for example, NON PATENT DOCUMENT 29, NON PATENT DOCUMENT 30), head and neck cancer (see, for example, NON PATENT DOCUMENT 31), glioma (see, for example, NON PATENT DOCUMENT 32, NON PATENT DOCUMENT 33), osteosarcoma (see, for example, NON PATENT DOCUMENT 34, NON PATENT DOCUMENT 35, NON PATENT DOCUMENT 36), bladder cancer (see, for example, NON PATENT DOCUMENT 37), acute lymphocytic leukemia (see, for example, NON PATENT DOCUMENT 38), acute myeloid leukemia (see, for example, NON PATENT DOCUMENT 39), chronic lymphocytic leukemia (see, for example, NON PATENT DOCUMENT 40), chronic myeloid leukemia (see, for example, NON PATENT DOCUMENT 41), B-cell lymphoma (see, for example, NON PATENT DOCUMENT 42, NON PATENT DOCUMENT 43, NON PATENT DOCUMENT 44), multiple myeloma (see, for example, NON PATENT DOCUMENT 45, NON PATENT DOCUMENT 46), T-cell lymphoma (see, for example, NON PATENT DOCUMENT 47), skin cancer (see, for example, NON PATENT DOCUMENT 48), Kaposi's sarcoma (see, for example, NON PATENT DOCUMENT 49), Hodgkin's lymphoma (see, for example, NON PATENT DOCUMENT 50), myeloproliferative tumor (see, for example, NON PATENT DOCUMENT 51), adenoid cystic carcinoma (see, for example, NON PATENT DOCUMENT 52), Ewing's sarcoma (see, for example, NON PATENT DOCUMENT 53), adult T-cell leukemia (see, for example, NON PATENT DOCUMENT 54), mesothelioma (see, for example, NON PATENT DOCUMENT 55), acute promyelocytic leukemia (see, for example, NON PATENT DOCUMENT 56), choriocarcinoma (see, for example, NON PATENT DOCUMENT 57), liposarcoma (see, for example, NON PATENT DOCUMENT 58), neuroblastoma (see, for example, NON PATENT DOCUMENT 59), seminoma (see, for example, NON PATENT DOCUMENT 60), lymphoblastic lymphoma (see, for example, NON PATENT DOCUMENT 46), Epstein-Barr virus infection and hemophagocytic syndrome in which Epstein-Barr virus is known to be involved (see, for example, NON PATENT DOCUMENT 61), influenza (see, for example, NON PATENT DOCUMENT 62), hepatitis C (see, for example, NON PATENT DOCUMENT 63), salmonellosis (see, for example, NON PATENT DOCUMENT 64), herpesvirus infection (see, for example, NON PATENT DOCUMENT 65), vaginal trichomonas infection (see, for example, NON PATENT DOCUMENT 66), human granulocytic ehrlichiosis (see, for example, NON PATENT DOCUMENT 67). In addition, PIM kinases have also been reported to contribute to the pathological conditions of aplastic anemia (see, for example, NON PATENT DOCUMENT 68), atherosclerosis (see, for example, NON PATENT DOCUMENT 69, NON PATENT DOCUMENT 70), pulmonary hypertension (see, for example, NON PATENT DOCUMENT 71), diabetes (see, for example, NON PATENT DOCUMENT 69, NON PATENT DOCUMENT 70), enlarged prostate (see, for example, NON PATENT DOCUMENT 72), Alzheimer's disease (see, for example, NON PATENT DOCUMENT 73).

The compound of the present invention can be used as a therapeutic agent for various disorders as described above, for example, for mammals such as humans, mice, rats, rabbits, dogs, cats, cows, horses, pigs, and monkeys when the compound of the present invention is mixed with a pharmacologically acceptable carrier or the like to prepare a pharmaceutical composition containing, for example, 0.001% to 99.5% and preferably 0.1% to 90% of the compound of the present invention.

The dose as a pharmaceutical is preferably adjusted taking into consideration of the conditions such as age, weight, type and severity of disease of the patient, administration route, type of the compound of the present invention, whether or not it is a salt, and the type of the salt. In general, the effective amount of the compound of the present invention or a pharmaceutically acceptable salt thereof for adult, in the case of oral administration, is preferably within a range of 0.01 mg to 5g/day, preferably 1 mg to 500 mg/day. In some cases, a smaller amount may be sufficient or a larger amount may be required. Usually, the dosage can be administered once a day or can be divided and administered several times a day, or in the case of intravenous administration, the dosage can be administered rapidly or sustainably within 24 hours.

One or more hydrogen, carbon and/or the other atoms in the compound of the present invention may be replaced with an isotope of thereof. Examples of such isotopes include ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl, i.e., hydrogen, carbon, nitrogen, oxygen, phosphorus, sulfur, fluorine, iodine and chlorine. The compound substituted with such isotope may be useful as a pharmaceutical and includes all radiolabeled compounds of the compound of the present invention.

The present invention is described more detail with reference to, but is not limited to, the following Comparative Examples, Examples and Test Examples.

The following abbreviations are used in Examples.
TFA: Trifluoroacetic acid
Pd-C: Palladium-carbon
Pd₂(dba)₃: Tris(dibenzylideneacetone)bispalladium
Pd(PPh3)4: Tetrakis triphenylphosphine palladium
PdCl₂(PPh₃)₂: Bis(triphenylphosphine)palladium(II) dichloride
Pd(OAc)₂: Palladium(II) acetate
Xantphos: 4,5-bis(diphenylphosphino)-9,9-dimethylxanthene
BINAP: 2,2'-bis(diphenylphosphino)-1,1'-binaphthyl
PPh₃: Triphenylphosphine
Boc₂O: Di-tert-butyl dicarbonate
HATU: O-(7-azabenzotriazol-1-yl)-N,N,N',N'-tetramethyluronium hexafluorophosphate
HBTU: O-(benzotriazol-1-yl)-N,N,N',N'-tetramethyluronium hexafluorophosphate
THF: Tetrahydrofuran
DME: Dimethoxyethane
DMF: Dimethylformamide
DMSO: Dimethylsulfoxide
NMP: N-methylpyrrolidone
DIPEA: N,N-diisopropylethylamine
TEA: Triethylamine
BH₃-THF: Borane-tetrahydrofuran complex
CDCl₃: Deuterated chloroform
TLC: Thin layer chromatography
MS: Mass spectrometry
LCMS: High performance liquid chromatography-Mass spectrometry
ESI: Electron Spray Ionization
M: Molar concentration (mol/L)

MS was performed using LCMS. ESI was used as a method for ionization. Observed values of the mass spectrometry are expressed as m/z.

The conditions for LCMS are as follows:
Instrument: ACQUITY UPLC MS/PDA system (Waters)
Mass spectrometry: Waters 3100 MS detector
Photodiode array detector: ACQUITY PDA detector (UV-detected wave length: 210-400nm)
Column: Acquity BEH C18, 1.7µm, 2.1x50mm
Follow rate: 0.5mL/min
Colum temperature: 40°C
Solvent;
   A: 0.1% formic acid/H₂O(v/v; the same hereinafter)
   B: 0.1% formic acid/acetonitrile

¹H NMR spectrum was obtained using by JNM-ECS400 Nuclear Magnetic Resonance Spectrometer (JEOL RESONANCE Ltd.). The observed peaks were shown as chemical shift values δ (ppm) (s = singlet, d = doublet, t = triplet, q = quartet, brs = broad singlet, m = multiplet, dd = double doublet, dt = double triplet).

In the experiment using microwave, Initiator 60 (Biotage) was used, which can achieve a temperature of 40-250°C and a pressure up to 20 bar.

The compounds described herein were named using naming software, ACD/NAME® (Advanced Chemistry Development Inc.) according to IUPAC nomenclature rules, or ChemBioDraw (version 14.0, Cambridge Soft), or named according to IUPAC nomenclature.

In a name of compound, the descriptors "r" and "s" (lower case) refer to the stereochemistry of pseudoasymmetric carbon atom according IUPAC rules.

### Reference Example 1: tert-Butyl[2-methyl-2-(piperidin-4-yl)propyl]carbamate [Step 1] Preparation of tert-butyl[2-methyl-2-(pyridin-4-yl)propyl]carbamate

TEA (0.51 mL) and Boc₂O (0.84 mL) were added to a solution of 2-methyl-2-(pyridin-4-yl)propan-1-amine (0.50 g) in dichloromethane (5 mL), and the mixture was stirred at room temperature. After monitoring the consumption of the starting material on TLC, the reaction solution was concentrated under reduced pressure. The obtained residue was purified by silica gel column chromatography to afford the title compound (0.61 g). MS (m/z): 251.3 [M+H]⁺

### [Step 2] Preparation of tert-butyl[2-methyl-2-(piperidin-4-yl)propyl]carbamate

To a solution of tert-butyl[2-methyl-2-(pyridin-4-yl)propyl]carbamate (0.60 g) obtained in Step 1 in methanol (9.75 mL), after degassing, 6M hydrochloric acid (0.48 mL) and platinum(IV) oxide (60 mg) were added with stirring at room temperature under argon atmosphere. The inside of the reaction system was replaced with hydrogen, and the mixture was stirred at room temperature under hydrogen atmosphere. After monitoring the consumption of the starting material on TLC, the reaction solution was filtered through Celite (registered trademark) containing sodium bicarbonate, and the solvent was removed under reduced pressure. The residue was dissolved in ethyl acetate, then hexane was added to suspend the residue, and the precipitate was collected by filtration to afford the title compound (0.54 g). MS (m/z): 257.3 [M+H]⁺

### Reference Example 2: tert-Butyl (2-{[tert-butyl(dimethyl)silylloxy}-2-(piperidin-4-yl)ethyl]carbamate

### [Step 1] Preparation of benzyl 4-(1-hydroxy-2-nitroethyl)piperidine-1-carboxylate

Nitromethane (0.22 mL) and potassium tert-butoxide (0.23 g) were added to a solution of benzyl 4-formylpiperidine-1-carboxylate (0.50 g) in THF (6.25 mL) and tert-butanol (6.25 mL) under ice-cooling, and the mixture was stirred at room temperature for 30 minutes. Acetic acid was added to the reaction solution, and the reaction solution was diluted with ethyl acetate. Then, the organic layer was washed with water and dried over anhydrous sodium sulfate. The solvent was removed under reduced pressure, and the residue was purified by silica gel column chromatography to afford the title compound (0.59 g).

### [Step 2] Preparation of benzyl 4-(1-{[tert-butyl(dimethyl)silyl]oxy}-2-nitroethyl)piperidine-1-carboxylate

Imidazole (0.13 g) and tert-butyldimethylchlorosilane (0.29 g) were added to a solution of benzyl 4-(1-hydroxy-2-nitroethyl)piperidine-1-carboxylate (0.25 g) obtained in Step 1 in DMF (1.25 mL), and the mixture was stirred at room temperature for 3 days. The reaction solution was diluted with saturated aq. sodium bicarbonate and then extracted with ethyl acetate. The organic layer was dried over anhydrous magnesium sulfate, then the solvent was removed under reduced pressure, and the residue was purified by silica gel column chromatography to afford the title compound (0.25 g).

### [Step 3] Preparation of benzyl 4-(2-amino-1-{[tert-butyl(dimethyl)silyl]oxy}ethyl)piperidine-1-carboxylate

Ammonium chloride (16 mg) and iron powder (0.33 g) were added to a solution of benzyl 4-(1-{[tert-butyl(dimethyl)silyl]oxy}-2-nitroethyl)piperidine-1-carboxylate (0.25 g) obtained in Step 2 in ethanol (0.25 mL) and water (0.5 mL), and the mixture was stirred at 80°C for 3 hours. The insolubles were filtered off through Celite (registered trademark), and the Celite was washed with ethyl acetate. The filtrate was washed with water and dried over anhydrous sodium sulfate. The solvent was removed under reduced pressure to afford the title compound (0.20 g).

### [Step 4] Preparation of benzyl 4-(2,2,3,3,10,10-hexamethyl-8-oxo-4,9-dioxa-7-aza-3-silaundecan-5-yl)piperidine-1-carboxylate

The title compound (0.24 g) was obtained as described in Reference Example 1, Step 1, using benzyl 4-(2-amino-1-{[tert-butyl(dimethyl)silyl]oxy}ethyl)piperidine-1-carboxylate (0.20 g) obtained in Step 3, instead of 2-methyl-2-(pyridin-4-yl)propan-1-amine.

### [Step 5] Preparation of tert-butyl[2-{[tert-butyl(dimethyl)silyl]oxy}-2-(piperidin-4-yl)ethyl]carbamate

To a solution of benzyl 4-(2,2,3,3,10,10-hexamethyl-8-oxo-4,9-dioxa-7-aza-3-silaundecan-5-yl)piperidine-1-carboxylate (0.24 g) obtained in Step 4 in methanol (4.40 mL), after degassing, 10% Pd-C (24 mg) was added with stirring at room temperature under argon atmosphere, and the mixture was stirred at room temperature under hydrogen atmosphere for 2 hours. The reaction solution was filtered through Celite (registered trademark), and then the solvent was removed under reduced pressure to afford the title compound (0.16 g). MS (m/z): 359.4 [M+H]⁺

### Reference Example 3: Benzyl {1-[(1r,4r)-4-aminocyclohexyl]ethyl}carbamate

### [Step 1] Preparation of tert-butyl [(lr,4r)-4-acetylcyclohexyl]carbamate

Methyl magnesium bromide (1 M in diethyl ether, 5.24 mL) was added to a solution of tert-butyl {(1r,4r)-4-[methoxy(methyl)carbamoyl]cyclohexyl]carbamate (0.60 g) in diethyl ether (8 mL) under ice-cooling, and the mixture was stirred at room temperature. After monitoring the consumption of the starting material on TLC, the reaction solution was diluted with saturated aq. ammonium chloride and extracted with ethyl acetate. The organic layer was washed with saturated saline and dried over anhydrous magnesium sulfate, and then the solvent was removed under reduced pressure. The residue was purified by silica gel column chromatography to afford the title compound (0.41 g).

### [Step 2] Preparation of tert-butyl [(1r,4r)-4-(1-aminoethyl)cyclohexyl]carbamate

Ammonium acetate (0.65 g) and sodium cyanotrihydridoborate (80 mg) were added to a solution of tert-butyl [(1r,4r)-4-acetylcyclohexyl]carbamate (0.25 g) obtained in Step 1 in methanol (5 mL), and the mixture was stirred at room temperature overnight. 2 M aq. sodium hydroxide was added to the reaction solution to make the reaction solution basic, and then the reaction solution was extracted with ethyl acetate. The organic layer was washed with water and saturated saline and dried over anhydrous sodium sulfate, and then the solvent was removed under reduced pressure to afford the title compound (0.38 g).

### [Step 3] Preparation of benzyl (1-{(1r,4r)-4-[(tertbutoxycarbonyl)amino]cyclohexyl}ethyl)carbamate

DIPEA (0.29 mL) and benzyl chloroformate (0.15 mL) were added to a solution of tert-butyl [(1r,4r)-4-(1-aminoethyl)cyclohexyl]carbamate (0.20 g) obtained in Step 2 in dichloromethane (5 mL) under ice-cooling, and the mixture was stirred at room temperature for 3 hours and 30 minutes. Saturated aq. sodium bicarbonate was added to the reaction solution, and the mixture was extracted with chloroform. The organic layer was washed with saturated saline and dried over anhydrous magnesium sulfate, and then the solvent was removed under reduced pressure to afford the title compound (0.30 g).

### [Step 4] Preparation of benzyl {1-[(1r,4r)-4-aminocyclohexyl]ethyl}carbamate

Hydrogen chloride (4 M in 1,4-dioxane, 3 mL) was added to benzyl (1-{(1r,4r)-4-[(tert-butoxycarbonyl)amino]cyclohexyl}ethyl)carbamate (0.30 g) obtained in Step 3, and the mixture was stirred at room temperature for 20 minutes. The reaction solution was made basic with 2 M aq. sodium hydroxide, and then extracted with chloroform. The organic layer was washed with water and saturated saline and dried over anhydrous sodium sulfate, and then the solvent was removed under reduced pressure to afford the title compound (0.20 g).

### Reference Example 4: Benzyl {2-[(1r,4r)-4-aminocyclohexyl]ethyl}carbamate

### [Step 1] Preparation of benzyl (2-{(1r,4r)-4-[(tertbutoxycarbonyl)amino]cyclohexyl} ethyl)carbamate

The title compound (0.21 g) was obtained as described in Reference Example 3, Step 3, using tert-butyl [(1r,4r)-4-(2-aminoethyl)cyclohexyl]carbamate (0.20 g) instead of tert-butyl [(1r,4r)-4-(1-aminoethyl)cyclohexyl]carbamate.

### [Step 2] Preparation of benzyl {2-[(1r,4r)-4-aminocyclohexyl]ethyl}carbamate

The title compound (90 mg) was obtained as described in Reference Example 3, Step 4, using benzyl (2-{(1r,4r)-4-[(tert-butoxycarbonyl)amino]cyclohexyl}ethyl)carbamate (0.21 g) obtained in Reference Example 4, Step 1, instead of benzyl (1-{(1r,4r)-4-[(tertbutoxycarbonyl)amino]cyclohexyl}ethyl)carbamate. MS (m/z): 277.2 [M+H]⁺

### Reference Example 5: tert-Butyl (1S)-1-amino-7-azaspiro[3.5]nonane-7-carboxylate

### [Step 1] Preparation of tert-butyl (1E)-1-{[(S)-2-methylpropane-2-sulfinyl]imino}-7-azaspiro[3 .5]nonane-7-carboxylate

A solution of tert-butyl 1-oxo-7-azaspiro[3.5]nonane-7-carboxylate (0.50 g) and (S)-(-)-2-methyl-2-propanesulfinamide (0.41 g) in tetraethyl orthotitanate (1.53 g) was stirred at 60°C overnight. The reaction solution was diluted with ethyl acetate, and water was added thereto. The reaction solution was filtered through Celite (registered trademark), and the solvent was removed under reduced pressure. The residue was purified by silica gel column chromatography to afford the title compound (0.60 g).

### [Step 2] Preparation of tert-butyl (1S)-1-{[(S)-2-methylpropane-2-sulfinyl]amino}-7-azaspiro[3.5]nonane-7-carboxylate

Sodium borohydride (0.13 g) was added to a solution of tert-butyl (1E)-1-{[(S)-2-methylpropane-2-sulfinyl]imino}-7-azaspiro[3.5]nonane-7-carboxylate (0.60 g) obtained in Step 1 in THF (8.36 mL) and water (0.44 mL) at -80°C, and the mixture was stirred for 4 hours while raising the temperature from -80°C to room temperature. The reaction solution was diluted with saturated aq. ammonium chloride and extracted with ethyl acetate. The organic layer was dried over anhydrous sodium sulfate, and then the solvent was removed under reduced pressure. The residue was purified by silica gel column chromatography to afford the title compound (465 mg) and tert-butyl (1R)-1-{[(S)-2-methylpropane-2-sulfinyl]amino}-7-azaspiro[3.5]nonane-7-carboxylate (23 mg). Stereochemistry was assigned (optionally) by bioactivity and established structural similarity.

### [Step 3] Preparation of tert-butyl (1S)-1-amino-7-azaspiro[3.5]nonane-7-carboxylate

Hydrogen chloride (4 M in 1,4-dioxane solution, 0.354 mL) was added to a solution of tert-butyl (1S)-1-{[(S)-2-methylpropane-2-sulfinyl]amino}-7-azaspiro[3.5]nonane-7-carboxylate (465 mg) obtained in Step 2 in methanol (2.7 mL) under ice-cooling, and the mixture was stirred for 3 hours. 2 M aq. sodium hydroxide was added to the reaction solution to make the reaction solution basic, and then the reaction solution was extracted with ethyl acetate. The organic layer was dried over anhydrous sodium sulfate, and then the solvent was removed under reduced pressure to afford the title compound (311 mg).

### Reference Example 6: tert-Butyl(1R)-1-amino-7-azaspiro[3.5]nonane-7-carboxylate

The title compound (14 mg) was obtained as described in Reference Example 5, Step 3, using tert-butyl (1R)-1-{[(S)-2-methylpropane-2-sulfinyl]amino}-7-azaspiro[3.5]nonane-7-carboxylate (23 mg) obtained in Reference Example 5, Step 2, instead of tert-butyl (1S)-1-{[(S)-2-methylpropane-2-sulfinyl]amino}-7-azaspiro[3.5]nonane-7-carboxylate.

### Reference Example 7: tert-Butvl {(1S,4r)-4-[(1S)-1-aminoethyl]cyclohexyl}carbamate

### [Step 1] Preparation of tert-butyl [(1r,4r)-4-{(1E)-N-[(S)-2-methylpropane-2-sulfinyl]ethaneimidoyl}cyclohexyl]carbamate

The title compound (20.9 g) was obtained as described in Reference Example 5, Step 1, using tert-butyl [(1r,4r)-4-acetylcyclohexyl]carbamate (15.4 g) obtained in Reference Example 3, Step 1, instead of tert-butyl 1-oxo-7-azaspiro[3.5]nonane-7-carboxylate.

### [Step 2] Preparation of tert-butyl {(1S,4r)-4-[(1S)-1-{[(S)-2-methylpropane-2-sulfinyl]amino}ethyl]cyclohexyl}carbamate

A suspension of dichloro(p-cymene)ruthenium(II), a dimer (3.72 g), 2-amino-2-methyl-1-propanol (1.16 mL), and Molecular Sieve 4A (20.9 g) in 2-propanol (140 mL) was degassed and stirred at 80°C for 30 minutes under argon atmosphere. Subsequently, potassium tert-butoxide (3.4g) was added to a solution of tert-butyl [(1r,4r)-4-{(1E)-N-[(S)-2-methylpropane-2-sulfinyl]ethaneimidoyl}cyclohexyl]carbamate (20.9 g) obtained in Step 1 in 2-propanol (70 mL) with stirring at 50°C, and the mixture was stirred for 3 hours. The reaction solution was filtered through Celite (registered trademark), and the solvent was removed under reduced pressure. The residue was purified by silica gel column chromatography to afford the title compound (17.4 g).

### [Step 3] Preparation of tert-butyl {(1S,4r)-4-[(1S)-1-aminoethyl]cyclohexyl}carbamate

The title compound (8.5 g) was obtained as described in Reference Example 5, Step 3, using tert-butyl {(1S,4r)-4-[(1S)-1-{[(S)-2-methylpropane-2-sulfinyl]amino}ethyl]cyclohexyl}carbamate (17.4 g) obtained in Step 2 instead of tert-butyl (1S)-1-{[(S)-2-methylpropane-2-sulfinyl]amino}-7-azaspiro[3.5]nonane-7-carboxylate.

### Reference Example 8: tert-Butyl {(1R,4r)-4-[(1R)-1-aminoethyl]cyclohexyl}carbamate

### [Step 1] Preparation of tert-butyl [(1r,4r)-4-{(1E)-N-[(R)-2-methylpropane-2-sulfinyl]ethaneimidoyl}cyclohexyl]carbamate

The title compound (6.0 g) was obtained as described in Reference Example 7, Step 1, using (R)-(+)-2-methyl-2-propanesulfinamide instead of (S)-(-)-2-methyl-2-propanesulfinamide.

### [Step 2] Preparation of tert-butyl {(1R,4r)-4-[(1R)-1-{[(R)-2-methylpropane-2-sulfinyl]amino}ethyl]cyclohexyl}carbamate

The title compound (4.8 g) was obtained as described in Reference Example 7, Step 2, using tert-butyl [(1r,4r)-4-{(1E)-N-[(R)-2-methylpropane-2-sulfinyl]ethaneimidoyl}cyclohexyl]carbamate (6.0 g) obtained in Step 1 instead of tert-butyl [(1r,4r)-4-{(1E)-N-[(S)-2-methylpropane-2-sulfinyl]ethaneimidoyl} cyclohexyl] carbamate.

### [Step 3] Preparation of tert-butyl {(1R,4r)-4-[(1R)-1-aminoethyl]cyclohexyl}carbamate

The title compound (2.7 g) was obtained as described in Reference Example 5, Step 3, using tert-butyl {(1R,4r)-4-[(1R)-1-{[(R)-2-methylpropane-2-sulfinyl]amino}ethyl]cyclohexyl}carbamate (4.8 g) obtained in Step 2 instead of tert-butyl (1S)-1-{[(S)-2-methylpropane-2-sulfinyl]amino}-7-azaspiro[3.5]nonane-7-carboxylate.

### Reference Example 9: Benzyl 4-[(1R)-1-amino-2,2-difluoroethyl]piperidine-1-carboxylate

### [Step 1] Preparation of benzyl 4-[(E)-{[(S)-2-methylpropane-2-sulfinyl]imino}methyl]piperidine-1-carboxylate

The title compound (1.15 g) was obtained as described in Reference Example 5, Step 1, using benzyl 4-formylpiperidine-1-carboxylate (1.0 g) instead of tert-butyl 1-oxo-7-azaspiro[3 .5]nonane-7-carboxylate.

### [Step 2] Preparation of benzyl 4-[(1R)-2,2-difluoro-1-{[(S)-2-methylpropane-2-sulfinyl]amino}ethyl]piperidine-1-carboxylate

A solution of benzyl 4-[(E)-{[(S)-2-methylpropane-2-sulfinyl]imino}methyl]piperidine-1-carboxylate (1.15 g) obtained in Step 1 and (difluoromethyl)trimethylsilane (0.90 mL) in THF (10 mL) was added dropwise to a suspension of potassium tert-butoxide (0.74 g) in THF (10 mL) at -78°C, and the mixture was stirred for 2 hours while raising the temperature to 0°C. The reaction solution was diluted with saturated aq. ammonium chloride and extracted with ethyl acetate. The organic layer was dried over anhydrous sodium sulfate, and then the solvent was removed under reduced pressure. The residue was purified by silica gel column chromatography to afford the title compound (164 mg).

### [Step 3] Preparation of benzyl 4-[(1R)-1-amino-2,2-difluoroethyl]piperidine-1-carboxylate

The title compound (104 mg) was obtained as described in Reference Example 5, Step 3, using benzyl 4-[(1R)-2,2-difluoro-1-{[(S)-2-methylpropane-2-sulfinyl]amino}ethyl]piperidine-1-carboxylate (164 mg) obtained in Step 2 instead of tert-butyl (1S)-1-{[(S)-2-methylpropane-2-sulfinyl]amino}-7-azaspiro[3.5]nonane-7-carboxylate.

### Reference Example 10: tert-Butyl 4-(1-aminoethyl)-4-methylpiperidine-1-carboxylate

The title compound (0.60 g) was obtained as described in Reference Example 3, Step 2, using tert-butyl 4-acetyl-4-methylpiperidine-1-carboxylate (0.66 g) (for example, synthesized according to the method described in WO2013/182546 A1) instead of tert-butyl [(1r,4r)-4-acetylcyclohexyl]carbamate.

### Reference Example 11: tert-Butyl[(1r,4r)-4-(sulfanylmethyl)cyclohexyl]carbamate

Potassium carbonate (96 mg) was added to a solution of S-({(1r,4r)-4-[(tertbutoxycarbonyl)amino]cyclohexyl} methyl) ethanethioate (100 mg) (for example, synthesized according to the method described in WO2013/007765 A1) in methanol (0.6 mL), and the mixture was stirred at 50°C for 2 hours. 2 M hydrochloric acid was added to the reaction solution to make the reaction solution acidic, and then the reaction solution was extracted with diethyl ether. The organic layer was dried, and then the solvent was removed under reduced pressure to afford the title compound (70 mg).

### Reference Example 12: tert-Butyl 4-[(1S)-1-aminoethyl]-4-fluoropiperidine-1-carboxylate

### [Step 1] Preparation of tert-butyl 4-fluoro-4-[(E)-{ [(S)-2-methylpropane-2-sulfinyl]imino}methyl]piperidine-1-carboxylate

The title compound (4.19 g) was obtained as described in Reference Example 5, Step 1, using tert-butyl 4-fluoro-4-formylpiperidine-1-carboxylate (4.34 g) instead of tert-butyl 1-oxo-7-azaspiro[3.5]nonane-7-carboxylate.

### [Step 2] Preparation of tert-butyl 4-fluoro-4-[(1S)-1-{[(S)-2-methylpropane-2-sulfinyl]amino}ethyl]piperidine-1-carboxylate

A solution of tert-butyl 4-fluoro-4-[(E)-{[(S)-2-methylpropane-2-sulfinyl]imino}methyl]piperidine-1-carboxylate (2.0 g) obtained in Step 1 in toluene (10 mL) was added dropwise to a solution of methyl lithium (1.14 M in diethyl ether, 10.5 mL) in toluene (50 mL) with stirring at -80°C, and the mixture was stirred at the same temperature for 1 hour. Saturated aq. ammonium chloride was added to the reaction solution, and the organic layer was washed with water and saturated saline and dried over anhydrous sodium sulfate. Then, the solvent was removed under reduced pressure to afford the title compound (2.13 g).

### [Step 3] Preparation of tert-butyl 4-[(1S)-1-aminoethyl]-4-fluoropiperidine-1-carboxylate

The title compound (1.49 g) was obtained as described in Reference Example 5, Step 3, using tert-butyl 4-fluoro-4-[(1S)-1-{[(S)-2-methylpropane-2-sulfinyl]amino}ethyl]piperidine-1-carboxylate (2.13 g) obtained in Step 2 instead of tert-butyl (1S)-1-{[(S)-2-methylpropane-2-sulfinyl]amino}-7-azaspiro[3.5]nonane-7-carboxylate.

### Reference Example 13: tert-Butyl [(1r,4r)-4-(aminomethyl)cyclohexyl]methylcarbamate

### [Step 1] Preparation of tert-butyl {(1r,4r)-4-[(dibenzylamino)methyl]cyclohexyl}carbamate

A mixture of tert-butyl [(1r,4r)-4-(aminomethyl)cyclohexyl]carbamate hydrochloride (150 mg), potassium carbonate (235 mg), benzyl bromide (194 mg), and acetonitrile (5 mL) was stirred at 75°C for 17 hours. The reaction solution was diluted with water and extracted with ethyl acetate. The organic layer was washed with saturated saline and dried over anhydrous sodium sulfate, and then the solvent was removed under reduced pressure. The residue was purified by silica gel column chromatography to afford the title compound (208 mg).

### [Step 2] Preparation of tert-butyl {(1r,4r)-4-[(dibenzylamino)methyl]cyclohexyl} methylcarbamate

60% sodium hydride (24 mg) was added to a solution of tert-butyl {(1r,4r)-4-[(dibenzylamino)methyl]cyclohexyl}carbamate (205 mg) obtained in Step 1 in DMF (3 mL) under ice-cooling, and the mixture was stirred at the same temperature for 30 minutes. Then, iodomethane (214 mg) was added to the mixture, and the mixture was stirred at the same temperature for 2.5 hours. 60% sodium hydride (120 mg) and iodomethane (456 mg) were added, and the mixture was further stirred at room temperature for 1 hour. Water was added to the reaction solution under ice-cooling, and the reaction solution was extracted with ethyl acetate. The organic layer was washed with saturated saline and dried over anhydrous sodium sulfate, and then the solvent was removed under reduced pressure to afford the title compound (192 mg).

### [Step 3] Preparation of tert-butyl [(1r,4r)-4-(aminomethyl)cyclohexyl]methylcarbamate

The title compound (101 mg) was obtained as described in Reference Example 2, Step 5, using tert-butyl {(1r,4r)-4-[(dibenzylamino)methyl]cyclohexyl}methylcarbamate (185 mg) obtained in Step 2 instead of benzyl 4-(2,2,3,3,10,10-hexamethyl-8-oxo-4,9-dioxa-7-aza-3-silaundecan-5-yl)piperidine-1-carboxylate.

### Reference Example 14: tert-Butyl (3S)-3-(2-aminopropyl)pyrrolidine-1-carboxylate

### [Step 1] Preparation of tert-butyl (3S)-3-{2-[methoxy(methyl)amino]-2-oxoethyl}pyrrolidine-1-carboxylate

HATU (915 mg), N,O-dimethylhydroxylamine hydrochloride (235 mg), and DIPEA (0.69 mL) were added to a solution of [(3S)-1-(tert-butoxycarbonyl)pyrrolidin-3-yl]acetic acid (460 mg) in DMF (4 mL), and the mixture was stirred at room temperature for 18 hours. The reaction solution was diluted with ethyl acetate, and the organic layer was washed with saturated aq. sodium bicarbonate and saturated saline and dried over anhydrous magnesium sulfate. Then, the solvent was removed under reduced pressure to afford the title compound (545 mg).

### [Step 2] Preparation of tert-butyl (3 S)-3-(2-oxopropyl)pyrrolidine-1-carboxylate

The title compound (340 mg) was obtained as described in Reference Example 3, Step 1, using tert-butyl (3S)-3-{2-[methoxy(methyl)amino]-2-oxoethyl}pyrrolidine-1-carboxylate (545 mg) obtained in Step 1 instead of tert-butyl {(1r,4r)-4-[methoxy(methyl)carbamoyl]cyclohexyl}carbamate.

### [Step 3] Preparation of tert-butyl (3S)-3-(2-aminopropyl)pyrrolidine-1-carboxylate

The title compound (300 mg) was obtained as described in Reference Example 3, Step 2, using tert-butyl (3S)-3-(2-oxopropyl)pyrrolidine-1-carboxylate (340 mg) obtained in Step 2 instead of tert-butyl [(lr,4r)-4-acetylcyclohexyl]carbamate.

### Reference Example 15: tert-Butyl (3R)-3-(2-aminopropyl)pyrrolidine-1-carboxylate

### [Step 1] Preparation of tert-butyl (3R)-3-{2-[methoxy(methyl)amino]-2-oxoethyl}pyrrolidine-1-carboxylate

The title compound (545 mg) was obtained as described in Reference Example 14, Step 1, using [(3R)-1-(tert-butoxycarbonyl)pyrrolidin-3-yl]acetic acid (460 mg) instead of [(3S)-1-(tert-butoxycarbonyl)pyrrolidin-3-yl]acetic acid.

### [Step 2] Preparation of tert-butyl (3R)-3-(2-oxopropyl)pyrrolidine-1-carboxylate

The title compound (380 mg) was obtained as described in Reference Example 3, Step 1, using tert-butyl (3R)-3-{2-[methoxy(methyl)amino]-2-oxoethyl}pyrrolidine-1-carboxylate (545 mg) obtained in Step 1 instead of tert-butyl {(1r,4r)-4-[methoxy(methyl)carbamoyl]cyclohexyl} carbamate.

### [Step 3] Preparation of tert-butyl (3R)-3-(2-aminopropyl)pyrrolidine-1-carboxylate

The title compound (190 mg) was obtained as described in Reference Example 3, Step 2, using tert-butyl (3R)-3-(2-oxopropyl)pyrrolidine-1-carboxylate (380 mg) obtained in Step 2 instead of tert-butyl [(1r,4r)-4-acetylcyclohexyl]carbamate.

### Reference Example 16: tert-Butyl{(1S,3R)-3-[(1S)-1-aminoethyl]-2,2-dimethylcyclobutyl}carbamate

### [Step 1] Preparation of tert-butyl [(1S,3R)-3-acetyl-2,2-dimethylcyclobutyl]carbamate

Diphenylphosphoryl azide (5.3 mL) and TEA (4.3 mL) were added to a suspension of (1S,3R)-3-acetyl-2,2-dimethylcyclobutane-1-carboxylic acid (3.5 g) (for example, synthesized according to the method described in Journal of Organic Chemistry, 2000, 65, 3934-3940) in tert-butanol (40 mL), and the mixture was stirred at 85°C for 15 hours. Saturated aq. sodium bicarbonate was added to the reaction solution, and the reaction solution was extracted with ethyl acetate. The organic layer was washed with saturated saline, and the solvent was removed under reduced pressure. The residue was purified by silica gel column chromatography to afford the title compound (1.4 g).

### [Step 2] Preparation of tert-butyl [(1S,3R)-2,2-dimethyl-3-{(1E)-N-[(S)-2-methylpropane-2-sulfinyl]ethaneimidoyl}cyclobutyl]carbamate

The title compound (700 mg) was obtained as described in Reference Example 5, Step 1, using tert-butyl [(1S,3R)-3-acetyl-2,2-dimethylcyclobutyl]carbamate (1.4 g) obtained in Step 1 instead of tert-butyl 1-oxo-7-azaspiro[3.5]nonane-7-carboxylate.

### [Step 3] Preparation of tert-butyl {(1S,3R)-2,2-dimethyl-3-[(1S)-1-{[(S)-2-methylpropane-2-sulfinyl]amino}ethyl]cyclobutyl}carbamate

The title compound (450 mg) was obtained as described in Reference Example 7, Step 2, using tert-butyl [(1S,3R)-2,2-dimethyl-3-{(1E)-N-[(S)-2-methylpropane-2-sulfinyl]ethaneimidoyl}cyclobutyl]carbamate (500 mg) obtained in Step 2 instead of tert-butyl [(1r,4r)-4-{(1E)-N-[(S)-2-methylpropane-2-sulfinyl]ethaneimidoyl}cyclohexyl]carbamate.

### [Step 4] Preparation of tert-butyl {(1S,3R)-3-[(1S)-1-aminoethyl]-2,2-dimethylcyclobutyl}carbamate

The title compound (300 mg) was obtained as described in Reference Example 5, Step 3, using tert-butyl {(1S,3R)-2,2-dimethyl-3-[(1S)-1-{[(S)-2-methylpropane-2-sulfinyl]amino}ethyl]cyclobutyl}carbamate (450 mg) obtained in Step 3 instead of tert-butyl (1S)-1-{[(S)-2-methylpropane-2-sulfinyl]amino}-7-azaspiro[3.5]nonane-7-carboxylate.

### Reference Example 17: tert-Butyl 4-[(1S)-1-aminopropyl]piperidine-1-carboxylate

### [Step 1] Preparation of tert-butyl 4-[(E)-{[(R)-2-methylpropane-2-sulfinyl]imino}methyl]piperidine-1-carboxylate

The title compound (5.0 g) was obtained as described in Reference Example 8, Step 1, using tert-butyl 4-formylpiperidine-1-carboxylate (4.0 g) instead of tert-butyl [(1r,4r)-4-acetylcyclohexyl]carbamate.

### [Step 2] Preparation of tert-butyl 4-[(1S)-1-{[(R)-2-methylpropane-2-sulfinyl]amino}propyl]piperidine-1-carboxylate

Ethyl magnesium bromide (3 M in diethyl ether, 10.6 mL) was added dropwise to a solution of tert-butyl 4-[(E)-{[(R)-2-methylpropane-2-sulfinyl]imino}methyl]piperidine-1-carboxylate (5.0 g) obtained in Step 1 in dichloromethane (64 mL) at -78°C, and the mixture was stirred at the same temperature for 15 minutes. Subsequently, the mixture was stirred for 2 hours while raising the temperature from -78°C to 0°C. Saturated aq. ammonium chloride was added to the reaction solution, and the reaction solution was extracted with ethyl acetate. The organic layer was washed with water and saturated saline and dried over anhydrous magnesium sulfate, and then the solvent was removed under reduced pressure to afford the title compound (5.4 g).

### [Step 3] Preparation of tert-butyl 4-[(1S)-1-aminopropyl]piperidine-1-carboxylate

The title compound (3.6 g) was obtained as described in Reference Example 5, Step 3, using tert-butyl 4-[(1S)-1-{[(R)-2-methylpropane-2-sulfinyl]amino}propyl]piperidine-1-carboxylate (5.4 g) obtained in Step 2 instead of tert-butyl (1S)-1-{[(S)-2-methylpropane-2-sulfinyl]amino}-7-azaspiro[3.5]nonane-7-carboxylate.

### Reference Example 18: tert-Butyl 4-[(1R)-1-amino-2,2-difluoroethyl]-4-fluoropiperidine-1-carboxylate

### [Step 1] Preparation of tert-butyl 4-[(1R)-2,2-difluoro-1-{[(S)-2-methylpropane-2-sulfinyl] amino }ethyl]-4-fluoropiperidine-1-carboxylate

The title compound (390 mg) was obtained as described in Reference Example 9, Step 2, using benzyl 4-[(E)-{[(S)-2-methylpropane-2-sulfinyl]imino}methyl]piperidine-1-carboxylate (1.15 g), and tert-butyl 4-fluoro-4-[(E)-{[(S)-2-methylpropane-2-sulfinyl]imino}methyl]piperidine-1-carboxylate (500 mg) obtained in Reference Example 12, Step 1 instead of (difluoromethyl)trimethylsilane.

### [Step 2] Preparation of tert-butyl 4-[(1R)-1-amino-2,2-difluoroethyl]-4-fluoropiperidine-1-carboxylate

The title compound (78 mg) was obtained as described in Reference Example 5, Step 3, using tert-butyl 4-[(1R)-2,2-difluoro-1-{[(S)-2-methylpropane-2-sulfinyl]amino}ethyl]-4-fluoropiperidine-1-carboxylate (320 mg) obtained in Reference Example 18, Step 1 instead of tert-butyl (1S)-1-{[(S)-2-methylpropane-2-sulfinyl]amino}-7-azaspiro[3.5]nonane-7-carboxylate.

### Reference Example 19: Benzyl4-(aminomethyl)-3-methylpil)eridine-1-carboxylate

### [Step 1] Preparation of tert-butyl [(3-methylpyridin-4-yl)methyl]carbamate

The title compound (236 mg) was obtained as described in Reference Example 1, Step 1, using 1-(3-methylpyridin-4-yl)methaneamine (150 mg) instead of 2-methyl-2-(pyridin-4-yl)propan-1-amine.

### [Step 2] Preparation of tert-butyl [(3-methylpiperidin-4-yl)methyl]carbamate

The title compound (46 mg) was obtained as described in Reference Example 1, Step 2, using tert-butyl [(3-methylpyridin-4-yl)methyl]carbamate (220 mg) obtained in Step 1 instead of tert-butyl[2-methyl-2-(pyridin-4-yl)propyl]carbamate. MS (m/z): 229.3 [M+H]⁺

### [Step 3] Preparation of benzyl 4-{[(tert-butoxycarbonyl)amino]methyl}-3-methylpiperidine-1-carboxylate

The title compound (186 mg) was obtained as described in Reference Example 3, Step 3, using tert-butyl [(3-methylpiperidin-4-yl)methyl]carbamate (110 mg) obtained in Step 2 instead of tert-butyl [(1r,4r)-4-(1-aminoethyl)cyclohexyl]carbamate.

### [Step 4] Preparation of benzyl 4-(aminomethyl)-3-methylpiperidine-1-carboxylate

The title compound (109 mg) was obtained as described in Reference Example 3, Step 4, using benzyl 4-{[(tert-butoxycarbonyl)amino]methyl}-3-methylpiperidine-1-carboxylate (186 g) obtained in Step 3 instead of benzyl (1-{(1r,4r)-4-[(tertbutoxycarbonyl)amino]cyclohexyl } ethyl)carbamate.

### Reference Example 20: tert-Butyl 2-[(1S)-1-aminoethyl]-7-azaspiro[3.5]nonane-7-carboxylate

### [Step 1] Preparation of tert-butyl 2-[(E)-{[(R)-2-methylpropane-2-sulfinyl]imino}methyl]-7-azaspiro[3.5]nonane-7-carboxylate

The title compound (342 mg) was obtained as described in Reference Example 8, Step 1, using tert-butyl 2-formyl-7-azaspiro[3.5]nonane-7-carboxylate (364 mg) instead of tert-butyl [(1r,4r)-4-acetylcyclohexyl]carbamate.

### [Step 2] Preparation of tert-butyl 2-[(1S)-1-{[(R)-2-methylpropane-2-sulfinyl]amino}ethyl]-7-azaspiro[3.5]nonane-7-carboxylate

The title compound (341 mg) was obtained as described in Reference Example 17, Step 2, using methyl magnesium bromide and tert-butyl 2-[(E)-{[(R)-2-methylpropane-2-sulfinyl]imino}methyl]-7-azaspiro[3.5]nonane-7-carboxylate (342 mg) obtained in Reference Example 20, Step 1.

### [Step 3] Preparation of tert-butyl 2-[(1S)-1-aminoethyl]-7-azaspiro[3.5]nonane-7-carboxylate

The title compound (249 mg) was obtained as described in Reference Example 5, Step 3, using tert-butyl 2-[(1S)-1-{[(R)-2-methylpropane-2-sulfinyl]amino}ethyl]-7-azaspiro[3.5]nonane-7-carboxylate (341 mg) obtained in Step 2 instead of tert-butyl (1S)-1-{[(S)-2-methylpropane-2-sulfinyl]amino}-7-azaspiro[3.5]nonane-7-carboxylate.

### Reference Example 21: Benzyl 4-(aminomethyl)-2-methylpiperidine-1-carboxylate

### [Step 1] Preparation of tert-butyl [(2-methylpyridin-4-yl)methyl]carbamate

The title compound (256 mg) was obtained as described in Reference Example 1, Step 1, using 1-(2-methylpyridin-4-yl)methaneamine (150 mg) instead of 2-methyl-2-(pyridin-4-yl)propan-1-amine. MS (m/z): 223.2 [M+H]⁺

### [Step 2] Preparation of tert-butyl [(2-methylpiperidin-4-yl)methyl]carbamate

The title compound (252 mg) was obtained as described in Reference Example 1, Step 2, using tert-butyl [(2-methylpyridin-4-yl)methyl]carbamate (256 mg) obtained in Step 1 instead of tert-butyl[2-methyl-2-(pyridin-4-yl)propyl]carbamate. MS (m/z): 229.3 [M+H]⁺

### [Step 3] Preparation of benzyl 4-{[(tert-butoxycarbonyl)amino]methyl}-2-methylpiperidine-1-carboxylate

The title compound (248 mg) was obtained as described in Reference Example 3, Step 3, using tert-butyl [(2-methylpiperidin-4-yl)methyl]carbamate (252 mg) obtained in Step 2 instead of tert-butyl [(1r,4r)-4-(1-aminoethyl)cyclohexyl]carbamate.

### [Step 4] Preparation of benzyl 4-(aminomethyl)-2-methylpiperidine-1-carboxylate

The title compound (209 mg) was obtained as described in Reference Example 3, Step 4, using benzyl 4-{[(tert-butoxycarbonyl)amino]methyl}-2-methylpiperidine-1-carboxylate (248g) obtained in Step 3 instead of benzyl (1-{(1r,4r)-4-[(tertbutoxycarbonyl)amino]cyclohexyl} ethyl)carbamate.

### Reference Example 22: tert-Butyl 4-[(1S)-1-aminoethyl]-4-methylpiperidine-1-carboxylate

### [Step 1] Preparation of tert-butyl 4-methyl-4-{(1E)-N-[(S)-2-methylpropane-2-sulfinyl]ethaneimidoyl}piperidine-1-carboxylate

The title compound (320 mg) was obtained as described in Reference Example 5, Step 1, using tert-butyl 4-acetyl-4-methylpiperidine-1-carboxylate (580 mg) instead of tert-butyl 1-oxo-7-azaspiro[3.5]nonane-7-carboxylate.

### [Step 2] Preparation of tert-butyl 4-methyl-4-[(1S)-1-{[(S)-2-methylpropane-2-sulfinyl]amino}ethyl]piperidine-1-carboxylate

The title compound (180 mg) was obtained as described in Reference Example 7, Step 2, using tert-butyl 4-methyl-4-{(1E)-N-[(S)-2-methylpropane-2-sulfinyl]ethaneimidoyl}piperidine-1-carboxylate (320 mg) obtained in Step 1 instead of tertbutyl [(1r,4r)-4-{(1E)-N-[(S)-2-methylpropane-2-sulfinyl]ethaneimidoyl}cyclohexyl]carbamate.

### [Step 3] Preparation of tert-butyl 4-[(1S)-1-aminoethyl]-4-methylpiperidine-1-carboxylate

The title compound (107 mg) was obtained as described in Reference Example 5, Step 3, using tert-butyl 4-methyl-4-[(1S)-1-{[(S)-2-methylpropane-2-sulfinyl]amino}ethyl]piperidine-1-carboxylate (180 mg) obtained in Step 2 instead of tert-butyl (1S)-1-{[(S)-2-methylpropane-2-sulfinyl]amino}-7-azaspiro[3.5]nonane-7-carboxylate.

### Reference Example 23: tert-Butyl {(1R,3s)-3-[(1S)-1-aminoethyl]cyclobutyl}carbamate

### [Step 1] Preparation of tert-butyl {(ls,3s)-3-[methoxy(methyl)carbamoyl]cyclobutyl}carbamate

The title compound (44.7 g) was obtained as described in Reference Example 14, Step 1, using (1s,3s)-3-[(tert-butoxycarbonyl)amino]cyclobutane-1-carboxylic acid (36.3 g) instead of [(3S)-1-(tert-butoxycarbonyl)pyrrolidin-3-yl]acetic acid. MS (m/z): 259.6 [M+H]⁺

### [Step 2] Preparation of tert-butyl [(1s,3s)-3-acetylcyclobutyl]carbamate

The title compound (33.8 g) was obtained as described in Reference Example 3, Step 1, using tert-butyl {(1s,3s)-3-[methoxy(methyl)carbamoyl]cyclobutyl}carbamate (44.7 g) obtained in Step 1 instead of tert-butyl {(1r,4r)-4-[methoxy(methyl)carbamoyl]cyclohexyl} carbamate.

### [Step 3] Preparation of tert-butyl [(1s,3s)-3-{(1E)-N-[(S)-2-methylpropane-2-sulfinyl]ethaneimidoyl}cyclobutyl]carbamate

The title compound (24 g) was obtained as described in Reference Example 5, Step 1, using tert-butyl [(1s,3s)-3-acetylcyclobutyl]carbamate (32.8 g) obtained in Step 2 instead of tert-butyl 1-oxo-7-azaspiro[3.5]nonane-7-carboxylate.

### [Step 4] Preparation of tert-butyl {(1R,3s)-3-[(1S)-1-{[(S)-2-methylpropane-2-sulfinyl]amino}ethyl]cyclobutyl}carbamate

The title compound (11.2 g) was obtained as described in Reference Example 7, Step 2, using tert-butyl [(1s,3s)-3-{(1E)-N-[(S)-2-methylpropane-2-sulfinyl]ethaneimidoyl}cyclobutyl]carbamate (23.4 g) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl {(1R,3s)-3-[(1S)-1-aminoethyl]cyclobutyl}carbamate

The title compound (7.2 g) was obtained as described in Reference Example 5, Step 3, using tert-butyl {(1R,3s)-3-[(1S)-1-{[(S)-2-methylpropane-2-sulfinyl]amino}ethyl]cyclobutyl}carbamate (11.2 g) obtained in Step 4 instead of tert-butyl (1S)-1-{[(S)-2-methylpropane-2-sulfinyl]amino}-7-azaspiro[3.5]nonane-7-carboxylate.

### Reference Example 24: tert-Butyl 4-[(1R)-1-amino-2,2,2-trifluoroethyl]piperidine-1-carboxylate

### [Step 1] Preparation of tert-butyl 4-{(1E)-2,2,2-trifluoro-N-[(S)-2-methylpropane-2-sulfinyl]ethaneimidoyl} piperidine-1 -carboxylate

The title compound (640 mg) was obtained as described in Reference Example 5, Step 1, using tert-butyl 4-(trifluoroacetyl)piperidine-1-carboxylate (1.26 g) instead of tert-butyl 1-oxo-7-azaspiro[3.5]nonane-7-carboxylate.

### [Step 2] Preparation of tert-butyl 4-[(1R)-2,2,2-trifluoro-1-{[(S)-2-methylpropane-2-sulfinyl]amino}ethyl]piperidine-1-carboxylate

Lithium tri-sec-butylborohydride (1 M in THF, 1.6 mL) was added to a solution of tert-butyl 4-{(1E)-2,2,2-trifluoro-N-[(S)-2-methylpropane-2-sulfinyl]ethaneimidoyl}piperidine-1-carboxylate (300 mg) obtained in Step 1 in THF (8 mL) under ice-cooling, and the mixture was stirred at the same temperature for 2 hours. Saturated aq. ammonium chloride was added to the reaction solution, and the reaction solution was extracted with ethyl acetate. The organic layer was dried over anhydrous magnesium sulfate, and then the solvent was removed under reduced pressure. The residue was purified by silica gel column chromatography to afford the title compound (133 mg).

### [Step 3] Preparation of tert-butyl 4-[(1R)-1-amino-2,2,2-trifluoroethyl]piperidine-1-carboxylate

The title compound (76 mg) was obtained as described in Reference Example 5, Step 3, using tert-butyl 4-[(1R)-2,2,2-trifluoro-1-{[(S)-2-methylpropane-2-sulfinyl]amino}ethyl]piperidine-1-carboxylate (133 mg) obtained in Step 2 instead of tert-butyl (1S)-1-{[(S)-2-methylpropane-2-sulfinyl]amino}-7-azaspiro[3.5]nonane-7-carboxylate.

### Reference Example 25: tert-Butyl 4-[(1S)-1-amino-2,2,2-trifluoroethyl]piperidine-1-carboxylate

### [Step 1] Preparation of tert-butyl 4-[(1S)-2,2,2-trifluoro-1-{[(S)-2-methylpropane-2-sulfinyl]amino}ethyl]piperidine-1-carboxylate

Sodium borohydride (30 mg) was added to a solution of tert-butyl 4-{(1E)-2,2,2-trifluoro-N-[(S)-2-methylpropane-2-sulfinyl]ethaneimidoyl}piperidine-1-carboxylate (300 mg) obtained in Reference Example 24, Step 1 in THF (8 mL) and water (0.16 mL) at -50°C, and the mixture was stirred at the same temperature for 2 hours. Saturated aq. ammonium chloride was added to the reaction solution, and the reaction solution was extracted with ethyl acetate. The organic layer was dried over anhydrous magnesium sulfate, and then the solvent was removed under reduced pressure. The residue was purified by silica gel column chromatography to afford the title compound (111 mg).

### [Step 2] Preparation of tert-butyl 4-[(1S)-1-amino-2,2,2-trifluoroethyl]piperidine-1-carboxylate

The title compound (67 mg) was obtained as described in Reference Example 5, Step 3, using tert-butyl 4-[(1S)-2,2,2-trifluoro-1-{[(S)-2-methylpropane-2-sulfinyl]amino}ethyl]piperidine-1-carboxylate (111 mg) obtained in Step 1 instead of tert-butyl (1S)-1-{[(S)-2-methylpropane-2-sulfinyl]amino}-7-azaspiro[3.5]nonane-7-carboxylate.

### Reference Example 26: tert-Butyl 6-[(1S)-1-aminoethyl]-2-azaspiro[3.3]heptane-2-carboxylate

### [Step 1] Preparation of tert-butyl 6-[(E)-{[(R)-2-methylpropane-2-sulfinyl]imino}methyl]-2-azaspiro [3.3]heptane-2-carboxylate

The title compound (371 mg) was obtained as described in Reference Example 8, Step 1, using tert-butyl 6-formyl-2-azaspiro[3.3]heptane-2-carboxylate (400 mg) instead of tert-butyl [(1r,4r)-4-acetylcyclohexyl]carbamate.MS (m/z): 329.6 [M+H]⁺

### [Step 2] Preparation of tert-butyl 6-[(1S)-1-{[(R)-2-methylpropane-2-sulfinyl]amino}ethyl]-2-azaspiro[3.3] heptane-2-carboxylate

The title compound (364 mg) was obtained as described in Reference Example 20, Step 2, using tert-butyl 6-[(E)-{[(R)-2-methylpropane-2-sulfinyl]imino}methyl]-2-azaspiro[3.3]heptane-2-carboxylate (371 mg) obtained in Step 1 instead of tert-butyl 2-[(E)-{[(R)-2-methylpropane-2-sulfinyl]imino}methyl]-7-azaspiro[3.5]nonane-7-carboxylate.

### [Step 3] Preparation of tert-butyl 6-[(1S)-1-aminoethyl]-2-azaspiro[3.3]heptane-2-carboxylate

The title compound (239 mg) was obtained as described in Reference Example 5, Step 3, using tert-butyl 6-[(1S)-1-{[(R)-2-methylpropane-2-sulfinyl)amino}ethyl]-2-azaspiro[3.3]heptane-2-carboxylate (364 mg) obtained in Step 2 instead of tert-butyl (1S)-1-{[(S)-2-methylpropane-2-sulfinyl]amino}-7-azaspiro[3.5]nonane-7-carboxylate. MS (m/z): 241.2 [M+H]⁺

### Reference Example 27: tert-Butyl 4-[(1R)-1-amino-2-fluoroethyl]piperidine-1-carboxylate

### [Step 1]Preparation of tert-butyl 4-[(E)-{[(S)-2-methylpropane-2-sulfinyl]imino}methyl]piperidine-1-carboxylate

The title compound (10.9 g) was obtained as described in Reference Example 5, Step 1, using tert-butyl 4-formylpiperidine-1-carboxylate (10 g) instead of tert-butyl 1-oxo-7-azaspiro[3.5]nonane-7-carboxylate.

### [Step 2] Preparation of tert-butyl 4-[(1R)-2-(benzenesulfonyl)-2-fluoro-1-{[(S)-2-methylpropane-2-sulfinyl]amino }ethyl]piperidine-1-carboxylate

Lithium bis(trimethylsilyl)amide (1.1 M in THF, 2.5 mL) was added dropwise to a solution of tert-butyl 4-[(E)-{[(S)-2-methylpropane-2-sulfinyl]imino}methyl]piperidine-1-carboxylate (800 mg) obtained in Step 1 and fluoromethylphenyl sulfone (462 mg) in THF (13 mL) at -80°C under argon atmosphere, and the mixture was stirred at the same temperature for 1 hour. Saturated aq. ammonium chloride was added to the reaction solution, and the reaction solution was extracted with ethyl acetate. The organic layer was dried over anhydrous sodium sulfate, and then the solvent was removed under reduced pressure to afford the title compound (1.26 g). MS (m/z): 491.7 [M+H]⁺

### [Step 3] Preparation of tert-butyl 4-[(1R)-2-fluoro-1-{[(S)-2-methylpropane-2-sulfinyl]amino}ethyl]piperidine-1-carboxylate

Magnesium (1.2 g) was added to a solution of tert-butyl 4-[(1R)-2-(benzenesulfonyl)-2-fluoro-1-{[(S)-2-methylpropane-2-sulfinyl]amino } ethyl]piperidine-1 - carboxylate (1.2 g) obtained in Step 2 in methanol (24 mL), and the mixture was stirred at room temperature for 4 hours. Saturated aq. ammonium chloride was added to the reaction solution, and the reaction solution was extracted with ethyl acetate. The organic layer was dried over anhydrous sodium sulfate, and then the solvent was removed under reduced pressure. The residue was purified by silica gel column chromatography to afford the title compound (283 mg).

### [Step 4] Preparation of tert-butyl 4-[(1R)-1-amino-2-fluoroethyl]piperidine-1-carboxylate

The title compound (201 mg) was obtained as described in Reference Example 5, Step 3, using tert-butyl 4-[(1R)-2-fhioro-1-{[(S)-2-methylpropane-2-sulfinyl]amino}ethyl]piperidine-1-carboxylate (283 mg) obtained in Step 3 instead of tert-butyl (1S)-1-{[(S)-2-methylpropane-2-sulfinyl]amino}-7-azaspiro[3.5]nonane-7-carboxylate.

### Reference Example 28: tert-Butyl {(1R,3S)-3-[(1S)-1-aminoethyl]-2,2-dimethylcyclobutyl}carbamate

### [Step 1] Preparation of methyl (1S,3R)-3-acetyl-2,2-dimethylcyclobutane-1-carboxylate

Iodomethane (15.1 mL) was added to a suspension of (1S,3R)-3-acetyl-2,2-dimethylcyclobutane-1-carboxylic acid (34.3 g) and cesium carbonate (78.8 g) in DMF (300 mL), and the mixture was stirred at room temperature for 20 hours. Water was added to the reaction solution, and the reaction solution was extracted with ethyl acetate. The organic layer was washed with saturated saline, and the solvent was removed under reduced pressure. The residue was purified by silica gel column chromatography to afford the title compound (37.2 g).

### [Step 2] Preparation of methyl (1S,3R)-3-acetamide-2,2-dimethylcyclobutane-1-carboxylate

Methyl (1S,3R)-3-acetyl-2,2-dimethylcyclobutane-1-carboxylate (15.0 g) obtained in Step 1 was dissolved in DME (250 mL), and sodium azide (15.9 g) was added to the solution with stirring at -78°C under argon atmosphere. Then, methanesulfonic acid (63.4 mL) was added dropwise to the mixture, and the mixture was stirred for 15 hours while raising the temperature from -78°C to room temperature. The reaction solution was neutralized by adding 28% aqueous ammonia dropwise thereto under ice-cooling, and then extracted with ethyl acetate. The organic layer was washed with water and saturated saline and dried over anhydrous magnesium sulfate, and then the solvent was removed under reduced pressure to afford the title compound (15.0 g).

### [Step 3] Preparation of (1S,3R)-3-[(tert-butoxycarbonyl)amino]-2,2-dimethylcyclobutane-1-carboxylic acid

4 M hydrochloric acid (164 mL) was added to methyl (1S,3R)-3-acetamide-2,2-dimethylcyclobutane-1-carboxylate (32.7 g) obtained in Step 2, and the mixture was stirred at 100°C for 8 hours. Under ice-cooling, the reaction solution was neutralized by adding 13 M aq. sodium hydroxide (50 mL) thereto, then DMF (180 mL), TEA (45.7 mL), and Boc₂O (41.4 mL) were added to the reaction solution, and the mixture was stirred at room temperature for 3 hours. 5% hydrochloric acid was added to the reaction solution, and the reaction solution was extracted with ethyl acetate. The organic layer was washed with saturated saline, and the solvent was removed under reduced pressure. The residue was purified by silica gel column chromatography to afford the title compound (39.4 g).

### [Step 4] Preparation of tert-butyl {(1R,3S)-3-[methoxy(methyl)carbamoyl]-2,2-dimethylcyclobutyl}carbamate

The title compound (33.4 g) was obtained as described in Reference Example 14, Step 1, using (1S,3R)-3-[(tert-butoxycarbonyl)amino]-2,2-dimethylcyclobutane-1-carboxylic acid (39.4 g) obtained in Step 3 instead of [(3S)-1-(tert-butoxycarbonyl)pyrrolidin-3-yl]acetic acid.

### [Step 5] Preparation of tert-butyl [(1R,3S)-3-acetyl-2,2-dimethylcyclobutyl]carbamate

The title compound (3.2 g) was obtained as described in Reference Example 3, Step 1, using tert-butyl {(1R,3S)-3-[methoxy(methyl)carbamoyl]-2,2-dimethylcyclobutyl}carbamate (8.0 g) obtained in Step 4 instead of tert-butyl {(1r,4r)-4-[methoxy(methyl)carbamoyl]cyclohexyl} carbamate.

### [Step 6] Preparation of tert-butyl[(1R,3S)-2,2-dimethyl-3-{(1E)-N-[(S)-2-methylpropane-2-sulfinyl]ethaneimidoyl} cyclobutyl]carbamate

The title compound (2.2 g) was obtained as described in Reference Example 5, Step 1, using tert-butyl [(1R,3S)-3-acetyl-2,2-dimethylcyclobutyl]carbamate (3.2 g) obtained in Step 5 instead of tert-butyl 1-oxo-7-azaspiro[3.5]nonane-7-carboxylate.

### [Step 7] Preparation of tert-butyl {(1R,3S)-2,2-dimethyl-3-[(1S)-1-{[(S)-2-methylpropane-2-sulfinyl]amino}ethyl]cyclobutyl}carbamate

The title compound (760 mg) was obtained as described in Reference Example 7, Step 2, using tert-butyl [(1R,3S)-2,2-dimethyl-3-{(1E)-N-[(S)-2-methylpropane-2-sulfinyl]ethaneimidoyl}cyclobutyl]carbamate (2.2 g) obtained in Step 6 instead of tert-butyl [(1r,4r)-4-{(1E)-N-[(S)-2-methylpropane-2-sulfinyl]ethaneimidoyl}cyclohexyl]carbamate.

### [Step 8] Preparation of tert-butyl {(1R,3s)-3-[(1S)-1-aminoethyl]-2,2-dimethylcyclobutyl}carbamate

The title compound (520 mg) was obtained as described in Reference Example 5, Step 3, using tert-butyl{(1R,3S)-2,2-dimethyl-3-[(1S)-1-{[(S)-2-methylpropane-2-sulfinyl]amino}ethyl]cyclobutyl}carbamate (760 mg) obtained in Step 7 instead of tert-butyl (1S)-1-{[(S)-2-methylpropane-2-sulfinyl]amino}-7-azaspiro[3.5]nonane-7-carboxylate.

### Reference Example 29: (1S)-1-[(1R,5S,8r)-3-Benzyl-3-azabicyclo[3.2.1]octan-8-yl]ethane-1-amine

### [Step 1] Preparation of (R)-N-{(E)-[(1R,5S,8r)-3-benzyl-3-azabicyclo[3.2.1]octan-8-yl]methylidenel-2-methylpropane-2-sulfinamide

The title compound (133 mg) and (R)-N-{(E)-[(lR,5S,8s)-3-benzyl-3-azabicyclo[3.2.1]octan-8-yl]methylidene}-2-methylpropane-2-sulfinamide (397 mg) were obtained as described in Reference Example 8, Step 1, using 3-benzyl-3-azabicyclo[3.2.1]octane-8-carbaldehyde (791 mg) (for example, synthesized according to the method described in WO2006035303 A1) instead of tert-butyl [(1r,4r)-4-acetylcyclohexyl]carbamate, and purifying the obtained residue by silica gel column chromatography. MS (m/z): 333.3 [M+H]⁺
Title compound: ¹H-NMR (400 MHz, CDCl₃) δ: 7.92 (d, 1H), 7.34-7.19 (m, 5H), 3.50 (s, 2H), 2.79-2.71 (m, 2H), 2.49-2.45 (m, 1H), 2.44-2.35 (m, 2H), 1.84-1.72 (m, 2H), 1.68-1.55 (m, 3H), 1.17 (s, 9H)
(R)-N-{(E)-[(1R,5S,8s)-3-benzyl-3-azabicyclo[3.2.1]octan-8-yl]methylidene}-2-methylpropane-2-sulfinamide: ¹H-NMR (400 MHz, CDCl₃) δ: 8.42 (d, 1H), 7.32-7.17 (m, 5H), 3.47 (s, 2H), 2.64 (q, 1H), 2.56-2.47 (m, 4H), 2.45-2.37 (m, 2H), 1.97-1.86 (m, 2H), 1.79-1.70 (m, 2H), 1.22 (s, 9H)

### [Step 2] Preparation of (R)-N-{(1S)-1-[(1R,5S,8r)-3-benzyl-3-azabicyclo[3.2.1]octan-8-yl]ethyl}-2-methylpropane-2-sulfinamide

The title compound (124 mg) was obtained as described in Reference Example 20, Step 2, using (R)-N-{(E)-[(1R,5S,8r)-3-benzyl-3-azabicyclo[3.2.1]octan-8-yl]methylidene}-2-methylpropane-2-sulfinamide (133 mg) obtained in Step 1 instead of tert-butyl 2-[(E)-{[(R)-2-methylpropane-2-sulfinyl]imino}methyl]-7-azaspiro[3.5]nonane-7-carboxylate.

### [Step 3] Preparation of (1S)-1-[(1R,5S,8r)-3-benzyl-3-azabicyclo[3.2.1]octan-8-yl]ethane-1-amine

The title compound (92 mg) was obtained as described in Reference Example 5, Step 3, using (R)-N-{(1S)-1-[(1R,5S,8r)-3-benzyl-3-azabicyclo[3.2.1]octan-8-yl]ethyl}-2-methylpropane-2-sulfinamide (124 mg) obtained in Step 2 instead of tert-butyl (1S)-1-{[(S)-2-methylpropane-2-sulfinyl]amino}-7-azaspiro[3.5]nonane-7-carboxylate.

### Reference Example 30: (1S)-1-[(1R,5S,8s)-3-Benzyl-3-azabicyclo[3.2.1]octan-8-yl]ethane-1-amine

### [Step 1] Preparation of (R)-N-{(1S)-1-[(1R,5S,8s)-3-benzyl-3-azabicyclo[3.2.1]octan-8-yl]ethyl}-2-methylpropane-2-sulfinamide

The title compound (280 mg) was obtained as described in Reference Example 20, Step 2, using (R)-N-{(E)-[(1R,5S,8s)-3-benzyl-3-azabicyclo[3.2.1]octan-8-yl]methylidene}-2-methylpropane-2-sulfinamide (397 mg) obtained in Reference Example 29, Step 1 instead of tert-butyl 2-[(E)-{[(R)-2-methylpropane-2-sulfinyl]imino}methyl]-7-azaspiro[3.5]nonane-7-carboxylate.

### [Step 2] Preparation of (1S)-1-[(1R,5S,8s)-3-benzyl-3-azabicyclo[3.2.1]octan-8-yl]ethane-1-amine

The title compound (201 mg) was obtained as described in Reference Example 5, Step 3, using (R)-N-{(1S)-1-[(1R,5S,8s)-3-benzyl-3-azabicyclo[3.2.1]octan-8-yl]ethyl}-2-methylpropane-2-sulfinamide (280 mg) obtained in Step 1 instead of tert-butyl (1S)-1-{[(S)-2-methylpropane-2-sulfinyl]amino }-7-azaspiro[3.5]nonane-7-carboxylate.

### Reference Example 31: tert-Butyl 4-[(1S)-1-aminoethyl]azepane-1-carboxylate

### [Step 1] Preparation of tert-butyl 4-[(E)-{[(R)-2-methylpropane-2-sulfinyl] imino } methyl] azepane-1 -carboxylate

The title compound (524 mg) was obtained as described in Reference Example 8, Step 1, using tert-butyl 4-formylazepane-1-carboxylate (600 mg) instead of tert-butyl [(1r,4r)-4-acetylcyclohexyl]carbamate.

### [Step 2] Preparation of tert-butyl 4-[(1S)-1-{[(R)-2-methylpropane-2-sulfinyl]amino}ethyl]azepane-1-carboxylate

The title compound (450 mg) was obtained as described in Reference Example 20, Step 2, using tert-butyl 4-[(E)-{[(R)-2-methylpropane-2-sulfinyl]imino}methyl]azepane-1-carboxylate (524 mg) obtained in Step 1 instead of tert-butyl 2-[(E)-{[(R)-2-methylpropane-2-sulfinyl] imino } methyl]-7-azaspiro [3.5]nonane-7-carboxylate.

### [Step 3] Preparation of tert-butyl 4-[(1S)-1-aminoethyl]azepane-1-carboxylate

The title compound (281 mg) was obtained as described in Reference Example 5, Step 3, using tert-butyl 4-[(1S)-1-{[(R)-2-methylpropane-2-sulfinyl]amino}ethyl]azepane-1-carboxylate (450 mg) obtained in Step 2 instead of tert-butyl (1S)-1-{[(S)-2-methylpropane-2-sulfinyl]amino}-7-azaspiro[3.5]nonane-7-carboxylate. MS (m/z): 243.3 [M+H]⁺

### Reference Example 32: {(1r,4r)-4-[(tert-Butoxycarbonyl)amino]cyclohexyl}methyl trifluoromethanesulfonate

Trifluoromethanesulfonic anhydride (1.35 mL) was added dropwise to a solution of tert-butyl [(1r,4r)-4-(hydroxymethyl)cyclohexyl]carbamate (1.72 g) and pyridine (0.73 mL) in dichloromethane (75 mL) under ice-cooling, and the mixture was stirred at the same temperature for 2 hours. The reaction solution was concentrated under reduced pressure, and the obtained residue was purified by silica gel column chromatography to afford the title compound (2.43 g).

### Reference Example 33: tert-Butyl 5-[(1S)-1-aminoethyl]azocane-1-carboxylate

### [Step 1] Preparation of tert-butyl 5-formylazocane-1-carboxylate

Potassium tert-butoxide (1 M in THF, 1.98 mL) was added to a mixture of tert-butyl 5-oxoazocane-1-carboxylate (225 mg), (methoxymethyl)triphenylphosphonium chloride (679 mg), and THF (8 mL) under ice-cooling, and the mixture was stirred at room temperature for 1 hour. Subsequently, 1 M hydrochloric acid (3.96 mL) was added to the mixture, and the mixture was stirred at room temperature for 3 days. Saturated aq. sodium bicarbonate was added to the reaction solution, and the reaction solution was extracted with ethyl acetate. The organic layer was dried over anhydrous sodium sulfate, then the solvent was removed under reduced pressure, and the obtained residue was purified by silica gel column chromatography to afford the title compound (238 mg).

### [Step 2] Preparation of tert-butyl 5-[(E)-{[(R)-2-methylpropane-2-sulfinyl]imino}methyl]azocane-1-carboxylate

The title compound (238 mg) was obtained as described in Reference Example 8, Step 1, using tert-butyl 5-formylazocane-1-carboxylate (238 mg) obtained Step 1 instead of tert-butyl [(1r,4r)-4-acetylcyclohexyl]carbamate.MS (m/z): 345.6 [M+H]⁺

### [Step 3] Preparation of tert-butyl 5-[(1S)-1-{[(R)-2-methylpropane-2-sulfinyl] amino } ethyl] azocane-1 -carboxylate

The title compound (255 mg) was obtained as described in Reference Example 20, Step 2, using tert-butyl 5-[(E)-{[(R)-2-methylpropane-2-sulfinyl]imino}methyl]azocane-1-carboxylate (238 mg) obtained in Step 2 instead of tert-butyl 2-[(E)-{[(R)-2-methylpropane-2-sulfinyl]imino}methyl]-7-azaspiro[3.5]nonane-7-carboxylate.

### [Step 4] Preparation of tert-butyl 5-[(1S)-1-aminoethyl]azocane-1-carboxylate

The title compound (140 mg) was obtained as described in Reference Example 5, Step 3, using tert-butyl 5-[(1S)-1-{[(R)-2-methylpropane-2-sulfinyl]amino)ethyl]azocane-1-carboxylate (255 mg) obtained in Step 3 instead of tert-butyl (1S)-1-{[(S)-2-methylpropane-2-sulfinyl]amino}-7-azaspiro[3.5]nonane-7-carboxylate.

### Reference Example 34: tert-Butyl [(1S,3R)-3-(aminomethyl)-2,2-dimethylcyclobutyl]carbamate

### [Step 1] Preparation of benzyl {[(1R,3S)-3-acetyl-2,2-dimethylcyclobutyl]methyl}carbamate

TEA (6.6 mL) and diphenylphosphoryl azide (8.2 mL) were added to a suspension of [(1S,3S)-3-acetyl-2,2-dimethylcyclobutyl]aceticacid (5.80 g) in toluene (63 mL), and the mixture was stirred at 70°C for 30 minutes. Then, benzyl alcohol (3.9 mL) was added to the mixture, and the mixture was stirred at the same temperature for 5 hours. The reaction solution was concentrated under reduced pressure, and the obtained residue was purified by silica gel column chromatography to afford the title compound (7.20 g).

### [Step 2] Preparation of benzyl {[(1R,3S)-3-acetamide-2,2-dimethylcyclobutyl]methyl}carbamate

The title compound (5.28 g) was obtained as described in Reference Example 28, Step 2, using benzyl{[(1R,3S)-3-acetyl-2,2-dimethylcyclobutyl]methyl}carbamate (7.19 g) obtained in Step 1 instead of methyl(1S,3R)-3-acetyl-2,2-dimethylcyclobutane-1-carboxylate.

### [Step 3] Preparation of N-[(1S,3R)-3-(aminomethyl)-2,2-dimethylcyclobutyl]acetamide

The title compound (3.23 g) was obtained as described in Reference Example 2, Step 5, using benzyl {[(1R,3S)-3-acetamide-2,2-dimethylcyclobutyl]methyl}carbamate (5.28 g) obtained in Step 2 instead of benzyl 4-(2,2,3,3,10,10-hexamethyl-8-oxo-4,9-dioxa-7-aza-3-silaundecan-5-yl)piperidine-1-carboxylate.

### [Step 4] Preparation of N-{(1S,3R)-3-[(dibenzylamino)methyl]-2,2-dimethylcyclobutyl} acetamide

The title compound (2.48 g) was obtained as described in Reference Example 13, Step 1, using N-[(1S,3R)-3-(aminomethyl)-2,2-dimethylcyclobutyl]acetamide (1.60 g) obtained in Step 3 instead of tert-butyl[(1r,4r)-4-(aminomethyl)cyclohexyl]carbamate hydrochloride.

### [Step 5] Preparation of tert-butyl{(1S,3R)-3-[(dibenzylamino)methyl]-2,2-dimethylcyclobutyl} carbamate

The title compound (2.54 g) was obtained as described in Reference Example 28, Step 3, using N-{(1S,3R)-3-[(dibenzylamino)methyl]-2,2-dimethylcyclobutyl}acetamide (2.48 g) obtained in Step 4 instead of methyl (1S,3R)-3-acetamide-2,2-dimethylcyclobutane-1-carboxylate.

### [Step 6] Preparation of tert-butyl[(1S,3R)-3-(aminomethyl)-2,2-dimethylcyclobutyl]carbamate

The title compound (1.29 g) was obtained as described in Reference Example 2, Step 5, using tert-butyl {(1S,3R)-3-[(dibenzylamino)methyl]-2,2-dimethylcyclobutyl}carbamate (2.54 g) obtained in Step 5 instead of benzyl 4-(2,2,3,3,10,10-hexamethyl-8-oxo-4,9-dioxa-7-aza-3-silaundecan-5-yl)piperidine-1-carboxylate.

### Reference Example 35: N-[(1R,3S)-3-(aminomethyl)-2,2-dimethylcyclobutyl]acetamide

### [Step 1] Preparation of benzyl{[(1S,3R)-3-acetyl-2,2-dimethylcyclobutyl]methyl}carbamate

The title compound (13.6 g) was obtained as described in Reference Example 34, Step 1, using [(1R,3R)-3-acetyl-2,2-dimethylcyclobutyl]acetic acid (10.0 g) instead of [(1S,3S)-3-acetyl-2,2-dimethylcyclobutyl]acetic acid.

### [Step 2] Preparation of benzyl {[(1S,3R)-3-acetamide-2,2-dimethylcyclobutyl]methyl}carbamate

The title compound (10.6 g) was obtained as described in Reference Example 28, Step 2, using benzyl {[(1S,3R)-3-acetyl-2,2-dimethylcyclobutyl]methyl}carbamate (13.6 g) obtained in Step 1 instead of methyl (1S,3R)-3-acetyl-2,2-dimethylcyclobutane-1-carboxylate.

### [Step 3] Preparation of N-[(1R,3S)-3-(aminomethyl)-2,2-dimethylcyclobutyl]acetamide

The title compound (6.5 g) was obtained as described in Reference Example 2, Step 5, using benzyl {[(1S,3R)-3-acetamide-2,2-dimethylcyclobutyl]methyl}carbamate (10.6 g) obtained in Step 2 instead of benzyl 4-(2,2,3,3,10,10-hexamethyl-8-oxo-4,9-dioxa-7-aza-3-silaundecan-5-yl)piperidine-1-carboxylate.

### Reference Example 36: tert-Butyl {(1S,4r)-4-[(1S)-1-aminopropyl]cyclohexyl}carbamate

### [Step 1] Preparation of tert-butyl [(1r,4r)-4-propanoylcyclohexyl]carbamate

The title compound (450 mg) was obtained as described in Reference Example 3, Step 1, using ethyl magnesium bromide instead of methyl magnesium bromide.

### [Step 2] Preparation of tert-butyl [(1r,4r)-4-{(1E)-N-[(S)-2-methylpropane-2-sulfinyl]propanimidoyl}cyclohexyl]carbamate

The title compound (300 mg) was obtained as described in Reference Example 5, Step 1, using tert-butyl [(1r,4r)-4-propanoylcyclohexyl]carbamate (450 mg) obtained in Step 1 instead of tert-butyl 1-oxo-7-azaspiro[3.5]nonane-7-carboxylate.

### [Step 3] Preparation of tert-butyl{(1S,4r)-4-[(1S)-1-{[(S)-2-methylpropane-2-sulfinyl]amino}propyl]cyclohexyl}carbamate

The title compound (115 mg) was obtained as described in Reference Example 7, Step 2, using tert-butyl [(1r,4r)-4-{(1E)-N-[(S)-2-methylpropane-2-sulfinyl]propanimidoyl}cyclohexyl]carbamate (300 mg) obtained in Step 2 instead of tert-butyl [(1r,4r)-4-{(1E)-N-[(S)-2-methylpropane-2-sulfinyl]ethaneimidoyl}cyclohexyl]carbamate.

### [Step 4] Preparation of tert-butyl {(1S,4r)-4-[(1S)-1-aminopropyl]cyclohexyl}carbamate

The title compound (70 mg) was obtained as described in Reference Example 5, Step 3, using tert-butyl {(1S,4r)-4-[(1S)-1-{[(S)-2-methylpropane-2-sulfinyl]amino}propyl]cyclohexyl}carbamate (112 mg) obtained in Step 3 instead of tert-butyl (1S)-1-{[(S)-2-methylpropane-2-sulfinyl]amino}-7-azaspiro[3.5]nonane-7-carboxylate.

### Reference Example 37: tert-Butyl{3-[(1S)-1-aminoethyl]bicyclo[1.1.1]pentan-1-yl}carbamate

### [Step 1] Preparation of tert-butyl {3-[methoxy(methyl)carbamoyl]bicyclo[1.1.1]pentan-1-yl} carbamate

The title compound (960 mg) was obtained as described in Reference Example 14, Step 1, using 3-[(tert-butoxycarbonyl)amino]bicyclo[1.1.1]pentane-1-carboxylic acid (850 mg) instead of [(3S)-1-(tert-butoxycarbonyl)pyrrolidin-3-yl]acetic acid.

### [Step 2] Preparation of tert-butyl (3-acetylbicyclo[1.1.1]pentan-1-yl)carbamate

The title compound (790 mg) was obtained as described in Reference Example 3, Step 1, using tert-butyl {3-[methoxy(methyl)carbamoyl]bicyclo[1.1.1]pentan-1-yl}carbamate (960 mg) obtained in Step 1 instead of tert-butyl {(1r,4r)-4-[methoxy(methyl)carbamoyl]cyclohexyl}carbamate.

### [Step 3] Preparation of tert-butyl (3-{(1E)-N-[(S)-2-methylpropane-2-sulfinyl]ethaneimidoyl}bicyclo [1.1.1]pentan-1-yl)carbamate

The title compound (493 mg) was obtained as described in Reference Example 5, Step 1, using tert-butyl (3-acetylbicyclo[1.1.1]pentan-1-yl)carbamate (500 mg) obtained in Step 2 instead of tert-butyl 1-oxo-7-azaspiro[3.5]nonane-7-carboxylate.

### [Step 4] Preparation of tert-butyl {3-[(1S)-1-{[(S)-2-methylpropane-2-sulfinyl]amino}ethyl]bicyclo[1.1.1]pentan-1-yl}carbamate

The title compound (444 mg) was obtained as described in Reference Example 7, Step 2, using tert-butyl (3-{(1E)-N-[(S)-2-methylpropane-2-sulfinyl]ethaneimidoyl]bicyclo[1.1.1]pentan-1-yl)carbamate(493 mg) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl {3-[(1S)-1-aminoethyl]bicyclo[1.1.1]pentan-1-yl}carbamate

The title compound (302 mg) was obtained as described in Reference Example 5, Step 3, using tert-butyl {3-[(1S)-1-{[(S)-2-methylpropane-2-sulfinyl]amino}ethyl]bicyclo[1.1.1]pentan-1-yl}carbamate (444 mg) obtained in Step 4 instead of tert-butyl (1S)-1-{[(S)-2-methylpropane-2-sulfinyl]amino}-7-azaspiro[3.5]nonane-7-carboxylate.

### Reference Example 38: tert-Butyl {4-r(1S)-1-aminoethyl)cuban-1-yl}carbamate

### [Step 1] Preparation of tert-butyl {4-[methoxy(methyl)carbamoyl]cuban-1-yl}carbamate

The title compound (440 mg) was obtained as described in Reference Example 14, Step 1, using 4-[(tert-butoxycarbonyl)amino]cubane-1-carboxylic acid instead of [(3S)-1-(tertbutoxycarbonyl)pyrrolidin-3-yl]acetic acid.

### [Step 2] Preparation of tert-butyl [4-acetylcuban-1-yl]carbamate

The title compound (358 mg) was obtained as described in Reference Example 3, Step 1, using tert-butyl {4-[methoxy(methyl)carbamoyl]cuban-1-yl}carbamate (440 mg) obtained in Step 1 instead of tert-butyl {(1r,4r)-4-[methoxy(methyl)carbamoyl]cyclohexyl} carbamate.

### [Step 3] Preparation of tert-butyl [4-{(1E)-N-[(S)-2-methylpropane-2-sulfinyl]ethaneimidoyl}cuban-1-yl]carbamate

The title compound (427 mg) was obtained as described in Reference Example 5, Step 1, using tert-butyl [4-acetylcuban-1-yl]carbamate (358 mg) obtained in Step 2 instead of tert-butyl 1-oxo-7-azaspiro[3.5]nonane-7-carboxylate.

### [Step 4] Preparation of tert-butyl {4-[(1S)-1-{[(S)-2-methylpropane-2-sulfinyl] amino }ethyl] cuban-1-yl}carbamate

The title compound (361 mg) was obtained as described in Reference Example 7, Step 2, using tert-butyl [4-{(1E)-N-[(S)-2-methylpropane-2-sulfinyl]ethaneimidoyl}cuban-1-yl]carbamate (427 mg) obtained in Step 3 instead of tert-butyl [(1r,4r)-4-{(1E)-N-[(S)-2-methylpropane-2-sulfinyl]ethaneimidoyl)cyclohexyl]carbamate.

### [Step 5] Preparation of tert-butyl {4-[(1S)-1-aminoethyl]cuban-1-yl}carbamate

The title compound (259 mg) was obtained as described in Reference Example 5, Step 3, using tert-butyl {4-[(1S)-1-{[(S)-2-methylpropane-2-sulfinyl]amino}ethyl]cuban-1-yl}carbamate (361 mg) obtained in Step 4 instead of tert-butyl (1S)-1-{[(S)-2-methylpropane-2-sulfinyl]amino}-7-azaspiro[3.5]nonane-7-carboxylate.

### Reference Example 39: tert-Butyl {4-[(1S)-1-aminoethyl]bicyclo[2.2.1]heptan-1-yl}carbamate

### [Step 1] Preparation of tert-butyl {4-[methoxy(methyl)carbamoyl]bicyclo[2.2.1]heptan-1-yl}carbamate

The title compound (231 mg) was obtained as described in Reference Example 14, Step 1, using 4-[(tert-butoxycarbonyl)amino]bicyclo[2.2.1]heptane-1-carboxylic acid (255 mg) instead of [(3S)-1-(tert-butoxycarbonyl)pyrrolidin-3-yl]acetic acid.

### [Step 2] Preparation of tert-butyl (4-acetylbicyclo[2.2.1]heptan-1-yl)carbamate

The title compound (183 mg) was obtained as described in Reference Example 3, Step 1, using tert-butyl {4-[methoxy(methyl)carbamoyl]bicyclo[2.2.1]heptan-1-yl}carbamate (231 mg) obtained in Step 1 instead of tert-butyl {(1r,4r)-4-[methoxy(methyl)carbamoyl]cyclohexyl} carbamate.

### [Step 3] Preparation of tert-butyl {4-[(1S)-1-{[(S)-2-methylpropane-2-sulfinyl] amino}ethyl]bicyclo[2.2.1]heptan-1-yl}carbamate

(S)-(-)-2-methyl-2-propanesulfinamide (219 mg) and tetraethyl orthotitanate (494 mg) were added to tert-butyl (4-acetylbicyclo[2.2.1]heptan-1-yl)carbamate (183 mg) obtained in Step 2, and the mixture was stirred overnight at 60°C overnight. Subsequently, THF (4 mL) was added to the mixture, sodium borohydride (55 mg) was added to the mixture at -50°C, and the mixture was stirred for 4 hours while raising the temperature to room temperature. The reaction solution was diluted with ethyl acetate, and water was added thereto. The reaction solution was filtered through Celite (registered trademark), and the solvent was removed under reduced pressure. The residue was purified by silica gel column chromatography to afford the title compound (130 mg).

### [Step 4] Preparation of tert-butyl {4-[(1S)-1-aminoethyl]bicyclo[2.2.1]heptan-1-yl}carbamate

The title compound (90 mg) was obtained as described in Reference Example 5, Step 3, using tert-butyl {4-[(1S)-1-{[(S)-2-methylpropane-2-sulfinyl]amino}ethyl]bicyclo[2.2.1]heptan-1-yl}carbamate (130 mg) obtained in Step 3 instead of tert-butyl (1S)-1-{[(S)-2-methylpropane-2-sulfinyl]amino} -7-azaspiro[3.5]nonane-7-carboxylate.

### Reference Example 40: tert-Butyl {(1S,4r)-4-[(1S)-1-aminoethyl]cyclohexyl}methylcarbamate

### [Step 1] Preparation of tert-butyl {(1r,4r)-4-[methoxy(methyl)carbamoyl]cyclohexyl}methylcarbamate

The title compound (1.68 g) was obtained as described in Reference Example 13, Step 2, using tert-butyl {(1r,4r)-4-[methoxy(methyl)carbamoyl]cyclohexyl}carbamate (2.0 g) instead of tert-butyl {(1r,4r)-4-[(dibenzylamino)methyl]cyclohexyl}carbamate.

### [Step 2] Preparation of tert-butyl [(1r,4r)-4-acetylcyclohexyl]methylcarbamate

The title compound (1.35 g) was obtained as described in Reference Example 3, Step 1, using tert-butyl {(1r,4r)-4-[methoxy(methyl)carbamoyl]cyclohexyl}methylcarbamate (1.68 g) obtained in Step 1 instead of tert-butyl {(1r,4r)-4-[methoxy(methyl)carbamoyl]cyclohexyl} carbamate.

### [Step 3] Preparation of tert-butyl methyl[(1r,4r)-4-{(1E)-N-[(S)-2-methylpropane-2-sulfinyl]ethaneimidoyl} cyclohexyl]carbamate

The title compound (1.69 g) was obtained as described in Reference Example 5, Step 1, using tert-butyl [(1r,4r)-4-acetylcyclohexyl]methylcarbamate (1.35 g) obtained in Step 2 instead of tert-butyl 1-oxo-7-azaspiro[3.5]nonane-7-carboxylate.

### [Step 4] Preparation of tert-butyl methyl{(1S,4r)-4-[(1S)-1-{[(S)-2-methylpropane-2-sulfinyl]amino}ethyl]cyclohexyl}carbamate

The title compound (1.5 g) was obtained as described in Reference Example 7, Step 2, using tert-butyl methyl[(1r,4r)-4-{(1E)-N-[(S)-2-methylpropane-2-sulfinyl]ethaneimidoyl}cyclohexyl]carbamate (1.69 g) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl {(1S,4r)-4-[(1S)-1-aminoethyl]cyclohexyl}methylcarbamate

The title compound (880 mg) was obtained as described in Reference Example 5, Step 3, using tert-butyl methyl{(1S,4r)-4-[(1S)-1-{[(S)-2-methylpropane-2-sulfinyl]amino}ethyl]cyclohexyl}carbamate (1.5 g) obtained in Step 4 instead of tert-butyl (1S)-1-{[(S)-2-methylpropane-2-sulfinyl]amino}-7-azaspiro[3.5]nonane-7-carboxylate.

### Reference Example 41: tert-Butyl {(1S,3R)-3-((1S)-1-aminoethyl]cyclohexyl}carbamate

### [Step 1] Preparation of tert-butyl {(1S,3R)-3-[methoxy(methyl)carbamoyl]cyclohexyl} carbamate

The title compound (1.50 g) was obtained as described in Reference Example 14, Step 1, using (1R,3S)-3-[(tert-butoxycarbonyl)amino]cyclohexane-1-carboxylic acid (1.23 g) instead of [(3 S)-1-(tert-butoxycarbonyl)pyrrolidin-3-yl] acetic acid.

### [Step 2] Preparation of tert-butyl [(1S,3R)-3-acetylcyclohexyl]carbamate

The title compound (850 mg) was obtained as described in Reference Example 3, Step 1, using tert-butyl {(1S,3R)-3-[methoxy(methyl)carbamoyl]cyclohexyl}carbamate (1.50 g) obtained in Step 1 instead of tert-butyl {(1r,4r)-4-[methoxy(methyl)carbamoyl]cyclohexyl}carbamate.

### [Step 3] Preparation of tert-butyl [(1S,3R)-3-{(1E)-N-[(S)-2-methylpropane-2-sulfinyl]ethaneimidoyl}cyclohexyl]carbamate

The title compound (700 mg) was obtained as described in Reference Example 5, Step 1, using tert-butyl [(1S,3R)-3-acetylcyclohexyl]carbamate (850 mg) obtained in Step 2 instead of tert-butyl 1-oxo-7-azaspiro[3.5]nonane-7-carboxylate.

### [Step 4] Preparation of tert-butyl {(1S,3R)-3-[(1S)-1-{[(S)-2-methylpropane-2-sulfinyl] amino } ethyl]cyclohexyl}carbamate

The title compound (555 mg) was obtained as described in Reference Example 7, Step 2, using tert-butyl [(1S,3R)-3-{(1E)-N-[(S)-2-methylpropane-2-sulfinyl]ethaneimidoyl}cyclohexyl]carbamate (700 mg) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl {(1S,3R)-3-[(1S)-1-aminoethyl]cyclohexyl}carbamate

The title compound (250 mg) was obtained as described in Reference Example 5, Step 3, using tert-butyl {(1S,3R)-3-[(1S)-1-{[(S)-2-methylpropane-2-sulfinyl]amino}ethyl]cyclohexyl}carbamate (555 mg) obtained in Step 4 instead of tert-butyl (1S)-1-{[(S)-2-methylpropane-2-sulfinyl]amino}-7-azaspiro[3.5]nonane-7-carboxylate.

### Reference Example 42: tert-Butyl {(1S,4r)-4-[(1S)-1-aminoethyl]cyclohexyl}ethylcarbamate

### [Step 1] Preparation of tert-butyl {(1S,4r)-4-[(1S)-1-(dibenzylamino)ethyl]cyclohexyl}carbamate

The title compound (1.72 g) was obtained as described in Reference Example 13, Step 1, using tert-butyl {(1S,4r)-4-[(1S)-1-aminoethyl]cyclohexyl}carbamate (1.0 g) obtained in Reference Example 7, Step 3 instead of tert-butyl [(1r,4r)-4-(aminomethyl)cyclohexyl]carbamate hydrochloride.

### [Step 2] Preparation of tert-butyl {(1S,4r)-4-[(1S)-1-(dibenzylamino)ethyl]cyclohexyl}ethylcarbamate

The title compound (170 mg) was obtained as described in Reference Example 13, Step 2, using tert-butyl {(1S,4r)-4-[(1S)-1-(dibenzylamino)ethyl]cyclohexyl}carbamate (400 mg) obtained in Step 1 instead of tert-butyl {(1r,4r)-4-[(dibenzylamino)methyl]cyclohexyl}carbamate, and iodoethane instead of iodomethane.

### [Step 3] Preparation of tert-butyl {(1S,4r)-4-[(1S)-1-aminoethyl]cyclohexyl}ethylcarbamate

The title compound (95 mg) was obtained as described in Reference Example 2, Step 5, using tert-butyl {(1S,4r)-4-[(1S)-1-(dibenzylamino)ethyl]cyclohexyl} ethylcarbamate (170 mg) obtained in Step 2 instead of benzyl 4-(2,2,3,3,10,10-hexamethyl-8-oxo-4,9-dioxa-7-aza-3-silaundecan-5-yl)piperidine-1-carboxylate.

### Reference Example 43: tert-Butyl {(1R,3S)-3-[(1S)-1-aminoethyl]cyclohexyl]carbamate

### [Step 1] Preparation of tert-butyl {(1R,3S)-3-[methoxy(methyl)carbamoyl]cyclohexyl}carbamate

The title compound (1.33 g) was obtained as described in Reference Example 14, Step 1, using (1S,3R)-3-[(tert-butoxycarbonyl)amino]cyclohexane-1-carboxylic acid (1.1 g) instead of [(3S)-1-(tert-butoxycarbonyl)pyrrolidin-3-yl]acetic acid.

### [Step 2] Preparation of tert-butyl [(1R,3S)-3-acetylcyclohexyl]carbamate

The title compound (780 mg) was obtained as described in Reference Example 3, Step 1, using tert-butyl {(1R,3S)-3-[methoxy(methyl)carbamoyl]cyclohexyl}carbamate (1.33 g) obtained in Step 1 instead of tert-butyl {(lr,4r)-4-[methoxy(methyl)carbamoyl]cyclohexyl} carbamate.

### [Step 3] Preparation of tert-butyl [(1R,3S)-3-{(1E)-N-[(S)-2-methylpropane-2-sulfinyl]ethaneimidoyl}cyclohexyl]carbamate

The title compound (883 mg) was obtained as described in Reference Example 5, Step 1, using tert-butyl [(1R,3S)-3-acetylcyclohexyl]carbamate (777 mg) obtained in Step 2 instead of tert-butyl 1-oxo-7-azaspiro[3.5]nonane-7-carboxylate.

### [Step 4] Preparation of tert-butyl {(1R,3s)-3-[(1S)-1-{[(S)-2-methylpropane-2-sulfinyl]amino} ethyl] cyclohexyl}carbamate

The title compound (630 mg) was obtained as described in Reference Example 7, Step 2, using tert-butyl [(1R,3S)-3-{(1E)-N-[(S)-2-methylpropane-2-sulfinyl]ethaneimidoyl}cyclohexyl]carbamate (883 mg) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl {(1R,3S)-3-[(1S)-1-aminoethyl]cyclohexyl}carbamate

The title compound (375 mg) was obtained as described in Reference Example 5, Step 3, using tert-butyl {(1R,3S)-3-[(1S)-1-{[(S)-2-methylpropane-2-sulfinyl]amino}ethyl]cyclohexyl}carbamate (630 mg) obtained in Step 4 instead of tert-butyl (1S)-1-{[(S)-2-methylpropane-2-sulfinyl]amino}-7-azaspiro[3.5]nonane-7-carboxylate.

### Reference Example 44: tert-Butyl {(1R,3S)-3-r(1S)-1-aminoethyl]cyclopentyl}carbamate

### [Step 1] Preparation of tert-butyl {(1R,3S)-3-[methoxy(methyl)carbamoyl]cyclopentyl}carbamate

The title compound was obtained as described in Reference Example 14, Step 1, using (1S,3R)-3-[(tert-butoxycarbonyl)amino]cyclopentane-1-carboxylic acid instead of [(3S)-1-(tert-butoxycarbonyl)pyrrolidin-3-yl]acetic acid.

### [Step 2] Preparation of tert-butyl [(1R,3S)-3-acetylcyclopentyl]carbamate

The title compound was obtained as described in Reference Example 3, Step 1, using tert-butyl {(1R,3S)-3-[methoxy(methyl)carbamoyl]cyclopentyl}carbamate obtained in Step 1 instead of tert-butyl {(1r,4r)-4-[methoxy(methyl)carbamoyl]cyclohexyl}carbamate.

### [Step 3] Preparation of tert-butyl {(1R,3S)-3-[(1S)-1-{[(S)-2-methylpropane-2-sulfinyl] amino } ethyl] cyclopentyl}carbamate

The title compound (336 mg) was obtained as described in Reference Example 39, Step 3, using tert-butyl [(1R,3S)-3-acetylcyclopentyl]carbamate (368 mg) obtained in Step 2 instead of tert-butyl (4-acetylbicyclo[2.2.1]heptan-1-yl)carbamate.

### [Step 4] Preparation of tert-butyl {(1R,3S)-3-[(1S)-1-aminoethyl]cyclopentyl}carbamate

The title compound (225 mg) was obtained as described in Reference Example 5, Step 3, using tert-butyl {(1R,3S)-3-[(1S)-1-{[(S)-2-methylpropane-2-sulfinyl]amino}ethyl]cyclopentyl}carbamate (336 mg) obtained in Step 3 instead of tert-butyl (1S)-1-{[(S)-2-methylpropane-2-sulfinyl]amino}-7-azaspiro[3.5]nonane-7-carboxylate.

### Reference Example 45: tert-Butyl (1S,3R)-3-[(1S)-1-aminoethyl]cyclopentyl}carbamate

### [Step 1] Preparation of tert-butyl {(1S,3R)-3-[methoxy(methyl)carbamoyl]cyclopentyl} carbamate

The title compound was obtained as described in Reference Example 14, Step 1, using (1R,3S)-3-[(tert-butoxycarbonyl)amino]cyclopentane-1-carboxylic acid instead of [(3S)-1-(tert-butoxycarbonyl)pyrrolidin-3 -yl] acetic acid.

### [Step 2] Preparation of tert-butyl [(1S,3R)-3-acetylcyclopentyl]carbamate

The title compound was obtained as described in Reference Example 3, Step 1, using tert-butyl {(1S,3R)-3-[methoxy(methyl)carbamoyl]cyclopentyl}carbamate obtained in Step 1 instead of tert-butyl {(1r,4r)-4-[methoxy(methyl)carbamoyl]cyclohexyl}carbamate.

### [Step 3] Preparation of tert-butyl {(1S,3R)-3-[(1S)-1-{[(S)-2-methylpropane-2-sulfinyl] amino } ethyl] cyclopentyl } carbamate

The title compound (215 mg) was obtained as described in Reference Example 39, Step 3, using tert-butyl [(1S,3R)-3-acetylcyclopentyl]carbamate (314 mg) obtained in Step 2 instead of tert-butyl (4-acetylbicyclo[2.2.1]heptan-1-yl)carbamate.

### [Step 4] Preparation of tert-butyl {(1S,3R)-3-[(1S)-1-aminoethyl]cyclopentyl}carbamate

The title compound (135 mg) was obtained as described in Reference Example 5, Step 3, using tert-butyl {(1S,3R)-3-[(1S)-1-{[(S)-2-methylpropane-2-sulfinyl]amino}ethyl]cyclopentyl}carbamate (215 mg) obtained in Step 3 instead of tert-butyl (1S)-1-{[(S)-2-methylpropane-2-sulfinyl]amino}-7-azaspiro[3.5]nonane-7-carboxylate.

### Reference Example 46: tert-Butyl 4-[(1S)-1-amino-2-fluoroethyl]-4-fluoropiperidine-1-carboxylate

### [Step 1] Preparation of tert-butyl 4-[(1R)-2-(benzenesulfonyl)-2-fluoro-1-{[(S)-2-methylpropane-2-sulfinyl]amino}ethyl]-4-fluoropiperidine-1-carboxylate

The title compound (1.33 g) was obtained as described in Reference Example 27, Step 2, using tert-butyl 4-fluoro-4-[(1S)-1-{[(S)-2-methylpropane-2-sulfinyl]amino}ethyl]piperidine-1-carboxylate (900 mg) obtained in Reference Example 12, Step 2 instead of tert-butyl 4-[(E)-{[(S)-2-methylpropane-2-sulfinyl]imino}methyl]piperidine-1-carboxylate.

### [Step 2] Preparation of tert-butyl 4-fluoro-4-[(1S)-2-fluoro-1-{[(S)-2-methylpropane-2-sulfinyl] amino}ethyl]piperidine-1-carboxylate

The title compound (300 mg) was obtained as described in Reference Example 27, Step 3, using tert-butyl 4-[(1R)-2-(benzenesulfonyl)-2-fluoro-1-{[(S)-2-methylpropane-2-sulfinyl]amino}ethyl]-4-fluoropiperidine-1-carboxylate (1.33 g) obtained in Step 1 instead of tert-butyl 4-[(1 R)-2-(benzenesulfonyl)-2-fluoro-1-{[(S)-2-methylpropane-2-sulfinyl]amino}ethyl]piperidine-1-carboxylate.

### [Step 3] Preparation of tert-butyl 4-[(1S)-1-amino-2-fluoroethyl]-4-fluoropiperidine-1-carboxylate

The title compound (165 mg) was obtained as described in Reference Example 5, Step 3, using tert-butyl 4-fluoro-4-[(1S)-2-fluoro-1-{[(S)-2-methylpropane-2-sulfinyl]amino}ethyl]piperidine-1-carboxylate (300 mg) obtained in Step 2 instead of tert-butyl (1S)-1-{[(S)-2-methylpropane-2-sulfinyl]amino}-7-azaspiro[3.5]nonane-7-carboxylate.

### Reference Example 47: tert-Butyl (3S,4S)-4-[(1S)-1-aminoethyl]-3-methylpiperidine-1-carboxylate or tert-butyl (3R,4R)-4-[(1S)-1-aminoethyl]-3-methylpiperidine-1-carboulate

### [Step 1] Preparation of tert-butyl 4-[methoxy(methyl)carbamoyl]-3-methylpiperidine-1-carboxylate

The title compound (4.62 g) was obtained as described in Reference Example 14, Step 1, using 1-(tert-butoxycarbonyl)-3-methylpiperidine-4-carboxylic acid (cis/trans = 4/1, 4.1 g) (for example, synthesized according to the method described in WO2010/013037 A1) instead of [(3S)-1-(tert-butoxycarbonyl)pyrrolidin-3-yl]acetic acid.

### [Step 2] Preparation of tert-butyl 4-acetyl-3-methylpiperidine-1-carboxylate

The title compound (3.98 g) was obtained as described in Reference Example 3, Step 1, using tert-butyl 4-[methoxy(methyl)carbamoyl]-3-methylpiperidine-1-carboxylate (4.62 g) obtained in Step 1 instead of tert-butyl {(1r,4r)-4-[methoxy(methyl)carbamoyl]cyclohexyl} carbamate.

### [Step 3] Preparation of tert-butyl (3S,4S)-3-methyl-4-{(lE)-N-[(S)-2-methylpropane-2-sulfinyl]ethaneimidoyl}piperidine-1-carboxylate or tert-butyl (3R,4R)-3-methyl-4-{(1E)-N-[(S)-2-methylpropane-2-sulfinyl]ethaneimidoyl}piperidine-1 -carboxylate

(S)-(-)-2-methyl-2-propanesulfinamide (1.04 g) and tetraethyl orthotitanate (3.27 g) were added to tert-butyl 4-acetyl-3-methylpiperidine-1-carboxylate (1.45 g) obtained in Step 2, and the mixture was stirred at 60°C overnight. The reaction solution was diluted with ethyl acetate, and water was added thereto. The reaction solution was filtered through Celite (registered trademark), and the solvent was removed under reduced pressure. The residue was purified by silica gel column chromatography to afford the title compound (683 mg) and tert-butyl (3R,4R)-3-methyl-4-{(1E)-N-[(S)-2-methylpropane-2-sulfinyl]ethaneimidoyl}piperidine-1-carboxylate (609 mg). Stereochemistry was assigned (optionally) by bioactivity and established structural similarity.

### [Step 4] Preparation of tert-butyl (3S,4S)-3-methyl-4-[(1S)-1-{[(S)-2-methylpropane-2-sulfinyl]amino}ethyl]piperidine-1-carboxylate

The title compound (426 mg) was obtained as described in Reference Example 7, Step 2, using tert-butyl (3S,4S)-3-methyl-4-{(lE)-N-[(S)-2-methylpropane-2-sulfinyl]ethaneimidoyl}piperidine-1-carboxylate (683 mg) obtained in Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl (3S,4S)-4-[(1S)-1-aminoethyl]-3-methylpiperidine-1-carboxylate

The title compound (291 mg) was obtained as described in Reference Example 5, Step 3, using tert-butyl (3S,4S)-3-methyl-4-[(1S)-1-{[(S)-2-methylpropane-2-sulfinyl]amino}ethyl]piperidine-1-carboxylate (426 mg) obtained in Step 4 instead of tert-butyl (1S)-1-{[(S)-2-methylpropane-2-sulfinyl]amino}-7-azaspiro[3.5]nonane-7-carboxylate.

### Reference Example 48: tert-Butyl(3R,4R)-4-[(1S)-1-aminoethyl]-3-methylpiperidine-1-carboxylate or tert-butyl(3S,4S)-4-[(1S)-1-aminoethyl]-3-methylpiperidine-1-carboxylate

### [Step 1] Preparation of tert-butyl (3R,4R)-3-methyl-4-[(1S)-1-{[(S)-2-methylpropane-2-sulfinyl]amino}ethyl]piperidine-1-carboxylate

The title compound (350 mg) was obtained as described in Reference Example 7, Step 2, using tert-butyl (3R,4R)-3-methyl-4-{(1E)-N-[(S)-2-methylpropane-2-sulfinyl]ethaneimidoyl}piperidine-1-carboxylate (609 mg) obtained in Reference Example 47, Step 3 instead of tert-butyl [(1r,4r)-4-{(1E)-N-[(S)-2-methylpropane-2-sulfinyl]ethaneimidoyl}cyclohexyl]carbamate.

### [Step 5] Preparation of tert-butyl (3R,4R)-4-[(1S)-1-aminoethyl]-3-methylpiperidine-1-carboxylate

The title compound (237 mg) was obtained as described in Reference Example 5, Step 3, using tert-butyl (3R,4R)-3-methyl-4-[(1S)-1-{[(S)-2-methylpropane-2-sulfinyl]amino}ethyl]piperidine-1-carboxylate (350 mg) obtained in Step 1 instead of tert-butyl (1S)-1-{[(S)-2-methylpropane-2-sulfinyl]amino}-7-azaspiro[3.5]nonane-7-carboxylate.

Reference Example 49: tert-Butyl 4-[(1S)-1-aminoethyl]-2-methylpiperidine-1-carboxylate

### [Step 1] Preparation of tert-butyl 4-[methoxy(methyl)carbamoyl]-2-methylpiperidine-1-carboxylate

The title compound (73.5 g) was obtained as described in Reference Example 14, Step 1, using 1-(tert-butoxycarbonyl)-2-methylpiperidine-4-carboxylic acid (57.8 g) instead of [(3S)-1-(tert-butoxycarbonyl)pyrrolidin-3-yl]aceticacid.

### [Step 2] Preparation of tert-butyl 4-acetyl-2-methylpiperidine-1-carboxylate

The title compound (54.7 g) was obtained as described in Reference Example 3, Step 1, using tert-butyl 4-[methoxy(methyl)carbamoyl]-2-methylpiperidine-1-carboxylate (73.5 g) obtained in Step 1 instead of tert-butyl {(1r,4r)-4-[methoxy(methyl)carbamoyl]cyclohexyl} carbamate.

### [Step 3] Preparation of tert-butyl 2-methyl-4-[(1S)-1-{[(R)-2-methylpropane-2-sulfinyl]amino}ethyl]piperidine-1-carboxylate

(R)-(+)-2-methyl-2-propanesulfinamide (35.7 g) and tetraisopropyl orthotitanate (193 g) were added sequentially to tert-butyl 4-acetyl-2-methylpiperidine-1-carboxylate (54.7 g) obtained in Step 2, and the mixture was stirred at 70°C for 12 hours. Subsequently, THF (453 mL) was added to the mixture, lithium tri-sec-butylborohydride (1 M in THF, 453 mL) was added to the mixture at -78°C, and the mixture was stirred for 2 hours while raising the temperature from -78°C to room temperature. The reaction solution was diluted with ethyl acetate, and water was added thereto. The reaction solution was filtered through Celite (registered trademark), and the solvent was removed under reduced pressure. The residue was purified by silica gel column chromatography to afford the title compound (85 g).

### [Step 4] Preparation of tert-butyl 4-[(1S)-1-aminoethyl]-2-methylpiperidine-1-carboxylate

The title compound (39.5 g) was obtained as described in Reference Example 5, Step 3, using tert-butyl 2-methyl-4-[(1S)-1-{[(R)-2-methylpropane-2-sulfinyl]amino}ethyl]piperidine-1-carboxylate(85 g) obtained in Step 3 instead of tert-butyl (1S)-1-{[(S)-2-methylpropane-2-sulfinyl]amino}-7-azaspiro[3.5]nonane-7-carboxylate.

### Reference Example 50: 2-Amino-3,7-anhydro-6-[(tert-butoxycarbonyl)aminol-1,2,4,5,6-pentadeoxy-L-arabino-heptitol

### [Step 1] Preparation of tert-butyl {(3R,6S)-6-[methoxy(methyl)carbamoyl]oxan-3-yl}carbamate

The title compound was obtained as described in Reference Example 14, Step 1, using 2,6-anhydro-5-[(tert-butoxycarbonyl)amino]-3,4,5-trideoxy-L-erythro-hexonic acid instead of [(3S)-1-(tert-butoxycarbonyl)pyrrolidin-3-yl]acetic acid.

### [Step 2] Preparation of tert-butyl [(3R,6S)-6-acetyloxan-3-yl]carbamate

The title compound (450 mg) was obtained as described in Reference Example 3, Step 1, using tert-butyl {(3R,6S)-6-[methoxy(methyl)carbamoyl]oxan-3-yl}carbamate obtained in Step 1 instead of tert-butyl {(1r,4r)-4-[methoxy(methyl)carbamoyl]cyclohexyl}carbamate.

### [Step 3] Preparation of 3,7-anhydro-6-[(tert-butoxycarbonyl)amino]-1,2,4,5,6-pentadeoxy-2-{[(R)-2-methylpropane-2-sulfinyl]amino}-L-arabino-heptitol

The title compound (240 mg) was obtained as described in Reference Example 49, Step 3, using tert-butyl [(3R,6S)-6-acetyloxan-3-yl]carbamate (450 mg) obtained in Step 2 instead of tert-butyl 4-acetyl-2-methylpiperidine-1-carboxylate.

### [Step 4] Preparation of 2-amino-3,7-anhydro-6-[(tert-butoxycarbonyl)amino]-1,2,4,5,6-pentadeoxy-L-arabino-heptitol

The title compound (90 mg) was obtained as described in Reference Example 5, Step 3, using 3,7-anhydro-6-[(tert-butoxycarbonyl)amino]-1,2,4,5,6-pentadeoxy-2-{[(R)-2-methylpropane-2-sulfinyl]amino}-L-arabino-heptitol (240 mg) obtained in Step 3 instead of tert-butyl (1S)-1-{[(S)-2-methylpropane-2-sulfinyl]amino}-7-azaspiro[3.5]nonane-7-carboxylate.

### Reference Example 51: tert-Butvl (3S,4R)-4-[(1S)-1-aminoethyl]-3-fluoropiperidine-1-carboxylate

### [Step 1] Preparation of tert-butyl (3S,4R)-3-fluoro-4-(hydroxymethyl)piperidine-1-carboxylate

Hydrogen chloride (2 M in methanol, 4 mL) was added to a solution of [(3S,4R)-1-benzyl-3-fluoropiperidin-4-yl]methanol (1.2 g) (for example, synthesized according to the method described in WO2006/069287 A1) in methanol (20 mL). After degassing, 10% Pd-C (500 mg) was added to the mixture with stirring at room temperature under argon atmosphere, and the mixture was stirred at room temperature under medium-pressure hydrogen atmosphere (0.4 MPa) overnight. Saturated aq. sodium bicarbonate and Boc₂O (1.9 mL) were added to the reaction solution, and the mixture was stirred at room temperature for 1 hour. The insolubles were filtered off, then the filtrate was diluted with ethyl acetate, and the organic layer was washed with saturated saline. The solvent was removed under reduced pressure, and the obtained residue was purified by silica gel column chromatography to afford the title compound (900 mg).

### [Step 2] Preparation of tert-butyl (3S,4R)-3-fluoro-4-[(E)-{[(R)-2-methylpropane-2-sulfinyl]imino} methyl]piperidine-1-carboxylate

2-Hydroxy-2-azaadamantane (29 mg) and iodobenzenediacetate (2.46 g) were added to a solution of tert-butyl (3S,4R)-3-fluoro-4-(hydroxymethyl)piperidine-1-carboxylate (890 mg) obtained in Step 1 in dichloromethane (7.6 mL), and the mixture was stirred at room temperature. After monitoring the consumption of the starting material on TLC, saturated aq. sodium carbonate and saturated aq. sodium thiosulfate were added to the reaction solution, and the mixture was stirred at room temperature for 10 minutes. The reaction solution was extracted with dichloromethane, and the solvent was removed under reduced pressure. A solution of (R)-(+)-2-methyl-2-propanesulfinamide (694 mg) in dichloromethane (7.6mL) was added to the obtained residue, and tetraisopropyl orthotitanate (3.3 mL) was added to the mixture under ice-cooling, and the mixture was stirred at room temperature for 2 hours. The reaction solution was diluted with ethyl acetate, and water was added thereto. The reaction solution was filtered through Celite (registered trademark), and the solvent was removed under reduced pressure. The residue was purified by silica gel column chromatography to afford the title compound (575 mg).

### [Step 3] Preparation of tert-butyl (3S,4R)-3-fluoro-4-[(1S)-1-{[(R)-2-methylpropane-2-sulfinyl] amino}ethyl]piperidine-1-carboxylate

The title compound (353 mg) was obtained as described in Reference Example 20, Step 2, using tert-butyl (3S,4R)-3-fluoro-4-[(E)-{[(R)-2-methylpropane-2-sulfinyl]imino}methyl]piperidine-1-carboxylate (575 mg) obtained in Step 2 instead of tert-butyl 2-[(E)-{[(R)-2-methylpropane-2-sulfinyl]imino}methyl]-7-azaspiro[3.5]nonane-7-carboxylate.

### [Step 4] Preparation of tert-butyl(3S,4R)-4-[(1S)-1-aminoethyl]-3-fluoropiperidine-1-carboxylate

The title compound (250 mg) was obtained as described in Reference Example 5, Step 3, using tert-butyl (3S,4R)-3-fluoro-4-[(1S)-1-{[(R)-2-methylpropane-2-sulfinyl]amino}ethyl]piperidine-1-carboxylate (353 mg) obtained in Step 3 instead of tert-butyl (1S)-1-{[(S)-2-methylpropane-2-sulfinyl]amino}-7-azaspiro[3.5]nonane-7-carboxylate.

### Reference Example 52: tert-Butyl (3S,4S)-4-[(1S)-1-aminoethyl]-3-ethylpiperidine-1-carboxylate or tert-butyl (3R,4R)-4-[(1S)-1-aminoethyl]-3-ethylpiperidine-1-carboxylate

### [Step 1] Preparation of 1-(tert-butoxycarbonyl)-3-ethylpiperidine-4-carboxylic acid

Methyl 3-ethynylpyridine-4-carboxylate (876 mg) was dissolved in methanol (10.9 mL). After degassing, 2 M hydrochloric acid (4.1 mL) and platinum(IV) oxide (87.6 mg) were added to the solution with stirring at room temperature under argon atmosphere, and the mixture was stirred at room temperature under medium-pressure hydrogen atmosphere (0.4 MPa) overnight. The reaction solution was filtered through Celite (registered trademark), and then the solvent was removed under reduced pressure. Boc₂O (1.87 mL) and 2 M aq. sodium hydroxide (16.3 mL) were added sequentially to the residue, and the mixture was stirred at 80°C for 1 hour. Hydrochloric acid was added to the reaction solution, and the reaction solution was extracted with ethyl acetate. The organic layer was washed with saturated saline, then the solvent was removed under reduced pressure, hexane was added to the obtained residue to suspend the residue, and the precipitate was collected by filtration to afford the title compound (870 mg).

### [Step 2] Preparation of tert-butyl 3-ethyl-4-[methoxy(methyl)carbamoyl]piperidine-1-carboxylate

The title compound (960 mg) was obtained as described in Reference Example 14, Step 1, using 1-(tert-butoxycarbonyl)-3-ethylpiperidine-4-carboxylic acid (870 mg) obtained in Step 1 instead of [(3S)-1-(tert-butoxycarbonyl)pyrrolidin-3-yl]acetic acid.

### [Step 3] Preparation of tert-butyl 4-acetyl-3-ethylpiperidine-1-carboxylate

The title compound was obtained as described in Reference Example 3, Step 1, using tert-butyl 3-ethyl-4-[methoxy(methyl)carbamoyl]piperidine-1-carboxylate obtained in Step 2 instead of tert-butyl {(1r,4r)-4-[methoxy(methyl)carbamoyl]cyclohexyl}carbamate.

### [Step 4] Preparation of tert-butyl (3S,4S)-3-ethyl-4-{(1E)-N-[(R)-2-methylpropane-2-sulfinyl]ethaneimidoyl}piperidine-1-carboxylate or tert-butyl (3R,4R)-3-ethyl-4-{(1E)-N-[(R)-2-methylpropane-2-sulfinyl]ethaneimidoyl}piperidine-1-carboxylate

The title compound (490 mg) and tert-butyl (3R,4R)-3-ethyl-4-{(1E)-N-[(R)-2-methylpropane-2-sulfinyl]ethaneimidoyl}piperidine-1-carboxylate (430 mg) were obtained as described in Reference Example 47, Step 3, using tert-butyl 4-acetyl-3-ethylpiperidine-1-carboxylate obtained in Step 3 instead of tert-butyl 4-acetyl-3-methylpiperidine-1-carboxylate. Stereochemistry was assigned (optionally) by bioactivity and established structural similarity.

### [Step 5] Preparation of tert-butyl (3S,4S)-3-ethyl-4-[(1S)-1-{[(R)-2-methylpropane-2-sulfinyl]amino}ethyl]piperidine-1-carboxylate

tert-Butyl(3S,4S)-3-ethyl-4-{(1E)-N-[(R)-2-methylpropane-2-sulfinyl]ethaneimidoyl}piperidine-1-carboxylate (490 mg) obtained in Step 4 was dissolved in THF (6.8 mL), and lithium tri-sec-butylborohydride (1 M in THF, 2.7 mL) was added to the solution at -78°C, and the mixture was stirred while raising the temperature from -78°C to room temperature. After monitoring the consumption of the starting material on TLC, saturated aq. ammonium chloride was added to the reaction solution, and the reaction solution was extracted with ethyl acetate. The organic layer was washed with saturated saline, and then the solvent was removed under reduced pressure. The residue was purified by silica gel column chromatography to afford the title compound (472 mg).

### [Step 6] Preparation of tert-butyl (3S,4S)-4-[(1S)-1-aminoethyl]-3-ethylpiperidine-1-carboxylate

The title compound (240 mg) was obtained as described in Reference Example 5, Step 3, using tert-butyl (3S,4S)-3-ethyl-4-[(1S)-1-{[(R)-2-methylpropane-2-sulfinyl]amino}ethyl]piperidine-1-carboxylate (472 mg) obtained in Step 5 instead of tert-butyl (1S)-1-{[(S)-2-methylpropane-2-sulfinyl]amino}-7-azaspiro[3.5]nonane-7-carboxylate.

### Reference Example 53: tert-Butvl (3R,4R)-4-[(1S)-1-aminoethyl]-3-ethylpiperidine-1-carboxylate or tert-butyl (3S,4S)-4-[(1S)-1-aminoethyl]-3-ethylpiperidine-1-carboxylate

### [Step 1] Preparation of tert-butyl (3R,4R)-3-ethyl-4-{(1E)-N-[(R)-2-methylpropane-2-sulfinyl]ethaneimidoyl}piperidine-1-carboxylate

The title compound (352 mg) was obtained as described in Reference Example 52, Step 5, using tert-butyl (3R,4R)-3-ethyl-4-{(1E)-N-[(R)-2-methylpropane-2-sulfinyl]ethaneimidoyl}piperidine-1-carboxylate (430 mg) obtained in Reference Example 52, Step 4 instead of tert-butyl (3S,4S)-3-ethyl-4-{(1E)-N-[(R)-2-methylpropane-2-sulfinyl] ethaneimidoyl} piperidine-1-carboxylate.

### [Step 5] Preparation of tert-butyl (3R,4R)-4-[(1S)-1-aminoethyl]-3-ethylpiperidine-1-carboxylate

The title compound (190 mg) was obtained as described in Reference Example 5, Step 3, using tert-butyl (3R,4R)-3-ethyl-4-{(1E)-N-[(R)-2-methylpropane-2-sulfinyl]ethaneimidoyl}piperidine-1-carboxylate (352 mg) obtained in Step 1 instead of tert-butyl (1S)-1-{[(S)-2-methylpropane-2-sulfinyl]amino}-7-azaspiro[3.5]nonane-7-carboxylate.

### Reference Example 54: tert-Butyl 4-[(1s,3s)-3-(hydroxymethyl)cyclobutyl]piperazine-1-carboxylate

### [Step 1] Preparation of tert-butyl 4-[(1s,3s)-3-(methoxycarbonyl)cyclobutyl]piperazine-1-carboxylate

Methyl 3-oxocyclobutane-1-carboxylate (1.0 g) was dissolved in dichloromethane (31 mL), 1-(tert-butoxycarbonyl)piperazine (1.7 g) was added to the solution, sodium triacetoxyborohydride (3.3 g) was added to the mixture under ice-cooling, and the mixture was stirred at room temperature overnight. Saturated aq. ammonium chloride was added to the reaction solution, and the reaction solution was extracted with ethyl acetate. The solvent was removed under reduced pressure, and the obtained residue was purified by silica gel column chromatography to afford the title compound (2.0 g). MS (m/z): 299.2 [M+H]⁺

### [Step 2] Preparation of tert-butyl 4-[(1s,3s)-3-(hydroxymethyl)cyclobutyl]piperazine-1-carboxylate

Sodium borohydride (101 mg) was added to a solution of tert-butyl 4-[(ls,3s)-3-(methoxycarbonyl)cyclobutyl]piperazine-1-carboxylate (200 mg) obtained in Step 1 in methanol (1.3 mL), and the mixture was stirred at room temperature overnight. Saturated aq. ammonium chloride was added to the reaction solution, and the reaction solution was extracted with ethyl acetate. The organic layer was washed with water and saturated saline and dried over anhydrous sodium sulfate. The solvent was removed under reduced pressure, and the obtained residue was purified by silica gel column chromatography to afford the title compound (125 mg). MS (m/z): 271.2 [M+H]⁺

### Reference Example 55: tert-Butvl 4-{(1S)-1-[(2-hydroxyethyl)(2-nitrobenzene-1-sulfonyl)amino]ethyl }piperidine-1-carboxylate

### [Step 1] Preparation of tert-butyl 4-{(1S)-1-[(2-nitrobenzene-1-sulfonyl)amino]ethyl}piperidine-1-carboxylate

2-Nitrobenzenesulfonyl chloride (1.07 g) was added to a mixture of tert-butyl 4-[(1S)-1-aminoethyl]piperidine-1-carboxylate (1.0 g), sodium bicarbonate (736 mg), water (9 mL), and THF (9 mL), and the mixture was stirred at room temperature for 1 hour. The reaction solution was diluted with ethyl acetate, and the organic layer was washed with water and saturated saline. The organic layer was dried over anhydrous sodium sulfate, then the solvent was removed under reduced pressure, and the obtained residue was purified by silica gel column chromatography to afford the title compound (860 mg).

### [Step 2] Preparation of tert-butyl 4-{(1S)-1-[(2-hydroxyethyl)(2-nitrobenzene-1-sulfonyl)amino]ethyl}piperidine-1 -carboxylate

2-Bromoethanol (598 mg) and potassium carbonate(662 mg) were added to a solution of tert-butyl 4-{(1S)-1-[(2-nitrobenzene-1-sulfonyl)amino]ethyl}piperidine-1-carboxylate (330 mg) obtained in Step 1 in NMP (1.6 mL), and the mixture was stirred at 80°C. After monitoring the consumption of the starting material on TLC, the solvent was removed under reduced pressure. The residue was purified by silica gel column chromatography to afford the title compound (100 mg).

### Reference Example 56: tert-Butyl[(1S,4r)-4-{(1S)-1-[(2-hydroxyethyl)(2-nitrobenzene-1-sulfonyl)amino]thyl}cyclohexyl]carbamate

### [Step 1] Preparation of tert-butyl [(1S,4r)-4-{(1S)-1-[(2-nitrobenzene-1-sulfonyl)amino] ethyl } cyclohexyl]carbamate

The title compound (1.41 g) was obtained as described in Reference Example 55, Step 1, using tert-butyl {(1S,4r)-4-[(1S)-1-aminoethyl]cyclohexyl}carbamate (1.0 g) obtained in Reference Example 7, Step 3 instead of tert-butyl 4-[(1S)-1-aminoethyl]piperidine-1-carboxylate.

### [Step 2] Preparation of tert-butyl [(1S,4r)-4-{(1S)-1-[(2-hydroxyethyl)(2-nitrobenzene-1-sulfonyl)amino]ethyl}cyclohexyl]carbamate

The title compound (140 mg) was obtained as described in Reference Example 55, Step 2, using tert-butyl [(1S,4r)-4-{(1S)-1-[(2-nitrobenzene-1-sulfonyl)amino]ethyl}cyclohexyl]carbamate (200 mg) obtained in Step 1 instead of tert-butyl 4-{(1S)-1-[(2-nitrobenzene-1-sulfonyl)amino]ethyl}piperidine-1-carboxylate.

### Reference Example 57: tert-Butyl 4-[(1S)-1-{(2-hydroxypropyl)[(S)-2-methylpropane-2-sulfinyl]amino}ethyl]piperidine-1-carboxylate

### [Step 1] Preparation of tert-butyl 4-{(1E)-N-[(S)-2-methylpropane-2-sulfinyl]ethaneimidoyl}piperidine-1-carboxylate

The title compound (106 g) was obtained as described in Reference Example 5, Step 1, using tert-butyl 4-acetylpiperidine-1-carboxylate (73.2 g) instead of tert-butyl 1-oxo-7-azaspiro[3.5]nonane-7-carboxylate.

### [Step 2] Preparation oftert-butyl 4-[(1S)-1-{[(S)-2-methylpropane-2-sulfinyl]amino}ethyl]piperidine-1-carboxylate

The title compound (91 g) was obtained as described in Reference Example 7, Step 2, using tert-butyl 4-{(1E)-N-[(S)-2-methylpropane-2-sulfinyl]ethaneimidoyl}piperidine-1-carboxylate (90 g) obtained in Step 1 instead of tert-butyl [(1r,4r)-4-{(1E)-N-[(S)-2-methylpropane-2-sulfinyl]ethaneimidoyl}cyclohexyl]carbamate.

### [Step 3] Preparation of tert-butyl 4-[(1S)-1-{[(S)-2-methylpropane-2-sulfinyl]](prop-2-en-1-yl)amino }ethyl]piperidine-1-carboxylate

The title compound (1.54 g) was obtained as described in Reference Example 13, Step 2, using tert-butyl 4-[(1S)-1-{[(S)-2-methylpropane-2-sulfinyl]amino}ethyl]piperidine-1-carboxylate (2.0 g) obtained in Reference Example 57, Step 2 and allyl bromide.

### [Step 4] Preparation of tert-butyl 4-[(1S)-1-{(2-hydroxypropyl)[(S)-2-methylpropane-2-sulfinyl]amino}ethyl]piperidine-1-carboxylate

BH₃-THF (0.9 M in THF, 3 mL) was added to a solution of tert-butyl 4-[(1S)-1-1[(S)-2-methylpropane-2-sulfinyl](prop-2-en-1-yl)amino}ethyl]piperidine-1-carboxylate (500 mg) obtained in Step 3 in THF (2.7 mL) under ice-cooling, and the mixture was stirred at the same temperature for 1 hour. Subsequently, sodium perborate tetrahydrate (1.03 g) was added to the mixture, and the mixture was stirred at room temperature for 2 hours. The reaction solution was diluted with ethyl acetate, and the organic layer was washed with saturated saline. The solvent was removed under reduced pressure, and the obtained residue was purified by silica gel column chromatography to afford the title compound (77 mg) and its isomer, tert-butyl 4-[(1S)-1-{(3-hydroxypropyl)[(S)-2-methylpropane-2-sulfinyl]amino}ethyl]piperidine-1- carboxylate (271 mg).

### Reference Example 58: tert-Butyl [(1S,4r)-4-{(1S)-1-[(2-hydroxypropyl)(2-nitrobenzene-1-sulfonyl)aminolethyl}cyclohexyl]carbamate

tert-Butyl [(1S,4r)-4-{(1S)-1-[(2-hydroxyethyl)(2-nitrobenzene-1-sulfonyl)amino]ethyl}cyclohexyl]carbamate (240 mg) obtained in Reference Example 56, Step 2, propylene oxide (98 mg), potassium carbonate (155 mg), and DMF (1.1 mL) were stirred at 80°C for 24 hours. The reaction solution was diluted with water and extracted with ethyl acetate. The solvent was removed under reduced pressure, and the obtained residue was purified by silica gel column chromatography to afford the title compound (71 mg).

### Reference Example 59: Ethyl 3-({4-[(tert-butoxvcarbonyl)amino]butyl}amino)-4-(trifluoromethyl)benzoate

Toluene (2 mL) was added to ethyl 3-iodo-4-(trifluoromethyl)benzoate (145 mg), tert-butyl (4-aminobutyl)carbamate (91 mg), BINAP (55 mg), cesium carbonate (286 mg), and Pd(OAC)₂ (10 mg). After degassing, the mixture was stirred at 110°C under argon atmosphere for 12 hours. The reaction solution was allowed to stand to cool, and then was purified by silica gel column chromatography to afford the title compound (135 mg).

### Reference Example 69: Methyl 4-(4-{2-[(tert-butoxycarbonyl)amino]ethyl}piperidin-1-yl)-1-{[2-(trimethylsilyl)ethoxy]methyl}-1H-indazole-6-carboxylate

### [Step 1] Preparation of methyl 4-bromo-1-{[2-(trimethylsilyl)ethoxy]methyl}-1H-indazole-6-carboxylate

2-(Chloromethoxy)ethyl trimethylsilane (1.1 mL) was added to a mixture of methyl 4-bromo-1H-indazole-6-carboxylate (1.58 g), potassium carbonate (942 mg), and DMF (12 mL), and the mixture was stirred at room temperature overnight. The reaction solution was diluted with ethyl acetate, and the organic layer was washed with water and saturated saline. The organic layer was dried over anhydrous sodium sulfate, then the solvent was removed under reduced pressure, and the obtained residue was purified by silica gel column chromatography to afford the title compound (1.01 g).

### [Step 2] Preparation of methyl 4-(4-{2-[(tert-butoxycarbonyl)amino]ethyl}piperidin-1-yl)-1-{[2-(trimethylsilyl)ethoxy]methyl}-1H-indazole-6-carboxylate

The title compound (52 mg) was obtained as described in Reference Example 59 using methyl 4-bromo-1-{[2-(trimethylsilyl)ethoxy]methyl}-1H-indazole-6-carboxylate (80 mg) obtained in Step 1 instead of ethyl 3-iodo-4-(trifluoromethyl)benzoate, and tert-butyl [2-(piperidin-4-yl)ethyl]carbamate instead of tert-butyl (4-aminobutyl)carbamate.

### Reference Example 74: Ethyl 3'-{[(tert-butoxycarbonyl)amino]methyl}-6-chloro[1,1'-biphenyl]-3-carboxylate

1,4-Dioxane (3 mL) was added to ethyl 4-chloro-3-iodobenzoate (150 mg), (3-{[(tert-butoxycarbonyl)amino]methyl}phenyl)boronic acid (127 mg), Pd(PPh3)4 (56 mg), and saturated aq. sodium bicarbonate (1 mL). After degassing, the mixture was stirred at 100°C under argon atmosphere for 2 hours. The reaction solution was diluted with ethyl acetate, and the organic layer was washed with water and saturated saline. The organic layer was dried over anhydrous magnesium sulfate, then the solvent was removed under reduced pressure, and the obtained residue was purified by silica gel column chromatography to afford the title compound (101 mg).

### Reference Example 78: tert-Butyl 4-({[5'-(methoxycarbonyl)-2'-(trifluoromethyl)[1,1'-biphenyl]-4-yl]oxy}methyl)piperidine-1-carboxylate

### [Step 1] Preparation of 4'-hydroxy-6-(trifluoromethyl)[1,1'-biphenyl]-3-carboxylic acid

The title compound (414 mg) was obtained as described in Reference Example 74, using methyl 3-bromo-4-(trifluoromethyl)benzoate (500 mg) instead of ethyl 4-chloro-3-iodobenzoate, and (4-hydroxyphenyl)boronic acid instead of (3-{[(tertbutoxycarbonyl)amino]methyl} phenyl)boronic acid.

### [Step 2] Preparation of methyl 4'-hydroxy-6-(trifluoromethyl)[1,1'-biphenyl]-3-carboxylate

Concentrated sulfuric acid (0.1 mL) was added to a solution of 4'-hydroxy-6-(trifluoromethyl)[1,1'-biphenyl]-3-carboxylic acid (350 mg) obtained in Step 1 in methanol (4 mL), and the mixture was stirred at 65°C for 4 hours. The reaction solution was concentrated under reduced pressure, water was added to the obtained residue, and the precipitate was collected by filtration and dried to afford the title compound (320 mg).

### [Step 3] Preparation of tert-butyl 4-({[5'-(methoxycarbonyl)-2'-(trifluoromethyl)[1,1'-biphenyl]-4-yl]oxy}methyl)piperidine-1-carboxylate

Bis(2-methoxyethyl)azodicarboxylate (24 mg) was added to a solution of methyl 4'-hydroxy-6-(trifluoromethyl)[1,1'-biphenyl]-3-carboxylate (20 mg) obtained in Step 2, tert-butyl 4-(hydroxymethyl)piperidine-1-carboxylate (22 mg), and PPh₃ (27 mg) in THF (0.7 mL), and the mixture was stirred at room temperature overnight. The reaction solution was concentrated under reduced pressure, and the obtained residue was purified by silica gel column chromatography to afford the title compound (20 mg).

### Reference Example 79: Methyl 4'-({(2S)-1-[(tert-butoxycarbonyl)amino]propan-2-yl}oxy)-6-(trifluoromethyl)[1,1'-biphenyl]-3-carboxylate

The title compound (20 mg) was obtained as described in Reference Example 78, Step 3, using tert-butyl [(2R)-2-hydroxypropyl]carbamate instead of tert-butyl 4-(hydroxymethyl)piperidine-1-carboxylate.

### Reference Example 81: tert-Butyl 2-[5-(methoxycarbonyl-2-(trifluoromethyl)phenyl]ethyl}piperidine-1-carboxylate

### [Step 1] Preparation of tert-butyl 4-{[5-(methoxycarbonyl-2-(trifluoromethyl)phenyl]ethynyl}piperidine-1-carboxylate

DMF (2 mL) was added to methyl 3-bromo-4-(trifluoromethyl)benzoate (406 mg), tert-butyl 4-ethynylpiperidine-1-carboxylate (300 mg), copper iodide (20 mg), Pd(PPh₃)₄ (83 mg), and TEA (2 mL). After degassing, the mixture was stirred at 50°C under argon atmosphere for 4 hours. The reaction solution was diluted with water, and the reaction solution was extracted with ethyl acetate. The organic layer was dried over anhydrous sodium sulfate, then the solvent was removed under reduced pressure, and the obtained residue was purified by silica gel column chromatography to afford the title compound (180 mg). MS (m/z): 412.7 [M+H]⁺

### [Step 2] Preparation of tert-butyl 4-{2-[5-(methoxycarbonyl-2-(trifluoromethyl)phenyl]ethyl}piperidine-1-carboxylate

The title compound was obtained as described in Reference Example 2, Step 5, using tert-butyl 4-{[5-(methoxycarbonyl-2-(trifluoromethyl)phenyl]ethynyl}piperidine-1- carboxylate obtained in Step 1 instead of benzyl 4-(2,2,3,3,10,10-hexamethyl-8-oxo-4,9-dioxa-7-aza-3-silaundecan-5-yl)piperidine-1-carboxylate.

### Reference Example 82: tert-Butyl [5-(methoxycarbonyl-2-(trifluoromethyl)phenyl]ethynyl}piperidine-1-carboxylate

The title compound (121 mg) was obtained as described in Reference Example 81, Step 1, using tert-butyl [(1r,4r)-4-ethynylcyclohexyl]carbamate (300 mg) instead of tert-butyl 4-ethynylpiperidine-1-carboxylate.

### Reference Example 83: Methyl 3-(2-{(1r,4s)-4-{(tert-butoxycarbonyl)amino]cyclohexyl}ethyl)-4-(trifluoromethyl)benzoate

The title compound (100 mg) was obtained as described in Reference Example 1, Step 2, using methyl 3-({(1r,4r)-4-[(tert-butoxycarbonyl)amino]cyclohexyl}ethynyl)-4-(trifluoromethyl)benzoate (126 mg) obtained in Reference Example 82 instead of tert-butyl[2-methyl-2-(pyridin-4-yl)propyl]carbamate.

### Reference Example 84: Methyl 3-[(E)-2-15-[(tert-butoxycarbonyl)aminol-1,3-dioxan-2-yl}ethenyl]-4-(trifluoromethyl)benzoate

### [Step 1] Preparation of methyl 3-[(1E)-3-oxoprop-1-en-1-yl]-4-(trifluoromethyl)benzoate

DMF (7.1 mL) was added to methyl 3-bromo-4-(trifluoromethyl)benzoate (500 mg), acrolein diethyl acetal (690 mg), potassium carbonate (366 mg), potassium chloride (132 mg), tetrabutylammonium acetate (1.07 g), and Pd(OAc)₂ (20 mg). After degassing, the mixture was stirred at 90°C under argon atmosphere for 3 hours. The reaction solution was filtered through Celite (registered trademark) and then diluted with ethyl acetate, and the organic layer was washed with 2 M hydrochloric acid and saturated saline. The solvent was removed under reduced pressure, and then the residue was purified by silica gel column chromatography to afford the title compound (250 mg).

### [Step 2] Preparation of methyl 3-{(E)-2-[5-(1,3-dioxo-1,3-dihydro-2H-isoindol-2-yl)-1,3-dioxan-2-yl]ethenyl]-4-(trifluoromethyl)benzoate

2-(1,3-Dihydroxypropan-2-yl)-1H-isoindole-1,3(2H)-dione (321 mg) and p-toluene sulfonic acid monohydrate (92 mg) were added to a solution of methyl 3-[(1E)-3-oxoprop-1-en-1-yl]-4-(trifluoromethyl)benzoate (250 mg) obtained in Step 1 in toluene (4.8 mL), and the mixture was stirred at 140°C for 6 hours. The reaction solution was allowed to stand to cool, then sodium bicarbonate was added to the reaction solution, and the reaction solution was filtered through Celite (registered trademark). The solvent was removed under reduced pressure, and then the residue was purified by silica gel column chromatography to afford the title compound (291 mg).

### [Step 3] Preparation of methyl 3-[(E)-2-{5-[(tert-butoxycarbonyl)amino]-1,3-dioxan-2-yl}ethenyl]-4-(trifluoromethyl)benzoate

Hydrazine monohydrate (13 mg) was added to a solution of methyl 3-{(E)-2-[5-(1,3-dioxo-1,3-dihydro-2H-isoindol-2-yl)-1,3-dioxan-2-yl]ethenyl}-4-(trifluoromethyl)benzoate (124 mg) obtained in Step 2 in THF (1.3 mL), the mixture was stirred at 60°C, then hydrazine acetate (25 mg) was added to the mixture, and the mixture was stirred at the same temperature. After monitoring the consumption of the starting material on TLC, Boc₂O (65 mg) was added to the mixture, and the mixture was stirred at the same temperature. After monitoring the consumption of the starting material on TLC, the reaction solution was diluted with ethyl acetate, and then the organic layer was washed with saturated saline and dried over anhydrous sodium sulfate. Then, the solvent was removed under reduced pressure. The residue was purified by silica gel column chromatography to afford the title compound (71 mg).

### Reference Example 85: tert-Butyl 4-{(E)-2-[5-(methoxycarbonyl)-2-(trifluoromethyl)phenyl]ethenyl}piperidine-1-carboxylate

### [Step 1] Preparation of tert-butyl 4-[(E)-2-(tributylstannyl)ethenyl]piperidine-1-carboxylate

Hydrogenated tributyl tin (334 mg) was added to tert-butyl 4-ethynylpiperidine-1-carboxylate (200 mg), PdCh(PPh₃)₂ (34 mg), and THF (4.8 mL). After degassing, the mixture was stirred at room temperature under argon atmosphere for 2 hours. The reaction solution was concentrated under reduced pressure, and the obtained residue was purified by silica gel column chromatography to afford the title compound (440 mg).

### [Step 2] Preparation of tert-butyl 4-{(E)-2-[5-(methoxycarbonyl)-2-(trifluoromethyl)phenyl] ethenyl} piperidine-1 -carboxylate

Toluene (4.8 mL) was added to tert-butyl 4-[(E)-2-(tributylstannyl)ethenyl]piperidine-1-carboxylate (383 mg) obtained in Step 1, methyl 3-bromo-4-(trifluoromethyl)benzoate (325 mg), lithium chloride (81 mg), and Pd(PPh₃)₄ (55 mg). After degassing, the mixture was stirred at 110°C under argon atmosphere overnight. The reaction solution was allowed to stand to cool, and then was purified by silica gel column chromatography to afford the title compound (150 mg).

### Reference Example 90: 4-Bromo-3-({(1r,4r)-4-[(tertbutoxycarbonyl)amino]cyclohexyl}amino)benzoicacid

### [Step 1] Preparation of ethyl 4-bromo-3-({(1r,4r)-4-[(tertbutoxycarbonyl)amino]cyclohexyl}amino)benzoate

The title compound (127 mg) was obtained as described in Reference Example 59, using ethyl 4-bromo-3-iodobenzoate (200 mg) instead of ethyl 3-iodo-4-(trifluoromethyl)benzoate, and tert-butyl [(1r,4r)-4-aminocyclohexyl]carbamate instead of tert-butyl (4-aminobutyl)carbamate. MS (m/z): 441.2 [M+H]⁺

### [Step 2] Preparation of 4-bromo-3-({(1r,4r)-4-[(tertbutoxycarbonyl)amino] cyclohexyl} amino)benzoic acid

2 M aq. sodium hydroxide (0.43 mL) was added to a solution of ethyl 4-bromo-3-({(1r,4r)-4-[(tert-butoxycarbonyl)amino]cyclohexyl}amino)benzoate (127 mg) obtained in Step 1 in ethanol (2 mL), and the mixture was stirred at 70°C for 1 hour. The solvent was removed under reduced pressure, and the residue was diluted with water, and the dilution was neutralized by adding 1 M hydrochloric acid thereto. The resulting precipitate was collected by filtration to afford the title compound (52 mg). MS (m/z): 413.2 [M+H]⁺

### Reference Example 91: Methyl3-({(1r,4r)-4-[(tert-butoxycarbonyl)amino]cyclohexyl}amino)-5-fluoro-4-(trifluoromethyl)benzoate

DMSO (3 mL) was added to methyl 3,5-difluoro-4-(trifluoromethyl)benzoate (160 mg), tert-butyl [(1r,4r)-4-aminocyclohexyl]carbamate (286 mg), and potassium carbonate (276 mg), and the mixture was stirred at 110°C for 2 hours. The reaction solution was diluted with ethyl acetate, and then the organic layer was washed with water and saturated saline and dried over anhydrous magnesium sulfate. The solvent was removed under reduced pressure, and then the residue was purified by silica gel column chromatography to afford the title compound (100 mg). MS (m/z): 435.3 [M+H]⁺

### Reference Example 126: Ethyl 2-[({(1r,4r)-4-[(tertbutoxycarbonyl)amino]cyclohexyl}methyl)amino][1,1'-biphenyl]-4-carboxylate

The title compound (47 mg) was obtained as described in Reference Example 74, using ethyl 4-bromo-3-[({(lr,4r)-4-[(tertbutoxycarbonyl)amino]cyclohexyl}methyl)amino]benzoate (52 mg) obtained in Reference Example 123 instead of ethyl 4-chloro-3-iodobenzoate, and phenylboronic acid instead of (3-{[(tert-butoxycarbonyl)amino]methyl}phenyl)boronic acid. MS (m/z): 453.4 [M+H]⁺

### Reference Example 130: 3-(((1S)-1-[1-(tert-Butoxycarbonyl)piperidin-4-yl]ethyl}amino)-5-fluoro-4-(trifluoromethyl)benzoicacid

### [Step 1] Preparation of tert-butyl 4-{(1S)-1-[5-bromo-3-fluoro-2-(trifluoromethyl)anilino]ethyl } piperidine-1-carboxylate

NMP (13 mL) was added to 5-bromo-1,3-difluoro-2-(trifluoromethyl)benzene (4.8 g), tert-butyl 4-[(1S)-1-aminoethyl]piperidine-1-carboxylate (3.0 g), and sodium bicarbonate (3.3 g), and the mixture was stirred at 120°C for 5 hours. The reaction solution was diluted with water, and the reaction solution was extracted with ethyl acetate. The organic layer was washed with saturated saline, and the solvent was removed under reduced pressure. The residue was purified by silica gel column chromatography to afford the title compound (3.3 g).

### [Step 2] Preparation of tert-butyl 4-{(1S)-1-[5-cyano-3-fluoro-2-(trifluoromethyl)anilino]ethyl}piperidine-1-carboxylate

A mixture of tert-butyl 4-{(1S)-1-[5-bromo-3-fluoro-2-(trifluoromethyl)anilino]ethyl}piperidine-1-carboxylate (2.4 g) obtained in Step 1, zinc cyanide (601 mg), and Pd(PPh₃)₄ (591 mg) in DMF (15 mL) was degassed and then stirred at 120°C under argon atmosphere for 2 hours. The reaction solution was diluted with ethyl acetate, and the organic layer was washed with saturated saline. The solvent was removed under reduced pressure, and the obtained residue was purified by silica gel column chromatography to afford the title compound (2.0 g).

### [Step 3] Preparation of 3-({(1S)-1-[1-(tert-butoxycarbonyl)piperidin-4-yl]ethyl}amino)-5-fluoro-4-(trifluoromethyl)benzoic acid

5.8 M aq. sodium hydroxide (25 mL) was added to a solution of tert-butyl 4-{(1S)-1-[5-cyano-3-fluoro-2-(trifluoromethyl)anilino]ethyl}piperidine-1-carboxylate (2.0 g) obtained in Step 2 in ethanol (25 mL), and the mixture was stirred at 70°C for 2 hours. The reaction solution was neutralized by hydrochloric acid under ice-cooling, and then extracted with ethyl acetate. The organic layer was washed with saturated saline and dried over anhydrous magnesium sulfate. The solvent was removed under reduced pressure to afford the title compound (2.0 g).

### Reference Example 137: tert-Butyl 4-{(1S)-1-[2-methoxy-5-(methoxycarbonyl)anilino]ethyl}piperidine-1-carboxylate

### [Step 1] Preparation of methyl 4-methoxy-3-[(trifluoromethanesulfonyl)oxy]benzoate

The title compound (850 mg) was obtained as described in Reference Example 32, using methyl 3-hydroxy-4-methoxybenzoate (515 mg) instead of.

### [Step 2] Preparation of tert-butyl 4-{(1S)-1-[2-methoxy-5-(methoxycarbonyl)anilino]ethyl}piperidine-1-carboxylate

The title compound (41 mg) was obtained as described in Reference Example 59, using methyl 4-methoxy-3-[(trifluoromethanesulfonyl)oxy]benzoate (100 mg) obtained in Reference Example 137, Step 1 instead of ethyl 3-iodo-4-(trifluoromethyl)benzoate, and tert-butyl 4-[(1S)-1-aminoethyl]piperidine-1-carboxylate instead of tert-butyl (4-aminobutyl)carbamate. MS (m/z): 449.7 [M+H]⁺

### Reference Example 142: tert-Butyl 4-{(1S)-1-[2,3-dichloro-6-fluoro-5-(methoxycarbonyl)anilino]ethyl}piperidine-1-carboxylate

### [Step 1] Preparation of 4,5-dichloro-2-fluoro-3-iodobenzoic acid

4,5-Dichloro-2-fluorobenzoic acid (500 mg) was dissolved in concentrated sulfuric acid (4 mL), and N-iodosuccinimide (600 mg) was added to the solution under ice-cooling, and the mixture was stirred at room temperature for 6 hours. The reaction solution was added to ice water, and the resulting precipitate was collected by filtration to afford the title compound (750 mg).

### [Step 2] Preparation of methyl 4,5-dichloro-2-fluoro-3-iodobenzoate

The title compound (734 mg) was obtained as described in Reference Example 78, Step 2, using 4,5-dichloro-2-fluoro-3-iodobenzoic acid (750 mg) obtained in Step 1 instead of 4'-hydroxy-6-(trifluoromethyl)[1,1'-biphenyl]-3-carboxylic acid.

### [Step 3] Preparation of tert-butyl 4-{(1S)-1-[2,3-dichloro-6-fluoro-5-(methoxycarbonyl)anilino]ethyl}piperidine-1-carboxylate

The title compound (23 mg) was obtained as described in Reference Example 59, using methyl 4,5-dichloro-2-fluoro-3-iodobenzoate (50 mg) obtained in Reference Example 142, Step 2 and tert-butyl 4-[(1S)-1-aminoethyl]piperidine-1-carboxylate. MS (m/z): 449.7 [M+H]⁺

### Reference Example 147: tert-Butyl 4-((1S)-1-[3-amino-2-chloro-5-(methoxycarbonyl)anilino]ethyl}piperidine-1-carboxylate

The title compound (16 mg) was obtained as described in Reference Example 2, Step 5, using tert-butyl 4-{(1S)-1-[2-chloro-5-(methoxycarbonyl)-3-nitroanilino]ethyl}piperidine-1-carboxylate (30 mg) obtained in Reference Example 146 instead of benzyl 4-(2,2,3,3,10,10-hexamethyl-8-oxo-4,9-dioxa-7-aza-3-silaundecan-5-yl)piperidine-1-carboxylate.

### Reference Example 158: tert-Butyl 4-[(1S)-1-{3-chloro-5-(methoxycarbonyl)-2-[(propan-2-yl)oxy]anilino}ethyl]piperidine-1-carboxylate

### [Step 1] Preparation of methyl 3-chloro-4-hydroxy-5-iodobenzoate

Methyl 3-chloro-4-hydroxybenzoate (2.0 g) was dissolved in dichloromethane (50 mL), N-iodosuccinimide (2.5 g) and titanium(IV) chloride (1.85 g) were added to the solution, and the mixture was stirred at room temperature for 24 hours. The reaction solution was diluted with saturated aq. sodium bicarbonate and extracted with dichloromethane. The organic layer was dried over anhydrous magnesium sulfate, and then the solvent was removed under reduced pressure. The residue was purified by silica gel column chromatography to afford the title compound (1.65 g).

### [Step 2] Preparation of methyl 3-chloro-5-iodo-4-[(propan-2-yl)oxy]benzoate

2-Bromopropane (276 mg) was added to a solution of methyl 3-chloro-4-hydroxy-5-iodobenzoate (350 mg) obtained in Step 1 and potassium carbonate (310 mg) in DMF (3 mL), and the mixture was stirred at 100°C for 9 hours. The reaction solution was diluted with ethyl acetate, and the organic layer was washed with water and saturated saline. The solvent was removed under reduced pressure, and then residue was purified by silica gel column chromatography to afford the title compound (157 mg).

### [Step 3] Preparation of tert-butyl 4-[(1S)-1-{3-chloro-5-(methoxycarbonyl)-2-[(propan-2-yl)oxy]anilino } ethyl]piperidine-1-carboxylate

The title compound (95 mg) was obtained as described in Reference Example 59, using methyl 3-chloro-5-iodo-4-[(propan-2-yl)oxy]benzoate (156 mg) obtained in Step 2 instead of ethyl 3-iodo-4-(trifluoromethyl)benzoate, and tert-butyl 4-[(1S)-1-aminoethyl]piperidine-1-carboxylate instead of tert-butyl (4-aminobutyl)carbamate.

### Reference Example 165: tert-Butyl (1S)-1-[2-chloro-3-cyano-5-(methoxycarbonyl)anilino]ethyl}piperidine-1-carboxylate

The title compound (20 mg) was obtained as described in Reference Example 130, Step 2, using tert-butyl 4-{(1S)-1-[3-bromo-2-chloro-5-(methoxycarbonyl)anilino]ethyl}piperidine-1-carboxylate (160 mg) obtained in Reference Example 152 instead of tert-butyl 4-{(1S)-1-[5-bromo-3-fluoro-2-(trifluoromethyl)anilino]ethyl}piperidine-1 -carboxylate.

### Reference Example 176: Methyl 4-bromo-1-{[1-(tert-butoxycarbonyl)piperidin-4-yl]methyl}-1H-indazole-6-carboxylate

The title compound (122 mg) was obtained as described in Reference Example 78, Step 3, using methyl 4-bromo-1H-indazole-6-carboxylate (150 mg) instead of methyl 4'-hydroxy-6-(trifluoromethyl)[1,1'-biphenyl]-3-carboxylate.

### Reference Example 181: tert-Butyl 4-1[5-(ethoxycarbonyl)-3-fluoro-2-(trifluoromethyl)anilino]methyl}-4-fluoropiperidine-1-carboxylate

### [Step 1] Preparation of ethyl 3,5-difluoro-4-(trifluoromethyl)benzoate

DMF (0.1 mL) was added to a solution of 3,5-difluoro-4-(trifluoromethyl)benzoic acid (2.0 g) in dichloromethane (40 mL), oxalyl chloride (1.12 mL) was added dropwise to the mixture under ice-cooling, and the mixture was stirred for 1 hour. Then, ethanol (20 mL) was added dropwise, and the mixture was stirred at room temperature for 2 hours. The reaction solution was concentrated under reduced pressure, and the obtained residue was purified by silica gel column chromatography to afford the title compound (1.95 g).

### [Step 2] Preparation of tert-butyl 4-{[5-(ethoxycarbonyl)-3-fluoro-2-(trifluoromethyl)anilino]methyl}-4-fluoropiperidine-1-carboxylate

The title compound (73 mg) was obtained as described in Reference Example 91, using ethyl 3,5-difluoro-4-(trifluoromethyl)benzoate (150 mg) obtained in Reference Example 181, Step 1 and tert-butyl 4-(aminomethyl)-4-fluoropiperidine-1-carboxylate.

### Reference Example 182: Methyl 3-[({(1r,4r)-4-[(tertbutoxycarbonyl)amino]cyclohexyl}methyl)(methy)amino]-4-(trifluoromethyl)benzoate

### [Step 1] Preparation of methyl 3-[({(1r,4r)-4-[(tertbutoxycarbonyl)amino]cyclohexyl}methyl)amino]-4-(trifluoromethyl)benzoate

The title compound (56 mg) was obtained as described in Reference Example 59, using methyl 3-bromo-4-(trifluoromethyl)benzoate (65 mg) and tert-butyl [(1r,4r)-4-(aminomethyl)cyclohexyl]carbamate. MS (m/z): 431.7 [M+H]⁺

### [Step 2] Preparation of methyl 3-[({(1r,4r)-4-[(tertbutoxycarbonyl)amino]cyclohexyl}methyl)(methyl)amino]-4-(trifluoromethyl)benzoate

A mixture of methyl 3-[({(1r,4r)-4-[(tertbutoxycarbonyl)amino]cyclohexyl}methyl)amino]-4-(trifluoromethyl)benzoate (56 mg), formaldehyde (37% aqueous solution, 0.63 mL), acetic acid (0.5 mL), and acetonitrile (3 mL) was stirred at room temperature for 15 minutes. Then, sodium cyanotrihydridoborate (522 mg) was added to the mixture, and the mixture was stirred at the same temperature overnight. Saturated aq. sodium bicarbonate was added to the reaction solution, and the reaction solution was extracted with ethyl acetate. The organic layer was washed with saturated saline and dried over anhydrous sodium sulfate, and then the solvent was removed under reduced pressure. The residue was purified by silica gel column chromatography to afford the title compound (22 mg). MS (m/z): 445.7 [M+H]⁺

### Reference Example 184: Methyl 3-[({(1r,4r)-4-[(tertbutoxycarbonyl)(methyl)amino]cyclohexyl}methyl)(methyl)amino]-4-(trifluoromethyl)benzoate

The title compound (82 mg) was obtained as described in Reference Example 13, Step 2, using methyl 3-[({(1r,4r)-4-[(tertbutoxycarbonyl)(methyl)amino]cyclohexyl}methyl)amino]-4-(trifluoromethyl)benzoate (70 mg) obtained in Reference Example 183 instead of tert-butyl {(1r,4r)-4-[(dibenzylamino)methyl]cyclohexyl}carbamate. MS (m/z): 459.7 [M+H]⁺

### Reference Example 207: tert-Butyl (1R,5S,8r)-8-{(1S)-1-[5-(methoxycarbonyl)-2-(trifluoromethyl)anilino]ethyl}-3-azabicyclo[3.2.1]octane-3-carboxylate

### [Step 1] Preparation of methyl 3-({(1S)-1-[(1R,5S,8r)-3-benzyl-3-azabicyclo[3.2.1]octan-8-yl]ethyl} amino)-4-(trifluoromethyl)benzoate

The title compound (54 mg) was obtained as described in Reference Example 59, using methyl 3-iodo-4-(trifluoromethyl)benzoate (110 mg) and (1S)-1-[(1R,5S,8r)-3-benzyl-3-azabicyclo[3.2.1]octan-8-yl]ethane-1-amine obtained in Reference Example 29. MS (m/z): 447.7 [M+H]⁺

### [Step 2] Preparation of tert-butyl (1R,5S,8r)-8-{(1S)-1-[5-(methoxycarbonyl)-2-(trifluoromethyl)anilino]ethyl)-3-azabicyclo[3.2.1]octane-3-carboxylate

The title compound (734 mg) was obtained as described in Reference Example 51, Step 1, using methyl 3-({(1S)-1-[(1R,5S,8r)-3-benzyl-3-azabicyclo[3.2.1]octan-8-yl]ethyl}amino)-4-(trifluoromethyl)benzoate (750 mg) obtained in Step 1 instead of [(3S,4R)-1-benzyl-3-fluoropiperidin-4-yl]methanol. MS (m/z): 357.2 [M+H]⁺

### Reference Example 211: 3-({(1S)-1-[1-(tert-Butoxcarbonyl)-4-fluoropiperidin-4-yl]ethyl}amino)-5-fluoro-4-(trifluoromethyl)benzoic acid

### [Step 1] Preparation of 3-bromo-5-fluoro-4-(trifluoromethyl)benzonitrile

Isoamyl nitrite (0.41 mL) was added to a mixture of 3-amino-5-fluoro-4-(trifluoromethyl)benzonitrile (480 mg), copper(II) bromide (630 mg), and acetonitrile (10 mL), and the mixture was stirred at 60°C for 8 hours. Hydrochloric acid was added to the reaction solution, and the reaction solution was extracted with ethyl acetate. The organic layer was washed with saturated saline, and then the solvent was removed under reduced pressure. The residue was purified by silica gel column chromatography to afford the title compound (580 mg).

### [Step 2] Preparation of tert-butyl 4-{(1S)-1-[5-cyano-3-fluoro-2-(trifluoromethyl)anilino]ethyl}-4-fluoropiperidine-1-carboxylate

The title compound (135 mg) was obtained as described in Reference Example 59, using 3-bromo-5-fluoro-4-(trifluoromethyl)benzonitrile (117 mg) obtained in Reference Example 211, Step 1 and tert-butyl 4-[(1S)-1-aminoethyl]-4-fluoropiperidine-1-carboxylate obtained in Reference Example 12.

### [Step 3] Preparation of 3-({(1S)-1-[1-(tert-butoxycarbonyl)-4-fluoropiperidin-4-yl]ethyl)amino)-5-fluoro-4-(trifluoromethyl)benzoic acid

The title compound (140 mg) was obtained as described in Reference Example 73, Step 3, using tert-butyl 4-1(1S)-1-[5-cyano-3-fluoro-2-(trifluoromethyl)anilino]ethyl)-4-fluoropiperidine-1-carboxylate (133 mg) obtained in Step 2 instead of tert-butyl 4-{(1S)-1-[5-cyano-3-fluoro-2-(trifluoromethyl)anilino]ethyl}piperidine-1-carboxylate.

### Reference Example 213: Methyl 3-(2-{(2r,5r)-5-[(tert-butoxycarbonyl)aminol-1,3-dioxan-2-yl}cyclopropyl}-4-(trifluoromethyl)benzoate

### [Step 1] Preparation of methyl 3-[(1E)-3-hydroxyprop-1-en-1-yl]-4-(trifluoromethyl)benzoate

Sodium borohydride (73 mg) was added to a solution of methyl 3-[(1E)-3-oxoprop-1-en-1-yl]-4-(trifluoromethyl)benzoate (500 mg) obtained in Reference Example 84, Step 1 in ethanol (9.7 mL), and the mixture was stirred at room temperature for 30 minutes. The reaction solution was diluted with saturated aq. ammonium chloride and extracted with ethyl acetate. The organic layer was dried over anhydrous sodium sulfate, and then the solvent was removed under reduced pressure. The residue was purified by silica gel column chromatography to afford the title compound (600 mg).

### [Step 2] Preparation of methyl 3-[2-(hydroxymethyl)cyclopropyl]-4-(trifluoromethyl)benzoate

Under ice-cooling, a solution of TFA (0.44 mL) in dichloromethane (5.8 mL) was added dropwise to a solution of diethylzinc (1 M in hexane, 5.8 mL) in dichloromethane (5.8 mL), then diiodomethane (1.54 g) was added, and the mixture was stirred at the same temperature for 30 minutes. Methyl 3-[(1E)-3-hydroxyprop-1-en-1-yl]-4-(trifluoromethyl)benzoate (300 mg) obtained in Reference Example 213, Step 1 was added to the reaction solution, and the mixture was stirred at room temperature overnight. Water was added to the reaction solution, and the reaction solution was filtered through Celite (registered trademark). The filtrate was extracted with ethyl acetate, the organic layer was dried over anhydrous sodium sulfate, and then the solvent was removed under reduced pressure. The residue was purified by silica gel column chromatography to afford the title compound (160 mg).

### [Step 3] Preparation of methyl 3-(2-formylcyclopropyl)-4-(trifluoromethyl)benzoate

1,1,1-Triacetoxy-1,1 -dihydro-1,2-benziodoxol-3 -(1 H)-one (Dess-Martin reagent) (495 mg) was added to a solution of methyl 3-[2-(hydroxymethyl)cyclopropyl]-4-(trifluoromethyl)benzoate (160 mg) obtained in Reference Example 213, Step 2 in dichloromethane (5 mL), and the mixture was stirred at room temperature. After monitoring the consumption of the starting material on TLC, the reaction solution was diluted with saturated aq. sodium bicarbonate, and then extracted with ethyl acetate. The organic layer was dried over anhydrous sodium sulfate, and then the solvent was removed under reduced pressure. The residue was purified by silica gel column chromatography to afford the title compound (159 mg).

### [Step 4] Preparation of methyl 3-{2-[5-(1,3-dioxo-1,3-dihydro-2H-isoindol-2-yl)-1,3-dioxan-2-yl]cyclopropyl}-4-(trifluoromethyl)benzoate

The title compound (235 mg) was obtained as described in Reference Example 84, Step 2, using methyl 3-(2-formylcyclopropyl)-4-(trifluoromethyl)benzoate (159 mg) obtained in Step 3 instead of methyl 3-[(1E)-3-oxoprop-1-en-1-yl]-4-(trifluoromethyl)benzoate.

### [Step 5] Preparation of methyl 3-(2-{5-[(tert-butoxycarbonyl)amino]-1,3-dioxan-2-yl}cyclopropyl}-4-(trifluoromethyl)benzoate

Sodium borohydride (34 mg) was added to a solution of methyl 3-{2-[5-(1,3-dioxo-1,3-dihydro-2H-isoindol-2-yl)-1,3-dioxan-2-yl]cyclopropyl}-4-(trifluoromethyl)benzoate (215 mg) obtained in Step 4 in 2-propanol (1.92 mL) and water (0.32 mL), and the mixture was stirred under ice-cooling for 2 hours. Acetic acid (2.26 mL) was added to the reaction solution, and the mixture was stirred at 60°C for 30 minutes. The reaction solution was concentrated under reduced pressure, THF (2.3 mL), sodium bicarbonate (190 mg), and Boc₂O (296 mg) were added to the obtained residue, and the mixture was stirred at room temperature. After monitoring the consumption of the starting material on TLC, the reaction solution was concentrated under reduced pressure, and the obtained residue was purified by silica gel column chromatography to afford the title compound (14 mg).

### Reference Example 215: tert-Butyl 4-{(1R)-2,2-difluoro-1-[3-fluoro-5-(methoxycarbonyl)-2-(trifluoromethyl)anilino]ethyl}-4-fluoropiperidine-1 -carboxylate

### [Step 1] Preparation of methyl 3-bromo-5-fluoro-4-(trifluoromethyl)benzoate

The title compound (500 mg) was obtained as described in Reference Example 211, Step 1, using methyl 3-amino-5-fluoro-4-(trifluoromethyl)benzoate (1.12 g) instead of 3-amino-5-fluoro-4-(trifluoromethyl)benzonitrile.

### [Step 2] Preparation of tert-butyl 4-{(1R)-2,2-difluoro-1-[3-fluoro-5-(methoxycarbonyl)-2-(trifluoromethyl)anilino]ethyl}-4-fluoropiperidine-1-carboxylate

The title compound (171 mg) was obtained as described in Reference Example 59, using methyl 3-bromo-5-fluoro-4-(trifluoromethyl)benzoate obtained in Reference Example 215, Step 1 and tert-butyl 4-[(1R)-1-amino-2,2-difluoroethyl]-4-fluoropiperidine-1-carboxylate (125 mg) obtained in Reference Example 18.

### Reference Example 228: Methyl 3-[({(1S,3R)-3-[acetyl(tert-butoxycarbonyl)amino]-2,2-dimethylcyclobutyl}methyl)aminol-4-(trifluoromethyl)benzoate

### [Step 1] Preparation of methyl 3-({[(1S,3R)-3-acetamide-2,2-dimethylcyclobutyl]methyl} amino)-4-(trifluoromethyl)benzoate

The title compound (298 mg) was obtained as described in Reference Example 59, using methyl 3-bromo-5-fluoro-4-(trifluoromethyl)benzoate (399 mg) and N-[(1R,3S)-3-(aminomethyl)-2,2-dimethylcyclobutyl]acetamide obtained in Reference Example 35. MS (m/z): 373.2 [M+H]⁺

### [Step 2] Preparation of methyl 3-[({(1S,3R)-3-[acetyl(tert-butoxycarbonyl)amino]-2,2-dimethylcyclobutyl}methyl)amino]-4-(trifluoromethyl)benzoate

The title compound (200 mg) was obtained as described in Reference Example 1, Step 1, using methyl 3-(([(1S,3R)-3-acetamide-2,2-dimethylcyclobutyl]methyl}amino)-4-(trifluoromethyl)benzoate (298 mg) obtained in Step 1 instead of 2-methyl-2-(pyridin-4-yl)propan-1-amine.

### Reference Example 244: Methyl 3-{[(1S)-1-{(1s,3R)-3-[(tertbutoxycarbonyl)(methyl)amino]cyclobutyl}ethyl]amino}-4-(trifluoromethyl)benzoate

### [Step 1] Preparation of methyl 3-{[(1S)-1-{(1s,3R)-3-[(2-nitrobenzene-1-sulfonyl)amino]cyclobutyl}ethyl]amino}-4-(trifluoromethyl)benzoate

Hydrogen chloride (2 M in methanol, 2 mL) was added to methyl 3-{[(1S)-1-{(1s,3R)-3-[(tert-butoxycarbonyl)amino]cyclobutyl}ethyl]amino)-4-(trifluoromethyl)benzoate (100 mg) obtained in Reference Example 197, and the mixture was stirred at room temperature for 1 hour. The reaction solution was concentrated under reduced pressure, THF (2 mL), sodium bicarbonate (61 mg), and 2-nitrobenzenesulfonyl chloride (80 mg) were added to the obtained residue, and the mixture was stirred at room temperature for 1 hour. The reaction solution was diluted with water and extracted with ethyl acetate. The organic layer was dried over anhydrous sodium sulfate, and then the solvent was removed under reduced pressure. The residue was purified by silica gel column chromatography to afford the title compound (98 mg).

### [Step 2] Preparation of methyl 3-{[(1S)-1-{(1s,3R)-3-[methyl(2-nitrobenzene-1-sulfonyl)amino]cyclobutyl} ethyl]amino} -4-(trifluoromethyl)benzoate

Iodomethane (13 mg) and cesium carbonate (97 mg) were added to a solution of methyl 3-{[(1S)-1-{(1s,3R)-3-[(2-nitrobenzene-1-sulfonyl)amino]cyclobutyl)ethyl]amino)-4-(trifluoromethyl)benzoate (30 mg) obtained in Reference Example 244, Step 1 in DMF (0.3 mL), and the mixture was stirred at room temperature for 2 hours. The reaction solution was diluted with ethyl acetate, and the organic layer was washed with water and saturated saline. The solvent was removed under reduced pressure, and then the residue was purified by silica gel column chromatography to afford the title compound (31 mg).

### [Step 3] Preparation of methyl 3-{[(1S)-1-{(1s,3R)-3-[(tertbutoxycarbonyl)(methyl)amino]cyclobutyl}ethyl]amino}-4-(trifluoromethyl)benzoate

4-Mercaptobenzoic acid (19 mg) and potassium carbonate (33 mg) were added to a solution of methyl 3-{[(1S)-1-{(1s,3R)-3-[methyl(2-nitrobenzene-1-sulfonyl)amino]cyclobutyl}ethyl]amino}-4-(trifluoromethyl)benzoate (31 mg) obtained in Step 2 in DMF (0.12 mL), and the mixture was stirred at 90°C. After monitoring the consumption of the starting material on TLC, the reaction solution was diluted with water, Boc₂O (66 mg) was added to the reaction solution, and the mixture was stirred at room temperature for 1 hour. Ethyl acetate was added to the reaction solution, the organic layer was washed with saturated saline and dried over anhydrous sodium sulfate, and then the solvent was removed under reduced pressure. The residue was purified by silica gel column chromatography to afford the title compound (15 mg).

### Reference Example 249: Methyl 3-{[(1S)-1-{(1r,4S)-4-[(2,2,2-trifluoroethyl)amino]cyclohexyl}ethyl]amino}-4-(trifluoromethyl)benzoate

Hydrogen chloride (4 M in 1,4-dioxane, 2 mL) was added to a solution of methyl 3-{[(1S)-1-{(1r,4S)-4-[(tert-butoxycarbonyl)amino]cyclohexyl}ethyl]amino}-4-(trifluoromethyl)benzoate (200 mg) obtained in Reference Example 161 in methanol (2 mL), and the mixture was stirred at room temperature for 1 hour. The reaction solution was concentrated under reduced pressure, THF (3 mL), TEA (0.36 mL), and 2,2,2-trifluoroethyl trifluoromethanesulfonate (274 mg) were added to the obtained residue, and the mixture was stirred at room temperature for 1 hour. The reaction solution was concentrated under reduced pressure, and the obtained residue was purified by silica gel column chromatography to afford the title compound (160 mg).

### Reference Example 253: Methyl 3-{[(1S)-1-{(1r,4S)-4-[(tert-butoxycarbonyl)(2-hydroxyethyl)amino]cyclohexyl}ethyl]amino}-4-(trifluoromethyl)benzoate

Hydrogen chloride (4 M in 1,4-dioxane, 1 mL) was added to a solution of methyl 3-{[(1S)-1-{(1r,4S)-4-[(tert-butoxycarbonyl)amino]cyclohexyl}ethyl]amino}-4-(trifluoromethyl)benzoate (100 mg) obtained in Reference Example 161 in methanol (1 mL), and the mixture was stirred at room temperature for 1 hour. The reaction solution was concentrated under reduced pressure, THF (2 mL), TEA (0.16 mL), and 2-iodoethanol (39 mg) were added to the obtained residue, and the mixture was stirred at 60°C for 30 minutes. Then, Boc₂O (491 mg) was added to the reaction solution, and the mixture was stirred at the same temperature for 12 hours. The reaction solution was concentrated under reduced pressure, and the obtained residue was purified by silica gel column chromatography to afford the title compound (70 mg).

### Reference Example 255: 3-{[(1S)-1-{(1s,3R)-3-[(tert-Butoxycarbonyl)(methyl)amino]cyclobutyl}ethyl]amino}-5-fluoro-4-(trifluoromethyl)benzoic acid

### [Step 1] Preparation of N-[(1R,3s)-3-{(1S)-1-[5-bromo-3-fluoro-2-(trifluoromethyl)anilino]ethyl}cyclobutyl]-2-nitrobenzene-1-sulfonamide

The title compound (1.08 g) was obtained as described in Reference Example 244, Step 1, using tert-butyl [(1R,3s)-3-{(1S)-1-[5-bromo-3-fluoro-2-(trifluoromethyl)anilino]ethyl}cyclobutyl]carbamate (767 mg) obtained in Reference Example 217 instead of methyl 3-1[(1S)-1-{(1s,3R)-3-[(tert-butoxycarbonyl)amino]cyclobutyl}ethyl]amino}-4-(trifluoromethyl)benzoate. [Step 2] Preparation of tert-butyl[(1R,3s)-3-{(1S)-1-[5-bromo-3-fluoro-2-(trifluoromethyl)anilino]ethyl}cyclobutyl]methylcarbamate

Iodomethane (236 mg) and potassium carbonate (921 mg) were added to a solution of N-[(1R,3s)-3- {(1S)-1-[5-bromo-3-fluoro-2-(trifluoromethyl)anilino]ethyl}cyclobutyl]-2-nitrobenzene-1-sulfonamide (600 mg) obtained in Step 1 in DMF (11 mL), and the mixture was stirred at room temperature. After monitoring the consumption of the starting material on TLC, 4-mercaptobenzoic acid (428 mg) was added to the reaction solution, and the reaction mixture was stirred at 80°C. After monitoring the consumption of the starting material on TLC, the reaction solution was diluted with water, Boc₂O (1.21 g) was added to the reaction solution, and the mixture was stirred at room temperature. After monitoring the consumption of the starting material on TLC, ethyl acetate was added to the reaction solution, the organic layer was washed with water and saturated saline and dried over anhydrous sodium sulfate, and then the solvent was removed under reduced pressure. The residue was purified by silica gel column chromatography to afford the title compound (396 mg).

### [Step 3] Preparation of tert-butyl [(1R,3s)-3-{(1S)-1-[5-cyano-3-fluoro-2-(trifluoromethyl)anilino]ethyl}cyclobutyl]methylcarbamate

The title compound (352 mg) was obtained as described in Reference Example 130, Step 2, using tert-butyl[(1R,3s)-3-{(1S)-1-[5-bromo-3-fluoro-2-(trifluoromethyl)anilino]ethyl}cyclobutyl]methylcarbamate (396 mg) obtained in Step 2 instead of tert-butyl 4-{(1S)-1-[5 -bromo-3 -fluoro-2-(trifluoromethyl)anilino]ethyl} piperidine-1 - carboxylate.

### [Step 4] Preparation of 3-{[(1S)-1-{(1s,3R)-3-[(tertbutoxycarbonyl)(methyl)amino]cyclobutyl}ethyl]amino}-5-fluoro-4-(trifluoromethyl)benzoic acid

The title compound was obtained as described in Reference Example 130, Step 3, using tert-butyl [(1R,3s)-3-{(1S)-1-[5-cyano-3-fluoro-2-(trifluoromethyl)anilino]ethyl}cyclobutyl]methylcarbamate (352 mg) obtained in Step 3 instead of tert-butyl 4-{(1S)-1-[5-cyano-3-fluoro-2-(trifluoromethyl)anilino]ethyl}piperidine-1-carboxylate. MS (m/z): 435.6 [M+H]⁺

### Reference Example 257: tert-Butyl 4-[{[2-chloro-5-(methoxycarbonyl)phenyl]methyl}(methyl)amino]piperidine-1-carboxylate

Methyl 3-(bromomethyl)-4-chlorobenzoate (400 mg) was dissolved in 2-propanol (7 mL), tert-butyl 4-(methylamino)piperidine-1-carboxylate (423 mg) and DIPEA (0.39 mL) were added to the solution, and the mixture was stirred at room temperature overnight. The reaction solution was concentrated under reduced pressure, and the obtained residue was purified by silica gel column chromatography to afford the title compound (447 mg).

### Reference Example 264: tert-Butvl 4-{(1S)-1-[2-(difluoromethoxy)-3-fluoro-5-(methoxycarbony)anilino]ethy}piperidine-1-carboxylate

### [Step 1] Preparation of methyl 3-bromo-4-(difluoromethoxy)-5-fluorobenzoate

Sodium chlorodifluoroacetate (612 mg) and potassium carbonate (416 mg) were added to a solution of methyl 3-bromo-5-fluoro-4-hydroxybenzoate (500 mg) in DMF (4 mL), and the mixture was stirred at 100°C for 4 hours. The reaction solution was diluted with ethyl acetate, and the organic layer was washed with water and saturated saline. The solvent was removed under reduced pressure, and then the residue was purified by silica gel column chromatography to afford the title compound (500 mg).

### [Step 2] Preparation of tert-butyl 4-{(1S)-1-[2-(difluoromethoxy)-3-fluoro-5-(methoxycarbonyl)anilino]ethyl} piperidine-1 -carboxylate

The title compound (200 mg) was obtained as described in Reference Example 59, using methyl 3-bromo-4-(difluoromethoxy)-5-fluorobenzoate (200 mg) obtained in Reference Example 277, Step 1 and tert-butyl 4-[(1S)-1-aminoethyl]piperidine-1-carboxylate.

### Reference Example 280: Methyl 3-[({(1r,4r)-4-[(tertbutoxycarbonyl)aminolcyclohexyl}methyl)sulfanyl]-4-(trifluoromethyl)benzoate

A mixture of methyl 3-bromo-4-(trifluoromethyl)benzoate (58 mg), tert-butyl [(1r,4r)-4-(sulfanylmethyl)cyclohexyl]carbamate (50 mg), Xantphos (5.9 mg), DIPEA (0.07 mL), Pd₂(dba)₃ (4.7 mg), and 1,4-dioxane (1.2 mL) was degassed and then stirred at 90°C under argon atmosphere for 2 hours. The reaction solution was filtered through Celite (registered trademark) and diluted with ethyl acetate, and then the organic layer was washed with water. The solvent was removed under reduced pressure, and then the residue was purified by silica gel column chromatography to afford the title compound (33 mg).

### Reference Example 290: Methyl 1-({(1r,4r)-4-[(tert-butoxycarbonyl)aminolcyclohexyl}methyl)-1,2,3,4-tetrahydroquinoline-7-carboxylate

{(1r,4r)-4-[(tert-Butoxycarbonyl)amino]cyclohexyl}methyl trifluoromethanesulfonate (491 mg) and potassium carbonate (188 mg) were added to a solution of methyl 1,2,3,4-tetrahydroquinoline-7-carboxylate (130 mg) in DMF (1 mL), and the mixture was stirred at 100°C for 5 hours. {(1r,4r)-4-[(tert-Butoxycarbonyl)amino]cyclohexyl}methyl trifluoromethanesulfonate (491 mg) and potassium carbonate (188 mg) were added to the reaction solution, and the mixture was further stirred at the same temperature for 22 hours. The reaction solution was diluted with ethyl acetate, and the organic layer was washed with water and saturated saline. The solvent was removed under reduced pressure, and then the residue was purified by silica gel column chromatography to afford the title compound (55 mg).

### Reference Example 291: Methyl 4-[(1S)-1-{(1r,4S)-4-[(tertbutoxycarbonyl)aminolcyclohexyl}ethyl]-3,4-dihydro-2H-1,4-benzoxazine-6-carboxylate

### [Step 1] Preparation of methyl 4-(benzyloxy)-3-{[(1S)-1-{(1r,4S)-4-[(tertbutoxycarbonyl)amino]cyclohexyl} ethyl]amino } benzoate

The title compound (375 mg) was obtained as described in Reference Example 59, using methyl 4-(benzyloxy)-3-bromobenzoate (729 mg) and tert-butyl {(1S,4r)-4-[(1S)-1-aminoethyl]cyclohexyl} carbamate.

### [Step 2] Preparation of methyl 3-{[(1S)-1-{(1r,4S)-4-[(tertbutoxycarbonyl)amino]cyclohexyl}ethyl]amino}-4-hydroxybenzoate

The title compound (300 mg) was obtained as described in Reference Example 2, Step 5, using methyl 4-(benzyloxy)-3-{[(1S)-1-{(1r,4S)-4-[(tertbutoxycarbonyl)amino]cyclohexyl}ethyl]amino}benzoate (375 mg) obtained in Step 1 instead of benzyl 4-(2,2,3,3,10,10-hexamethyl-8-oxo-4,9-dioxa-7-aza-3-silaundecan-5-yl)piperidine-1-carboxylate. MS (m/z): 393.7 [M+H]⁺

### [Step 3] Preparation of methyl 4-[(1S)-1-{(1r,4S)-4-[(tertbutoxycarbonyl)amino]cyclohexyl} ethyl]-3,4-dihydro-2H-1,4-benzoxazine-6-carboxylate

1,2-Dibromoethane (48 mg) and potassium carbonate (53 mg) were added to a solution of methyl 3-{ [(1S)-1-{(1r,4S)-4-[(tert-butoxycarbonyl)amino]cyclohexyl}ethyl]amino}-4-hydroxybenzoate (50 mg) obtained in Step 2 in DMF (0.64 mL), and the mixture was stirred at 80°C overnight. The reaction solution was diluted with ethyl acetate, and then the organic layer was washed with water and saturated saline and dried over anhydrous sodium sulfate. The solvent was removed under reduced pressure, and then the residue was purified by silica gel column chromatography to afford the title compound (4 mg). MS (m/z): 419.6 [M+H]⁺

### Reference Example 292: Methyl 4-{(1S)-1-[1-(tert-butoxycarbonyl)piperidin-4-yl]ethyl}-8-fluoro-3,4-dihydro-2H-1,4-benzoxazine-6-carboxylate

### [Step 1] Preparation of tert-butyl 4-{(1S)-1-[{2-[2-bromo-6-fluoro-4-(methoxycarbonyl)phenoxy]ethyl}(2-nitrobenzene-1-sulfonyl)amino]ethyl}piperidine-1-carboxylate

The title compound (72 mg) was obtained as described in Reference Example 78, Step 3, using tert-butyl 4-{(1S)-1-[(2-hydroxyethyl)(2-nitrobenzene-1-sulfonyl)amino]ethyl}piperidine-1-carboxylate (100 mg) obtained in Reference Example 55, Step 2 instead of tert-butyl 4-(hydroxymethyl)piperidine-1-carboxylate.

### [Step 2] Preparation of tert-butyl 4-[(1S)-1-({2-[2-bromo-6-fluoro-4-(methoxycarbonyl)phenoxy]ethyl}amino)ethyl]piperidine-1-carboxylate

Potassium carbonate (43 mg) was added to a solution of tert-butyl 4-{(1S)-1-[{2-[2-bromo-6-fluoro-4-(methoxycarbonyl)phenoxy] ethyl}(2-nitrobenzene-1- sulfonyl)amino]ethyl)piperidine-1-carboxylate (72 mg) obtained in Step 1 in acetonitrile (0.52 mL), and the mixture was stirred at room temperature for 6 hours. The reaction solution was concentrated under reduced pressure, and the obtained residue was purified by silica gel column chromatography to afford the title compound (41 mg).

### [Step 3] Preparation of methyl 4-{(1S)-1-[1-(tert-butoxycarbonyl)piperidin-4-yl]ethyl}-8-fluoro-3,4-dihydro-2H-1,4-benzoxazine-6-carboxylate

A mixture of tert-butyl 4-[(1S)-1-({2-[2-bromo-6-fluoro-4-(methoxycarbonyl)phenoxy]ethyl)amino)ethyl]piperidine-1-carboxylate (41 mg) obtained in Step 2, Xantphos (4.7 mg), cesium carbonate (53 mg), Pd₂(dba)₃ (3.7 mg), and toluene (0.81 mL) was degassed and then stirred at 110°C under argon atmosphere overnight. The reaction solution was allowed to stand to cool, and then was purified by silica gel column chromatography to afford the title compound (30 mg).

### Reference Example 294: Methyl (2R)-4-{(1S)-1-[1-(tert-butoxycarbonyl)piperidin-4-yl]ethyl}-8-fluoro-2-methyl-3,4-dihydro-2H-1,4-benzoxazine-6-carboxylate

### [Step 1] Preparation of tert-butyl 4-[(1S)-1-({(2R)-2-[2-bromo-6-fluoro-4-(methoxycarbonyl)phenoxy]propyl}[(S)-2-methylpropane-2-sulfinyl]amino)ethyl]piperidine-1-carboxylate

A crude product containing tert-butyl 4-[(1S)-1-({(2R)-2-[2-bromo-6-fluoro-4-(methoxycarbonyl)phenoxy]propyl}[(S)-2-methylpropane-2-sulfinyl]amino)ethyl]piperidine-1-carboxylate was obtained as described in Reference Example 78, Step 3, using tert-butyl 4-[(1S)-1-{(2-hydroxypropyl)[(S)-2-methylpropane-2-sulfinyl]amino}ethyl]piperidine-1-carboxylate (77 mg) obtained in Reference Example 57, Step 4 instead of tert-butyl 4-(hydroxymethyl)piperidine-1-carboxylate. The obtained crude product was purified by silica gel column chromatography to afford the title compound (98 mg) and tert-butyl 4-[(1S)-1-({(2S)-2-[2-bromo-6-fluoro-4-(methoxycarbonyl)phenoxy]propyl} [(S)-2-methylpropane-2-sulfinyl]amino)ethyl]piperidine-1-carboxylate (50 mg). Stereochemistry was assigned optionally by bioactivity and established structural similarity.

### [Step 2] Preparation of tert-butyl 4-[(1S)-1-({(2R)-2-[2-bromo-6-fluoro-4-(methoxycarbonyl)phenoxy]propyl} amino)ethyl]piperidine-1-carboxylate

The title compound (74 mg) was obtained as described in Reference Example 5, Step 3, using tert-butyl 4-[(1S)-1-({(2R)-2-[2-bromo-6-fluoro-4-(methoxycarbonyl)phenoxy]propyl}[(S)-2-methylpropane-2-sulfinyl]amino)ethyl]piperidine-1-carboxylate (98 mg) obtained in Step 1 instead of tert-butyl (1S)-1-{[(S)-2-methylpropane-2-sulfinyl]amino}-7-azaspiro[3.5]nonane-7-carboxylate.

### [Step 3] Preparation of methyl (2R)-4-{(1S)-1-[1-(tert-butoxycarbonyl)piperidin-4-yl]ethyl}-8-fluoro-2-methyl-3,4-dihydro-2H-1,4-benzoxazine-6-carboxylate

The title compound (50 mg) was obtained as described in Reference Example 292, Step 3, using tert-butyl 4-[(1S)-1-({(2R)-2-[2-bromo-6-fluoro-4-(methoxycarbonyl)phenoxy]propyl}amino)ethyl]piperidine-1-carboxylate (74 mg) obtained in Step 2 instead of tert-butyl 4-[(1S)-1-({2-[2-bromo-6-fluoro-4-(methoxycarbonyl)phenoxy]ethyl}amino)ethyl]piperidine-1-carboxylate.

### Example 1: 5-{3-[(4-aminobutyl)amino]-4-(trifluoromethyl)phenyl}-1,3,4-oxadiazol-2(3H)-one hydrochloride

### [Step 1] Preparation of tert-butyl {4-[5-(hydrazinecarbonyl)-2-(trifluoromethyl)anilino]butyl} carbamate

Hydrazine monohydrate (3.5 mL) was added to a solution of ethyl 3-({4-[(tertbutoxycarbonyl)amino]butyl}amino)-4-(trifluoromethyl)benzoate (138 mg) obtained in Reference Example 59 in ethanol (7.5 mL), and the mixture was stirred at 90°C for 2 hours. The reaction solution was diluted with ethyl acetate, and then the organic layer was washed with water and saturated saline and dried over anhydrous sodium sulfate. The solvent was removed under reduced pressure to afford the title compound (130 mg). MS (m/z): 391.6 [M+H]⁺

### [Step 2] Preparation of tert-butyl {4-[5-(5-oxo-4,5-dihydro-1,3,4-oxadiazol-2-yl)-2-(trifluoromethyl)anilino]butyl}carbamate

1,1'-Carbonyldiimidazole (60 mg) was added to a solution of tert-butyl {4-[5-(hydrazinecarbonyl)-2-(trifluoromethyl)anilino]butyl}carbamate (97 mg) obtained in Step 1 in THF (1 mL), and the mixture was stirred at room temperature for 12 hours. The reaction solution was concentrated under reduced pressure, and the obtained residue was purified by silica gel column chromatography to afford the title compound (100 mg). MS (m/z): 417.5 [M+H]⁺

### [Step 3] Preparation of 5-{3-[(4-aminobutyl)amino]-4-(trifluoromethyl)phenyl}-1,3,4-oxadiazol-2(3H)-one hydrochloride

Hydrogen chloride (2 M in ethanol, 2 mL) was added to tert-butyl {4-[5-(5-oxo-4,5-dihydro-1,3,4-oxadiazol-2-yl)-2-(trifluoromethyl)anilino]butyl}carbamate (130 mg) obtained in Step 2, and the mixture was stirred at 50°C for 1 hour. The reaction solution was concentrated under reduced pressure, the residue was suspended in diethyl ether, and the precipitate was collected by filtration, washed with diethyl ether, and then dried to afford the title compound (84 mg).

### Example 7: 5- {3-[(4-aminobutyl)amino]-4-(trifluoromethyl)phenyl}-1 ,3,4-oxadiazol-2(3H)-one hydrochloride

### [Step 1] Preparation of tert-butyl 4-[2-chloro-5-(hydrazinecarbonyl)phenyl]piperazine-1-carboxylate

The title compound (81 mg) was obtained as described in Example 1, Step 1, using tert-butyl 4-[2-chloro-5-(ethoxycarbonyl)phenyl]piperazine-1-carboxylate (89 mg) obtained in Reference Example 65 instead of ethyl 3-({4-[(tertbutoxycarbonyl)amino]butyl}amino)-4-(trifluoromethyl)benzoate. MS (m/z): 355.2 [M+H]⁺

### [Step 2] Preparation of tert-butyl 4-[2-chloro-5-(5-oxo-4,5-dihydro-1,3,4-oxadiazol-2-yl)phenyl]piperazine-1-carboxylate

The title compound (75 mg) was obtained as described in Example 1, Step 2, using tert-butyl 4-[2-chloro-5-(hydrazinecarbonyl)phenyl]piperazine-1-carboxylate (81 mg) obtained in Step 1 instead of tert-butyl {4-[5-(hydrazinecarbonyl)-2-(trifluoromethyl)anilino]butyl}carbamate.

### Example 8: 5-[4-chloro-3-(piperazin-1-yl)phenyl]-1,3,4-oxadiazol-2(3H)-one hydrochloride

The title compound (55 mg) was obtained as described in Example 1, Step 3, using tert-butyl 4-[2-chloro-5-(5-oxo-4,5-dihydro-1,3,4-oxadiazol-2-yl)phenyl]piperazine-1-carboxylate (71 mg) obtained in Example 7, Step 2 instead of tert-butyl {4-[5-(5-oxo-4,5-dihydro-1,3,4-oxadiazol-2-yl)-2-(trifluoromethyl)anilino]butyl}carbamate. Example 28: 5-[3-{2-[(2r,5r)-5-amino-1,3-dioxan-2-yl]ethyl}-4-(trifluoromethyl)phenyl]-1,3,4-oxadiazol-2(3H)-one hydrochloride

### [Step 1] Preparation of tert-butyl [2-{(E)-2-[5-(hydrazinecarbonyl)-2-(trifluoromethyl)phenyl]ethenyl}-1,3-dioxan-5-yl]carbamate

The title compound was obtained as described in Example 1, Step 1, using methyl 3-[(E)-2-{5-[(tert-butoxycarbonyl)amino]-1,3-dioxan-2-yl}ethenyl]-4-(trifluoromethyl)benzoate (71 mg) obtained in Reference Example 84 instead of ethyl 3-({4-[(tertbutoxycarbonyl)amino]butyl}amino)-4-(trifluoromethyl)benzoate.

### [Step 2] Preparation of tert-butyl [2-{(E)-2-[5-(5-oxo-4,5-dihydro-1,3,4-oxadiazol-2-yl)-2-(trifluoromethyl)phenyl]ethenyl} -1 ,3-dioxan-5-yl]carbamate

The title compound (25 mg) was obtained as described in Example 1, Step 2, using tert-butyl [2-{(E)-2-[5-(hydrazinecarbonyl)-2-(trifluoromethyl)phenyl]ethenyl}-1,3-dioxan-5-yl]carbamate obtained in Step 1 instead of tert-butyl {4-[5-(hydrazinecarbonyl)-2-(trifluoromethyl)anilino]butyl} carbamate.

### [Step 3] Preparation of tert-butyl [2-{2-[5-(5-oxo-4,5-dihydro-1,3,4-oxadiazol-2-yl)-2-(trifluoromethyl)phenyl] ethyl } -1,3 -dioxan-5-yl]carbamate

The title compound (10 mg) was obtained as described in Reference Example 2, Step 5, using tert-butyl [2-{(E)-2-[5-(5-oxo-4,5-dihydro-1,3,4-oxadiazol-2-yl)-2-(trifluoromethyl)phenyl]ethenyl}-1,3-dioxan-5-yl]carbamate (15 mg) obtained in Step 2 instead of benzyl 4-(2,2,3,3,10,1 0-hexamethyl-8-oxo-4,9-dioxa-7-aza-3-silaundecan-5-yl)piperidine-1-carboxylate.

### [Step 4] Preparation of 5-[3-{2-[5-amino-1,3-dioxan-2-yl]ethyl}-4-(trifluoromethyl)phenyl]-1,3,4-oxadiazol-2(3H)-one hydrochloride

The title compound (5 mg) was obtained as described in Example 1, Step 3, using tert-butyl [2-{2-[5-(5-oxo-4,5-dihydro-1,3,4-oxadiazol-2-yl)-2-(trifluoromethyl)phenyl]ethyl}-1,3-dioxan-5-yl]carbamate (10 mg) obtained in Step 3 instead of tert-butyl {4-[5-(5-oxo-4,5-dihydro-1,3,4-oxadiazol-2-yl)-2-(trifluoromethyl)anilino]butyl} carbamate.

### Example 35: 5-(3-{[(1r,4r)-4-aminocyclohexyl]amino}-4-bromophenyl)-1,3,4-oxadiazol-2(3H)-one hydrochloride

### [Step 1] Preparation of tert-butyl{(1r,4r)-4-[2-bromo-5-(hydrazinecarbonyl)anilino]cyclohexyl} carbamate

HBTU (108 mg) and DIPEA (0.054 mL) were added to a mixture of 4-bromo-3-({(1r,4r)-4-[(tert-butoxycarbonyl)amino]cyclohexyl}amino)benzoic acid (107 mg) obtained in Reference Example 90, Step 2 and THF (5.2 mL), and the mixture was stirred at room temperature for 1 hour. Then, hydrazine monohydrate (0.024 mL) was added, and the mixture was stirred at room temperature for 1 hour. The reaction solution was diluted with ethyl acetate, and then the organic layer was washed with water and saturated saline and dried over anhydrous magnesium sulfate. Then, the solvent was removed under reduced pressure. The residue was purified by silica gel column chromatography to afford the title compound (68 mg). MS (m/z): 427.2 [M+H]⁺

### [Step 2] Preparation of tert-butyl {(1r,4r)-4-[2-bromo-5-(5-oxo-4,5-dihydro-1,3,4-oxadiazol-2-yl)anilino] cyclohexyl} carbamate

The title compound (38 mg) was obtained as described in Example 1, Step 2, using tert-butyl {(1r,4r)-4-[2-bromo-5-(hydrazinecarbonyl)anilino]cyclohexyl}carbamate (68 mg) obtained in Step 1 instead of tert-butyl {4-[5-(hydrazinecarbonyl)-2-(trifluoromethyl)anilino]butyl}carbamate. MS (m/z): 453.2 [M+H]⁺

### [Step 3] Preparation of 5-(3-{[(1r,4r)-4-aminocyclohexyl]amino}-4-bromophenyl)-1,3,4-oxadiazol-2(3H)-one hydrochloride

The title compound (32 mg) was obtained as described in Example 1, Step 3, using tert-butyl{(1r,4r)-4-[2-bromo-5-(5-oxo-4,5-dihydro-1,3,4-oxadiazol-2-yl)anilino]cyclohexyl}carbamate (38 mg) obtained in Step 2 instead of tert-butyl {4-[5-(5-oxo-4,5-dihydro-1,3,4-oxadiazol-2-yl)-2-(trifluoromethyl)anilino]butyl}carbamate.

### Example 37: 5-[3-{[(1r,4r)-4-(aminomethyl)cyclohexyl]amino}-4-(trifluoromethyl)phenyl]-1,3,4-oxadiazol-2(3H)-one hydrochloride

### [Step 1] Preparation of benzyl ({(lr,4r)-4-[5-(hydrazinecarbonyl)-2-(trifluoromethyl)anilino]cyclohexyl}methyl)carbamate

The title compound was obtained as described in Example 1, Step 1, using ethyl 3-{[(1r,4r)-4-({[(benzyloxy)carbonyl]amino}methyl)cyclohexyl]amino}-4-(trifluoromethyl)benzoate obtained in Reference Example 92 instead of ethyl 3-({4-[(tertbutoxycarbonyl)amino]butyl} amino)-4-(trifluoromethyl)benzoate.

### [Step 2] Preparation of benzyl({(1r,4r)-4-[5-(5-oxo-4,5-dihydro-1,3,4-oxadiazol-2-yl)-2-(trifluoromethyl)anilino]cyclohexyl}methyl)carbamate

The title compound was obtained as described in Example 1, Step 2, using benzyl ({(1r,4r)-4-[5-(hydrazinecarbonyl)-2-(trifluoromethyl)anilino]cyclohexyl}methyl)carbamate obtained in Step 1 instead of tert-butyl {4-[5-(hydrazinecarbonyl)-2-(trifluoromethyl)anilino]butyl} carbamate.

### [Step 3] Preparation of 5-[3-{[(1r,4r)-4-(aminomethyl)cyclohexyl]amino}-4-(trifluoromethyl)phenyl]-1,3,4-oxadiazol-2(3H)-one hydrochloride

To a solution of benzyl ({(1r,4r)-4-[5-(5-oxo-4,5-dihydro-1,3,4-oxadiazol-2-yl)-2-(trifluoromethyl)anilino]cyclohexyl}methyl)carbamate (75 mg) obtained in Step 2 in methanol (5 mL), after degassing, 10% Pd-C (50 mg) was added with stirring at room temperature under argon atmosphere, and the mixture was stirred at room temperature under medium-pressure hydrogen atmosphere (0.3 MPa) for 2 hours. The insolubles were filtered off, then the solvent was removed under reduced pressure, the obtained residue was dissolved in methanol (2 mL), hydrogen chloride (2 M in ethanol, 0.5 mL) was added to the solution, and the mixture was stirred at room temperature. The reaction solution was concentrated under reduced pressure, the residue was suspended in diethyl ether, and the precipitate was collected by filtration, washed with diethyl ether, and then dried to afford the title compound (45 mg).

### Example 44: 5-[3-{[(1r,4r)-4-(2-aminoethyl)cyclohexyl]amino}-4-(trifluoromethyl)phenyl] - 1,3,4-oxadiazol-2(3H)-one hydrochloride

### [Step 1] Preparation of benzyl(2-{(1r,4r)-4-[5-(hydrazinecarbonyl)-2-(trifluoromethyl)anilino]cyclohexyl} ethyl)carbamate

The title compound was obtained as described in Example 1, Step 1, using methyl 3-{[(1r,4r)-4-(2-{[(benzyloxy)carbonyl]amino}ethyl)cyclohexyl]amino}-4-(trifluoromethyl)benzoate obtained in Reference Example 99 instead of ethyl 3-({4-[(tertbutoxycarbonyl)amino]butyl}amino)-4-(trifluoromethyl)benzoate.

### [Step 2] Preparation of benzyl(2-{(1r,4r)-4-[5-(5-oxo-4,5-dihydro-1,3,4-oxadiazol-2-yl)-2-(trifluoromethyl)anilino]cyclohexyl}ethyl)carbamate

The title compound was obtained as described in Example 1, Step 2, using benzyl (2-{(1r,4r)-4-[5-(hydrazinecarbonyl)-2-(trifluoromethyl)anilino]cyclohexyl}ethyl)carbamate obtained in Step 1 instead of tert-butyl {4-[5-(hydrazinecarbonyl)-2-(trifluoromethyl)anilino]butyl}carbamate. MS (m/z): 505.7 [M+H]⁺

### [Step 3] Preparation of 5-[3-{[(1r,4r)-4-(2-aminoethyl)cyclohexyl]amino}-4-(trifluoromethyl)phenyl]-1,3,4-oxadiazol-2(3H)-one hydrochloride

Hydrogen chloride (2 M in ethanol, 12 mL) was added to benzyl (2-{(1r,4r)-4-[5-(5-oxo-4,5-dihydro-1,3,4-oxadiazol-2-yl)-2-(trifluoromethyl)anilino]cyclohexyl} ethyl)carbamate (32 mg) obtained in Step 2, and the mixture was stirred at 50°C for 4 days. The reaction solution was concentrated under reduced pressure, the residue was suspended in diethyl ether, and the precipitate was collected by filtration, washed with diethyl ether, and then dried to afford the title compound (12 mg).

### Example 119: (1r,4r)-4-{[5-(5-oxo-4,5-dihydro-1,3,4-oxadiazol-2-yl)-2-(trifluoromethyl)anilino]methyl}cyclohexane-1 -carboxylic acid hydrochloride

TFA (1 mL) was added to a solution of tert-butyl (1r,4r)-4-{[5-(5-oxo-4,5-dihydro-1,3,4-oxadiazol-2-yl)-2-(trifluoromethyl)anilino]methyl}cyclohexane-1-carboxylate (280 mg) obtained in Example 118 in dichloromethane (2 mL), and the mixture was stirred at room temperature for 2 hours. The reaction solution was concentrated under reduced pressure, water was added to the obtained residue, and the mixture was extracted with ethyl acetate. The organic layer was washed with saturated saline and dried over anhydrous magnesium sulfate. The solvent was removed under reduced pressure to afford the title compound (240 mg). Example 120: 5-[3-({[(1r,4r)-4-(hydroxymethyl)cyclohexyl]methyl}amino)-4-(trifluoromethyl)phenyl]-1,3,4-oxadiazol-2(3H)-one

BH₃-THF (0.9 M in THF, 0.29 mL) was added to a solution of (1r,4r)-4-{[5-(5-oxo-4,5-dihydro-1,3,4-oxadiazol-2-yl)-2-(trifluoromethyl)anilino]methyl}cyclohexane-1-carboxylic acid (50 mg) obtained in Example 119 in THF (1 mL) under ice-cooling, and the mixture was stirred at room temperature for 1 hour. Water was added to the reaction solution, and the residue was purified by silica gel column chromatography to afford the title compound (30 mg).

### Example 121: (1r,4r)-4-{[5-(5-oxo-4,5-dihydro-1,3,4-oxadiazol-2-yl)-2-(trifluoromethyl)anilino]methyl}cyclohexane-1-carboxamide

HATU (59 mg), ammonium chloride (56 mg), and DIPEA (0.18 mL) were added to a mixture of (1r,4r)-4-{[5-(5-oxo-4,5-dihydro-1,3,4-oxadiazol-2-yl)-2-(trifluoromethyl)anilino]methyl}cyclohexane-1-carboxylic acid (40 mg) obtained in Example 119 in DMF (2 mL), and the mixture was stirred at room temperature for 6 hours. The reaction solution was diluted with ethyl acetate, then the organic layer was washed with saturated aq. sodium bicarbonate and saturated saline, and the solvent was removed under reduced pressure. The residue was purified by silica gel column chromatography to afford the title compound (5 mg).

### Example 149: 5-[3-{[(1S)-1-(2-azaspiro[3.3]heptan-6-yl)ethyl]amino}-4-(trifluoromethyl)phenyl]-1,3,4-oxadiazol-2(3H)-one trifluoroacetate

### [Step 1] Preparation of tert-butyl 6-{(1S)-1-[5-(hydrazinecarbonyl)-2-(trifluoromethyl)anilino]ethyl}-2-azaspiro[3.3]heptane-2-carboxylate

The title compound (118 mg) was obtained as described in Example 1, Step 1, using tert-butyl 6-{(1S)-1-[5-(methoxycarbonyl)-2-(trifluoromethyl)anilino]ethyl}-2-azaspiro[3.3]heptane-2-carboxylate (119 mg) obtained in Reference Example 214 instead of ethyl 3-({4-[(tert-butoxycarbonyl)amino]butyl}amino)-4-(trifluoromethyl)benzoate.

### [Step 2] Preparation of tert-butyl 6-{(1S)-1-[5-(5-oxo-4,5-dihydro-1,3,4-oxadiazol-2-yl)-2-(trifluoromethyl)anilino]ethyl}-2-azaspiro[3.3]heptane-2-carboxylate

The title compound (113 mg) was obtained as described in Example 1, Step 2, using tert-butyl 6-{(1S)-1-[5-(hydrazinecarbonyl)-2-(trifluoromethyl)anilino]ethyl}-2-azaspiro[3.3]heptane-2-carboxylate (118 mg) obtained in Step 1 instead of tert-butyl {4-[5-(hydrazinecarbonyl)-2-(trifluoromethyl)anilino]butyl} carbamate.

### [Step 3] Preparation of 5-[3-{[(1S)-1-(2-azaspiro[3.3]heptan-6-yl)ethyl]amino}-4-(trifluoromethyl)phenyl]-1,3,4-oxadiazol-2(3H)-one trifluoroacetate

TFA (1 mL) was added to a solution of tert-butyl 6-{(1S)-1-[5-(5-oxo-4,5-dihydro-1,3,4-oxadiazol-2-yl)-2-(trifluoromethyl)anilino]ethyl}-2-azaspiro[3.3]heptane-2-carboxylate (90 mg) obtained in Step 2 in dichloromethane (2 mL), and the mixture was stirred at room temperature for 2 hours. The reaction solution was concentrated under reduced pressure, water was added to the obtained residue, and the mixture was extracted with ethyl acetate. The organic layer was washed with saturated saline and dried over anhydrous magnesium sulfate. The solvent was removed under reduced pressure to afford the title compound (240 mg).

### Example 215: 5-(3-{ [(1 -acetylpiperidin-4-yl)(methyl)amino]methyl} -4-chlorophenyl)-1,3,4-oxadiazol-2(3H)-one hydrochloride

### [Step 1] Preparation of 5-(3-{[(1-acetylpiperidin-4-yl)(methyl)amino]methyl}-4-chlorophenyl)-1,3,4-oxadiazol-2(3H)-one

TEA (0.088 mL) and acetyl chloride (11 mg) were added to a mixture of 5-(4-chloro-3-{[methyl(piperidin-4-yl)amino]methyl}phenyl)-1,3,4-oxadiazol-2(3H)-one dihydrochloride (50 mg) obtained in Example 205 in THF (1.5 mL) under ice-cooling, and the mixture was stirred at room temperature for 4 hours. The reaction solution was concentrated under reduced pressure, and the obtained residue was purified by silica gel column chromatography to afford the title compound (26 mg).

### [Step 2] Preparation of 5-(3-{[(1-acetylpiperidin-4-yl)(methyl)amino]methyl}-4-chlorophenyl)-1,3,4-oxadiazol-2(3H)-one hydrochloride

Hydrogen chloride (2 M in ethanol, 0.071 mL) was added to a solution of 5-(3-{[(1-acetylpiperidin-4-yl)(methyl)amino]methyl}-4-chlorophenyl)-1,3,4-oxadiazol-2(3H)-one (26 mg) obtained in Step 1 in ethanol, and the mixture was stirred at room temperature for 1 hour. The reaction mixture was concentrated under reduced pressure to afford the title compound (17 mg).

### Example 223: 5-[3-fluoro-5-({(1S)-1-[(3S,4R)-3-fluoropiperidin-4-yl]ethyl}amino)-4-(trifluoromethyl)phenyl]-1,3,4-oxadiazol-2(3H)-one hydrochloride

### [Step 1]Preparation of tert-butyl(3S,4R)-4-{(1S)-1-[5-bromo-3-fluoro-2-(trifluoromethyl)anilino]ethyl}-3-fluoropiperidine-1-carboxylate

The title compound (173 mg) was obtained as described in Reference Example 130, Step 1, using tert-butyl (3S,4R)-4-[(1S)-1-aminoethyl]-3-fluoropiperidine-1-carboxylate (110 mg) obtained in Reference Example 51 instead of tert-butyl 4-[(1S)-1-aminoethyl]piperidine-1-carboxylate.

### [Step 2] Preparation of tert-butyl (3S,4R)-3-fluoro-4-{(1S)-1-[3-fluoro-5-(hydrazinecarbonyl)-2-(trifluoromethyl)anilino]ethyl}piperidine-1-carboxylate

A solution of 2,4,6-trichlorophenyl formate (240 mg) in toluene (3.6 mL) was added dropwise to a mixture of tert-butyl(3S,4R)-4-{(1S)-1-[5-bromo-3-fluoro-2-(trifluoromethyl)anilino]ethyl}-3-fluoropiperidine-1-carboxylate (173 mg) obtained in Step 1, Xantphos (21 mg), tripropylamine (153 mg), Pd(OAc)2 (4 mg), and toluene (3.6 mL) at 100°C under argon atmosphere over 3 hours, and the mixture was stirred at the same temperature for 1 hour. The reaction solution was concentrated under reduced pressure, and a solution of hydrazine monohydrate (130 mg) in THF (1.8 mL) was added dropwise to a solution of the obtained residue in THF (1.8 mL) under ice-cooling, and the mixture was stirred at the same temperature. After monitoring the consumption of the starting material on TLC, the reaction solution was diluted with ethyl acetate, and the organic layer was washed with saturated saline. The solvent was removed under reduced pressure to afford the title compound.

### [Step 3] Preparation of tert-butyl (3S,4R)-3-fluoro-4-{(1S)-1-[3-fluoro-5-(5-oxo-4,5-dihydro-1,3,4-oxadiazol-2-yl)-2-(trifluoromethyl)anilino]ethyl}piperidine-1-carboxylate

The title compound (151 mg) was obtained as described in Example 1, Step 2, using tert-butyl (3S,4R)-3-fluoro-4-{(1S)-1-[3-fluoro-5-(hydrazinecarbonyl)-2-(trifluoromethyl)anilino]ethyl}piperidine-1-carboxylate obtained in Step 2 instead of tert-butyl {4-[5-(hydrazinecarbonyl)-2-(trifluoromethyl)anilino]butyl}carbamate.

### [Step 4] Preparation of 5-[3-fluoro-5-({(1S)-1-[(3S,4R)-3-fluoropiperidin-4-yl]ethyl}amino)-4-(trifluoromethyl)phenyl]-1,3,4-oxadiazol-2(3H)-one hydrochloride

The title compound (110 mg) was obtained as described in Example 1, Step 3, using tert-butyl (3S,4R)-3-fluoro-4-{(1S)-1-[3-fluoro-5-(5-oxo-4,5-dihydro-1,3,4-oxadiazol-2-yl)-2-(trifluoromethyl)anilino]ethyl}piperidine-1-carboxylate (151 mg) obtained in Step 3 instead of tert-butyl {4-[5-(5-oxo-4,5-dihydro-1,3,4-oxadiazol-2-yl)-2-(trifluoromethyl)anilino]butyl} carbamate.

### Example 231: 5-[3-({(1S)-1-[(1r,4S)-4-aminocyclohexyl]ethyl}amino)-4-ethyl-5-fluorophenyl] - 1,3,4-oxadiazol-2(3H)-one hydrochloride

### [Step 1]Preparation of tert-butyl[(1S,4r)-4-{(1S)-1-[2-ethyl-3-fluoro-5-(hydrazinecarbonyl)anilino]ethyl} cyclohexyl]carbamate

The title compound was obtained as described in Example 1, Step 1, using methyl 3-{[(1S)-1-{(1r,4S)-4-[(tert-butoxycarbonyl)amino] cyclohexyl} ethyl] amino } -4-ethenyl-5 - fluorobenzoate obtained in Reference Example 278 instead of ethyl 3-({4-[(tertbutoxycarbonyl)amino]butyl}amino)-4-(trifluoromethyl)benzoate.

### [Step 2]Preparation of tert-butyl[(1S,4r)-4-{(1S)-1-[2-ethyl-3-fluoro-5-(5-oxo-4,5-dihydro-1,3,4-oxadiazol-2-yl)anilino]ethyl}cyclohexyl]carbamate

The title compound was obtained as described in Example 1, Step 2, using tert-butyl[(1S,4r)-4-{(1S)-1-[2-ethyl-3-fluoro-5-(hydrazinecarbonyl)anilino]ethyl}cyclohexyl]carbamate obtained in Step 1 instead of tert-butyl {4-[5-(hydrazinecarbonyl)-2-(trifluoromethyl)anilino]butyl} carbamate.

### [Step 3] Preparation of 5-[3-({(1S)-1-[(1r,4S)-4-aminocyclohexyl]ethyl}amino)-4-ethyl-5-fluorophenyl]-1,3,4-oxadiazol-2(3H)-one hydrochloride

The title compound (27 mg) was obtained as described in Example 1, Step 3, using tert-butyl [(1S,4r)-4-{(1S)-1-[2-ethyl-3-fluoro-5-(5-oxo-4,5-dihydro-1,3,4-oxadiazol-2-yl)anilino]ethyl}cyclohexyl]carbamate obtained in Step 2 instead of tert-butyl {4-[5-(5-oxo-4,5-dihydro-1,3,4-oxadiazol-2-yl)-2-(trifluoromethyl)anilino]butyl}carbamate. Example 233: 5-[3-({(1S)-1-[(1r,4S)-4-aminocyclohexyl]ethyl}amino)-5-fluoro-4-(propan-2-yl)phenyl]-1,3,4-oxadiazol-2(3H)-one hydrochloride

The title compound (15 mg) was obtained as described in Reference Example 2, Step 5, using 5-[3-({(1S)-1-[(1r,4S)-4-aminocyclohexyl]ethyl}amino)-5-fluoro-4-(prop-1-en-2-yl)phenyl]-1,3,4-oxadiazol-2(3H)-one hydrochloride (15 mg) obtained in Example 232 instead of benzyl 4-(2,2,3,3,10,1 0-hexamethyl-8-oxo-4,9-dioxa-7-aza-3-silaundecan-5-yl)piperidine-1-carboxylate.

### Example 245: 5-fluoro-1-{(1S)-1-[(3S,4S)-3-methylpiperidin-4-yl]ethyl}-7-(5-oxo-4,5-dihydro-1,3,4-oxadiazol-2-yl)-2,3-dihydroquinolin-4(1H)-one hydrochloride

### [Step 1] Preparation of tert-butyl (3S,4S)-4-[(1S)-1-(7-bromo-5-fluoro-4-oxo-3,4-dihydroquinolin-1(2H)-yl)ethyl]-3-methylpiperidine-1-carboxylate

A solution of tert-butyl (3S,4S)-4-[(1S)-1-aminoethyl]-3-methylpiperidine-1-carboxylate (118 mg) obtained in Reference Example 47, 1-(4-bromo-2,6-difluorophenyl)prop-2-en-1-one (100 mg), and sodium bicarbonate (68 mg) in NMP (2 mL) was stirred at 70°C for 3 hours. The reaction solution was diluted with ethyl acetate, then the organic layer was washed with water and saturated saline, and the solvent was removed under reduced pressure. The residue was purified by silica gel column chromatography to afford the title compound (104 mg).

### [Step 2] Preparation of tert-butyl (3S,4S)-4-{(1S)-1-[5-fluoro-7-(hydrazinecarbonyl)-4-oxo-3,4-dihydroquinolin-1(2H)-yl]ethyl}-3-methylpiperidine-1-carboxylate

The title compound was obtained as described in Reference Example 224, Step 2, using tert-butyl (3S,4S)-4-[(1S)-1-(7-bromo-5-fluoro-4-oxo-3,4-dihydroquinolin-1(2H)-yl)ethyl]-3-methylpiperidine-1-carboxylate obtained in Step 1 instead of tert-butyl (3S,4R)-4-{(1S)-1-[5-bromo-3-fluoro-2-(trifluoromethyl)anilino]ethyl}-3-fluoropiperidine-1-carboxylate.

### [Step 3] Preparation of tert-butyl (3S,4S)-4-{(1S)-1-[5-fluoro-4-oxo-7-(5-oxo-4,5-dihydro-1,3,4-oxadiazol-2-yl)-3,4-dihydroquinolin-1(2H)-yl]ethyl}-3-methylpiperidine-1-carboxylate

The title compound (17 mg) was obtained as described in Example 1, Step 2, using tert-butyl (3S,4S)-4-{(1S)-1-[5-fluoro-7-(hydrazinecarbonyl)-4-oxo-3,4-dihydroquinolin-1(2H)-yl]ethyl}-3-methylpiperidine-1-carboxylate obtained in Step 2 instead of tert-butyl {4-[5-(hydrazinecarbonyl)-2-(trifluoromethyl)anilino]butyl} carbamate.

### [Step 4] Preparation of 5-fluoro-1-{(1S)-1-[(3S,4S)-3-methylpiperidin-4-yl]ethyl}-7-(5-oxo-4,5-dihydro-1,3,4-oxadiazol-2-yl)-2,3-dihydroquinoline-4(1H)-one hydrochloride

The title compound (9 mg) was obtained as described in Example 1, Step 3, using tert-butyl (3S,4S)-4-{(1S)-1-[5-fluoro-4-oxo-7-(5-oxo-4,5-dihydro-1,3,4-oxadiazol-2-yl)-3,4-dihydroquinolin-1(2H)-yl]ethyl}-3-methylpiperidine-1-carboxylate (17 mg) obtained in Step 3 instead of tert-butyl {4-[5-(5-oxo-4,5-dihydro-1,3,4-oxadiazol-2-yl)-2-(trifluoromethyl)anilino]butyl}carbamate.

### Example 246: 5-[(4E)-5-fluoro-4-(methoxyimino)-1-{(1S)-1-[(3S,4S)-3-methylpiperidin-4-yl]ethyl}-1,2,3,4-tetrahydroquinolin-7-yl]-1,3,4-oxadiazol-2(3H)-one hydrochloride

### [Step 1] Preparation of tert-butyl (3S,4S)-4-{(1S)-1-[(4E)-5-fluoro-7-(hydrazinecarbonyl)-4-(methoxyimino)-3,4-dihydroquinolin-1(2H)-yl] ethyl}-3-methylpiperidine-1-carboxylate

A mixture of tert-butyl (3S,4S)-4-{(1S)-1-[5-fluoro-7-(hydrazinecarbonyl)-4-oxo-3,4-dihydroquinolin-1(2H)-yl]ethyl}-3-methylpiperidine-1-carboxylate (20 mg) obtained in Example 245, Step 2, O-methylhydroxylamine hydrochloride (7 mg), pyridine (11 mg), and ethanol (1 mL) was stirred at 70°C for 2 hours. The reaction solution was diluted with ethyl acetate, and the organic layer was washed with saturated saline. The solvent was removed under reduced pressure to afford the title compound.

### [Step 2] Preparation of tert-butyl (3S,4S)-4-{(1S)-1-[(4E)-5-fluoro-4-(methoxyimino)-7-(5-oxo-4,5-dihydro-1,3,4-oxadiazol-2-yl)-3,4-dihydroquinolin-1(2H)-yl]ethyl}-3-methylpiperidine-1-carboxylate

The title compound (7 mg) was obtained as described in Example 1, Step 2, using tert-butyl (3S,4S)-4-{(1S)-1-[(4E)-5-fluoro-7-(hydrazinecarbonyl)-4-(methoxyimino)-3,4-dihydroquinolin-1(2H)-yl]ethyl}-3-methylpiperidine-1-carboxylate obtained in Step 1 instead of tert-butyl {4-[5-(hydrazinecarbonyl)-2-(trifluoromethyl)anilino]butyl}carbamate.

### [Step 3] Preparation of 5-[(4E)-5-fluoro-4-(methoxyimino)-1-{(1S)-1-[(3S,4S)-3-methylpiperidin-4-yl]ethyl}-1,2,3,4-tetrahydroquinolin-7-yl]-1,3,4-oxadiazol-2(3H)-one hydrochloride

The title compound (4 mg) was obtained as described in Example 1, Step 3, using tert-butyl (3S,4S)-4-{(1S)-1-[(4E)-5-fluoro-4-(methoxyimino)-7-(5-oxo-4,5-dihydro-1,3,4-oxadiazol-2-yl)-3,4-dihydroquinolin-1 (2H)-yl]ethyl}-3 -methylpiperidine-1 -carboxylate (7 mg) obtained in Step 2 instead of tert-butyl{4-[5-(5-oxo-4,5-dihydro-1,3,4-oxadiazol-2-yl)-2-(trifluoromethyl)anilino]butyl} carbamate.

### Example 252: 5-[(2S)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one hydrochloride [Step 1] Preparation of tert-butyl[(1S,4r)-4-{(1S)-1-[8-fluoro-6-(hydrazinecarbonyl)-2-methyl-2,3-dihydro-4H-1,4-benzoxazin-4-yl]ethyl}cyclohexyl]carbamate

The title compound was obtained as described in Example 1, Step 1, using methyl 4-[(1S)-1-{(1r,4S)-4-[(tert-butoxycarbonyl)amino]cyclohexyl}ethyl]-8-fluoro-2-methyl-3,4-dihydro-2H-1,4-benzoxazine-6-carboxylate (30 mg) obtained in Reference Example 296 instead of ethyl 3-({4-[(tert-butoxycarbonyl)amino]butyl}amino)-4-(trifluoromethyl)benzoate.

### [Step 2] Preparation of tert-butyl [(1S,4r)-4-{(1S)-1-[(2S)-8-fluoro-2-methyl-6-(5-oxo-4,5-dihydro-1,3,4-oxadiazol-2-yl)-2,3-dihydro-4H-1,4-benzoxazin-4-yl]ethyl}cyclohexyl]carbamate

Acrude product containing tert-butyl [(1S,4r)-4-{(1S)-1-[(2S)-8-fluoro-2-methyl-6-(5-oxo-4,5-dihydro-1,3,4-oxadiazol-2-yl)-2,3-dihydro-4H-1,4-benzoxazin-4-yl]ethyl}cyclohexyl]carbamate was obtained as described in Example 1, Step 2, using tert-butyl [(1S,4r)-4-{(1S)-1-[8-fluoro-6-(hydrazinecarbonyl)-2-methyl-2,3-dihydro-4H-1,4-benzoxazin-4-yl]ethyl}cyclohexyl]carbamate obtained in Step 1 instead of tert-butyl {4-[5-(hydrazinecarbonyl)-2-(trifluoromethyl)anilino]butyl}carbamate. The obtained crude product was purified by silica gel column chromatography to afford the title compound (12 mg) and tert-butyl [(1S,4r)-4-{(1S)-1-[(2R)-8-fluoro-2-methyl-6-(5-oxo-4,5-dihydro-1,3,4-oxadiazol-2-yl)-2,3-dihydro-4H-1,4-benzoxazin-4-yl]ethyl}cyclohexyl]carbamate (8 mg). Stereochemistry was assigned (optionally) by bioactivity and established structural similarity.

### [Step 3] Preparation of 5-[(2S)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one hydrochloride

Hydrogen chloride (2 M in ethanol, 5 mL) was added to tert-butyl [(1S,4r)-4-{(1S)-1-[(2S)-8-fluoro-2-methyl-6-(5-oxo-4,5-dihydro-1,3,4-oxadiazol-2-yl)-2,3-dihydro-4H-1,4-benzoxazin-4-yl]ethyl}cyclohexyl]carbamate (12 mg) obtained in Step 2, and the mixture was stirred at room temperature for 3 hours. The reaction mixture was concentrated under reduced pressure to afford the title compound (16 mg).

### Example 253: 5-[(2R)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one hydrochloride

Hydrogen chloride (2 M in ethanol, 2 mL) was added to tert-butyl [(1S,4r)-4-{(1S)-1-[(2R)-8-fluoro-2-methyl-6-(5-oxo-4,5-dihydro-1,3,4-oxadiazol-2-yl)-2,3-dihydro-4H-1,4-benzoxazin-4-yl]ethyl}cyclohexyl]carbamate (8 mg) obtained in Example 252, Step 2, and the mixture was stirred at room temperature for 3 hours. The reaction mixture was concentrated under reduced pressure to afford the title compound (16 mg).

Compounds of Reference Examples and Examples are further provided below in Tables 2 to 55. In the tables, PREx means the Reference Example No. where the compound was prepared according to the method as described in said Reference Example using a corresponding starting material. For example, the compound of the following Reference Example with the indication of PREx No. as 1 was prepared using the method as described in Reference Example 1. Also, in the tables, PEx means the Example No. where the compound was prepared according to the method as described in said Example using a corresponding starting material. For example, the compound of the following Example with the indication of PEx No. as 1 was prepared using the method as described in Example 1. Further, in the tables, Chemical Name refers to the name of the Reference Example (REx) or the Example (Ex). In addition, Data means the instrumental analytical data, such as mass spectrometric data (m/z values), 1H NMR data (δ (ppm) of peaks), and elemental analytical data (composition (%) of C, H, and N).

**[Table 2]**

| REx | PREx | Chemical Name |
|---|---|---|
| 59 | 59 | Ethyl 3-({4-[(tert-butoxycarbonyl)amino]butyl}amino)-4-(trifluoromethyl)benzoate |
| 60 | 59 | Ethyl 3-({3-[(tert-butoxycarbonyl)amino]propyl}amino)-4-(trifluoromethyl)benzoate |
| 61 | 59 | Methyl 3-({5-[(tert-butoxycarbonyl)amino]pentyl}amino)-4-(trifluoromethyl)benzoate |
| 62 | 59 | Methyl 3-({6-[(tert-butoxycarbonyl)amino]hexyl}amino)-4-(trifluoromethyl)benzoate |
| 63 | 59 | Methyl 3-({6-[(tert-butoxycarbonyl)amino]hexan-2-yl}amino)-4-(trifluoromethyl)benzoate |
| 64 | 59 | Ethyl 3-(4-{[(tert-butoxycarbonyl)amino]methyl}piperidin-1-yl)-4-chlorobenzoate |
| 65 | 59 | tert-Butyl 4-[2-chloro-5-(ethoxycarbonyl)phenyl]piperazine-1-carboxylate |
| 66 | 59 | Ethyl 3-{4-[(tert-butoxycarbonyl)amino]piperidin-1-yl}-4-(trifluoromethyl)benzoate |
| 67 | 59 | Ethyl 3-(4-{2-[(tert-butoxycarbonyl)amino]ethyl}piperidin-1-yl)-4-(trifluoromethyl)benzoate |
| 68 | 59 | Ethyl 3-(3-{2-[(tert-butoxycarbonyl)amino]ethyl }piperidin-1-yl)-4-(trifluoromethyl)benzoate |
| 69 | 69 | Methyl 4-(4-{2-[(tert-butoxycarbonyl)amino]ethyl}piperidin-1-yl)-1- {[2-(trimethylsilyl)ethoxy]methyl}-1H-indazole-6-carboxlate |
| 70 | 59 | Methyl 3-(4-{1-[(tert-butoxycarbonyl)amino]-2-methylpropan-2-yl}piperidin-1-yl)-4-(trifluoromethyl)benzoate |
| 71 | 59 | Methyl 3-[4-(2,2,3,3,10,10-hexamethyl-8-oxo-4,9-dioxa-7-aza-3-silaundecan-5-yl)piperidin-1-yl]-4-(trifluoromethyl)benzoate |
| 72 | 59 | tert-Butyl 9-[5-(methoxycarbonyl)-2-(trifluoromethyl)phenyl]-3,9-diazaspiro[5.5]undecane-3-carboxylate |
| 73 | 59 | tert-Butyl 7-[5-(methoxycarbonyl)-2-(trifluoromethyl)phenyl]-2, 7-diazaspiro[3.5]nonane-2-carboxylate |
| 74 | 74 | Ethyl 3'-{[(tert-butoxycarbonyl)amino]methyl}-6-chloro[1,1'-biphenyl]-3-carboxylate |

**[Table 3]**

| REx | PREx | Chemical Name |
|---|---|---|
| 75 | 74 | Ethyl 4'-{[(tert-butoxycarbonyl)amino]methyl}-6-(trifluoromethyl)[1,1'-biphenyl]-3-carboxylate |
| 76 | 74 | Ethyl 3'-{[(tert-butoxycarbonyl)amino]methyl}-6-(trifluoromethyl)[1,1'-biphenyl]-3-carboxylate |
| 77 | 74 | Methyl 4'-{2-[(tert-butoxycarbonyl)amino]ethyl}-6-(trifluoromethyl)(1,1'-biphenyl]-3-carboxylate |
| 78 | 78 | tert-Butyl 4-({[5'-(methoxycarbonyl)-2'-(trifluoromethyl)[1,1'-biphenyl]-4-yl]oxy}methyl)piperidine-1-carboxylate |
| 79 | 79 | Methyl 4'-({(2S)-1-[(tert-butoxycarbonyl)amino]propan-2-yl }oxy)-6-(trifluoromethyl)[1,1'-biphenyl]-3-carboxylate |
| 80 | 74 | tert-Butyl 4-{5-[5-(ethoxycarbonyl)-2-(trifluoromethyl)phenyl]pyridin-3-yl}piperazine-1-carboxylate |
| 81 | 81 | tert-Butyl 4-{2-[5-(methoxycarbonyl-2-(trifluoromethyl)phenyl]ethyl}piperidine-1-carboxylate |
| 83 | 83 | Methyl 3-(2- {(1r,4s)-4-[(tert-butoxycarbonyl)amino]cyclohexyl} ethyl)-4-(trifluoromethyl)benzoate |
| 84 | 84 | Methyl 3-[(E)-2-{5-[(tert-butoxycarbonyl)amino]-1,3-dioxan-2-yl}ethenyl]-4-(trifluoromethyl)benzoate |
| 82 | 82 | tert-Butyl 4-{[5-(methoxycarbonyl-2-(trifluoromethyl)phenyl]ethynyl}piperidine- 1 -carboxylate |
| 85 | 85 | tert-Butyl 4-{(E)-2-[5-(methoxycarbonyl)-2-(trifluoromethyl)phenyl]ethenyl)piperidine-1-carboxylate |
| 86 | 59 | tert-Butyl 4-[2-chloro-5-(ethoxycarbonyl)anilino]piperidine-1-carboxylate |
| 87 | 59 | Ethyl 3-({(1r,4r)-4-[(tert-butoxycarbonyl)amino]cyclohexyl}amino)-4-chlorobenzoate |
| 88 | 59 | Ethyl 3-({(1s,4s)-4-[(tert-butoxycarbonyl)amino]cyclohexyl}amino)-4-chlorobenzoate |

**[Table 4]**

| REx | PREx | Chemical Name |
|---|---|---|
| 89 | 59 | Ethyl 3-({(1r,4r)-4-[(tert-butoxycarbonyl)amino]cyclohexyl}amino)-4-(trifluoromethyl)benzoate |
| 90 | 90 | 4-Bromo-3-({(1r,4r)-4-[(tert-butoxycarbonyl)amino]cyclohexyl}amino)benzoic acid |
| 91 | 91 | Methyl 3-({(1r,4r)-4-[(tert-butoxycarbonyl)amino]cyclohexyl}amino)-5-fluoro-4-(trifluoromethyl)benzoate |
| 92 | 59 | Ethyl 3-{[(1r,4r)-4-({[(benzyloxy)carbonyl]amino}methyl)cyclohexyl]amino}-4-(trifluoromethyl)benzoate |
| 93 | 59 | Ethyl 3-({(1r,4r)-4-[(tert-butoxycarbonyl)amino]cyclohexyl} amino)-4-(trifluoromethyl)benzoate |
| 94 | 59 | Ethyl 3-{[(1r,4r)-4-(1-{[(benzyloxy)carbonyl]amino}ethyl)cyclohexyl]amino}-4-(trifluoromethyl)benzoate |
| 95 | 59 | Methyl 3-({(1r,4r)-4-[(tert-butoxycarbonyl)amino]cyclohexyl}amino)-4-chloro- 5-fluorobenzoate |
| 96 | 59 | Methyl 3-(4-{[(tert-butoxycarbonyl)amino]methyl}anilino)-4-(trifluoromethyl)benzoate |
| 97 | 59 | tert-Butyl 1-[5-(methoxycarbonyl)-2-(trifluoromethyl)anilino]-6-azaspiro[2.5]octane-6-carboxylate |
| 98 | 59 | Methyl 3-({6-[(tert-butoxycarbonyl)amino]spiro[3.3]heptan-2-yl}amino)-4-(trifluoromethyl)benzoate |
| 99 | 59 | Methyl 3-{[(1r,4r)-4-(2-{[(benzyloxy)carbonyl]amino}ethyl)cyclohexyl]amino}-4-(trifluoromethyl)benzoate |
| 100 | 59 | tert-Butyl (1S)-1-[5-(methoxycarbonyl)-2-(trifluoromethyl)anilino]-7-azaspiro[3.5]nonane-7-carboxylate |
| 101 | 59 | tert-Butyl (1R)-1-[5-(methoxycarbonyl)-2-(trifluoromethyl)anilino]-7-azaspiro[3.5]nonane-7-carboxylate |
| 102 | 59 | tert-Butyl 4-{[2-chloro-5-(ethoxycarbonyl)anilino]methyl}piperidine-1-carboxylate |
| 103 | 59 | tert-Butyl 4-{[5-(ethoxycarbonyl)-2-(trifluoromethyl)anilino]methyl}piperidine-1-carboxylate |

**[Table 5]**

| REx | PREx | Chemical Name |
|---|---|---|
| 104 | 59 | tert-Butyl(3S)-3-{[2-chloro-5-(ethoxycarbonyl)anilino]methyl}pyrrolidine-1-carboxylate |
| 105 | 90 | 4-bromo-3-({[1-(tert-butoxycarbonyl)piperidin-4-yl]methyl}amino)benzoic acid |
| 106 | 59 | tert-Butyl 4-({[5-(ethoxycarbonyl)-2-(trifluoromethyl)phenyl](methyl)amino}methyl)piperidine-1-carboxylate |
| 107 | 59 | tert-Butyl 4-{1-[5-(ethoxycarbonyl)-2-(trifluoromethyl)anilino]ethyl}piperidine-1-carboxylate |
| 108 | 59 | tert-Butyl (3R)-3-{[5-(ethoxycarbonyl)-2-(trifluoromethyl)anilino]methyl}piperidine-1-carboxylate |
| 109 | 59 | tert-Butyl (3S)-3-{[5-(ethoxycarbonyl)-2-(trifluoromethyl)anilimo]methyl}piperidine-1-carboxylate |
| 110 | 59 | Ethyl 3-[({(1r,4r)-4-[(tert-butoxycarbonyl)amino]cyclohexyl}methyl)amino]-4-(trifluoromethyl)benzoate |
| 111 | 59 | tert-Butyl 4-{1-[5-(ethoxycarbonyl)-2-(trifluoromethyl)anilino]propyl}piperidine-1-carboxylate |
| 112 | 59 | tert-Butyl 4-{[5-(ethoxycarbonyl)-2-(trifluoromethyl)anilino]methyl}-4-methylpiperidine-1-carboxylate |
| 113 | 59 | tert-Butyl 4-{(1S)-1-[5-(ethoxycarbonyl)-2-(trifluoromethyl)anilino]ethyl}piperidine-1-carboxylate |
| 114 | 59 | tert-Butyl 4-{(1R)-1-[5-(ethoxycarbonyl)-2-(trifluoromethyl)anilino]ethyl}piperidine-1-carboxylate |
| 115 | 59 | tert-Butyl 3-{2-[5-(ethoxycarbonyl)-2-(trifluoromethyl)anilino]ethyl}piperidine-1-carboxylate |
| 116 | 59 | Ethyl 3-[({(1r,4r)-4-[(tert-butoxycarbonyl)amino]cyclohexyl}methyl)amino]-4-chlorobenzoate |
| 117 | 59 | Ethyl 3-[({(1r,4r)-4-[(tert-butoxycarbonyl)amino]cyclohexyl} methyl)amino]benzoate |
| 118 | 59 | Ethyl 3-[(1-{(1r,4r)-4- [(tert-butoxycarbonyl)amino]cyclohexyl}ethyl)amino]-4-(trifluoromethyl)benzoate |

**[Table 6]**

| REx | PREx | Chemical Name |
|---|---|---|
| 119 | 59 | tert-Butyl 4-{2-[5-(ethoxycarbonyl)-2-(trifluoromethyl)anilino]ethyl}piperidine-1-carboxylate |
| 120 | 59 | tert-Butyl 4-{2-[5-(ethoxycarbonyl)-2-(trifluoromethyl)anilino]ethyl}piperazine-1-carboxylate |
| 121 | 59 | Ethyl 3-[({(1r,4r)-4-[(tertbutoxycarbonyl)amino]cyclohexyl}methyl)amino]-4-(trifluoromethyl)benzoate |
| 122 | 59 | Ethyl 3-[({(1r,4r)-4-[(tertbutoxycarbonyl)amino]cyclohexyl}methyl)amino]-4-methylbenzoate |
| 123 | 90 | 4-bromo-3-[({(1r,4r)-4-[(tertbutoxycarbonyl)amino]cyclohexyl}methyl)amino]benzoic acid |
| 124 | 91 | Methyl 3-[({(1r,4r)-4-[(tertbutoxycarbonyl)amino]cyclohexyl}methyl)amino]-5-fluoro-4-(trifluoromethyl)benzoate |
| 125 | 59 | tert-Butyl 4-(2-[5-(ethoxycarbonyl)-2-(trifluoromethyl)anilino]ethyl}-4-methylpiperidine-1-carboxylate |
| 126 | 126 | Ethyl 2-[({(1r,4r)-4-[(tertbutoxycarbonyl)amino]cyclohexyl}methyl)amino][1,1'-biphenyl]-4-carboxylate |
| 127 | 59 | Ethyl 3-[(2-{(3R)-3-[(tert-butoxycarbonyl)amino]piperidin-1-yl}ethyl)amino]-4-(trifluoromethyl)benzoate |
| 128 | 59 | Ethyl 3-[(2-{(3 S)-3-[(tert-butoxycarbonyl)amino]piperidin-1 - yl}ethyl)amino]-4-(trifluoromethyl)benzoate |
| 129 | 59 | Ethyl 3-[({(1s,4s)-4-[(tertbutoxycarbonyl)amino]cyclohexyl}methyl)amino]-4-(trifluoromethyl)benzoate |
| 130 | 130 | 3-({(1S)-1-[1-(tert-Butoxycarbonyl)piperidin-4-yl]ethyl}amino)-5-fluoro-4-(trifluoromethyl)benzoic acid |
| 131 | 59 | tert-Butyl 4-{(1S)-1-[2-chloro-3-fluoro-5-(methoxycarbonyl)anilino]ethyl}piperidine-1-carboxylate |
| 132 | 59 | tert-Butyl 4-{(1S)-1-[2-chloro-5-(ethoxycarbonyl)anilino]ethyl}piperidine-1-carboxylate |
| 133 | 90 | 4-bromo-3-({(1S)-1- [1-(tert-butoxycarbonyl)piperidin-4-yl]ethyl}amino)benzoic acid |

**[Table 7]**

| REx | PREx | Chemical Name |
|---|---|---|
| 134 | 59 | tert-Butyl 4-{(1S)-1-[2,3-dichloro-5-(methoxycarbonyl)anilino]ethyl}piperidine-1-carboxylate |
| 135 | 59 | tert-Butyl 4-{(1S)-1-[2-fluoro-5-(methoxycarbonyl)anilino]ethyl}piperidine-1-carboxylate |
| 136 | 59 | tert-Butyl 4-{2-[5-(ethoxycarbonyl)-2-(trifluoromethyl)anilino]propyl}piperidine-1-carboxylate |
| 137 | 137 | tert-Butyl 4-{(1S)-1-[2-methoxy-5-(methoxycarbonyl)anilino]ethyl}piperidine-1-carboxylate |
| 138 | 59 | tert-Butyl 4-{(1S)-1-[2-bromo-3-fluoro-5-(methoxycarbonyl)anilino]ethyl}piperidine-1-carboxylate |
| 139 | 59 | tert-Butyl 4-{(1S)-1-[2-chloro-5-(methoxycarbonyl)-3-methylanilino]ethyl}piperidine-1-carboxlate |
| 140 | 59 | tert-Butyl 4-{(1S)-1-[2-cyano-5-(ethoxycarbonyl)anilino]ethyl}piperidine-1-carboxylate |
| 141 | 59 | Methyl 3-[({(1r,4r)-4-[(tertbutoxycarbonyl)amino]cyclohexyl}methyl)amino]-4-chloro-5 - fluorobenzoate |
| 142 | 142 | tert-Butyl 4-{(1S)-1-[2,3-dichloro-6-fluoro-5-(methoxycarbonyl)anilino]ethyl}piperidine-1-carboxylate |
| 143 | 142 | tert-Butyl 4-{(1S)-1-[2-chloro-3,6-difluoro-5-(methoxycarbonyl)anilino]ethyl}piperidine-1-carboxylate |
| 144 | 59 | tert-Butyl 4-{(1S)-1-[2,3-difluoro-5-(methoxycarbonyl)anilin]ethyl}piperidine-1-carboxylate |
| 145 | 59 | tert-Butyl 4-{(1S)-1-[2-(difluoromethyl)-5-(methoxycarbonyl)anilino]ethyl}piperidine-1-carboxylate |
| 146 | 59 | tert-Butyl 4-{(1S)-1-[2-chloro-5-(methoxycarbonyl)-3 - nitroanilino]ethyl}piperidine-1-carboxylate |
| 147 | 147 | tert-Butyl 4-{(1S)-1-[3 -amino-2-chloro-5 - (methoxycarbonyl)anilino]ethyl}piperidine-1-carboxylate |

**[Table 8]**

| REx | PREx | Chemical Name |
|---|---|---|
| 148 | 142 | tert-Butyl 4-{(1S)-1-[2,3-dichloro-5-(methoxycarbonyl)-6-methylanilino]ethyl}piperidine-1-carboxylate |
| 149 | 142 | tert-Butyl 4-{(1S)-1-[2-chloro-5-(methoxycarbonyl)-4-methylanilino]ethyl}piperidine-1-carboxylate |
| 150 | 59 | tert-Butyl 4-{(1S)-1-[2-chloro-4-fluoro-5-(methoxycarbonyl)anilino]ethyl}piperidine-1-carboxylate |
| 151 | 59 | tert-Butyl 4-{(1S)-1-[2,4-dichloro-5-(methoxycarbonyl)anilino]ethyl}piperidine-1-carboxylate |
| 152 | 90 | 3-bromo-5-({(1S)-1-[1-(tert-butoxycarbonyl)piperidin-4-yl]ethyl}amino)-4-chlorobenzoic acid |
| 153 | 59 | tert-Butyl 4-{(1S)-1-[3-chloro-5-(methoxycarbonyl)-2-methylanilino]ethyl}piperidine-1-carboxylate |
| 154 | 59 | tert-Butyl 4-{(1S)-1-[3-fluoro-5-(methoxycarbonyl)-2-methylanilino]ethyl}piperidine-1-carboxylate |
| 155 | 59 | Methyl 4-({[1-(tert-butoxycarbonyl)piperidin-4-yl]methyl}amino)-1-{[2-(trimethylsilyl)ethoxy]methyl}-1H-indazole-6-carboxylate |
| 156 | 142 | tert-Butyl 4-{(1S)-1-[2-chloro-5-(methoxycarbonyl)-3-(trifluoromethyl)anilinol]ethyl}piperidine-1-carboxylate |
| 157 | 126 | tert-Butyl 4-{(1S)-1-[2-chloro-3-cyclopropyl-5-(methoxycarbonyl)anilino]ethyl} piperidine- 1 -carboxylate |
| 158 | 158 | tert-Butyl 4-[(1S)-1-{3-chloro-5-(methoxycarbonyl)-2-[(propan-2-yl)oxy]anilino}ethyl]piperidine-1-carboxylate |
| 159 | 59 | tert-Butyl 4-{(1S)-1-[3-chloro-2-methoxy-5-(methoxycarbonyl)anilino)ethyl}piperidine-1-carboxylate |
| 160 | 59 | tert-Butyl 4-{(1S)-1-[3-chloro-5-(methoxycarbonyl)anilino]ethyl}piperidine-1-carboxylate |
| 161 | 59 | Methyl 3-{ [(1S)-1-{(1r,4S)-4-[(tertbutoxycarbonyl)amino]cyclohexyl}ethyl]amino}-4-(trifluoromethyl)benzoate |

**[Table 9]**

| REx | PREx | Chemical Name |
|---|---|---|
| 162 | 59 | tert-Butyl 4-{(1S)-1-[3-(methoxycarbonyl)-5-(pentafluoro-λ⁶-sulfanyl)anilino]ethyl}piperidine-1-carboxylate |
| 163 | 59 | tert-Butyl 4-fluoro-4-{[5-(methoxycarbonyl)-2-(trifluoromethyl)anilino]methyl}piperidine-1-carboxylate |
| 164 | 59 | tert-Butyl 3-fluoro-3-{[5-(methoxycarbonyl)-2-(trifluoromethyl)anilino]methyl}piperidine-1-carboxylate |
| 165 | 165 | tert-Butyl 4-{(1S)-1-[2-chloro-3-cyano-5-(methoxycarbonyl)anilino]ethyl}piperidine-1-carboxylate |
| 166 | 59 | Methyl 3-{ [(1R)-1-{(1r,4R)-4-[(tertbutoxycarbonyl)amino]cyclohexyl}ethyl]amino}-4-(trifluoromethyl)benzoate |
| 167 | 59 | Methyl 2-({(1S)-1-[1-(tert-butoxycarbonyl)piperidin-4-yl]ethyl} amino)pyridine-4-carboxylate |
| 168 | 59 | Benzyl 4-{(1R)-2,2-difluoro-1-[5-(methoxycarbonyl)-2-(trifluoromethyl)anilino]ethyl}piperidine-1-carboxylate |
| 169 | 59 | Methyl 6-({(1S)-1-[1-(tert-butoxycarbonyl)piperidin-4-yl]ethyl}amino)-5-(trifluoromethyl)pyridine-2-carboxylate |
| 170 | 59 | tert-Butyl 4-{1-[5-(methoxycarbonyl)-2-(trifluoromethyl)anilino]ethyl}-4-methylpiperidine-1-carboxylate |
| 171 | 59 | Methyl 4-({[1-(tert-butoxycarbonyl)-4-fluoropiperidin-4-yl]methyl}amino)-1-{[2-(trimethylsilyl)ethoxy]methyl}-1H-indazole-6-carboxylate |
| 172 | 59 | Methyl 3-[(2-{(1r,4r)-4-[(tertbutoxycarbonyl)amino]cyclohexyl}ethyl)amino]-4-(trifluoromethyl)benzoate |
| 173 | 59 | Methyl 3-({[(1r,4r)-4-(tert-butoxycarbonyl)cyclohexyl]methyl}amino)-4-(trifluoromethyl)benzoate |
| 174 | 59 | tert-Butyl 4-ethyl-4-{[5-(methoxycarbonyl)-2-(trifluoromethyl)anilino]methyl}piperidine-1-carboxylate |
| 175 | 59 | tert-Butyl 4-{ [2-chloro-3-fluoro-5-(methoxycarbonyl)anilino]methyl } -4-fluoropiperidine-1-carboxylate |

**[Table 10]**

| REx | PREx | Chemical Name |
|---|---|---|
| 176 | 176 | Methyl 4-bromo-1-{[1-(tert-butoxycarbonyl)piperidin-4-yl]methyl}-1H-indazole-6-carboxylate |
| 177 | 59 | tert-Butyl 4-fluoro-4-{(1S)-1-[5-(methoxycarbonyl)-2-(trifluoromethyl)anilino]ethyl}piperidine-1-carboxylate |
| 178 | 59 | tert-Butyl cis-3-fluoro-4-{[5-(methoxycarbonyl)-2-(trifluoromethyl)anilino}piperidine-1-carboxylate |
| 179 | 59 | tert-Butyl trans-3-fluoro-4-{[5-(methoxycarbonyl)-2-(trifluoromethyl)anilino]methyl}piperidine-1-carboxylate |
| 180 | 59 | tert-Butyl 4-{(1S)-1-[2-(methanesulfonyl)-5-(methoxycarbonyl)anilino]ethyl}piperidine-1-carboxylate |
| 181 | 181 | tert-Butyl 4-{[5-(ethoxycarbonyl)-3-fluoro-2-(trifluoromethyl)anilino]methyl}-4-fluoropiperidine-1-carboxylate |
| 182 | 182 | Methyl 3-[({(1r,4r)-4-[(tertbutoxycarbonyl)amino]cyclohexyl}methyl)(methyl)amino]-4-(trifluoromethyl)benzoate |
| 183 | 59 | Methyl 3-[({ (1r,4r)-4-[(tertbutoxycarbonyl)(methyl)amino]cyclohexyl}methyl)amino]-4-(trifluoromethyl)benzoate |
| 184 | 184 | Methyl 3-[({(1r,4r)-4-[(tertbutoxycarbonyl)(methyl)amino]cyclohexyl}methyl)(methyl)amino]-4-(trifluoromethyl)benzoate |
| 185 | 59 | tert-Butyl 8-{[5-(methoxycarbonyl)-2-(trifluoromethyl)anilino]methyl}-3-azabicyclo[3.2.1]octane-3-carboxylate |
| 186 | 59 | tert-Butyl (3S)-3-{2-[5-(mebuthoxycarbonyl)-2-(trifluoromethyl)anilino]propyl} pyrrolidine- 1 -carboxylate |
| 187 | 59 | tert-Butyl (1R,3s,5S)-3-{[5-(methoxycarbonyl)-2-(trifluoromethyl)anilino]methyl}-8-azabicyclo[3.2.1]octane-8-carboxylate |
| 188 | 59 | tert-Butyl (3R)-3-{2-[5-(mebutoxycarbonyl)-2-(trifluoromethyl)anilino]propyl}pyrrolidine-1-carboxylate |
| 189 | 59 | Methyl 3-{ [(1S)-1-{(1R,3S)-3-[(tert-butoxycarbonyl)amino]-2,2-dimethylcyclobutyl}ethyl]amino}-4-(trifluoromethyl)benzoate |

**[Table 11]**

| REx | PREx | Chemical Name |
|---|---|---|
| 190 | 59 | tert-Butyl 4-{(1S)-1-[5-(methoxycarbonyl)-2-(trifluoromethyl)anilino]propyl}piperidine-1-carboxylate |
| 191 | 59 | tert-Butyl 4-{(1S)-1-[2-chloro-3-fluoro-5-(methoxycarbonyl)anilino]propyl} piperidine-1-carboxylate |
| 192 | 59 | tert-Butyl 4-{(1R)-2,2-difluoro-1-[5-(methoxycarbonyl)-2-(trifluoromethyl)anilino]ethyl}-4-fluoropiperidine-1-carboxylate |
| 193 | 59 | benzyl 4-{[5-(methoxycarbonyl)-2-(trifluoromethyl)anilino]methyl}-3-methylpiperidine-1 -carboxylate |
| 194 | 59 | tert-Butyl 2-{(1S)-1-[5-(methoxycarbonyl)-2-(trifluoromethyl)anilino]ethyl}-7-azaspiro[3.5]nonane-7-carboxylate |
| 195 | 59 | benzyl 4-{[5-(methoxycarbonyl)-2-(trifluoromethyl)anilino] methyl } -2-methylpiperidine-1-carboxylate |
| 196 | 59 | tert-Butyl 4-{(1S)-1-[5-(methoxycarbonyl)-2-(trifluoromethyl)anilinolethyl}-4-methylpiperidine-1-carboxylate |
| 197 | 59 | Methyl 3-{[(1S)-1-{(1s,3R)-3-[(tertbutoxycarbonyl)amino]cyclobutyl}ethyl]amino}-4-(trifluoromethyl)benzoate |
| 198 | 59 | tert-Butyl 4-{(1R)-2,2,2-trifluoro-1-[5-(methoxycarbonyl)-2-(trifluoromethyl)anilino]ethyl}piperidine-1-carboxylate |
| 199 | 59 | tert-Butyl 4-{(1S)-2,2,2-trifluoro-1-[5-(methoxycarbonyl)-2-(trifluoromethyl)anilino]ethyl}piperidine-1-carboxylate |
| 200 | 59 | Methyl 3-{[(1S)-1-{(1r,4S)-4-[(tertbutoxycarbonyl)amino}ethyl]amino}-4-chlorobenzoate |
| 201 | 59 | tert-Butyl 6-{(1S)-1-[5-(methoxycarbonyl)-2-(trifluoromethyl)anilino]ethyl}-2-azaspiro[3.3]heptane-2-carboxylate |
| 202 | 91 | Ethyl 3-{[(1S)-1-{(1R,3S)-3-[(tert-butoxycarbonyl)amino]-2,2-dimethylcyclobutyl}ethyl]amino}-5-fluoro-4-(trifluoromethyl)benzoate |
| 203 | 91 | Ethyl 3-{[(1S)-1-{(1r,4S)-4-[(tertbutoxycarbonyl)amino]cyclohexyl}ethyl]amino}-5-fluoro-4-(trifluoromethyl)benzoate |

**[Table 12]**

| REx | PREx | Chemical Name |
|---|---|---|
| 204 | 59 | tert-Butyl 4-1(1R)-2-fluoro-1-[5-(methoxycarbonyl)-2-(trifluoromethyl)anilino]ethyl}piperidine-1-carboxylate |
| 205 | 59 | Methyl 3-{[(1S)-1-{(1S,3R)-3-[(tert-butoxycarbonyl)amino]-2,2-dimethylcyclobutyl}ethyl]amino}-4-(trifluoromethyl)benzoate |
| 206 | 90 | 4-bromo-3-{[(1S)-1-{(1r,4S)-4-[(tertbutoxycarbonyl)amino]cyclohexyl}ethyl]amino}benzoic acid |
| 207 | 207 | tert-Butyl (1R,5S,8r)-8-{(1S)-1-[5-(methoxycarbonyl)-2-(trifluoromethyl)anilino)ethyl}-3-azabicyclo[3.2.1]octane-3-carboxylate |
| 208 | 207 | tert-Butyl (1R,5S,8s)-8-{(1S)-1-[5-(methoxycarbonyl)-2-(trifluoromethyl)anilino]ethyl}-3-azabicyclo[3.2.1]octane-3-carboxylate |
| 209 | 59 | tert-Butyl 4-((1S)-1-[5-(methoxycarbonyl)-2-(trifluoromethyl)anilino]ethyl}azepane-1-carboxylate |
| 210 | 59 | Methyl 3-{ [(1S)-1-{(1R,3S)-3-[(tert-butoxycarbonyl)amino]-2,2-dimethylcyclobutyl}ethyl]amino}-4-chlorobenzoate |
| 211 | 211 | 3-({(1S)-1-[1-(tert-Butoxycarbonyl)-4-fluoropiperidin-4-yl]ethyl}amino)-5-fluoro-4-(trifluoromethyl)benzoic acid |
| 212 | 211 | 3-({(1R)-1-[1-(tert-Butoxycarbonyl)piperidin-4-yl]-2-fluoroethyl}amino)-5-fluoro-4-(trifluoromethyl)benzoic acid |
| 213 | 213 | Methyl 3-(2-{(2r,5r)-5-[(tert-butoxycarbonyl)amino]-1,3-dioxan-2-yl} cyclopropyl}-4-(trifluoromethyl)benzoate |
| 214 | 59 | tert-Butyl 5-{(1S)-1-[5-(methoxycarbonyl)-2-(trifluoromethyl)anilino]ethyl}azocane-1-carboxylate |
| 215 | 215 | tert-Butyl 4-{(1R)-2,2-difluoro-1-[3-fluoro-5-(methoxycarbonyl)-2-(trifluoromethyl)anilino]ethyl}-4-fluoropiperidine-1-carboxylate |

**[Table 13]**

| REx | PREx | Chemical Name |
|---|---|---|
| 216 | 59 | Methyl 3-{[(1S)-1-{(1S,3R)-3-[(tert-butoxycarbonyl)amino]-2,2-dimethylcyclobutyl}ethyl]amino}-5-fluoro-4-(trifluoromethyl)benzoate |
| 217 | 130 | 3-{[(1S)-1-{(1s,3R)-3-[(tert-Butoxycarbonyl)amino]cyclobutyl}ethyl]amino}-5-fluoro-4-(trifluoromethyl)benzoic acid |
| 218 | 59 | Methyl 3-[({(1R,3S)-3-[(tert-butoxycarbonyl)amino]-2,2-dimethylcyclobutyl}methyl)amino]-4-(trifluoromethyl)benzoate |
| 219 | 59 | Methyl 3-{[(1S)-1-{(1r,4S)-4-[(tertbutoxycarbonyl)amino]cyclohexyl}ethyl]amino}-5-fluoro-4-methoxybenzoate |
| 220 | 59 | Methyl 3-{[(1S)-1-{(1S,3R)-3-[(tert-butoxycarbonyl)amino]-2,2-dimethylcyclobutyl }ethyl]amino}-4-chlorobenzoate |
| 221 | 59 | Methyl 3-{[(1S)-1-{(1r,4S)-4-[(tertbutoxycarbonyl)amino]cyclohexyl}ethyl]amino}-4-chloro-2,5-difluorobenzoate |
| 222 | 130 | 3-{[(1S)-1-{(1r,4S)-4-[(tert-Butoxycarbonyl)amino]cyclohexyl}ethyl]amino}-5-fluoro-4-(trifluoromethoxy)benzoic acid |
| 223 | 90 | 3-Bromo-5-{[(1S)-1-{(1r,4S)-4-[(tert-butoxycarbonyl)amino]cyclohexyl}ethyl]amino}-4-chlorobenzoic acid |
| 224 | 59 | Methyl 3-{[(1S)-1-{(1r,4S)-4-[(tert-butoxycarbonyl)amino]cyclohexyl}ethyl]amino}-5-fluoro-4-methylbenzoate |
| 225 | 158 | Methyl 3-{[(1S)-1-{(1r,4S)-4-[(tert-butoxycarbonyl)amino]cyclohexyl}ethyl]amino}-4-ethoxy-5-fluoro benzoate |
| 226 | 90 | 3-{[(1S)-1-{(1r,4S)-4-[(tert-butoxycarbonyl)amino]cyclohexyl}ethyl]amino}-4,5-dichlorobenzoic acid |
| 227 | 158 | Methyl 3-{[(1S)-1-{(1r,4S)-4-[(tert-butoxycarbonyl)amino]cyclohexyl}ethyl]amino}-5-fluoro-4-[(propan-2-yl)oxy]benzoate |
| 228 | 228 | Methyl 3-[({(1S,3R)-3-[acetyl(tert-butoxycarbonyl)amino]-2,2-dimethylcyclobutyl}methyl)amino-4-(trifluoromethyl)benzoate |

**[Table 14]**

| REx | PREx | Chemical Name |
|---|---|---|
| 229 | 90 | 3-{[(1S)-1-{(1r,4S)-4-[(tert-Butoxycarbonyl)amino]cyclohexyl}ethyl]amino}-4-chloro-5-fluorobenzoic acid |
| 230 | 59 | Methyl 3-{[(1S)-1-{(1s,3R)-3-[(tert-butoxycarbonyl)amino]cyclobutyl}ethyl]amino}-4-chloro-5-fluorobenzoate |
| 231 | 59 | Methyl 3-{[(1S)-1-{(1s,3R)-3-[(tert-butoxycarbonyl)amino)cyclobutyl}ethyl] amino }-4,5-dichlorobenzoate |
| 232 | 130 | 3-{[(1S)-1-{(1s,3R)-3-[(tert-Butoxycarbonyl)amino]cyclobutyl}ethyl]amino}-5-fluoro-4-(trifluoromethoxy)benzoic acid |
| 233 | 59 | Methyl 3-{[(1S)-1-{(1r,4S)-4-[(tert-butoxycarbonyl)amino]cyclohexyl}propyl]amino}-4-(trifluoromethyl)benzoate |
| 234 | 90 | 3-Bromo-5-{[(1S)-1-{(1s,3R)-3-[(tert-butoxycarbonyl)amino]cyclobutyl}ethyl]amino}-4-chlorobenzoic acid |
| 235 | 90 | 3-{[(1S)-1-{(1s,3R)-3-[(tert-Butoxycarbonyl)amino] cyclobutyl } ethyl] amino }-4-chloro-5-(trifluoromethyl)benzoic acid |
| 236 | 59 | Methyl 3-({[(1R,3 S)-3 -acetamide-2,2-dimethylcyclobutyl]methyl } amino)-4-(trifluoromethyl)benzoate |
| 237 | 59 | Methyl 3-{[(1S)-1-{3-[(tert-butoxycarbonyl)amino]bicyclo[1.1.1 ]pentan-1-yl}ethyl]amino}-4-(trifluoromethyl)benzoate |
| 238 | 59 | Methyl 3-{[(1S)-1-{4-[(tert-butoxycarbonyl)amino]cuban-1-yl}ethyl]amino}-4-(trifluoromethyl)benzoate |
| 239 | 59 | Methyl 3-{[(1S)-1-{4-[(tert-butoxycarbonyl)amino]bicyclo[2.2.1 ]heptan-1-yl}ethyl]amino}-4-(trifluoromethyl)benzoate |
| 240 | 59 | 3,7-Anhydro-6-[(tert-butoxycarbonyl)amino]-1,2,4,5,6-pentadeoxy-2-[5-(methoxycarbonyl)-2-(trifluoromethyl)anilino]-L-arabino-heptitol |
| 241 | 59 | Methyl 3-{[(1S)-1-{(1r,4S)-4-[(tert-butoxycarbonyl)(methyl)amino]cyclohexyl}ethyl]amino}-4-(trifluoromethyl)benzoate |

**Table 15**

| REx | PREx | Chemical Name |
|---|---|---|
| 242 | 59 | Ethyl 3-{[(1S)-1-{(1r,4S)-4-[(tert-butoxycarbonyl)(methyl)amino]cyclohexyl}ethyl]amino}-5-fluoro-4-(trifluoromethyl)benzoate |
| 243 | 59 | Methyl 3-{[(1S)-1-{(1R,3S)-3-[(tert-butoxycarbonyl)amino]cyclohexyl}ethyl]amino}-4-(trifluoromethyl)benzoate |
| 244 | 244 | Methyl 3-{[(1S)-1-{(1s,3R)-3-[(tert-butoxycarbonyl)(methyl)amino]cyclobutyl}ethyl]amino}-4-(trifluoromethyl)benzoate |
| 245 | 59 | Methyl 3-{[(1S)-1-{(1r,4S)-4-[(tert-butoxycarbonyl)(ethyl)amino] cyclohexyl } ethyl] amino }-4-(trifluoromethyl)benzoate |
| 246 | 59 | Methyl 3-{[(1S)-1-{(1S,3R)-3-[(tert-butoxycarbonyl)amino]cyclohexyl} ethyl] amino} -4-(trifluoromethyl)benzoate |
| 247 | 244 | Methyl3-{[(1S)-1-{(1s,3R)-3-[(tert-butoxycarbonyl)(ethyl)amino]cyclobutyl} ethyl]amino} -4-(trifluoromethyl)benzoate |
| 248 | 244 | Methyl 3-{[(1S)-1-{(1s,3R)-3-[(tert-butoxycarbonyl)(cyclopropylmethyl)amino]cyclobutyl}ethyl]amino}-4-(trifluoromethyl)benzoate |
| 249 | 249 | Methyl 3-{[(1S)-1-{(1r,4S)-4-[(2,2,2-trifluoroethyl)amino]cyclohexyl}ethyl]amino}-4-(trifluoromethyl)benzoate |
| 250 | 130 | 3-{[(1S)-1-{(1r,4S)-4-[(tert-Butoxycarbonyl)amino]cyclohexyl}propyl]amino}-5-fluoro-4-(trifluoromethyl)benzoic acid |
| 251 | 59 | Methyl 3-{[(1S)-1-{(1S,3R)-3-[(tert-butoxycarbonyl)amino]cyclopentyl}ethyl]amino}-4-(trifluoromethyl)benzoate |
| 252 | 59 | Methyl 3-{[(1S)-1-{(1R,3S)-3-[(tert-butoxycarbonyl)amino]cyclopentyl}ethyl]amino}-4-(trifluoromethyl) benzoate |
| 253 | 253 | Methyl 3-{[(1S)-1-{(1r,4S)-4-[(tert-butoxycarbonyl)(2-hydroxyethyl)amino]cyclohexyl}ethyl]amino}-4-(trifluoromethyl)benzoate |
| 254 | 130 | 3-({(1S)-1-[1-(tert-Butoxycarbonyl)piperidin-4-yl]ethyl}amino)-5-fluoro-4-(trifluoromethoxy)benzoic acid |

**[Table 16]**

| REx | PREx | Chemical Name |
|---|---|---|
| 255 | 255 | 3-{[(1S)-1-{(1r,3R)-3-[(tert-butoxycarbonyl)(methyl)amino]cyclobutyl}ethyl]amino}-5-fluoro-4-(trifluoromethyl) benzoate |
| 256 | 130 | 3-{[(1S)-1-{(1r,4S)-4-[(tert-butoxycarbonyl)(methyl)amino]cyclohexyl}ethyl]amino}-5-fluoro-4-(trifluoromethoxy)benzoate |
| 257 | 257 | tert-butyl4-[{[2-chloro-5-(methoxycarbonyl)phenyl]methyl}(methyl)amino]piperidine-1-carboxylate |
| 258 | 130 | 3-({(1S)-1-[1-(tert-butoxycarbonyl)-4-methylpiperidin-4-yl]ethyl}amino)-5-fluoro-4-(trifluoromethyl) benzoate |
| 259 | 130 | 3-({(1S)-1-[1-(tert-butoxycarbonyl)-4-methylpiperidin-4-yl]ethyl}amino)-5-fluoro-4-(trifluoromethoxy)benzoate |
| 260 | 255 | 3-{[(1S)-1-{(1s,3R)-3-[(tert-butoxycarbonyl)(methyl)amino]cyclobutyl}ethyl]amino}-5-fluoro-4-(trifluoromethoxy) benzoate |
| 261 | 59 | tert-butyl 4-fluoro-4-{(1S)-2-fluoro-1-[5-(methoxycarbonyl)-2-(trifluoromethyl)anilino]ethyl}piperidine-1-carboxylate |
| 262 | 130 | 3-({(1S)-1-[(3S,4S)-1-(tert-butoxycarbonyl)-3-methylpiperidin-4-yl]ethyl} amino)-5-fluoro-4-(trifluoromethyl) benzoate |
| 263 | 130 | 3-({(1S)-1-[(3R,4R)-1-(tert-butoxycarbonyl)-3-methylpiperidin-4-yl]ethyl}amino)-5-fluoro-4-(trifluoromethyl) benzoate |
| 264 | 264 | tert-butyl 4-{(1S)-1-[2-(difluoromethoxy)-3-fluoro-5-(methoxycarbonyl)anilino] ethyl } piperidine- 1 -carboxylate |
| 265 | 264 | tert-butyl 4-{(1S)-1-[2-(difluoromethoxy)-3-fluoro-5-(methoxycarbonyl)anilino]ethyl}-4-methylpiperidine-1-carboxylate |
| 266 | 264 | tert-butyl (3S,4S)-4-((1S)-1-[2-(difluoromethoxy)-3-fluoro-5-(methoxycarbonyl)anilino]ethyl}-3-methylpiperidine-1-carboxylate |
| 267 | 264 | tert-butyl 4-{(1S)-1-[2-(difluoromethoxy)-3-fluoro-5-(methoxycarbonyl)anilino]ethyl}-2-methylpiperidine-1-carboxylate |
| 268 | 59 | tert-butyl (3S,4S)-4-{(1S)-1-[5-(methoxycarbonyl)-2-(trifluoromethyl)anilino]ethyl}-3-methylpiperidine-1-carboxylate |

**[Table 17]**

| REx | PREx | Chemical Name |
|---|---|---|
| 269 | 59 | tert-Butyl (3S,4S)-4-{(1S)-1-[5-(methoxycarbonyl)-2-(trifluoromethoxy)anilino]ethyl}-3-methylpiperidine-1-carboxylate |
| 270 | 130 | 3-({(1S)-1-[(3S,4S)-1-(tert-Butoxycarbonyl)-3-methylpiperidin-4-yl]ethyl}amino)-5 -fluoro-4-(trifluoromethoxy)benzoic acid |
| 271 | 59 | tert-Butyl (3S,4S)-4-{(1S)-1-[2-(difluoromethoxy)-5-(methoxycarbonyl)anilino]ethyl}-3-methylpiperidine-1-carboxylate |
| 272 | 90 | 3-({(1S)-1-[(3S,4S)-1-(tert-Butoxycarbonyl)-3-methylpiperidin-4-yl]ethyl}amino)-4-chlorobenzoic acid |
| 273 | 90 | 3-({(1S)-1-[(3S,4S)-1-(tert-Butoxycarbonyl)-3-methylpiperidin-4-yl]ethyl}amino)-4-chloro-5-fluorobenzoic acid |
| 274 | 59 | tert-Butyl (3S,4R)-3-fluoro-4-{(1S)-1-[3-fluoro-5-(methoxycarbonyl)-2-methylanilino]ethyl}piperidine-1-carboxylate |
| 275 | 59 | tert-Butyl (3S,4S)-4-{(1S)-1-[3-fluoro-5-(methoxycarbonyl)-2-methylanilino]ethyl}-3-methylpiperidine-1-carboxylate |
| 276 | 59 | tert-Butyl (3S,4S)-3-ethyl-4-{(1S)-1-[3-fluoro-5-(methoxycarbonyl)-2-methylanilino]ethyl}piperidine-1-carboxylate |
| 277 | 126 | Methyl 3-{[(1S)-1-{(1r,4S)-4-[(tert-butoxycarbonyl)amino]cyclohexyl}ethyl]amino}-4-cyclopropyl-5-fluorobenzoate |
| 278 | 126 | Methyl 3-{[(1S)-1-{(1r,4S)-4-[(tert-butoxycarbonyl)amino]cyclohexyl}ethyl]amino}-4-ethenyl-5-fluorobenzoate |
| 279 | 126 | Methyl 3-{[(1S)-1-{(1r,4S)-4-[(tert-butoxycarbonyl)amino]cyclohexyl}ethyl]amino}-5-fluoro-4-(prop-1-en-2-yl)benzoate |
| 280 | 280 | Methyl 3-[({(1r,4r)-4-[(tert-butoxycarbonyl)amino]cyclohexyl}methyl)sulfanyl]-4-(trifluoromethyl)benzoate |
| 281 | 79 | tert-Butyl 4-{ [2-bromo-5-(methoxycarbonyl)phenoxy]methyl }piperidine-1-carboxylate |
| 282 | 79 | tert-Butyl 4-{1-[2-bromo-5-(methoxycarbonyl)phenoxy]ethyl}piperidine-1-carboxylate |

**[Table 18]**

| REx | PREx | Chemical Name |
|---|---|---|
| 283 | 79 | Methyl 4-bromo-3-(1-{(1r,4r)-4-[(tert-butoxycarbonyl)amino]cyclohexyl}ethoxy)benzoate |
| 284 | 79 | Methyl 4-bromo-3-({(1r,4r)-4-[(tert-butoxycarbonyl)amino}methoxy)benzoate |
| 285 | 79 | tert-Butyl 4-{1-[5-(methoxycarbonyl)-2-(trifluoromethyl)phenoxy]ethyl}piperidine-1-carboxylate |
| 286 | 79 | Methyl 3-({(1r,4r)-4-[(tert-butoxycarbonyl)amino]cyclohexyl}methoxy)-4-(trifluoromethyl)benzoate |
| 287 | 79 | Methyl 3-({(1r,4r)-4-[(tert-butoxycarbonyl)amino]cyclohexyl}methoxy)-4-chlorobenzoate |
| 288 | 79 | tert-Butyl 4-[(1s,3s)-3-{[2-chloro-5-(methoxycarbonyl)phenoxy]methyl}cyclobutyl]piperazine-1-carboxylate |
| 289 | 79 | Methyl 3-({(1s,4s)-4-[(tert-butoxycarbonyl)amino]cyclohexyl}methoxy)-4-chlorobenzoate |
| 290 | 290 | Methyl 1-({(1r,4r)-4-[(tert-butoxycarbonyl)amino]cyclohexyl}methyl)-1,2,3,4-tetrahydroquinoline-7-carboxylate |
| 291 | 291 | Methyl 4-[(1S)-1-{(1r,4S)-4-[(tert-butoxycarbonyl)amino]cyclohexyl} ethyl]-3,4-dihydro-2H-1,4-benzoxazine-6-carboxylate |
| 292 | 292 | Methyl 4-{(1S)-1-[1-(tert-butoxycarbonyl)piperidin-4-yl]ethyl}-8-fluoro-3,4-dihydro-2H-1,4-benzoxazine-6-carboxylate |
| 293 | 292 | Methyl 4-[(1S)-1-{(1r,4S)-4-[(tert-butoxycarbonyl)amino]cyclohexyl}ethyl]-8-fluoro-3,4-dihydro-2H-1,4-benzoxazine-6-carboxylate |
| 294 | 294 | Methyl (2R)-4-{(1S)-1-[1-(tert-butoxycarbonyl)piperidin-4-yl]ethyl}-8-fluoro-2-methyl-3,4-dihydro-2H-1,4-benzoxazine-6-carboxylate |
| 295 | 294 | Methyl (2S)-4-{(1S)-1-[1-(tert-butoxycarbonyl)piperidin-4-yl]ethyl}-8-fluoro-2-methyl-3,4-dihydro-2H-1,4-benzoxazine-6-carboxylate |

**[Table 19]**

| REx | PREx | Chemical Name |
|---|---|---|
| 296 | 292 | Methyl 4-[(1S)-1-{(1r,4S)-4-[(tert-butoxycarbonyl)amino]cyclohexyl}ethyl]-8-fluoro-2-methyl-3,4-dihydro-2H-1,4-benzoxazine-6-carboxylate |
| 297 | 294 | Methyl 5-{(1S)-1-[1-(tert-butoxycarbonyl)piperidin-4-yl]ethyl}-9-fluoro-2,3,4,5-tetrahydro-1,5-benzoxazepine-7-carboxylate |

**[Table 20]**

| Ex | REx | Chemical Name |
|---|---|---|
| 1 | 1 | 5-{3-[(4-aminobutyl)amino]-4-(trifluoromethyl)phenyl}-1,3,4-oxadiazol-2(3H)-one hydrochloride |
| 2 | 1 | 5-{3-[(3-aminopuropyl)amino]-4-(trifluoromethyl)phenyl}-1,3,4-oxadiazol-2(3H)-one hydrochloride |
| 3 | 1 | 5-{3-[(5-aminopentyl)amino]-4-(trifluoromethyl)phenyl}-1,3,4-oxadiazol-2(3H)-one hydrochloride |
| 4 | 1 | 5-{3-[(6-aminohexyl)amino]-4-(trifluoromethyl)phenyl)-1,3,4-oxadiazol-2(3H)-one hydrochloride |
| 5 | 1 | 5-{3-[(6-aminohexan-2-yl)amino]-4-(trifluoromethyl)phenyl}-1,3,4-oxadiazol-2(3H)-one hydrochloride |
| 6 | 1 | 5-{3-[(4-aminomethyl)piperidin-1-yl]-4-chlorophenyl}-1,3,4-oxadiazol-2(3H)-one hydrochloride |
| 7 | 7 | tert-butyl 4-[2-chloro-5-(5-oxo-4,5-dihydro-1,3,4-oxadiazol-2-yl)phenyl]piperadine-1-carboxylate |
| 8 | 8 | 5-[4-chloro-3-(piperadin-1-yl)phenyl]-1,3,4-oxadiazol-2(3H)-one hydrochloride |
| 9 | 1 | 5-[3-(4-aminopiperidin-1-yl)-4-(trifluoromethyl)phenyl]-1,3,4-oxadiazol-2(3H)-one hydrochloride |
| 10 | 1 | 5-{3-[4-(2-aminoethyl)piperidin-1-yl]-4-(trifluoromethyl)phenyl}-1,3,4-oxadiazol-2(3H)-one hydrochloride |

**[Table 21]**

| Ex | PEx | Chemical Name |
|---|---|---|
| 11 | 1 | 5-{3-[3-(2-aminoethyl)piperidin-1-yl]-4-(trifluoromethyl)phenyl}-1,3,4-oxadiazol-2(3H)-one hydrochloride |
| 12 | 1 | 5-{4-[4-(2-aminoethyl)piperidin-1-yl]-1H-indazol-6-yl}-1,3,4-oxadiazol-2(3H)-one dihydrochloride |
| 13 | 1 | 5-{3-[4-(1-amino-2-methylpropan-2-yl)piperidin-1-yl]-4-(trifluoromethyl)phenyl}-1,3,4-oxadiazol-2(3H)-onehydrochloride |
| 14 | 1 | 5-{3-[4-(2-amino-1-hydroxyethyl)piperidin-1-yl]-4-(trifluoromethyl)phenyl}-1,3,4-oxadiazol-2(3H)-one hydrochloride |
| 15 | 1 | 5-[3-(3,9-diazaspiro[5.5]undecan-3-yl)-4-(trifluoromethyl)phenyl]-1,3,4-oxadiazol-2(3H)-one hydrochloride |
| 16 | 1 | 5-[3-(2,7-diazaspiro[3.5]nonan-7-yl)-4-(trifluoromethyl)phenyl]-1,3,4-oxadiazol-2(3H)-one hydrochloride |
| 17 | 7 | tert-butyl {[2'-chloro-5'-(5-oxo-4,5-dihydro-1,3,4-oxadiazol-2-yl)[1,1'-biphenyl]-3-yl]methyl}carbamate |
| 18 | 8 | 5-[3'-(aminomethyl)-6-chloro[1,1'-biphenyl]-3-yl]-1,3,4-oxadiazol-2(3H)-one hydrochloride |
| 19 | 7 | tert-butyl {[5'-(5-oxo-4,5-dihydro-1,3,4-oxadiazol-2-yl)-2'-(trifluoromethyl)[1,1'-biphenyl]-4-yl]methyl}carbamate |
| 20 | 8 | 5-[4'-(aminomethyl)-6-(trifluoromethyl)[1,1'-biphenyl]-3-yl]-1,3,4-oxadiazol-2(3H)-one hydrochloride |
| 21 | 1 | 5-[3'-(aminomethyl)-6-(trifluoromethyl)[1,1'-biphenyl]-3-yl]-1,3,4-oxadiazol-2(3H)-one hydrochloride |
| 22 | 1 | 5-[4'-(2-aminoethyl)-6-(trifluoromethyl)[1,1'-biphenyl]-3-yl]-1,3,4-oxadiazol-2(3H)-one hydrochloride |
| 23 | 1 | 5-{,1'-biphenyl]-3-yl}-1,3,4-oxadiazol-2(3H)-one hydrochloride |

**[Table 22]**

| Ex | PEx | Chemical Name |
|---|---|---|
| 24 | 1 | 5-[4'-{[(2S)-1-aminopropan-2-yl]oxy}-6-(trifluoromethyl)[1,1'-biphenyl]-3-yl]-1,3,4-oxadiazol-2(3H)-one hydrochloride |
| 25 | 1 | 5-{3-[5-(piperazin-1-yl)pyridin-3-yl]-4-(trifluoromethyl)phenyl}-1,3,4-oxadiazol-2(3H)-one dihydrochloride |
| 26 | 1 | 5-{3-[2-(piperidin-4-yl)ethyl]-4-(trifluoromethyl)phenyl}-1,3,4-oxadiazol-2(3H)-one hydrochloride |
| 27 | 1 | 5-[3-{2-[(1r,4s)-4-aminocyclohexyl]ethyl}-4-(trifluoromethyl)phenyl]-1,3,4-oxadiazol-2(3H)-one hydrochloride |
| 28 | 28 | 5-[3-{2-[(2r,5r)-5-amino-1,3-dioxan-2-yl]ethyl}-4-(trifluoromethyl)phenyl]-1,3,4-oxadiazol-2(3H)-one hydrochloride |
| 29 | 1 | 5-{3-[(piperidin-4-yl)ethynyl]-4-(trifluoromethyl)phenyl}-1,3,4-oxadiazol-2(3H)-one hydrochloride |
| 30 | 1 | 5-{3-[(E)-2-(piperidin-4-yl)ethenyl]-4-(trifluoromethyl)phenyl}-1,3,4-oxadiazol-2(3H)-one hydrochloride |
| 31 | 1 | 5-{4-chloro-3-[(piperidin-4-yl)amino]phenyl}-1,3,4-oxadiazol-2(3H)-one hydrochloride |
| 32 | 1 | 5-(3-{[(1r,4r)-4-aminocyclohexyl)amino}-4-chlorophenyl)-1,3,4-oxadiazol-2(3H)-one hydrochloride |
| 33 | 1 | 5-(3-{ [(1 s,4s)-4-aminocyclohexyl]amino} -4-chlorophenyl)-1 ,3,4-oxadiazol-2(3H)-one hydrochloride |
| 34 | 1 | 5-[3-{[(1r,4r)-4-aminocyclohexyl]amino}-4-(trifluoromethyl)phenyl]-1,3,4-oxadiazol-2(3H)-one hydrochloride |
| 35 | 35 | 5-(3-{[(1r,4r)-4-aminocyclohexyl]amino}-4-bromophenyl)-1,3,4-oxadiazol-2(3H)-one hydrochloride |
| 36 | 1 | 5-[3-{[(1r,4r)-4-aminocyclohexyl]amino}-5-fluoro-4-(trifluoromethyl)phenyl]-1,3,4-oxadiazol-2(3H)-one hydrochloride |
| 37 | 37 | 5-[3-{[(1r,4r)-4-(aminomethyl)cyclohexyl]amino}-4-(trifluoromethyl)phenyl]-1,3,4-oxadiazol-2(3H)-one hydrochloride |

**[Table 23]**

| Ex | PEx | Chemical Name |
|---|---|---|
| 38 | 1 | 5-[3-{[(1r,4r)-4-aminocyclohexyl]amino}-4-(trifluoromethoxy)phenyl]-1,3,4-oxadiazol-2(3H)-one hydrochloride |
| 39 | 37 | 5-[3-{[(1r,4r)-4-(1-aminoethyl)cyclohexyl]amino}-4-(trifluoromethyl)phenyl]-1,3,4-oxadiazol-2(3H)-one hydrochloride |
| 40 | 1 | 5-(3-{[(1r,4r)-4-aminocyclohexyl]amino}-4-chloro-5-fluorophenyl)-1,3,4-oxadiazol-2(3H)-one hydrochloride |
| 41 | 1 | 5-{3-[4-(aminomethyl)anilino]-4-(trifluoromethyl)phenyl}-1,3,4-oxadiazol-2(3H)-one hydrochloride |
| 42 | 1 | 5-{3-[(6-azaspiro[2.5]octan-1-yl)amino]-4-(trifluoromethyl)phenyl}-1,3,4-oxadiazol-2(3H)-one hydrochloride |
| 43 | 1 | 5-{3-[(6-aminospiro[3.3]heptan-2-yl)amino]-4-(trifluoromethyl)phenyl}-1,3,4-oxadiazol-2(3H)-one hydrochloride |
| 44 | 44 | 5-[3-{[(1r,4r)-4-(2-aminoethyl)cyclohexyl]amino}-4-(trifluoromethyl)phenyl]-1,3,4-oxadiazol-2(3H)-one hydrochloride |
| 45 | 1 | 5-[3-{[(1S)-7-azaspiro[3.5]nonan-1-yl]amino}-4-(trifluoromethyl)phenyl]-1,3,4-oxadiazol-2(3H)-one hydrochloride |
| 46 | 1 | 5-[3-{[(1R)-7-azaspiro[3.5]nonan-1-yl]amino}-4-(trifluoromethyl)phenyl]-1,3,4-oxadiazol-2(3H)-one hydrochloride |
| 47 | 1 | 5 -(4-chloro-3-{[(piperidin-4-yl)methyl]amino}phenyl)-1,3,4-oxadiazol-2(3H)-one hydrochloride |
| 48 | 1 | 5-[3-{[(piperidin-4-yl)methyl]amino}-4-(trifluoromethyl)phenyl]-1,3,4-oxadiazol-2(3H)-one hydrochloride |
| 49 | 1 | 5-[4-chloro-3-({[(3R)-pyrrolidin-3-yl]methyl}amino)phenyl]-1,3,4-oxadiazol-2(3H)-one hydrochloride |
| 50 | 35 | 5-(4-bromo-3-{[(piperidin-4-yl)methyl]amino}phenyl)-1,3,4-oxadiazol-2(3H)-one hydrochloride |
| 51 | 1 | 5-[3-{methyl[(piperidin-4-yl)methyl]amino}-4-(trifluoromethyl)phenyl]-1,3,4-oxadiazol-2(3H)-one hydrochloride |

**[Table 24]**

| Ex | PEx | Chemical Name |
|---|---|---|
| 52 | 1 | 5-[3-{[1-(piperidin-4-yl)ethyl]amino}-4-(trifluoromethyl)phenyl]-1,3,4-oxadiazol-2(3H)-one hydrochloride |
| 53 | 1 | 5-[3-({[(3S)-piperidin-3-yl]methyl}amino)-4-(trifluoromethyl)phenyl]-1,3,4-oxadiazol-2(3H)-one hydrochloride |
| 54 | 1 | 5-[3-({[(3R)-piperidin-3-yl]methyl}amino)-4-(trifluoromethyl)phenyl]-1,3,4-oxadiazol-2(3H)-one hydrochloride |
| 55 | 1 | 5-[3-({[(1r,4r)-4-aminocyclohexyl]methyl}amino)-4-(trifluoromethyl)phenyl]-1,3,4-oxadiazol-2(3H)-one hydrochloride |
| 56 | 1 | 5-[3-{[1-(piperidin-4-yl)propyl]amino}-4-(trifluoromethyl)phenyl]-1,3,4-oxadiazol-2(3H)-one hydrochloride |
| 57 | 1 | 5-[3-{[(4-methylpiperidin-4-yl)methyl]amino}-4-(trifluoromethyl)phenyl]-1,3,4-oxadiazol-2(3H)-one hydrochloride |
| 58 | 1 | 5-[3-{[(1S)-1-(piperidin-4-yl)ethyl]amino}-4-(trifluoromethyl)phenyl]-1,3,4-oxadiazol-2(3H)-one hydrochloride |
| 59 | 1 | 5-[3-{[(1R)-1-(piperidin-4-yl)ethyl]amino}-4-(trifluoromethyl)phenyl]-1,3,4-oxadiazol-2(3H)-one hydrochloride |
| 60 | 1 | 5-[3-{[2-(piperidin-3-yl)ethyl]amino}-4-(trifluoromethyl)phenyl]-1,3,4-oxadiazol-2(3H)-one hydrochloride |
| 61 | 1 | 5-[3-({[(1r,4r)-4-aminocyclohexyl]methyl}amino)-4-chlorophenyl]-1,3,4-oxadiazol-2(3H)-one hydrochloride |
| 62 | 1 | 5-[3-({[(1r,4r)-4-aminocyclohexyl]methyl}amino)phenyl]-1,3,4-oxadiazol-2(3H)-one hydrochloride |
| 63 | 1 | 5-[3-({1-[(1r,4r)-4-aminocyclohexyl]ethyl}amino)-4-(trifluoromethyl)phenyl]-1,3,4-oxadiazol-2(3H)-one hydrochloride |
| 64 | 1 | 5-[3-{[2-(piperidin-4-yl)ethyl]amino}-4-(trifluoromethyl)phenyl]-1,3,4-oxadiazol-2(3H)-one hydrochloride |
| 65 | 1 | 5-[3-{[2-(piperazin-1-yl)ethyl]amino}-4-(trifluoromethyl)phenyl]-1 ,3,4-oxadiazol-2(3H)-one dihydrochloride |

**[Table 25]**

| Ex | PEx | Chemical Name |
|---|---|---|
| 66 | 1 | 5-[3-({[(1r,4r)-4-aminocyclohexyl]methyl}amino)-4-(trifluoromethoxy)phenyl]-1,3,4-oxadiazol-2(3H)-one hydrochloride |
| 67 | 1 | 5-[3-({[(1r,4r)-4- aminocyclohexyl]methyl}amino)-4-methylphenyl]-1,3,4-oxadiazol-2(3H)-one hydrochloride |
| 68 | 35 | 5-[3-({[(1r,4r)-4-aminocyclohexyl]methyl}amino)-4-bromophenyl]-1,3,4-oxadiazol-2(3H)-one hydrochloride |
| 69 | 1 | 5-[3-({[(1r,4r)-4-aminocyclohexyl]methyl}amino)-5-fluoro-4-(trifluoromethyl)phenyl]-1,3,4-oxadiazol-2(3H)-one hydrochloride |
| 70 | 1 | 5-[3-{[2-(4-methylpiperidin-4-yl)ethyl]amino}-4-(trifluoromethyl)phenyl]-1,3,4-oxadiazol-2(3 H)-one hydrochloride |
| 71 | 1 | 5-[2-({[(1r,4r)-4-aminocyclohexyl]methyl}amino)[1,1'-biphenyl]-4-yl]-1,3,4-oxadiazol-2(3H)-one hydrochloride |
| 72 | 1 | 5-[3-({2-[(3R)-3-aminopiperidin-1-yl]ethyl}amino)-4-(trifluoromethyl)phenyl]-1,3,4-oxadiazol-2(3H)-one dihydrochloride |
| 73 | 1 | 5-[3-({2-[(3S)-3-aminopiperidin-1-yl]ethyl}amino)-4-(trifluoromethyl)phenyl]-1,3,4-oxadiazol-2(3H)-one dihydrochloride |
| 74 | 1 | 5-[3-({[(1s,4s)-4-aminocyclohexyl]methyl}amino)-4-(trifluoromethyl)phenyl] -1,3,4-oxadiazol-2(3H)-one hydrochloride |
| 75 | 35 | 5-[3-fluoro-5-{[(1S)-1-(piperidin-4-yl)ethyl]amino}-4-(trifluoromethyl)phenyl]-1,3,4-oxadiazol-2(3H)-one hydrochloride |
| 76 | 1 | 5-(4-chloro-3-fluoro-5-{[(1S)-1-(piperidin-4-yl)ethyl]amino}phenyl)-1,3,4-oxadiazol-2(3H)-one hydrochloride |
| 77 | 1 | 5-(4-chloro-3-{[(1S)-1-(piperidin-4-yl)ethyl]amino}phenyl)-1,3,4-oxadiazol-2(3H)-one dihydrochloride |
| 78 | 35 | 5-(4-bromo-3-{[(1S)-1-(piperidin-4-yl)ethyl]amino}phenyl)-1,3,4-oxadiazol-2(3H)-one dihydrochloride |
| 79 | 1 | 5-(3,4-dichloro-5-{[(1S)-1-(piperidin-4-yl)ethyl]amino}phenyl)-1,3,4-oxadiazol-2(3H)-one dihydrochloride |

**[Table 26]**

| Ex | PEx | Chemical Name |
|---|---|---|
| 80 | 1 | 5-(4-fluoro-3-{[(1S)-1-(piperidin-4-yl)ethyl]amino}phenyl)-1,3,4-oxadiazol-2(3H)-one hydrochloride |
| 81 | 1 | 5-[3-{[1-(piperidin-4-yl)propan-2-yl]amino}-4-(trifluoromethyl)phenyl]-1,3,4-oxadiazol-2(3H)-one hydrochloride |
| 82 | 1 | 5-(4-methoxy-3-{[(1S)-1-(piperidin-4-yl)ethyl]amino}phenyl)-1,3,4-oxadiazol-2(3H)-one dihydrochloride |
| 83 | 1 | 5-(4-bromo-3-fluoro-5-{[(1S)-1-(piperidin-4-yl)ethyl]amino}phenyl)-1,3,4-oxadiazol-2(3H)-one hydrochloride |
| 84 | 1 | 5-(4-chloro-3-methyl-5-{[(1S)-1-(piperidin-4-yl)ethyl]amino}phenyl)-1,3,4-oxadiazol-2(3H)-one dihydrochloride |
| 85 | 1 | 4-(5-oxo-4,5-dihydro-1,3,4-oxadiazol-2-yl)-2-{[(1S)-1-(piperidin-4-yl)ethyl]amino}benzonitrile hydrochloride |
| 86 | 1 | 5-[3-({[(1r,4r)-4-aminocyclohexyl]methyl}amino)-4-chloro-5-fluorophenyl]-1,3,4-oxadiazol-2(3H)-one hydrochloride |
| 87 | 1 | 5-(4,5-dichloro-2-fluoro-3-{[(1S)-1-(piperidin-4-yl)ethyl]amino}phenyl)-1,3,4-oxadiazol-2(3H)-one hydrochloride |
| 88 | 1 | 5-(4-chloro-2,5-difluoro-3-{[(1S)-1-(piperidin-4-yl)ethyl] amino}phenyl)-1,3,4-oxadiazol-2(3H)-one hydrochloride |
| 89 | 1 | 5-(3,4-difluoro-5-1[(1S)-1-(piperidin-4-yl)ethyl]amino}phenyl)-1,3,4-oxadiazol-2(3H)-one hydrochloride |
| 90 | 1 | 5-[4-(difluoromethyl)-3-{[(1S)-1-(piperidin-4-yl)ethyl]amino}phenyl]-1,3,4-oxadiazol-2(3H)-one |
| 91 | 1 | 5-(4-chloro-3-nitro-5-{[(1S)-1-(piperidin-4-yl)ethyl]amino}phenyl)-1,3,4-oxadiazol-2(3H)-one hydrochloride |
| 92 | 1 | 5-(3-amino-4-chloro-5-{[(1S)-1-(piperidin-4-yl)ethyl]amino}phenyl)-1,3,4-oxadiazol-2(3H)-one dihydrochloride |
| 93 | 1 | 5-(4,5-dichloro-2-methyl-3-{[(1S)-1-(piperidin-4-yl)ethyl]amino}phenyl)-1,3,4-oxadiazol-2(3H)-one hydrochloride |

**[Table 27]**

| Ex | PEx | Chemical Name |
|---|---|---|
| 94 | 1 | 5-(4-chloro-2-methyl-5-{[(1S)-1-(piperidin-4-yl)ethyl]amino}phenyl)-1,3,4-oxadiazol-2(3H)-one hydrochloride |
| 95 | 1 | 5-(4-chloro-2-fluoro-5-{[(1S)-1-(piperidin-4-yl)ethyl]amino}phenyl)-1,3,4-oxadiazol-2(3H)-one hydrochloride |
| 96 | 1 | 5-(2,4-dichloro-5-{[(1S)-1-(piperidin-4-yl)ethyl]amino}phenyl)-1,3,4-oxadiazol-2(3H)-one hydrochloride |
| 97 | 35 | 5-(3-bromo-4-chloro-5-{[(1S)-1-(piperidin-4-yl)ethyl]amino}phenyl)-1,3,4-oxadiazol-2(3H)-one dihydrochloride |
| 98 | 1 | 5-(3-chloro-4-methyl-5-{[(1S)-1-(piperidin-4-yl)ethyl]amino}phenyl)-1,3,4-oxadiazol-2(3H)-one dihydrochloride |
| 99 | 1 | 5-(3-fluoro-4-methyl-5-{[(1S)-1-(piperidin-4-yl)ethyl]amino} phenyl)-1,3,4-oxadiazol-2(3H)-one dihydrochloride |
| 100 | 1 | 5-(4-{[(1S)-1-(piperidin-4-yl)ethyl]amino}-1H-indazol-6-yl)-1,3,4-oxadiazol-2(3H)-one dihydrochloride |
| 101 | 1 | 5-[4-chloro-3-{[(1S)-1-(piperidin-4-yl)ethyl]amino}-5-(trifluoromethyl)phenyl]-1,3,4-oxadiazol-2(3H)-one dihydrochloride |
| 102 | 1 | 5-(4-chloro-3-cyclopropy1-5-{[(1S)-1-(piperidin-4-yl)ethyl]amino}phenyl)-1,3,4-oxadiazol-2(3H)-one hydrochloride |
| 103 | 1 | 5-(3-chloro-5-{[(1S)-1-(piperidin-4-yl)ethyl]amino}-4-[(propan-2-yl)oxy]phenyl)-1,3,4-oxadiazol-2(3H)-one hydrochloride |
| 104 | 1 | 5-(3-chloro-4-methoxy-5-{[(1S)-1-(piperidin-4-yl)ethyl]amino}phenyl)-1,3,4-oxadiazol-2(3H)-one hydrochloride |
| 105 | 1 | 5-(3-chloro-5-{[(1S)-1-(piperidin-4-yl)ethyl]amino}phenyl)-1,3,4-oxadiazol-2(3H)-one dihydrochloride |
| 106 | 1 | 5-[3-({(1S)-1-[(1r,4S)-4-aminocyclohexyl]ethyl}amino)-4-(trifluoromethyl)phenyl]-1,3,4-oxadiazol-2(3H)-one hydrochloride |
| 107 | 1 | 5-[3-(pentafluoro-λ⁶-sulfanyl)-5-{[(1S)-1-(piperidin-4-yl)ethyl]amino}phenyl]-1,3,4-oxadiazol-2(3H)-one hydrochloride |

**[Table 28**

| Ex | PEx | Chemical Name |
|---|---|---|
| 108 | 1 | 5-[3-{[(4-fluoropiperidin-4-yl)methyl]amino}-4-(trifluoromethyl)phenyl]-1,3,4-oxadiazol-2(3H)-one hydrochloride |
| 109 | 1 | 5-[3-{[(3-fluoropiperidin-3-yl)methyl]amino}-4-(trifluoromethyl)phenyl]-1,3,4-oxadiazol-2(3H)-one hydrochloride |
| 110 | 1 | 2-chloro-5-(5-oxo-4,5-dihydro-1,3,4-oxadiazol-2-yl)-3-{[(1S)-1-(piperidin-4-yl)ethyl]amino}benzonitrile hydrochloride |
| 111 | 1 | 5-[3-({(1R)-1-[(1r,4R)-4-aminocyclohexyl]ethyl}amino)-4-(trifluoromethyl)phenyl]-1,3,4-oxadiazol-2(3H)-one hydrochloride |
| 112 | 1 | 5-(2-{[(1S)-1-(piperidin-4-yl)ethyl]amino}pyridin-4-yl)-1,3,4-oxadiazol-2(3H)-one dihydrochloride |
| 113 | 44 | 5-[3-{[(1R)-2,2-difluoro-1-(piperidin-4-yl)ethyl]amino}-4-(trifluoromethyl)phenyl] -1,3,4-oxadiazol-2(3 H)-one hydrochloride |
| 114 | 1 | 5-[6-{[(1S)-1-(piperidin-4-yl)ethyl]amino}-5-(trifluoromethyl)pyridin-2-yl]-1,3,4-oxadiazol-2(3H)-one hydrochloride hydrochloride |
| 115 | 1 | 5-[3-{[1-(4-methylpiperidin-4-yl)ethyl]amino)-4-(trifluoromethyl)phenyl]-1,3,4-oxadiazol-2(3H)-one hydrochloride |
| 116 | 1 | 5-(4-{[(4-fluoropiperidin-4-yl)methyl]amino}-1H-indazol-6-yl)-1,3,4-oxadiazol-2(3H)-one hydrochloride |
| 117 | 1 | 5-[3-({2-[(1r,4r)-4-aminocyclohexyl]ethyl}amino)-4-(trifluoromethyl)phenyl]-1,3,4-oxadiazol-2(3H)-one hydrochloride |
| 118 | 7 | tert-butyl (1r,4r)-4-{[5-(5-oxo-4,5-dihydro-1,3,4-oxadiazol-2-yl)-2-(trifluoromethyl)anilino]methyl}cyclohexane-1-carboxylate |
| 119 | 119 | (1r,4r)-4-{[5-(5-oxo-4,5-dihydro-1,3,4-oxadiazol-2-yl)-2-(trifluoromethyl)anilino]methyl}cyclohexane-1-carboxylic acid |
| 120 | 120 | 5-[3-({[(1r,4r)-4-(hydroxymethyl)cyclohexyl]methyl}amino)-4-(trifluoromethyl)phenyl]-1,3,4-oxadiazol-2(3H)-one |

**[Table 29]**

| Ex | PEx | Chemical Name |
|---|---|---|
| 121 | 121 | (1r,4r)-4-{[5-(5-oxo-4,5-dihydro-1,3,4-oxadiazol-2-yl)-2-(trifluoromethyl)anilino]methyl}cyclohexane-1-carboxamide |
| 122 | 1 | 5-[3-{[(4-ethylpiperidin-4-yl)methyl]amino}-4-(trifluoromethyl)phenyl]-1,3,4-oxadiazol-2(3H)-one hydrochloride |
| 123 | 1 | 5-(4-chloro-3-fluoro-5-{[(4-fluoropiperidin-4-yl)methyl]amino}phenyl)-1,3,4-oxadiazol-2(3H)-one hydrochloride |
| 124 | 1 | 5-{4-bromo-1-[(piperidin-4-yl)methyl]-1H-indazol-6-yl}-1,3,4-oxadiazol-2(3H)-one hydrochloride |
| 125 | 1 | 5-[3-{[(1S)-1-(4-fluoropiperidin-4-yl)ethyl]amino}-4-(trifluoromethyl)phenyl]-1,3,4-oxadiazol-2(3H)-one hydrochloride |
| 126 | 1 | 5-[3-({[(3S,4R)-3-fluoropiperidin-4-yl]methyl}amino)-4-(trifluoromethyl)phenyl]-1,3,4-oxadiazol-2(3H)-one hydrochloride |
| 127 | 1 | 5-[3-({[(3S,4S)-3-fluoropiperidin-4-yl]methyl}amino)-4-(trifluoromethyl)phenyl]-1,3,4-oxadiazol-2(3H)-one hydrochloride |
| 128 | 1 | 5-[4-(methanesulfonyl)-3-{[(1S)-1-(piperidin-4-yl)ethyl]amino}phenyl]-1,3,4-oxadiazol-2(3H)-one hydrochloride |
| 129 | 1 | 5-[3-fluoro-5-{[(4-fluoropiperidin-4-yl)methyl]amino}-4-(trifluoromethyl)phenyl]-1,3,4-oxadiazol-2(3H)-one hydrochloride |
| 130 | 1 | 5-{3-[{[(1r,4r)-4-aminocyclohexyl]methyl} (methyl)amino]-4-(trifluoromethyl)phenyl}-1,3,4-oxadiazol-2(3H)-one hydrochloride |
| 131 | 1 | 5-[3-({[(1r,4r)-4-(methylamino)cyclohexyl]methyl}amino)-4-(trifluoromethyl)phenyl]-1,3,4-oxadiazol-2(3H)-one hydrochloride |
| 132 | 1 | 5-[3-(methyl{[(1r,4r)-4-(methylamino)cyclohexyl]methyl}amino)-4-(trifluoromethyl)phenyl]-1,3,4-oxadiazol-2(3H)-one hydrochloride |
| 133 | 1 | 5-[3-{[(3-azabicyclo[3.2.1]octan-8-yl)methyl]amino}-4-(trifluoromethyl)phenyl]-1,3,4-oxadiazol-2(3H)-one hydrochloride |

**[Table 30]**

| Ex | PEx | Chemical Name |
|---|---|---|
| 134 | 1 | 5-[3-({1-[(3S)-pyrrolidin-3-yl]propan-2-yl}amino)-4-(trifluoromethyl)phenyl]-1,3,4-oxadiazol-2(3H)-one hydrochloride |
| 135 | 1 | 5-[3-({[(1R,3s,5S)-8-azabicyclo[3.2.1]octan-3-yl]methyl}amino)-4-(trifluoromethyl)phenyl]-1,3,4-oxadiazol-2(3H)-one hydrochloride |
| 136 | 1 | 5-[3-({1-[(3R)-pyrrolidin-3-yl]propan-2-yl}amino)-4-(trifluoromethyl)phenyl]-1,3,4-oxadiazol-2(3H)-one hydrochloride |
| 137 | 1 | 5-[3-({(1S)-1-[(1R,3S)-3-amino-2,2-dimethylcyclobutyl]ethyl}amino)-4-(trifluoromethyl)phenyl]-1,3,4-oxadiazol-2(3H)-one hydrochloride |
| 138 | 1 | 5-[3-{[(1S)-1-(piperidin-4-yl)propyl]amino}-4-(trifluoromethyl)phenyl]-1,3,4-oxadiazol-2(3H)-one hydrochloride |
| 139 | 1 | 5-(4-chloro-3-fluoro-5-{[(1S)-1-(piperidin-4-yl)propyl]amino}phenyl)-1,3,4-oxadiazol-2(3H)-one hydrochloride |
| 140 | 1 | 5-[3- {[(1R)-2,2-difluoro-1-(4-fluoropiperidin-4-yl)ethyl]amino}-4-(trifluoromethyl)phenyl]-1,3,4-oxadiazol-2(3H)-one hydrochloride |
| 141 | 44 | 5-[3-({[(3R,4R)-3-methylpiperidin-4-yl]methyl}amino)-4-(trifluoromethyl)phenyl]-1,3,4-oxadiazol-2(3H)-one hydrochloride |
| 142 | 1 | 5-[3-{[(1S)-1-(7-azaspiro[3.5]nonan-2-yl)ethyl]amino}-4-(trifluoromethyl)phenyl]-1,3,4-oxadiazol-2(3H)-one hydrochloride |
| 143 | 44 | 5-[3-{[(2-methylpiperidin-4-yl)methyl]amino}-4-(trifluoromethyl)phenyl]-1,3,4-oxadiazol-2(3H)-one hydrochloride |
| 144 | 1 | 5-[3-{[(1S)-1-(4-methylpiperidin-4-yl)ethyl]amino}-4-(trifluoromethyl)phenyl]-1,3,4-oxadiazol-2(3H)-one hydrochloride |
| 145 | 1 | 5-[3-({(1S)-1-[(1s,3R)-3-aminocyclobutyl]ethyl}amino)-4-(trifluoromethyl)phenyl]-1,3,4-oxadiazol-2(3H)-one hydrochloride |
| 146 | 1 | 5-[4-(trifluoromethyl)-3-{[(1R)-2,2,2-trifluoro-1-(piperidin-4-yl)ethyl]amino}phenyl]-1,3,4-oxadiazol-2(3H)-one hydrochloride |

**[Table 31]**

| Ex | PEx | Chemical Name |
|---|---|---|
| 147 | 1 | 5-[4-(trifluoromethyl)-3-{[(1S)-2,2,2-trifluoro-1-(piperidin-4-yl)ethyl]amino}phenyl]-1,3,4-oxadiazol-2(3H)-one hydrochloride |
| 148 | 1 | 5-[3-({(1S)-1-[(1r,4S)-4-aminocyclohexyl]ethyl}amino)-4-chlorophenyl]-1,3,4-oxadiazol-2(3H)-one hydrochloride |
| 149 | 149 | 5-[3-{[(1S)-1-(2-azaspiro[3.3]heptan-6-yl)ethyl]amino}-4-(trifluoromethyl)phenyl]-1,3,4-oxadiazol-2(3H)-one trifluoroacetate |
| 150 | 1 | 5-[3-({(1S)-1-[(1R,3S)-3-amino-2,2-dimethylcyclobutyl]ethyl}amino)-5-fluoro-4-(trifluoromethyl)phenyl]-1,3,4-oxadiazol-2(3H)-one hydrochloride |
| 151 | 1 | 5-[3-({(1S)-1-[(1r,4S)-4-aminocyclohexyl]ethyl}amino)-5-fluoro-4-(trifluoromethyl)phenyl]-1,3,4-oxadiazol-2(3H)-one hydrochloride |
| 152 | 1 | 5-[3-{[(1R)-2-fluoro-1-(piperidin-4-yl)ethyl]amino}-4-(trifluoromethyl)phenyl]-1,3,4-oxadiazol-2(3H)-one hydrochloride |
| 153 | 1 | 5-[3-({(1S)-1-[(1S,3R)-3-amino-2,2-dimethylcyclobutyl]ethyl}amino)-4-(trifluoromethyl)phenyl]-1,3,4-oxadiazol-2(3H)-one hydrochloride |
| 154 | 35 | 5-[3-({(1S)-1-[(1r,4S)-4-aminocyclohexyl]ethyl}amino)-4-bromophenyl]-1,3,4-oxadiazol-2(3H)-one hydrochloride |
| 155 | 1 | 5-[3-({(1S)-1-[(1R,5S,8r)-3-azabicyclo[3.2.1]octan-8-yl]ethyl}amino)-4-(trifluoromethyl)phenyl]-1,3,4-oxadiazol-2(3H)-one hydrochloride |
| 156 | 1 | 5-[3-({(1S)-1-[(1R,5S,8s)-3-azabicyclo[3.2.1]octan-8-yl]ethyl}amino)-4-(trifluoromethyl)phenyl]-1,3,4-oxadiazol-2(3H)-one hydrochloride |
| 157 | 1 | 5-[3-{[(1S)-1-(azepan-4-yl)ethyl]amino}-4-(trifluoromethyl)phenyl]-1,3,4-oxadiazol-2(3H)-one hydrochloride |
| 158 | 1 | 5-[3-({(1S)-1-[(1R,3S)-3-amino-2,2-dimethylcyclobutyl]ethyl}amino)-4-chlorophenyl]-1,3,4-oxadiazol-2(3H)-one hydrochloride |

**[Table 32]**

| Ex | PEx | Chemical Name |
|---|---|---|
| 159 | 35 | 5-[3-fluoro-5-{[(1S)-1-(4-fluoropiperidin-4-yl)ethyl]amino}-4-(trifluoromethyl)phenyl]-1,3,4-oxadiazol-2(3H)-onehydrochloride |
| 160 | 35 | 5-[3-fluoro-5-{[(1R)-2-fluoro-1-(piperidin-4-yl)ethyl]amino}-4-(trifluoromethyl)phenyl]-1,3,4-oxadiazol-2(3H)-one hydrochloride |
| 161 | 1 | 5-[3-{2-[(2r,5r)-5-amino-1,3-dioxan-2-yl]cyclopropyl}-4-(trifluoromethyl)phenyl]-1,3,4-oxadiazol-2(3H)-one hydrochloride |
| 162 | 1 | 5-[3-{[(1S)-1-(azocan-5-yl)ethyl]amino}-4-(trifluoromethyl)phenyl]-1,3,4-oxadiazol-2(3H)-one hydrochloride |
| 163 | 1 | 5-[3-{ [(1R)-2,2-difluoro-1-(4-fluoropiperidin-4-yl)ethyl]amino}-5-fluoro-4-(trifluoromethyl)phenyl]-1,3,4-oxadiazol-2(3H)-one hydrochloride |
| 164 | 1 | 5-[3-({(1S)-1-[(1S,3R)-3-amino-2,2-dimethylcyclobutyl]ethyl}amino)-5-fluoro-4-(trifluoromethyl)phenyl]-1,3,4-oxadiazol-2(3H)-one hydrochloride |
| 165 | 35 | 5-[3-({(1S)-1-[(1S,3R)-3-aminocyclobutyl]ethyl}amino)-5-fluoro-4-(trifluoromethyl)phenyl]-1,3,4-oxadiazol-2(3H)-one hydrochloride |
| 166 | 1 | 5-[3-({[(1R,3S)-3-amino-2,2-dimethylcyclobutyl]methyl}amino)-4-(trifluoromethyl)phenyl]-1,3,4-oxadiazol-2(3H)-one hydrochloride |
| 167 | 1 | 5-[3-({(1S)-1-[(1r,4S)-4-aminocyclohexyl]ethyl}amino)-5-fluoro-4-methoxyphenyl]-1,3,4-oxadiazol-2(3H)-one hydrochloride |
| 168 | 1 | 5-[3-({(1S)-1-[(1S,3R)-3-amino-2,2-dimethylcyclobutyl]ethyl}amino)-4-chlorophenyl)-1,3,4-oxadiazol-2(3H)-one, hydrochloride |
| 169 | 1 | 5-[3-({(1S)-1-[(1r,4S)-4-aminocyclohexyl]ethyl}amino)-4-chloro-2,5-difluorophenyl]-1,3,4-oxadiazol-2(3H)-one hydrochloride |
| 170 | 35 | 5-[3-({(1S)-1-[(1r,4S)-4-aminocyclohexyl]ethyl}amino)-5-fluoro-4-(trifluoromethoxy)phenyl]-1,3,4-oxadiazol-2(3H)-one hydrochloride |

**[Table 33]**

| Ex | PEx | Chemical Name |
|---|---|---|
| 171 | 35 | 5-[3-({(1S)-1-[(1r,4S)-4-aminocyclohexyl]ethyl}amino)-5-bromo-4-chlorophenyl]-1,3,4-oxadiazol-2(3H)-one dihydrochloride |
| 172 | 1 | 5-[3-({(1S)-1-[(1r,4S)-4-aminocyclohexyl]ethyl}amino)-5-fluoro-4-methylphenyl]-1,3,4-oxadiazol-2(3H)-one dihydrochloride |
| 173 | 1 | 5-[3-({(1S)-1-[(1r,4S)-4-aminocyclohexyl]ethyl}amino)-4-ethoxy-5-fluorophenyl]-1,3,4-oxadiazol-2(3H)-one dihydrochloride |
| 174 | 35 | 5-[3-({(1S)-1-[(1r,4S)-4-aminocyclohexyl]ethyl}amino)-4,5-dichlorophenyl]-1,3,4-oxadiazol-2(3H)-one hydrochloride |
| 175 | 1 | 5-{3-({(1S)-1-[(1r,4S)-4-aminocyclohexyl]ethyl}amino)-5-fluoro-4-[(propan-2-yl)oxy]phenyl}-1,3,4-oxadiazol-2(3H)-one dihydrochloride |
| 176 | 1 | 5-[3-(([(1S,3R)-3-amino-2,2-dimethylcyclobutyl]methyl}amino)-4-(trifluoromethyl)phenyl]-1,3,4-oxadiazol-2(3H)-one hydrochloride |
| 177 | 35 | 5-[3-({(1S)-1-[(1r,4S)-4-aminocyclohexyl]ethyl}amino)-4-chloro-5-fluorophenyl]-1,3,4-oxadiazol-2(3H)-one hydrochloride |
| 178 | 1 | 5-[3-({(1S)-1-[(1s,3R)-3-aminocyclobutyl]ethyl}amino)-4-chloro-5-fluorophenyl]-1,3,4-oxadiazol-2(3H)-one hydrochloride |
| 179 | 1 | 5-[3-({(1S)-1-[(1s,3R)-3-aminocyclobutyl]ethyl}amino)-4,5-dichlorophenyl]-1,3,4-oxadiazol-2(3H)-one dihydrochloride |
| 180 | 35 | 5-[3-({(1S)-1-[(1s,3R)-3-aminocyclobutyl]ethyl}amino)-5-fluoro-4-(trifluoromethoxy)phenyl]-1,3,4-oxadiazol-2(3H)-one hydrochloride |
| 181 | 1 | 5-[3-({(1S)-1-[(1r,4S)-4-aminocyclohexyl]propyl}amino)-4-(trifluoromethyl)phenyl]-1,3,4-oxadiazol-2(3H)-one hydrochloride |
| 182 | 35 | 5-[3-({(1S)-1-[(1s,3R)-3-aminocyclobutyl]ethyl}amino)-5-bromo-4-chlorophenyl]-1,3,4-oxadiazol-2(3H)-one hydrochloride |
| 183 | 35 | 5-[3-({(1S)-1-[(1s,3R)-3-aminocyclobutyl]ethyl}amino)-4-chloro-5-(trifluoromethyl)phenyl]-1,3,4-oxadiazol-2(3H)-one hydrochloride |

**[Table 34]**

| Ex | PEx | Chemical Name |
|---|---|---|
| 184 | 7 | N-[(1S,3R)-2,2-dimethyl-3-{[5-(5-oxo-4,5-dihydro-1,3,4-oxadiazol-2-yl)-2-(trifluoromethyl)anilino]methyl}cyclobutyl]acetamide |
| 185 | 1 | 5-[3-({(1S)-1-[(1s,3R)-3-aminocyclobutyl]ethyl}amino)-4-chloro-5-(trifluoromethyl)phenyl]-1,3,4-oxadiazol-2(3H)-one hydrochloride |
| 186 | 1 | 5-[3-({(1S)-1-[(2S,3R)-4-aminocuban-1-yl]ethyl}amino)-4-(trifluoromethyl)phenyl]-1,3,4-oxadiazol-2(3H)-one hydrochloride |
| 187 | 1 | 5-[3-{[(1S)-1-(4-aminobicyclo[2.2.1]heptan-1-yl)ethyl]amino}-4-(trifluoromethyl)phenyl]-1,3,4-oxadiazol-2(3H)-one hydrochloride |
| 188 | 1 | 6-amino-3,7-anhydro-1,2,4,5,6-pentadeoxy-2-[5-(5-oxo-4,5-dihydro-1,3,4-oxadiazol-2-yl)-2-(trifluoromethyl)anilino]-L-arabino-heptitol hydrochloride hydrochloride |
| 189 | 1 | 5-[3-({(1S)-1-[(1r,4S)-4-(methylamino)cyclohexyl]ethyl}amino)-4-(trifluoromethyl)phenyl]-1,3,4-oxadiazol-2(3H)-one hydrochloride |
| 190 | 1 | 5-[3-fluoro-5-({(1S)-1-[(1r,4S)-4-(methylamino)cyclohexyl]ethyl}amino)-4-(trifluoromethyl)phenyl]-1,3,4-oxadiazol-2(3H)-one hydrochloride |
| 191 | 1 | 5-[3-({(1S)-1-[(1R,3S)-3-aminocyclohexyl]ethyl}amino)-4-(trifluoromethyl)phenyl]-1,3,4-oxadiazol-2(3H)-one hydrochloride |
| 192 | 1 | 5-[3-({(1S)-1-[(1s,3R)-3-(methylamino)cyclobutyl]ethyl}amino)-4-(trifluoromethyl)phenyl]-1,3,4-oxadiazol-2(3H)-one hydrochloride |
| 193 | 1 | 5-[3-({(1S)-1-[(1r,4S)-4-(ethylamino)cyclohexyl]ethyl}amino)-4-(trifluoromethyl)phenyl]-1,3,4-oxadiazol-2(3H)-one hydrochloride |
| 194 | 1 | 5-[3-({(1S)-1-[(1S,3R)-3-aminocyclohexyl]ethyl}amino)-4-(trifluoromethyl)phenyl]-1,3,4-oxadiazol-2(3H)-one hydrochloride |

**[Table 35]**

| Ex | PEx | Chemical Name |
|---|---|---|
| 195 | 1 | 5-[3-({(1S)-1-[(1s,3R)-3-(ethylamino)cyclobutyl]ethyl}amino)-4-(trifluoromethyl)phenyl]-1,3,4-oxadiazol-2(3H)-one hydrochloride |
| 196 | 1 | 5-[3-{[(1S)-1-{(1s,3R)-3-[(cyclopropylmethyl)amino]cyclobutyl} ethyl]amino}-4-(trifluoromethyl)phenyl]-1 ,3,4-oxadiazol-2(3H)-one hydrochloride |
| 197 | 1 | 5-[3-{[(1S)-1-{(1r,4S)-4-[(2,2,2-trifluoroethyl)amino]cyclohexyl}ethyl]amino}-4-(trifluoromethyl)phenyl]-1,3,4-oxadiazol-2(3H)-one hydrochloride |
| 198 | 35 | 5-[3-({(1S)-1-[(1r,4S)-4-aminocyclohexyl]propyl}amino)-5-fluoro-4-(trifluoromethyl)phenylj-1,3,4-oxadiazol-2(3H)-one hydrochloride |
| 199 | 1 | 5-[3-({(1S)-1-[(1S,3R)-3-aminocyclopentyl]ethyl}amino)-4-(trifluoromethyl)phenyl]-1,3,4-oxadiazol-2(3H)-one hydrochloride |
| 200 | 1 | 5-[3-({(1S)-1-[(1R,3S)-3-aminocyclopentyl]ethyl}amino)-4-(trifluoromethyl)phenyl]-1,3,4-oxadiazol-2(3H)-one hydrochloride |
| 201 | 1 | 5-[3-{ [(1S)-1-{(1r,4S)-4-[(2-hydroxyethyl)amino]cyclohexyl}ethyl]amino}-4-(trifluoromethyl)phenyl]-1,3,4-oxadiazol-2(3H)-one hydrochloride |
| 202 | 35 | 5-[3-fluoro-5-{[(1S)-1-(piperidin-4-yl)ethyl]amino}-4-(trifluoromethoxy)phenyl]-1,3,4-oxadiazol-2(3H)-one hydrochloride |
| 203 | 35 | 5-[3-fluoro-5-({(1S)-1-[(1s,3R)-3-(methylamino)cyclobutyl]ethyl}amino)-4-(trifluoromethyl)phenyl]-1,3,4-oxadiazol-2(3H)-one hydrochloride |
| 204 | 35 | 5-[3-fluoro-5-({(1S)-1-[(1r,4S)-4-(methylamino)cyclohexyl]ethyl} amino)-4-(trifluoromethoxy)phenyl]-1,3,4-oxadiazol-2(3H)-one hydrochloride |
| 205 | 1 | 5-(4-chloro-3-{[methyl(piperidin-4-yl)amino]methyl}phenyl)-1,3,4-oxadiazol-2(3H)-one dihydrochloride |

**[Table 36]**

| Ex | PEx | Chemical Name |
|---|---|---|
| 206 | 35 | 5-[3-fluoro-5-{[(1S)-1-(4-methylpiperidin-4-yl)ethyl]amino}-4-(trifluoromethyl)phenyl]-1,3,4-oxadiazol-2(3H)-one hydrochloride |
| 207 | 35 | 5-[3-fluoro-5-{[(1S)-1-(4-methylpiperidin-4-yl)ethyl]amino}-4-(trifluoromethoxy)phenyl]-1,3,4-oxadiazol-2(3H)-one hydrochloride |
| 208 | 35 | 5-[3-fluoro-5-({(1S)-1-[(1s,3R)-3-(methylamino)cyclobutyl]ethyl}amino)-4-(trifluoromethoxy)phenyl]-1,3,4-oxadiazol-2(3H)-one hydrochloride |
| 209 | 1 | 5-[3-{[(1S)-2-fluoro-1-(4-fluoropiperidin-4-yl)ethyl]amino}-4-(trifluoromethyl)phenyl]-1,3,4-oxadiazol-2(3H)-one hydrochloride |
| 210 | 35 | 5-[3-fluoro-5-({(1S)-1-[(3S,4S)-3-methylpiperidin-4-yl]ethyl}amino)-4-(trifluoromethyl)phenyl]-1,3,4-oxadiazol-2(3H)-one hydrochloride |
| 211 | 35 | 5-[3-fluoro-5-({(1S)-1-[(3R,4R)-3-methylpiperidin-4-yl]ethyl}amino)-4-(trifluoromethyl)phenyl]-1,3,4-oxadiazol-2(3H)-one hydrochloride |
| 212 | 1 | 5-[4-(difluoromethoxy)-3-fluoro-5-{[(1S)-1-(piperidin-4-yl)ethyl]amino}phenyl]-1,3,4-oxadiazol-2(3H)-one hydrochloride |
| 213 | 1 | 5-[4-(difluoromethoxy)-3-fluoro-5-{[(1S)-1-(4-methylpiperidin-4-yl)ethyl]amino}phenyl]-1,3,4-oxadiazol-2(3H)-one hydrochloride |
| 214 | 1 | 5-[4-(difluoromethoxy)-3-fluoro-5-({(1S)-1-[(3S,4S)-3-methylpiperidin-4-yl]ethyl}amino)phenyl]-1,3,4-oxadiazol-2(3H)-one hydrochloride |
| 215 | 215 | 5-(3-{[(1-acetylpiperidin-4-yl)(methyl)amino]methyl}-4-chlorophenyl)-1,3,4-oxadiazol-2(3H)-one hydrochloride |
| 216 | 1 | 5-[4-(difluoromethoxy)-3-fluoro-5-({(1S)-1-[(2R,4R)-2-methylpiperidin-4-yl]ethyl}amino)phenyl]-1,3,4-oxadiazol-2(3H)-one hydrochloride |

**[Table 37]**

| Ex | PEx | Chemical Name |
|---|---|---|
| 217 | 1 | 5-[3-({(1S)-1-[(3S,4S)-3-methylpiperidin-4-yl]ethyl}amino)-4-(trifluoromethyl)phenyl]-1,3,4-oxadiazol-2(3H)-one hydrochloride |
| 218 | 1 | 5-[3-({(1S)-1-[(3S,4S)-3-methylpiperidin-4-yl]ethyl}amino)-4-(trifluoromethoxy)phenyl]-1,3,4-oxadiazol-2(3H)-one hydrochloride |
| 219 | 35 | 5-[3-fluoro-5-({(1S)-1-[(3S,4S)-3-methylpiperidin-4-yl]ethyl}amino)-4-(trifluoromethoxy)phenyl]-1,3,4-oxadiazol-2(3H)-one hydrochloride |
| 220 | 1 | 5-[4-(difluoromethoxy)-3-({(1S)-1-[(3S,4S)-3-methylpiperidin-4-yl]ethyl}amino)phenyl]-1,3,4-oxadiazol-2(3H)-one hydrochloride |
| 221 | 35 | 5-[4-chloro-3-({(1S)-1-[(3S,4S)-3-methylpiperidin-4-yl]ethyl}amino)phenyl]-1,3,4-oxadiazol-2(3H)-one hydrochloride |
| 222 | 35 | 5-[4-chloro-3-fluoro-5-({(1S)-1-[(3S,4S)-3-methylpiperidin-4-yl]ethyl}amino)phenyl]-1,3,4-oxadiazol-2(3H)-one hydrochloride |
| 223 | 223 | 5-[3-fluoro-5-({(1S)-1-[(3S,4R)-3-fluoropiperidin-4-yl]ethyl}amino)-4-(trifluoromethyl)phenyl]-1,3,4-oxadiazol-2(3H)-one hydrochloride |
| 224 | 223 | 5-[3-fluoro-5-({(1S)-1-[(3S,4R)-3-fluoropiperidin-4-yl]ethyl}amino)-4-(trifluoromethoxy)phenyl]-1,3,4-oxadiazol-2(3H)-one hydrochloride |
| 225 | 223 | 5-[3-({(1S)-1-[(3S,4S)-3-ethylpiperidin-4-yl]ethyl}amino)-5-fluoro-4-(trifluoromethyl)phenyl]-1,3,4-oxadiazol-2(3H)-one hydrochloride |
| 226 | 223 | 5-[3-({(1S)-1-[(3R,4R)-3-ethylpiperidin-4-yl]ethyl}amino)-5-fluoro-4-(trifluoromethyl)phenyl]-1,3,4-oxadiazol-2(3H)-one hydrochloride |
| 227 | 1 | 5-[3-fluoro-5-({(1S)-1-[(3S,4R)-3-fluoropiperidin-4-yl]ethyl}amino)-4-methylphenyl]-1,3,4-oxadiazol-2(3H)-one hydrochloride |

**[Table 38]**

| Ex | PEx | Chemical Name |
|---|---|---|
| 228 | 1 | 5-[3-fluoro-4-methyl-5-({(1S)-1-[(3S,4S)-3-methylpiperidin-4-yl]ethyl}amino)phenyl]-1,3,4-oxadiazol-2(3H)-one hydrochloride |
| 229 | 1 | 5-[3-({(1S)-1-[(3S,4S)-3-ethylpiperidin-4-yl]ethyl}amino)-5-fluoro-4-methylphenyl]-1,3,4-oxadiazol-2(3H)-one hydrochloride |
| 230 | 1 | 5-[3-({(1S)-1-[(1r,4S)-4-aminocyclohexyl]ethyl}amino)-4-cyclopropyl-5-fluorophenyl]-1,3,4-oxadiazol-2(3H)-one hydrochloride |
| 231 | 231 | 5-[3-({(1S)-1-[(1r,4S)-4-aminocyclohexyl]ethyl}amino)-4-ethyl-5-fluorophenyl]-1,3,4-oxadiazol-2(3H)-one hydrochloride |
| 232 | 1 | 5-[3-({(1S)-1-[(1r,4S)-4-aminocyclohexyl]ethyl}amino)-5-fluoro-4-(prop-1-en-2-yl)phenyl]-1,3,4-oxadiazol-2(3H)-one hydrochloride |
| 233 | 233 | 5-[3-({(1S)-1-[(1r,4S)-4-aminocyclohexyl]ethyl}amino)-5-fluoro-4-(propan-2-yl)phenyl]-1,3,4-oxadiazol-2(3H)-one hydrochloride |
| 234 | 1 | 5-[3-({[(1r,4r)-4-aminocyclohexyl]methyl}sulfanyl)-4-(trifluoromethyl)phenyl]-1,3,4-oxadiazol-2(3H)-one hydrochloride |
| 235 | 1 | 5-{4-bromo-3-[(piperidin-4-yl)methoxy]phenyl}-1,3,4-oxadiazol-2(3H)-one hydrochloride |
| 236 | 1 | 5-{4-bromo-3-[1-(piperidin-4-yl)ethoxy]phenyl}-1,3,4-oxadiazol-2(3H)-one hydrochloride |
| 237 | 1 | 5-(3-{1-[(1r,4r)-4-aminocyclohexyl]ethoxy}-4-bromophenyl)-1 ,3,4-oxadiazol-2(3H)-one hydrochloride |
| 238 | 1 | 5-(3-{[(1r,4r)-4-aminocyclohexyl]methoxyl-4-bromophenyl)-1,3,4-oxadiazol-2(3H)-one hydrochloride |
| 239 | 1 | 5-{3-[1-(piperidin-4-yl)ethoxy]-4-(trifluoromethyl)phenyl}-1,3,4-oxadiazol-2(3H)-one hydrochloride |
| 240 | 1 | 5-[3- {[(1r,4r)-4-aminocyclohexyl]methoxy} -4-(trifluoromethyl)phenyl]-1,3,4-oxadiazol-2(3H)-one hydrochloride |

**[Table 39]**

| Ex | PEx | Chemical Name |
|---|---|---|
| 241 | 1 | 5-(3-{[(1r,4r)-4-aminocyclohexyl]methoxy}-4-chlorophenyl)-1,3,4-oxadiazol-2(3H)-one hydrochloride |
| 242 | 1 | 5-(4-chloro-3-{[(1s,3s)-3-(piperazin-1-yl)cyclobutyl]methoxy}phenyl)-1,3,4-oxadiazol-2(3H)-one dihydrochloride |
| 243 | 1 | 5-(3-{[(1s,4s)-4-aminocyclohexyl]methoxy} -4-chlorophenyl)-1,3 ,4-oxadiazol-2(3H)-one hydrochloride |
| 244 | 1 | 5-(1-{[(1r,4r)-4-aminocyclohexyl]methyl}-1,2,3,4-tetrahydroquinolin-7-yl)-1,3,4-oxadiazol-2(3H)-one dihydrochloride |
| 245 | 245 | 5-fluoro-1-{(1S)-1-[(3S,4S)-3-methylpiperidin-4-yl]ethyl}-7-(5-oxo-4,5-dihydro-1,3,4-oxadiazol-2-yl)-2,3-dihydroquinolin-4(1H)-one hydrochloride |
| 246 | 246 | 5-[(4E)-5-fluoro-4-(methoxyimino)-1-{(1S)-1-[(3S,4S)-3-methylpiperidin-4-yl]ethyl} -1 ,2,3,4-tetrahydroquinolin-7-yl]-1 ,3,4-oxadiazol-2(3H)-one hydrochloride |
| 247 | 1 | 5-(4-{(1S)-1-[(1r,4S)-4-aminocyclohexyl]ethyl}-3,4-dihydro-2H-1,4-benzoxazin-6-yl)-1,3,4-oxadiazol-2(3H)-one hydrochloride |
| 248 | 1 | 5-{8-fluoro-4-[(1S)-1-(piperidin-4-yl)ethyl]-3,4-dihydro-2H-1,4-benzoxazin-6-yl}-1,3,4-oxadiazol-2(3H)-one hydrochloride |
| 249 | 1 | 5-(4-{(1S)-1-[(1r,4S)-4-aminocyclohexyl]ethyl}-8-fluoro-3,4-dihydro-2H-1,4-benzoxazin-6-yl)-1,3,4-oxadiazol-2(3H)-one hydrochloride |
| 250 | 1 | 5-{(2R)-8-fluoro-2-methyl-4-[(1 S)-1-(piperidin-4-yl)ethyl]-3,4-dihydro-2H-1,4-benzoxazin-6-yl}-1,3,4-oxadiazol-2(3H)-one hydrochloride |
| 251 | 1 | 5-{(2S)-8-fluoro-2-methyl-4-[(1S)-1-(piperidin-4-yl)ethyl]-3,4-dihydro-2H-1,4-benzoxazin-6-yl}-1,3,4-oxadiazol-2(3H)-one hydrochloride |

**[Table 40]**

| Ex | PEx | Chemical Name |
|---|---|---|
| 252 | 252 | 5-[(2S)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one hydrochloride |
| 253 | 253 | 5-[(2R)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one hydrochloride |
| 254 | 1 | benzoxazepin-7-yl}-1,3,4-oxadiazol-2(3H)-one hydrochloride |

**[Table 41]**

| Example No. | Data |
|---|---|
| 1 | MS (ESI+) m/z: 317.3 (M+H)⁺ |
| 2 | MS (ESI+) m/z: 303.3 (M+H)⁺ |
| 3 | MS (ESI+) m/z: 331.2 (M+H)⁺ |
| 4 | MS (ESI+) m/z: 345.1 (M+H)⁺ |
| 5 | MS (ESI+) m/z: 345.1 (M+H)⁺ |
| | Elemental analysis value as C₁₅H₁₉F₃N₄O₂•HCl |
| | Calculated (%) C: 47.31 H: 5.29 N: 14.71 |
| | Found (%) C: 47.17 H: 5.20 N: 14.58 |
| 6 | MS (ESI+) m/z: 309.2 (M+H)⁺ |
| 7 | MS (ESI+) m/z: 381.2 (M+H)⁺ |
| 8 | MS (ESI+) m/z: 281.2 (M+H)⁺ |
| 9 | MS (ESI+) m/z: 329.3 (M+H)⁺ |
| 10 | MS (ESI+) m/z: 357.3 (M+H)⁺ |
| 11 | MS (ESI+) m/z: 357.3 (M+H)⁺ |
| 12 | MS (ESI+) m/z: 329.7 (M+H)⁺ |
| 13 | MS (ESI+) m/z: 385.2 (M+H)⁺ |
| 14 | MS (ESI+) m/z: 373.3 (M+H)⁺ |
| 15 | MS (ESI+) m/z: 383.6 (M+H)⁺ |
| | Elemental analysis value as C₁₈H₂₁F₃N₄O₂•HCl |
| | Calculated (%) C: 51.62 H: 5.29 N: 13.38 |
| | Found (%) C: 51.47 H: 5.13 N: 13.27 |

**[Table 42]**

| Example No. | Data |
|---|---|
| 16 | MS (ESI+) m/z: 355.2 (M+H)⁺ |
| 17 | MS (ESI+) m/z: 400.4 (M-H)⁻ |
| 18 | MS (ESI+) m/z: 302.2 (M+H)⁺ |
| 19 | MS (ESI+) m/z: 436.2 (M+H)⁺ |
| 20 | MS (ESI+) m/z: 336.2 (M+H)⁺ |
| 21 | MS (ESI+) m/z: 336.2 (M+H)⁺ |
| 22 | MS (ESI+) m/z: 350.2 (M+H)⁺ |
| 23 | MS (ESI+) m/z: 420.1 (M+H)⁺ |
| 24 | MS (ESI+) m/z: 380.1 (M+H)⁺ |
| 25 | MS (ESI+) m/z: 392.3 (M+H)⁺ |
| 26 | MS (ESI+) m/z: 342.6 (M+H)⁺ |
| | Elemental analysis value as C₁₆H₁₈F₃N₃O₂•HCl |
| | Calculated (%) C: 50.87 H: 5.07 N: 11.12 |
| | Found (%) C: 50.93 H: 5.18 N: 11.04 |
| 27 | MS (ESI+) m/z: 356.2 (M+H)⁺ |
| 28 | MS (ESI+) m/z: 360.1 (M+H)⁺ |
| 29 | MS (ESI+) m/z: 338.6 (M+H)⁺ |
| | Elemental analysis value as C₁₆H₁₄F₃N₃O₂•HCl + 0.7H₂O |
| | Calculated (%) C: 49.74 H: 4.28 N: 10.88 |
| | Found (%) C: 49.70 H: 3.92 N: 10.94 |
| 30 | MS (ESI+) m/z: 340.5 (M+H)⁺ |
| 31 | MS (ESI+) m/z: 295.2 (M+H)⁺ |
| 32 | MS (ESI+) m/z: 309.2 (M+H)⁺ |
| 33 | MS (ESI+) m/z: 309.2 (M+H)⁺ |
| 34 | MS (ESI+) m/z: 343.3 (M+H)⁺ |
| 35 | MS (ESI+) m/z: 353.1 (M+H)⁺ |
| 36 | MS (ESI+) m/z: 361.3 (M+H)⁺ |
| 37 | MS (ESI+) m/z: 357.3 (M+H)⁺ |
| 38 | MS (ESI+) m/z: 359.3 (M+H)⁺ |
| 39 | MS (ESI+) m/z: 371.3 (M+H)⁺ |
| 40 | MS (ESI+) m/z: 327.1 (M+H)⁺ |
| | Elemental analysis value as C₁₄H₁₆ClFN₄O₂•HCl + 1.7H₂O |
| | Calculated (%) C: 42.70 H: 5.22 N: 14.23 |
| | Found (%) C: 42.92 H: 5.00 N: 14.31 |

**[Table 43]**

| Example No. | Data |
|---|---|
| 41 | MS (ESI+) m/z: 349.5 (M-H)⁻ |
| 42 | MS (ESI+) m/z: 355.1 (M+H)⁺ |
| 43 | MS (ESI+) m/z: 355.6 (M+H)⁺ |
| 44 | MS (ESI+) m/z: 371.6 (M+H)⁺ |
| 45 | MS (ESI+) m/z: 369.2 (M+H)⁺ |
| 46 | MS (ESI+) m/z: 369.2 (M+H)⁺ |
| 47 | MS (ESI+) m/z: 309.2 (M+H)⁺ |
| 48 | MS (ESI+) m/z: 343.2 (M+H)⁺ |
| 49 | MS (ESI+) m/z: 295.2 (M+H)⁺ |
| 50 | MS (ESI+) m/z: 353.2 (M+H)⁺ |
| 51 | MS (ESI+) m/z: 357.3 (M+H)⁺ |
| 52 | MS (ESI+) m/z: 357.3 (M+H)⁺ |
| 53 | MS (ESI+) m/z: 343.3 (M+H)⁺ |
| 54 | MS (ESI+) m/z: 343.3 (M+H)⁺ |
| 55 | MS (ESI+) m/z: 357.3 (M+H)⁺ |
| | Elemental analysis value as C₁₆H₁₉F₃N₄O₂.HCl + 0.4H₂O |
| | Calculated (%) C: 48.04 H: 5.24 N: 14.01 |
| | Found (%) C: 48.05 H: 5.26 N: 14.39 |
| 56 | MS (ESI+) m/z: 371.3 (M+H)⁺ |
| 57 | MS (ESI+) m/z: 357.3 (M+H)⁺ |
| 58 | MS (ESI+) m/z: 357.7 (M+H)⁺ |
| | Elemental analysis value as C₁₆H₁₉F₃N₄O₂•HCl + 0.3H₂O |
| | Calculated (%) C: 48.26 H: 5.21 N: 14.07 |
| | Found (%) C: 48.02 H: 5.00 N: 14.07 |
| 59 | MS (ESI+) m/z: 357.3 (M+H)⁺ |
| 60 | MS (ESI+) m/z: 357.3 (M+H)⁺ |
| 61 | MS (ESI+) m/z: 323.3 (M+H)⁺ |
| 62 | MS (ESI+) m/z: 289.3 (M+H)⁺ |
| 63 | MS (ESI+) m/z: 371.3 (M+H)⁺ |
| 64 | MS (ESI+) m/z: 357.3 (M+H)⁺ |
| 65 | MS (ESI+) m/z: 358.3 (M+H)⁺ |
| 66 | MS (ESI+) m/z: 373.3 (M+H)⁺ |

**[Table 44]**

| Example No. | Data |
|---|---|
| 67 | MS (ESI+) m/z: 303.3 (M+H)⁺ |
| 68 | MS (ESI+) m/z: 367.2 (M+H)⁺ |
| 69 | MS (ESI+) m/z: 375.3 (M+H)⁺ |
| 70 | MS (ESI+) m/z: 371.3 (M+H)⁺ |
| 71 | MS (ESI+) m/z: 365.3 (M+H)⁺ |
| 72 | MS (ESI+) m/z: 372.3 (M+H)⁺ |
| 73 | MS (ESI+) m/z: 372.3 (M+H)⁺ |
| 74 | MS (ESI+) m/z: 357.3 (M+H)⁺ |
| 75 | MS (ESI+) m/z: 375.5 (M+H)⁺ |
| | Elemental analysis value as C₁₆H₁₈F₄N₄O₂•HCl |
| | Calculated (%) C: 46.78 H: 4.66 N: 13.64 |
| | Found (%) C: 46.53 H: 4.85 N: 13.58 |
| 76 | MS (ESI+) m/z: 341.1 (M+H)⁺ |
| | Elemental analysis value as C₁₅H₁₈ClFN₄O₂•HCl + 2.5H₂O |
| | Calculated (%) C: 42.66 H: 5.73 N: 13.27 |
| | Found (%) C: 42.86 H: 5.54 N: 13.39 |
| 77 | MS (ESI+) m/z: 323.1 (M+H)⁺ |
| 78 | MS (ESI+) m/z: 367.1 (M+H)⁺ |
| | Elemental analysis value as C₁₅H₁₉BrN₄O₂•2HCl + H₂O |
| | Calculated (%) C: 39.32 H: 5.06 N: 12.23 |
| | Found (%) C: 39.04 H: 4.86 N: 12.11 |
| 79 | MS (ESI+) m/z: 357.6 (M+H)⁺ |
| 80 | MS (ESI+) m/z: 307.6 (M+H)⁺ |
| | Elemental analysis value as C₁₅H₁₉FN₄O₂•HCl + 2.8H₂O |
| | Calculated (%) C: 45.81 H: 6.56 N: 14.25 |
| | Found (%) C: 45.89 H: 6.28 N: 13.92 |
| 81 | MS (ESI+) m/z: 371.6 (M+H)⁺ |
| | Elemental analysis value as C₁₇H₂₁F₃N₄O₂•HCl + 0.8H₂O |
| | Calculated (%) C: 48.47 H: 5.65 N: 13.30 |
| | Found (%) C: 48.27 H: 5.26 N: 13.26 |
| 82 | MS (ESI+) m/z: 319.3 (M+H)⁺ |
| | Elemental analysis value as C₁₆H₂₂N₄O₃•2HCl + 0.9H₂O |
| | Calculated (%) C: 47.16 H: 6.38 N: 13.75 |
| | Found (%) C: 47.30 H: 6.66 N: 13.94 |
| 83 | MS (ESI+) m/z: 385.2 (M+H)⁺ |
| 84 | MS (ESI+) m/z: 337.2 (M+H)⁺ |
| 85 | MS (ESI+) m/z: 314.2 (M+H)⁺ |
| 86 | MS (ESI+) m/z: 341.6 (M+H)⁺ |

**[Table 45]**

| Example No. | Data |
|---|---|
| 87 | MS (ESI+) m/z: 375.6 (M+H)⁺ |
| | Elemental analysis value as C₁₅H₁₈ClFN₄O₂•HCl + 0.3H₂O |
| | Calculated (%) C: 43.19 H: 4.49 N: 13.43 |
| | Found (%) C: 42.82 H: 4.09 N: 13.31 |
| 88 | MS (ESI+) m/z: 359.6 (M+H)⁺ |
| 89 | MS (ESI+) m/z: 325.6 (M+H)⁺ |
| | Elemental analysis value as C₁₅H₁₈F₂N₄O₂•HCl + 1.5H₂O |
| | Calculated (%) C: 46.46 H: 5.72 N: 14.45 |
| | Found (%) C: 46.18 H: 5.64 N: 14.12 |
| 90 | MS (ESI+) m/z: 339.6 (M+H)⁺ |
| 91 | MS (ESI+) m/z: 368.2 (M+H)⁺ |
| 92 | MS (ESI+) m/z: 338.7 (M+H)⁺ |
| 93 | MS (ESI+) m/z: 371.6 (M+H)⁺ |
| | Elemental analysis value as C₁₆H₂₀Cl₂N₄O₂•HCl + 1.2H₂O |
| | Calculated (%) C: 44.76 H: 5.49 N: 13.05 |
| | Found (%) C: 44.82 H: 5.70 N: 13.08 |
| 94 | MS (ESI+) m/z: 337.7 (M+H)⁺ |
| | Elemental analysis value as C₁₆H₂₁ClN₄O₂•HCl + 1.8H₂O |
| | Calculated (%) C: 47.37 H: 6.36 N: 13.81 |
| | Found (%) C: 47.55 H: 6.64 N: 13.89 |
| 95 | MS (ESI+) m/z: 341.6 (M+H)⁺ |
| | Elemental analysis value as C₁₅H₁₈ClFN₄O₂•HCl + 0.5H₂O |
| | Calculated (%) C: 46.64 H: 5.22 N: 14.51 |
| | Found (%) C: 46.47 H: 5.07 N: 14.37 |
| 96 | MS (ESI+) m/z: 357.6 (M+H)⁺ |
| | Elemental analysis value as C₁₅Hₗ₈Cl₂N₄O₂•HCl + 1.7H₂O |
| | Calculated (%) C: 42.46 H: 5.32 N: 13.20 |
| | Found (%) C: 42.61 H: 5.50 N: 13.05 |
| 97 | MS (ESI+) m/z: 401.6 (M+H)⁺ |
| | Elemental analysis value as C₁₅H₁₈BrClN₄O₂•2HCl + 0.3H₂O |
| | Calculated (%) C: 37.53 H: 4.33 N: 11.67 |
| | Found (%) C: 37.56 H: 4.46 N: 11.65 |
| 98 | MS (ESI+) m/z: 337.7 (M+H)⁺ |
| | Elemental analysis value as C₁₆H₂₁CN₄O₂•2HCl + H₂O |
| | Calculated (%) C: 44.93 H: 5.89 N: 13.10 |
| | Found (%) C: 45.15 H: 5.59 N: 13.10 |
| 99 | MS (ESI+) m/z: 321.7 (M+H)⁺ |
| | Elemental analysis value as C₁₆H₂₁FN₄O₂•2HCl + H₂O |
| | Calculated (%) C: 46.72 H: 6.13 N: 13.62 |
| | Found (%) C: 46.45 H: 5.84 N: 13.50 |
| 100 | MS (ESI+) m/z: 329.7 (M+H)⁺ |

**[Table 46]**

| Example No. | Data |
|---|---|
| 101 | MS (ESI+) m/z: 391.0 (M+H)⁺ |
| | Elemental analysis value as C₁₆H₁₈ClF₃N₄O₂•2HCl |
| | Calculated (%) C: 41.44 H: 4.35 N: 12.08 |
| | Found (%) C: 41.80 H: 4.48 N: 12.19 |
| 102 | MS (ESI+) m/z: 363.7 (M+H)⁺ |
| | Elemental analysis value as C₁₈H₂₃ClN₄O₂•HCl + 1.6H₂O |
| | Calculated (%) C: 50.50 H: 6.40 N: 13.09 |
| | Found (%) C: 50.53 H: 6.50 N: 13.08 |
| 103 | MS (ESI+) m/z: 381.7 (M+H)⁺ |
| | Elemental analysis value as C₁₈H₂₅ClN₄O₃•HCl + H₂O |
| | Calculated (%) C: 49.66 H: 6.48 N: 12.87 |
| | Found (%) C: 49.86 H: 6.62 N: 12.74 |
| 104 | MS (ESI+) m/z: 353.6 (M+H)⁺ |
| | Elemental analysis value as C₁₆H₂₁ClN₄O₃•HCl + 1.5H₂O |
| | Calculated (%) C: 46.16 H: 6.05 N: 13.46 |
| | Found (%) C: 46.06 H: 6.39 N: 13.13 |
| 105 | MS (ESI+) m/z: 323.6 (M+H)⁺ |
| 106 | MS (ESI+) m/z: 371.3 (M+H)⁺ |
| 107 | MS (ESI+) m/z: 415.6 (M+H)⁺ |
| | Elemental analysis value as C₁₅H₁₉F₅N₄O₂S•HCl + 1.3H₂O |
| | Calculated (%) C: 37.99 H: 4.80 N: 11.81 |
| | Found (%) C: 38.18 H: 4.75 N: 11.60 |
| 108 | MS (ESI+) m/z: 361.6 (M+H)⁺ |
| | Elemental analysis value as C₁₅H₁₆F₄N₄O₂•HCl + 0.2H₂O |
| | Calculated (%) C: 45.00 H: 4.38 N: 13.99 |
| | Found (%) C: 44.86 H: 4.06 N: 13.84 |
| 109 | MS (ESI+) m/z: 361.6 (M+H)⁺ |
| | Elemental analysis value as C₁₅H₁₆F₄N₄O₂•HCl + 2.4H₂O |
| | Calculated (%) C: 40.95 H: 4.99 N: 12.73 |
| | Found (%) C: 41.09 H: 4.89 N: 12.53 |
| 110 | MS (ESI+) m/z: 348.2 (M+H)⁺ |
| 111 | MS (ESI+) m/z: 371.3 (M+H)⁺ |
| 112 | MS (ESI+) m/z: 290.2 (M+H)⁺ |
| | Elemental analysis value as C₁₄H₁₉N₅O₂•2HCl + H₂O |
| | Calculated (%) C: 44.32 H: 6.10 N: 18.42 |
| | Found (%) C: 44.40 H: 6.28 N: 18.38 |
| 113 | MS (ESI+) m/z: 393.3 (M+H)⁺ |
| | Elemental analysis value as C₁₆H₁₇F₅N₄O₂•HCl + 0.7H₂O |
| | Calculated (%) C: 43.54 H: 4.43 N: 12.69 |
| | Found (%) C: 43.57 H: 4.20 N: 12.87 |

**[Table 47]**

| Example No. | Data |
|---|---|
| 114 | MS (ESI+) m/z: 358.1 (M+H)⁺ |
| 115 | MS (ESI+) m/z: 371.7 (M+H)⁺ |
| | Elemental analysis value as C₁₇H₂₁F₃N₄O₂•HCl + 0.7H₂O |
| | Calculated (%) C: 48.68 H: 5.62 N: 13.36 |
| | Found (%) C: 48.90 H: 6.01 N: 13.33 |
| 116 | MS (ESI+) m/z: 333.1 (M+H)⁺ |
| 117 | MS (ESI+) m/z: 371.6 (M+H)⁺ |
| 118 | MS (ESI+) m/z: 442.7 (M+H)⁺ |
| 119 | MS (ESI+) m/z: 386.6 (M+H)⁺ |
| 120 | MS (ESI+) m/z: 372.6 (M+H)⁺ |
| 121 | MS (ESI+) m/z: 385.6 (M+H)⁺ |
| 122 | MS (ESI+) m/z: 371.1 (M+H)⁺ |
| 123 | MS (ESI+) m/z: 345.5 (M+H)⁺ |
| | Elemental analysis value as C₁₄H₁₅ClF₂N₄O₂•HCl + 0.4H₂O |
| | Calculated (%) C: 43.29 H: 4.36 N: 14.42 |
| | Found (%) C: 43.55 H: 4.25 N: 14.50 |
| 124 | MS (ESI+) m/z: 378.5 (M+H)⁺ |
| 125 | MS (ESI+) m/z: 375.6 (M+H)⁺ |
| | Elemental analysis value as C₁₆H₁₈F₄N₄O₂•HCl + 0.1H₂O |
| | Calculated (%) C: 46.58 H: 4.69 N: 13.58 |
| | Found (%) C: 46.43 H: 4.71 N: 13.64 |
| 126 | MS (ESI+) m/z: 361.6 (M+H)⁺ |
| 127 | MS (ESI+) m/z: 361.6 (M+H)⁺ |
| | Elemental analysis value as C₁₅H₁₆F₄N₄O₂•HCl + 0.9H₂O |
| | Calculated (%) C: 43.62 H: 4.59 N: 13.57 |
| | Found (%) C: 43.91 H: 4.52 N: 13.31 |
| 128 | MS (ESI+) m/z: 367.1 (M+H)⁺ |
| 129 | MS (ESI+) m/z: 379.1 (M+H)⁺ |
| | Elemental analysis value as C₁₅H₁₅F₅N₄O₂•HCl + 0.2H₂O |
| | Calculated (%) C: 43.06 H: 3.95 N: 13.39 |
| | Found (%) C: 43.02 H: 3.78 N: 13.43 |
| 130 | MS (ESI+) m/z: 371.6 (M+H)⁺ |
| 131 | MS (ESI+) m/z: 371.2 (M+H)⁺ |
| | Elemental analysis value as C₁₇H₂₁F₄N₄O₂•HCl |
| | Calculated (%) C: 50.19 H: 5.45 N: 13.77 |
| | Found (%) C: 50.22 H: 5.27 N: 13.59 |

**[Table 48]**

| Example No. | Data |
|---|---|
| 132 | MS (ESI+) m/z: 385.6 (M+H)⁺ |
| | Elemental analysis value as C₁₈H₂₃F₄N₄O₂•HCl |
| | Calculated (%) C: 51.37 H: 5.75 N: 13.31 |
| | Found (%) C: 51.09 H: 5.39 N: 13.22 |
| 133 | MS (ESI+) m/z: 369.6 (M+H)⁺ |
| 134 | MS (ESI+) m/z: 357.1 (M+H)⁺ |
| 135 | MS (ESI+) m/z: 369.6 (M+H)⁺ |
| 136 | MS (ESI+) m/z: 357.1 (M+H)⁺ |
| 137 | MS (ESI+) m/z: 371.6 (M+H)⁺ |
| | Elemental analysis value as C₁₇H₂₁F₃N₄O₂•HCl + 0.7H₂O |
| | Calculated (%) C: 48.68 H: 5.62 N: 13.36 |
| | Found (%) C: 48.93 H: 5.62 N: 12.98 |
| 138 | MS (ESI+) m/z: 371.6 (M+H)⁺ |
| | Elemental analysis value as C₁₇H₂₁F₃N₄O₂•HCl + 0.5H₂O |
| | Calculated (%) C: 49.10 H: 5.57 N: 13.47 |
| | Found (%) C: 49.40 H: 5.62 N: 13.07 |
| 139 | MS (ESI+) m/z: 355.6 (M+H)⁺ |
| 140 | MS (ESI+) m/z: 411.5 (M+H)⁺ |
| | Elemental analysis value as C₁₆H₁₆F₆N₄O₂•HCl + 0.3H₂O |
| | Calculated (%) C: 42.50 H: 3.92 N: 12.39 |
| | Found (%) C: 42.59 H: 4.20 N: 12.30 |
| 141 | MS (ESI+) m/z: 357.2 (M+H)⁺ |
| 142 | MS (ESI+) m/z: 397.6 (M+H)⁺ |
| 143 | MS (ESI+) m/z: 357.2 (M+H)⁺ |
| 144 | MS (ESI+) m/z: 371.6 (M+H)⁺ |
| 145 | MS (ESI+) m/z: 343.6 (M+H)⁺ |
| | Elemental analysis value as C₁₅H₁₇F₃N₄O₂•HCl + 0.4H₂O |
| | Calculated (%) C: 46.68 H: 4.91 N: 14.52 |
| | Found (%) C: 46.78 H: 5.17 N: 14.25 |
| 146 | MS (ESI+) m/z: 411.6 (M+H)⁺ |
| 147 | MS (ESI+) m/z: 411.6 (M+H)⁺ |
| 148 | MS (ESI+) m/z: 337.3 (M+H)⁺ |
| 149 | MS (ESI+) m/z: 369.6 (M+H)⁺ |
| 150 | MS (ESI+) m/z: 389.6 (M+H)⁺ |
| | Elemental analysis value as C₁₇H₂₀F₄N₄O₂•HCl + 1.16H₂O |
| | Calculated (%) C: 45.81 H: 5.27 N: 12.57 |
| | Found (%) C: 46.12 H: 5.55 N: 12.17 |

**[Table 49]**

| Example No. | Data |
|---|---|
| 151 | MS (ESI+) m/z: 389.2 (M+H)⁺ |
| | Elemental analysis value as C₁₇H₂₀F₄N₄O₂•HCl + 0.2H₂O |
| | Calculated (%) C: 47.66 H: 5.03 N: 13.08 |
| | Found (%) C: 47.67 H: 4.84 N: 12.78 |
| 152 | MS (ESI+) m/z: 375.3 (M+H)⁺ |
| | Elemental analysis value as C₁₆H₁₈F₄N₄O₂•HCl |
| | Calculated (%) C: 46.78 H: 4.66 N: 13.64 |
| | Found (%) C: 46.91 H: 4.68 N: 13.67 |
| 153 | MS (ESI+) m/z: 371.2 (M+H)⁺ |
| | Elemental analysis value as C₁₇H₂₁F₃N₄O₂•HCl + H₂O |
| | Calculated (%) C: 48.06 H: 5.69 N: 13.19 |
| | Found (%) C: 48.22 H: 5.88 N: 13.09 |
| 154 | MS (ESI+) m/z: 381.1 (M+H)⁺ |
| | Elemental analysis value as C₁₆H₂₁BrN₄O₂•2HCl + 0.1H₂O |
| | Calculated (%) C: 42.14 H: 5.13 N: 12.29 |
| | Found (%) C: 42.35 H: 5.22 N: 12.02 |
| 155 | MS (ESI+) m/z: 383.6 (M+H)⁺ |
| 156 | MS (ESI+) m/z: 383.2 (M+H)⁺ |
| | Elemental analysis value as C₁₈H₂₁F₃N₄O₂•HCl + 0.9H₂O |
| | Calculated (%) C: 49.69 H: 5.51 N: 12.88 |
| | Found (%) C: 49.95 H: 5.78 N: 12.55 |
| 157 | MS (ESI+) m/z: 371.6 (M+H)⁺ |
| | Elemental analysis value as C₁₇H₂₁F₃N₄O₂•HCl + 0.7H₂O |
| | Calculated (%) C: 48.68 H: 5.62 N: 13.36 |
| | Found (%) C: 48.72 H: 5.55 N: 13.09 |
| 158 | MS (ESI+) m/z: 337.5 (M+H)⁺ |
| | Elemental analysis value as C₁₆H₂₁ClN₄O₂•HCl + 2H₂O |
| | Calculated (%) C: 46.95 H: 6.40 N: 13.69 |
| | Found (%) C: 46.74 H: 6.33 N: 13.40 |
| 159 | MS (ESI+) m/z: 393.5 (M+H)⁺ |
| | Elemental analysis value as C₁₆H₁₇F₅N₄O₂•HCl + 0.5H₂O |
| | Calculated (%) C: 43.90 H: 4.37 N: 12.80 |
| | Found (%) C: 43.95 H: 4.73 N: 12.70 |
| 160 | MS (ESI+) m/z: 393.6 (M+H)⁺ |
| 161 | MS (ESI+) m/z: 372.7 (M+H)⁺ |
| 162 | MS (ESI+) m/z: 385.6 (M+H)⁺ |
| | Elemental analysis value as C₁₈H₂₃F₄N₄O₂•HCl+ 0.6H₂O |
| | Calculated (%) C: 50.08 H: 5.88 N: 12.98 |
| | Found (%) C: 50.06 H: 6.01 N: 12.94 |
| 163 | MS (ESI+) m/z: 429.2 (M+H)⁺ |

**[Table 50]**

| Example No. | Data |
|---|---|
| 164 | MS (ESI+) m/z: 389.6 (M+H)⁺ |
| | Elemental analysis value as C₁₇H₂₀F₄N₄O₂•HCl + 0.7H₂O |
| | Calculated (%) C: 46.68 H: 5.16 N: 12.81 |
| | Found (%) C: 46.85 H: 5.46 N: 12.69 |
| 165 | MS (ESI+) m/z: 361.1 (M+H)⁺ |
| | Elemental analysis value as C₁₅H₁₆F₄N₄O₂•HCl |
| | Calculated (%) C: 45.41 H: 4.32 N: 14.12 |
| | Found (%) C: 45.52 H: 4.40 N: 13.90 |
| 166 | MS (ESI+) m/z: 357.2 (M+H)⁺ |
| | Elemental analysis value as C₁₆H₁₉F₃N₄O₂•HCl + H₂O |
| | Calculated (%) C: 46.78 H: 5.49 N: 13.64 |
| | Found (%) C: 46.70 H: 5.73 N: 13.76 |
| 167 | MS (ESI+) m/z: 351.6 (M+H)⁺ |
| 168 | MS (ESI+) m/z: 337.5 (M+H)⁺ |
| 169 | MS (ESI+) m/z: 373.5 (M+H)⁺ |
| 170 | MS (ESI+) m/z: 405.6 (M+H)⁺ |
| | Elemental analysis value as C₁₇H₂₀F₄N₄O₃•HCl + 0.6H₂O |
| | Calculated (%) C: 45.21 H: 4.95 N: 12.41 |
| | Found (%) C: 45.38 H: 4.87 N: 12.15 |
| 171 | MS (ESI+) m/z: 415.5 (M+H)⁺ |
| 172 | MS (ESI+) m/z: 335.6 (M+H)⁺ |
| | Elemental analysis value as C₁₇H₂₃FN₄O₂•2HCl |
| | Calculated (%) C: 50.13 H: 6.19 N: 13.76 |
| | Found (%) C: 50.41 H: 6.59 N: 13.49 |
| 173 | MS (ESI+) m/z: 365.6 (M+H)⁺ |
| 174 | MS (ESI+) m/z: 371.6 (M+H)⁺ |
| | Elemental analysis value as C₁₆H₂₀Cl₂N₄O₂•HCl + 0.4H₂O |
| | Calculated (%) C: 46.31 H: 5.30 N: 13.50 |
| | Found (%) C: 46.15 H: 5.28 N: 13.46 |
| 175 | MS (ESI+) m/z: 379.6 (M+H)⁺ |
| | Elemental analysis value as C₁₉H₂₇FN₄O₃•2HCl |
| | Calculated (%) C: 50.56 H: 6.48 N: 12.41 |
| | Found (%) C: 50.67 H: 6.55 N: 12.34 |
| 176 | MS (ESI+) m/z: 357.6 (M+H)⁺ |
| | Elemental analysis value as C₁₆H₁₉F₃N₄O₂•HCl + 1.2H₂O |
| | Calculated (%) C: 46.37 H: 5.45 N: 13.52 |
| | Found (%) C: 46.61 H: 5.66 N: 13.75 |
| 177 | MS (ESI+) m/z: 355.6 (M+H)⁺ |
| | Elemental analysis value as C₁₆H₂₀ClFN₄O₂•HCl + 0.8H₂O |
| | Calculated (%) C: 47.37 H: 5.62 N: 13.81 |
| | Found (%) C: 47.40 H: 5.54 N: 13.56 |

**[Table 51]**

| Example No. | Data |
|---|---|
| 178 | MS (ESI+) m/z: 327.5 (M+H)⁺ |
| | Elemental analysis value as C₁₄H₁₆ClFN₄O₂•HCl + 1.4H₂O |
| | Calculated (%) C: 43.29 H: 5.14 N: 14.42 |
| | Found (%) C: 43.32 H: 4.87 N: 14.39 |
| 179 | MS (ESI+) m/z: 343.5 (M+H)⁺ |
| 180 | MS (ESI+) m/z: 377.2 (M+H)⁺ |
| | Elemental analysis value as C₁₅H₁₆F₄N₄O₃•HCl + 0.8H₂O |
| | Calculated (%) C: 42.17 H: 4.39 N: 13.12 |
| | Found (%) C: 42.32 H: 4.37 N: 12.83 |
| 181 | MS (ESI+) m/z: 385.7 (M+H)⁺ |
| 182 | MS (ESI+) m/z: 387.1 (M+H)⁺ |
| | Elemental analysis value as C₁₄H₁₆BrClN₄O₂•HCl + H₂O |
| | Calculated (%) C: 38.03 H: 4.33 N: 12.67 |
| | Found (%) C: 38.07 H: 4.38 N: 12.61 |
| 183 | MS (ESI+) m/z: 377.1 (M+H)⁺ |
| | Elemental analysis value as C₁₅H₁₆ClF₃N₄O₂•HCl + H₂O |
| | Calculated (%) C: 41.78 H: 4.44 N: 12.99 |
| | Found (%) C: 41.98 H: 4.24 N: 13.01 |
| 184 | MS (ESI+) m/z: 399.3 (M+H)⁺ |
| 185 | MS (ESI+) m/z: 355.6 (M+H)⁺ |
| 186 | MS (ESI+) m/z: 391.7 (M+H)⁺ |
| 187 | MS (ESI+) m/z: 383.6 (M+H)⁺ |
| 188 | MS (ESI+) m/z: 373.5 (M+H)⁺ |
| 189 | MS (ESI+) m/z: 385.6 (M+H)⁺ |
| | Elemental analysis value as C₁₈H₂₃F₄N₄O₂•HCl + 0.15H₂O |
| | Calculated (%) C: 51.04 H: 5.78 N: 13.23 |
| | Found (%) C: 51.08 H: 5.86 N: 12.96 |
| 190 | MS (ESI+) m/z: 403.6 (M+H)⁺ |
| | Elemental analysis value as C₁₈H₂₂F₄N₄O₂•HCl |
| | Calculated (%) C: 49.26 H: 5.28 N: 12.77 |
| | Found (%) C: 49.13 H: 5.28 N: 12.62 |
| 191 | MS (ESI+) m/z: 371.6 (M+H)⁺ |
| | Elemental analysis value as C₁₇H₂₁F₃N₄O₂•HCl + 1.5H₂O |
| | Calculated (%) C: 47.06 H: 5.81 N: 12.91 |
| | Found (%) C: 46.96 H: 5.67 N: 12.80 |
| 192 | MS (ESI+) m/z: 357.6 (M+H)⁺ |
| | Elemental analysis value as C₁₆H₁₄F₃N₃O₂•HCl + 0.7H₂O |
| | Calculated (%) C: 49.74 H: 4.28 N: 10.88 |
| | Found (%) C: 49.70 H: 3.92 N: 10.262 |

**[Table 52]**

| Example No. | Data |
|---|---|
| 193 | MS (ESI+) m/z: 399.8 (M+H)⁺ |
| | Elemental analysis value as C₁₉H₂₅F₃N₄O₂•HCl + 0.8H₂O |
| | Calculated (%) C: 50.79 H: 6.19 N: 12.47 |
| | Found (%) C: 50.97 H: 6.18 N: 12.43 |
| 194 | MS (ESI+) m/z: 371.2 (M+H)⁺ |
| | Elemental analysis value as C₁₇H₂₁F₃N₄O₂•HCl |
| | Calculated (%) C: 50.19 H: 5.45 N: 13.77 |
| | Found (%) C: 50.24 H: 5.37 N: 13.65 |
| 195 | MS (ESI+) m/z: 371.6 (M+H)⁺ |
| 196 | MS (ESI+) m/z: 397.6 (M+H)⁺ |
| 197 | MS (ESI+) m/z: 453.6 (M+H)⁺ |
| | Elemental analysis value as C₁₉H₂₂F₆N₄O₂•HCl |
| | Calculated (%) C: 46.68 H: 4.74 N: 11.46 |
| | Found (%) C: 46.59 H: 4.72 N: 11.28 |
| 198 | MS (ESI+) m/z: 403.6 (M+H)⁺ |
| 199 | MS (ESI+) m/z: 357.6 (M+H)⁺ |
| 200 | MS (ESI+) m/z: 357.6 (M+H)⁺ |
| 201 | MS (ESI+) m/z: 415.6 (M+H)⁺ |
| 202 | MS (ESI+) m/z: 391.5 (M+H)⁺ |
| | Elemental analysis value as C₁₆H₁₈F₄N₄O₃•HCl + 0.6H₂O |
| | Calculated (%) C: 43.91 H: 4.65 N: 12.80 |
| | Found (%) C: 44.07 H: 4.68 N: 12.77 |
| 203 | MS (ESI+) m/z: 375.5 (M+H)⁺ |
| | Elemental analysis value as C₁₆H₁₈F₄N₄O₂•HCl + 0.8H₂O |
| | Calculated (%) C: 45.20 H: 4.88 N: 13.18 |
| | Found (%) C: 45.19 H: 4.73 N: 13.08 |
| 204 | MS (ESI+) m/z: 419.6 (M+H)⁺ |
| | Elemental analysis value as C₁₈H₂₂F₄N₄O₃•HCl + 0.4H₂O |
| | Calculated (%) C: 46.79 H: 5.19 N: 12.13 |
| | Found (%) C: 46.84 H: 5.25 N: 12.02 |
| 205 | MS (ESI+) m/z: 323.3 (M+H)⁺ |
| 206 | MS (ESI+) m/z: 389.5 (M+H)⁺ |
| | Elemental analysis value as C₁₇H₂₀F₄N₄O₂•HCl + 0.1H₂O |
| | Calculated (%) C: 47.86 H: 5.01 N: 13.13 |
| | Found (%) C: 47.91 H: 5.20 N: 12.87 |
| 207 | MS (ESI+) m/z: 405.5 (M+H)⁺ |
| | Elemental analysis value as C₁₇H₂₀F₄N₄O₃•HCl |
| | Calculated (%) C: 46.32 H: 4.80 N: 12.71 |
| | Found (%) C: 46.30 H: 4.87 N: 12.48 |
| 208 | MS (ESI+) m/z: 391.5 (M+H)⁺ |

**[Table 53]**

| Example No. | Data |
|---|---|
| 209 | MS (ESI+) m/z: 393.5 (M+H)⁺ |
| 210 | MS (ESI+) m/z: 389.5 (M+H)⁺ |
| 211 | MS (ESI+) m/z: 389.5 (M+H)⁺ |
| 212 | MS (ESI+) m/z: 373.2 (M+H)⁺ |
| | Elemental analysis value as C₁₆H₁₉F₃N₄O₃•HCl + 0.5H₂O |
| | Calculated (%) C: 45.99 H: 5.07 N: 13.41 |
| | Found (%) C: 46.00 H: 5.09 N: 13.22 |
| 213 | MS (ESI+) m/z: 387.5 (M+H)⁺ |
| | Elemental analysis value as C₁₇H₂₁F₃N₄O₃•HCl + 0.5H₂O |
| | Calculated (%) C: 47.28 H: 5.37 N: 12.97 |
| | Found (%) C: 47.27 H: 5.57 N: 12.77 |
| 214 | MS (ESI+) m/z: 387.5 (M+H)⁺ |
| | Elemental analysis value as C₁₇H₂₁F₃N₄O₃•HCl + 0.4H₂O |
| | Calculated (%) C: 47.48 H: 5.34 N: 13.03 |
| | Found (%) C: 47.67 H: 5.72 N: 12.78 |
| 215 | MS (ESI+) m/z: 365.3 (M+H)⁺ |
| 216 | MS (ESI+) m/z: 387.5 (M+H)⁺ |
| 217 | MS (ESI+) m/z: 371.5 (M+H)⁺ |
| | Elemental analysis value as C₁₇H₂₁F₃N₄O₂•HCl + 0.7H₂O |
| | Calculated (%) C: 48.68 H: 5.62 N: 13.36 |
| | Found (%) C: 48.76 H: 5.60 N: 13.49 |
| 218 | MS (ESI+) m/z: 387.2 (M+H)⁺ |
| | Elemental analysis value as C₁₇H₂₁F₃N₄O₃•HCl + 0.9H₂O |
| | Calculated (%) C: 46.51 H: 5.46 N: 12.76 |
| | Found (%) C: 46.65 H: 5.33 N: 12.74 |
| 219 | MS (ESI+) m/z: 405.4 (M+H)⁺ |
| | Elemental analysis value as C₁₇H₂₀F₄N₄O₃•HCl + H₂O |
| | Calculated (%) C: 44.50 H: 5.05 N: 12.21 |
| | Found (%) C: 44.71 H: 5.00 N: 12.24 |
| 220 | MS (ESI+) m/z: 369.5 (M+H)⁺ |
| 221 | MS (ESI+) m/z: 337.5 (M+H)⁺ |
| 222 | MS (ESI+) m/z: 355.5 (M+H)⁺ |
| 223 | MS (ESI+) m/z: 393.5 (M+H)⁺ |
| | Elemental analysis value as C₁₆H₁₇F₅N₄O₂•HCl + 0.7H₂O |
| | Calculated (%) C: 43.54 H: 4.43 N: 12.69 |
| | Found (%) C: 43.57 H: 4.72 N: 12.57 |
| 224 | MS (ESI+) m/z: 409.5 (M+H)⁺ |

**[Table 54]**

| Example No. | Data |
|---|---|
| 225 | MS (ESI+) m/z: 403.6 (M+H)⁺ |
| | Elemental analysis value as C₁₈H₂₂F₄N₄O₂•HCl + 0.3H₂O |
| | Calculated (%) C: 48.66 H: 5.35 N: 12.61 |
| | Found (%) C: 48.67 H: 5.27 N: 12.61 |
| 226 | MS (ESI+) m/z: 403.4 (M+H)⁺ |
| | Elemental analysis value as C₁₈H₂₂F₄N₄O₂•HCl + 0.4H₂O |
| | Calculated (%) C: 48.47 H: 5.38 N: 12.56 |
| | Found (%) C: 48.37 H: 5.23 N: 12.50 |
| 227 | MS (ESI+) m/z: 339.5 (M+H)⁺ |
| | Elemental analysis value as C₁₆H₂₀F₂N₄O₂•2HCl + H₂O |
| | Calculated (%) C: 44.76 H: 5.64 N: 13.05 |
| | Found (%) C: 44.94 H: 5.87 N: 12.99 |
| 228 | MS (ESI+) m/z: 335.5 (M+H)⁺ |
| 229 | MS (ESI+) m/z: 349.5 (M+H)⁺ |
| | Elemental analysis value as C₁₈H₂₅FN₄O₂•HCl + 1.4H₂O |
| | Calculated (%) C: 52.72 H: 7.08 N: 13.66 |
| | Found (%) C: 53.02 H: 7.15 N: 13.31 |
| 230 | MS (ESI+) m/z: 361.5 (M+H)⁺ |
| 231 | MS (ESI+) m/z: 349.5 (M+H)⁺ |
| 232 | MS (ESI+) m/z: 361.5 (M+H)⁺ |
| 233 | MS (ESI+) m/z: 363.5 (M+H)⁺ |
| 234 | MS (ESI+) m/z: 374.2 (M+H)⁺ |
| 235 | MS (ESI+) m/z: 354.0 (M+H)⁺ |
| 236 | MS (ESI+) m/z: 368.2 (M+H)⁺ |
| 237 | MS (ESI+) m/z: 382.2 (M+H)⁺ |
| 238 | MS (ESI+) m/z: 368.5 (M+H)⁺ |
| 239 | MS (ESI+) m/z: 358.5 (M+H)⁺ |
| 240 | MS (ESI+) m/z: 304.5 (M+H)⁺ |
| 241 | MS (ESI+) m/z: 324.5 (M+H)⁺ |
| 242 | MS (ESI+) m/z: 365.1 (M+H)⁺ |
| 243 | MS (ESI+) m/z: 324.1 (M+H)⁺ |
| 244 | MS (ESI+) m/z: 329.5 (M+H)⁺ |
| 245 | MS (ESI+) m/z: 375.4 (M+H)⁺ |
| 246 | MS (ESI+) m/z: 404.3 (M+H)⁺ |
| 247 | MS (ESI+) m/z: 345.6 (M+H)⁺ |

**[Table 55]**

| Example No. | Data |
|---|---|
| 248 | MS (ESI+) m/z: 349.5 (M+H)⁺ |
| 249 | MS (ESI+) m/z: 363.5 (M+H)⁺ |
| 250 | MS (ESI+) m/z: 363.5 (M+H)⁺ |
| 251 | MS (ESI+) m/z: 363.5 (M+H)⁺ |
| 252 | MS (ESI+) m/z: 377.5 (M+H)⁺ |
| 253 | MS (ESI+) m/z: 377.5 (M+H)⁺ |
| 254 | MS (ESI+) m/z: 363.5 (M+H)⁺ |

Biological test examples of the compounds of the present invention are described below.

### <Test Example 1: PIM1, 2, 3 serine/threonine kinase inhibitory effects>

### 1. Preparation of test substances

Each test substance was prepared to 10 mM with dimethylsulfoxide (DMSO), and further diluted with DMSO so as to have concentrations of 1000, 300, 100, 30, 10, 3, 1, 0.3, 0.1, 0.03, 0.01, and 0.001 µM. Moreover, each dilution was further diluted 33.3-fold with an assay buffer to prepare a test substance solution. The composition of the assay buffer was 50 mM HEPES (pH 7.0), 0.02% NaN₃, 0.01% Bovine Serum Albumin, 0.1 mM Orthovanadate, 1 mM Dithiothreitol, and 5 mM MgCl₂.

### 2. Measurement of PIM1, 2, 3 serine/threonine kinase inhibitory effects

5 µL of each test substance solution was added to 384-well plates (n = 2), and then 2.5 µL of a substrate solution (substrate 3-biotin (Cisbio Bioassays SAS), final concentration in the reaction solution: 0.5 µM) and 2.5 µL of an ATP solution (in the cases of PIM1, 2, 3, the final concentrations in the reaction solutions were 40, 2, and 10 µL, respectively) were added, 5 µL of each of PIM1, 2, 3 serine/threonine kinase solutions (Carna Bioscience, Inc., the final concentrations in the reaction solutions were 0.15, 1.6, and 0.25 µg/ml, respectively) was finally added, and the mixture was reacted at 30°C for 30 minutes. The test substance concentrations in the reaction solutions are 10000, 3000, 1000, 300, 100, 30, 10, 3, 1, 0.3, 0.1, and 0.01 nM.

Thereafter, 15 µL of each detection solution (62.5 nM Streptavidin-XL665 (Cisbio Bioassays SAS), 0.0038test STK antibody-Cryptate (Cisbio Bioassays SAS), 50 mM HEPES (pH 7.0), 0.8 M KF, 0.1% Bovine Serum Albumin, 20 mM ethylenediaminetetraacetic acid) was added to each well. After stirring, the mixture was reacted at 30°C for 1 hour. Absorbance (OD₆₂₀, OD₆₆₅) at 620 and 665 nm was measured with a microplate reader (SpectraMax M5e, Molecular Devices, LLC).

### 3. Analysis of measurement results

Non-linear regression analysis was performed by an SAS system (SAS Institute Inc.) using the ratio (OD₆₆₅/OD₆₂₀) of the measured absorbance to calculate a test substance concentration (IC₅₀) that inhibits PIM1 serine/threonine kinase activity by 50%. The results are shown in Tables 56 to 58 below.

**[Table 56]**

| Example No. | PIM1 IC₅₀ (nM) | Example No. | PIM1 IC₅₀ (nM) | Example No. | PIM1 IC₅₀ (nM) |
|---|---|---|---|---|---|
| 1 | 15 | 31 | 70 | 61 | 1.0 |
| 2 | 27 | 32 | 5.7 | 62 | 140 |
| 3 | 11 | 33 | 6.2 | 63 | 0.7 |
| 4 | 50 | 34 | 4.0 | 64 | 9.0 |
| 5 | 11 | 35 | 4.9 | 65 | 14 |
| 6 | 59 | 36 | 3.1 | 66 | 4.7 |
| 7 | 4100 | 37 | 2.0 | 67 | 26 |
| 8 | 810 | 38 | 20 | 68 | 1.4 |
| 9 | 45 | 39 | 2.2 | 69 | 1.1 |
| 10 | 6.6 | 40 | 6.2 | 70 | 4.2 |
| 11 | 13 | 41 | 40 | 71 | 140 |
| 12 | 13 | 42 | 10 | 72 | 8.4 |
| 13 | 30 | 43 | 9.2 | 73 | 21 |
| 14 | 12 | 44 | 4.2 | 74 | 2.2 |
| 15 | 15 | 45 | 20 | 75 | 1.4 |
| 16 | 53 | 46 | 290 | 76 | 1.9 |
| 17 | 1300 | 47 | 7.6 | 77 | 5.8 |
| 18 | 64 | 48 | 4.4 | 78 | 3.3 |
| 19 | 1500 | 49 | 18 | 79 | 0.8 |
| 20 | 33 | 50 | 3.7 | 80 | 56 |
| 21 | 160 | 51 | 14 | 81 | 4.3 |
| 22 | 27 | 52 | 4.2 | 82 | 71 |
| 23 | 110 | 53 | 15 | 83 | 1.0 |
| 24 | 39 | 54 | 13 | 84 | 1.1 |
| 25 | 180 | 55 | 0.7 | 85 | 4.7 |
| 26 | 12 | 56 | 7.0 | 86 | 0.6 |
| 27 | 4.1 | 57 | 6.0 | 87 | 1.2 |
| 28 | 38 | 58 | 2.0 | 88 | 2.4 |
| 29 | 18 | 59 | 16 | 89 | 5.4 |
| 30 | 24 | 60 | 13 | 90 | 21 |

**[Table 57]**

| Example No. | PIM1 IC₅₀ (nM) | Example No. | PIM1 IC₅₀ (nM) | Example No. | PIM1 IC₅₀ (nM) |
|---|---|---|---|---|---|
| 91 | 1.5 | 121 | 120 | 151 | 1.8 |
| 92 | 2.6 | 122 | 6.5 | 152 | 3.3 |
| 93 | 36 | 123 | 8.2 | 153 | 3.1 |
| 94 | 8.4 | 124 | 26 | 154 | 2.4 |
| 95 | 11 | 125 | 2.9 | 155 | 5.8 |
| 96 | 6.2 | 126 | 11 | 156 | 14 |
| 97 | 1.2 | 127 | 21 | 157 | 3.3 |
| 98 | 2.0 | 128 | 82 | 158 | 3.3 |
| 99 | 3.9 | 129 | 8.2 | 159 | 2.8 |
| 100 | 1.9 | 130 | 19 | 160 | 1.2 |
| 101 | 2.1 | 131 | 3.0 | 161 | 29 |
| 102 | 36 | 132 | 17 | 162 | 8.7 |
| 103 | 5.0 | 133 | 8.0 | 163 | 2.0 |
| 104 | 4.0 | 134 | 14 | 164 | 1.7 |
| 105 | 12 | 135 | 29 | 165 | 2.2 |
| 106 | 1.5 | 136 | 16 | 166 | 2.3 |
| 107 | 25 | 137 | 2.2 | 167 | 3.1 |
| 108 | 19 | 138 | 4.0 | 168 | 10 |
| 109 | 28 | 139 | 2.6 | 169 | 6.5 |
| 110 | 3.5 | 140 | 4.1 | 170 | 1.8 |
| 111 | 6.4 | 141 | 7.1 | 171 | 2.0 |
| 112 | 600 | 142 | 11 | 172 | 2.5 |
| 113 | 3.6 | 143 | 14 | 173 | 10 |
| 114 | 8.4 | 144 | 2.9 | 174 | 1.6 |
| 115 | 12 | 145 | 4.0 | 175 | 17 |
| 116 | 4.3 | 146 | 12 | 176 | 9.6 |
| 117 | 31 | 147 | 23 | 177 | 0.8 |
| 118 | 7300 | 148 | 3.7 | 178 | 3.2 |
| 119 | 1800 | 149 | 9.1 | 179 | 2.0 |
| 120 | 590 | 150 | 2.0 | 180 | 2.2 |

**[Table 58]**

| Example No. | PIM1 IC₅₀ (nM) | Example No. | PIM1 IC₅₀ (nM) | Example No. | PIM1 IC₅₀ (nM) |
|---|---|---|---|---|---|
| 181 | 3.5 | 206 | 1.7 | 231 | 3.4 |
| 182 | 1.7 | 207 | 2.6 | 232 | 2.0 |
| 183 | 1.8 | 208 | 8.5 | 233 | 5.5 |
| 184 | 65 | 209 | 5.7 | 234 | 2.6 |
| 185 | 5.5 | 210 | 1.3 | 235 | 7.7 |
| 186 | 11 | 211 | 4.9 | 236 | 8.7 |
| 187 | 8.7 | 212 | 2.1 | 237 | 5.3 |
| 188 | 2.9 | 213 | 2.0 | 238 | 2.5 |
| 189 | 2.2 | 214 | 1.8 | 239 | 10 |
| 190 | 1.9 | 215 | 8800 | 240 | 1.9 |
| 191 | 11 | 216 | 3.5 | 241 | 3.7 |
| 192 | 2.6 | 217 | 2.8 | 242 | 10 |
| 193 | 6.1 | 218 | 2.9 | 243 | 4.1 |
| 194 | 12 | 219 | 1.8 | 244 | 11 |
| 195 | 8.1 | 220 | 6.1 | 245 | 4.2 |
| 196 | 9.2 | 221 | 7.2 | 246 | 1.9 |
| 197 | 140 | 222 | 2.4 | 247 | 2.8 |
| 198 | 3.5 | 223 | 1.6 | 248 | 10 |
| 199 | 3.9 | 224 | 0.8 | 249 | 4.2 |
| 200 | 8.5 | 225 | 1.1 | 250 | 5.7 |
| 201 | 8.7 | 226 | 5.4 | 251 | 12 |
| 202 | 1.5 | 227 | 7.7 | 252 | 6.6 |
| 203 | 3.6 | 228 | 4.7 | 253 | 3.0 |
| 204 | 3.5 | 229 | 5.1 | 254 | 15 |
| 205 | 290 | 230 | 3.3 | | |

For the compounds with the following Example numbers, in addition to the above PIM1 serine/threonine kinase activity (IC₅₀), test substance concentrations (IC₅₀) that inhibit PIM2 serine/threonine kinase activity and PIM3 serine/threonine kinase activity by 50% were calculated. The results are shown in Table 59 below.

**[Table 59]**

| Example No. | PIM1 IC₅₀ (nM) | PIM2 IC₅₀ (nM) | PIM3 IC₅₀ (nM) |
|---|---|---|---|
| 18 | 64 | 220 | 340 |
| 25 | 180 | 770 | 740 |
| 34 | 4.0 | 25 | 16 |
| 37 | 2.0 | 11 | 10 |
| 52 | 4.2 | 19 | 12 |
| 55 | 0.7 | 14 | 5.0 |
| 58 | 2.0 | 18 | 16 |
| 59 | 16 | 98 | 85 |
| 61 | 1.0 | 19 | 5.0 |
| 63 | 0.7 | 13 | 5.0 |
| 66 | 4.7 | 90 | 18 |
| 68 | 1.4 | 18 | 5.0 |
| 69 | 1.1 | 7.0 | 4.0 |
| 75 | 1.4 | 2.3 | 10 |

### <Test Example 2: Proliferation inhibitory effects on PIM1, 2, 3 transgenic cells>

### 1. Preparation of PIM1 transgenic cells

Human PIM1, 2, and 3 genes were inserted into the multi-cloning sites of retrovirus expression vectors pMYs-IRES-GFP, respectively, to prepare gene transfer vectors. Then, each gene transfer vector was introduced into packaging cells PLAT-E derived from a human fetal kidney cell line in the logarithmic growth phase, using a transfection reagent (FuGENE6, Promega Corporation). Since the culture supernatant of PLAT-E after gene transfer contained virus particles for gene transfer, the culture supernatant was collected and used as a medium for gene transfer. The medium for gene transfer was added to a plate coated with RetroNectin, and the plate was incubated to attach the virus particles to the plate. Then, a mouse pro-B cell line Ba/F3 in the logarithmic growth phase was seeded on the plate and infected with the virus to prepare PIM1, 2, or 3 transgenic cells that proliferate in a PIM 1, 2, or 3-dependent manner. The expression of PIM1, 2, or 3 protein in each cell was confirmed by Western blotting.

### 2. Preparation of test substances

Each test substance was prepared to 10 mM with dimethylsulfoxide (DMSO), and diluted with DMSO so as to have concentrations of 3000, 1000, 300, 100, 30, 10, 3, and 1 µM. Moreover, each dilution was further diluted 10-fold with distilled water to prepare a test substance solution.

### 3. Measurement of PIM1, 2, 3 expression cell proliferation inhibitory effects

PIM1, 2, 3-expressing cells were seeded on a 96-well plate, and each test substance solution prepared the next day was added such that final concentrations were 10000, 3000, 1000, 300, 100, 30, 10, 3, and 1 nM. 72 hours after the addition, 10 µL of Cell Counting Kit-8 (DOJINDO LABORATORIES) was added to each well. After incubation for 1 to 4 hours, absorbance OD₄₅₀ at 450 nm was measured. In addition, absorption OD₆₅₀ at 650 nm as a reference wavelength was measured.

### 4. Analysis of measurement results

OD₄₅₀ - OD₆₅₀ for each condition was calculated, and then the inhibition rates when OD₄₅₀ - OD₆₅₀ of negative control (DMSO only) and OD₄₅₀ - OD₆₅₀ of Blank (without cells) were set to 0% and 100%, respectively, was calculated. Then, non-linear regression analysis was performed for logarithmic dose and inhibition rate using a two-parameter logistic model to estimate IC₅₀ values. The results of the proliferation inhibitory effect on PIM1 transgenic cells are shown in Tables 60 to 62 below.

**[Table 60]**

| Example No. | PIM1 IC₅₀ (nM) | Example No. | PIM1 IC₅₀ (nM) | Example No. | PIM1 IC₅₀ (nM) |
|---|---|---|---|---|---|
| 1 | 1900 | 42 | 270 | 73 | 570 |
| 3 | 7900 | 43 | 190 | 74 | 150 |
| 4 | 3000 | 44 | 330 | 75 | 24 |
| 5 | 500 | 45 | 390 | 76 | 31 |
| 6 | 3600 | 46 | 2600 | 77 | 180 |
| 10 | 630 | 47 | 820 | 78 | 170 |
| 11 | 330 | 48 | 1200 | 79 | 53 |
| 12 | 5100 | 49 | 7600 | 81 | 600 |
| 13 | 540 | 50 | 1800 | 83 | 75 |
| 14 | 1300 | 51 | 3100 | 84 | 36 |
| 15 | 600 | 52 | 59 | 85 | 610 |
| 16 | 2400 | 53 | 1600 | 86 | 61 |
| 20 | 4500 | 54 | 1200 | 87 | 64 |
| 22 | 2200 | 55 | 34 | 88 | 110 |
| 24 | 870 | 56 | 200 | 89 | 250 |
| 26 | 910 | 57 | 370 | 90 | 3800 |
| 27 | 200 | 58 | 33 | 91 | 210 |
| 28 | 3500 | 59 | 320 | 92 | 280 |
| 29 | 1100 | 60 | 3100 | 94 | 140 |
| 30 | 970 | 61 | 44 | 95 | 590 |
| 31 | 3300 | 63 | 28 | 96 | 380 |
| 32 | 410 | 64 | 1300 | 97 | 55 |
| 33 | 730 | 65 | 1000 | 98 | 79 |
| 34 | 910 | 66 | 190 | 99 | 97 |
| 35 | 510 | 67 | 610 | 100 | 260 |
| 36 | 570 | 68 | 61 | 101 | 61 |
| 37 | 280 | 69 | 37 | 103 | 120 |
| 38 | 550 | 70 | 1700 | 104 | 110 |
| 39 | 100 | 71 | 4200 | 105 | 550 |
| 40 | 440 | 72 | 920 | 106 | 19 |

**[Table 61]**

| Example No. | PIM1 IC₅₀ (nM) | | PIM1 IC₅₀ (nM) | Example No. | PIM1 IC₅₀ (nM) |
|---|---|---|---|---|---|
| 107 | 6500 | 145 | 61 | 176 | 88 |
| 108 | 270 | 146 | 89 | 177 | 26 |
| 109 | 480 | 148 | 45 | 178 | 34 |
| 110 | 2100 | 149 | 380 | 179 | 23 |
| 111 | 140 | 150 | 9.2 | 180 | 30 |
| 113 | 81 | 151 | 17 | 181 | 68 |
| 114 | 300 | 152 | 57 | 182 | 45 |
| 115 | 100 | 153 | 40 | 183 | 45 |
| 116 | 4200 | 154 | 35 | 184 | 880 |
| 117 | 1300 | 155 | 92 | 185 | 87 |
| 122 | 220 | 156 | 160 | 186 | 2300 |
| 123 | 340 | 157 | 65 | 187 | 110 |
| 124 | 4100 | 158 | 37 | 188 | 56 |
| 125 | 40 | 159 | 30 | 189 | 44 |
| 126 | 600 | 160 | 40 | 190 | 41 |
| 127 | 440 | 161 | 1000 | 191 | 160 |
| 129 | 360 | 162 | 140 | 192 | 47 |
| 130 | 280 | 163 | 24 | 193 | 160 |
| 131 | 71 | 164 | 25 | 194 | 280 |
| 132 | 440 | 165 | 25 | 195 | 140 |
| 133 | 200 | 166 | 26 | 196 | 250 |
| 135 | 3500 | 167 | 94 | 197 | 1900 |
| 137 | 27 | 168 | 110 | 198 | 100 |
| 138 | 73 | 169 | 310 | 199 | 110 |
| 139 | 80 | 170 | 17 | 200 | 100 |
| 140 | 57 | 171 | 28 | 201 | 560 |
| 141 | 250 | 172 | 49 | 202 | 26 |
| 142 | 220 | 173 | 250 | 203 | 54 |
| 143 | 610 | 174 | 18 | 204 | 57 |
| 144 | 64 | 175 | 320 | 206 | 32 |

**[Table 62]**

| Example No. | PIM1 IC₅₀ (nM) | Example No. | PIM1 IC₅₀ (nM) | Example No. | PIM1 IC₅₀ (nM) |
|---|---|---|---|---|---|
| 207 | 50 | 224 | 7.1 | 240 | 87 |
| 208 | 130 | 225 | 7.6 | 241 | 79 |
| 209 | 100 | 226 | 83 | 242 | 2600 |
| 210 | 15 | 227 | 24 | 243 | 150 |
| 211 | 110 | 228 | 15 | 244 | 140 |
| 212 | 73 | 229 | 19 | 245 | 3200 |
| 213 | 85 | 230 | 22 | 246 | 4900 |
| 214 | 34 | 231 | 37 | 247 | 120 |
| 216 | 45 | 232 | 28 | 248 | 250 |
| 217 | 26 | 233 | 44 | 249 | 110 |
| 218 | 27 | 234 | 120 | 250 | 190 |
| 219 | 11 | 235 | 650 | 251 | 260 |
| 220 | 110 | 236 | 180 | 252 | 210 |
| 221 | 46 | 237 | 90 | 253 | 81 |
| 222 | 19 | 238 | 87 | 254 | 680 |
| 223 | 11 | 239 | 250 | | |

For the compounds with the following Example numbers, in addition to the above proliferation inhibitory effect on PIM1 transgenic cells, the proliferation inhibitory effect on PIM2 transgenic cells and the proliferation inhibitory effect on PIM3 transgenic cells were calculated as IC₅₀. The results are shown in Table 63.

**[Table 63]**

| Example No. | PIM1 IC₅₀ (nM) | PIM2 IC₅₀ (nM) | PIM3 IC₅₀ (nM) |
|---|---|---|---|
| 58 | 33 | 3600 | 570 |
| 75 | 24 | 620 | 120 |
| 202 | 26 | 790 | 78 |
| 219 | 11 | 1100 | 40 |
| 223 | 11 | 290 | 59 |
| 224 | 7.1 | 510 | 35 |
| 225 | 7.6 | 390 | 51 |

### <Test Example 3: Examination of lymphocyte proliferation inhibitory effect using graft-versus-host disease model animals>

### 1. Preparation of animal models

BALB/c mice (8-week-aged female) were euthanized, and then the spleen was removed from each mouse. Then, hemolysis treatment was performed to isolate spleen cells. The obtained spleen cells were intravenously transplanted into CB6F1 mice (8-week-aged female), which are C57BL/6 and BALB/c F1 mice, to prepare a model, and splenomegaly was induced. For transplantation, spleen cells for 1.2 BALB/c mouse were used for one CB6F1 mouse.

### 2. Preparation of test substances

Each test substance shown in Table 64 was weighed and dissolved in a 0.5% methyl cellulose solution, thereby preparing test substance solutions of 0.3 to 10 mg/mL.

### 3. Drug administration and measurement of spleen weight

A vehicle (0.5% methyl cellulose solution) or test substance was orally administered to each CB6F1 mouse after transplantation at a dose of 3 to 50 mg/kg in terms of body weight twice a day for 10 days from the day after transplantation. The mouse was euthanized on the 11th day from the start of administration, then the spleen was removed therefrom, and the wet weight of the spleen was measured. For each of CB6F1 mice that had not undergone transplantation as a control group, the wet weight of the spleen was also measured. The test substances and doses administered are shown in Table 64.

### 4. Analysis of measurement results

The splenomegaly formation inhibition rate when the spleen weights of the mice to which the vehicle was administered after transplantation and the mice on which transplantation had not been performed were 0% and 100%, respectively, was calculated. The experimental results are shown in Table 64.

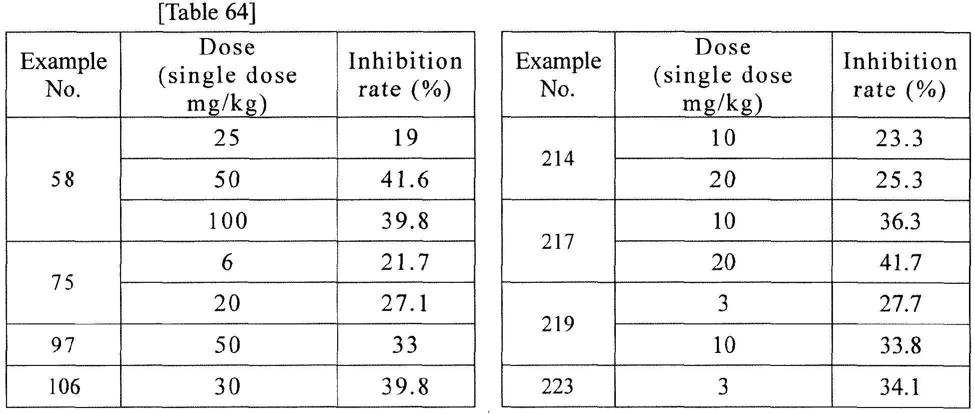

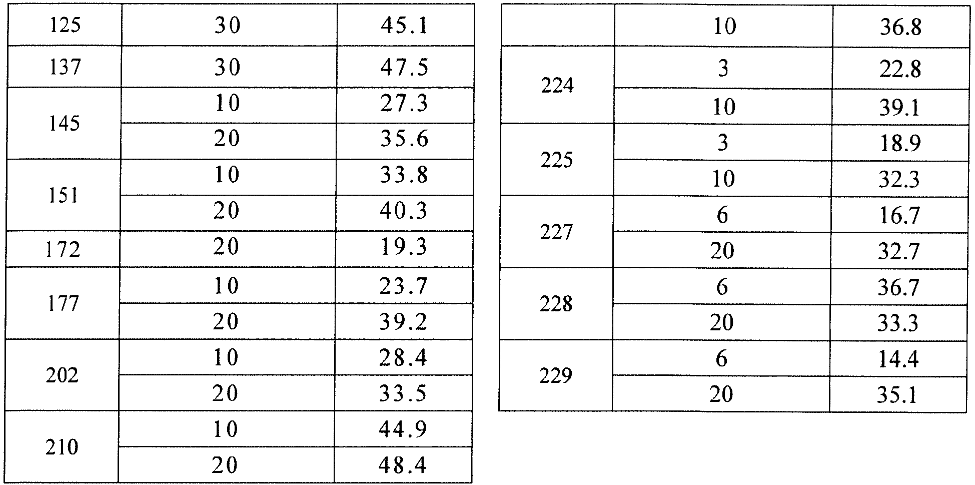

As shown in Table 64, all the compounds of the Examples were found to inhibit splenomegaly formation in the GVHD model. Splenomegaly in the GVHD model is known to be caused by the proliferation of lymphocytes as a result of immune activation. It was shown that the compounds of the Examples have an immunosuppressive effect and a lymphocyte proliferation inhibitory effect in the animal model.

### <Test Example 4: Inhibitory effect on systemic lupus erythematosus and lupus nephritis model animals>

### 1. Preparation of experimental animals

For the evaluation of test substances, NZB/W F1 mice (29, 30-week-aged female), which are model mice with systemic lupus erythematosus and lupus nephritis, were used. These mice are a spontaneous animal model, and an increase in autoantibodies such as anti-double-stranded DNA antibody, which is considered to be a cause of systemic lupus erythematosus, and proteinuria, which is a characteristic of lupus nephritis, are observed for the mice, and the mice are known to die from nephritis.

Urine was collected before the start of the test, and individuals with a urinary albumin concentration ÷ urinary creatinine concentration (UACR) value of 5 or more were excluded. Furthermore, the anti-double-stranded DNA antibody titer in blood and the body weight were measured before the start of the test, and 53 mice were grouped into 5 groups of 10 or 13 mice based on blood anti-double-stranded DNA antibody titer, age, and body weight. Since early death was expected for the vehicle-administered group, the test was conducted with 13 mice as this group.

### 2. Preparation of test substances

Example 75 and Example 202 were used as test substances, and a 0.5% methyl cellulose solution which is a vehicle was used as a control. Example 75 and Example 202 were prepared into solutions of 1.5 and 3.0 mg/mL by being dissolved in a 0.5% methyl cellulose solution.

### 3. Administration of test substances and measurement of number of dead/euthanized individuals

After grouping, the 0.5% methyl cellulose solution or the 1.5 or 3.0 mg/mL solution of Example 75 or Example 202 was administered to each of the mice of the 7 groups. The single dose was set to 10 mL per 1 kg of body weight, and the dose was administered twice a day. Under these conditions, the dose per time is 15, 30 mg per 1 kg of body weight. Administration was started on 29 or 30-week-aged mice, and was performed daily for 15 weeks until the age of 44 or 45 weeks. The number of death and euthanasia cases until the final day of administration is shown in Table 65.

### 4. Measurement and analysis of anti-double-stranded DNA antibody titer

Blood was collected from the tail vein of each of all the surviving individuals on the final day of administration. Then, the blood was centrifuged and the plasma was separated and taken. The plasma was diluted 3000-fold, and the anti-double-stranded DNA antibody titer was measured by ELISA method (Levis anti-dsDNA-mouse ELISA Kit, FUJIFILM Wako Shibayagi Corporation). Measurement and analysis were performed according to the protocol of the Kit. The median of the anti-double-stranded DNA antibody titers for each group is shown in Table 65.

### 5. Measurement and analysis of urinary albumin concentration and urinary creatinine concentration

All the surviving individuals on the final day of administration were bred in a mouse metabolism cage (CLEA Japan, Inc.) for 16 hours, and urine was collected from each individual. The solids were removed by centrifuging the urine and collecting the supernatant. An automatic analyzer (JCA-BM6050 BioMajesty, JEOL Ltd.) was used to measure the albumin concentration and creatinine concentration in urine after centrifugation. A urinary albumin/creatinine ratio (UACR) was calculated by dividing the albumin concentration by the creatinine concentration, and individuals with UACR > 20 were defined as proteinuria-positive individuals. In addition, for almost all the individuals that died before the final day of administration, UACR > 20 was confirmed in urinalysis immediately before death, and thus these individuals were treated as proteinuria-positive individuals. The number of proteinuria-positive individuals is shown in Table 65.

**[Table 65]**

| Test substance | Dose (single dose mg/kg) | Anti-double-stranded DNA antibody titer (U/mL) | Number of proteinuria-positive individuals | Number of dead/euthanized individuals |
|---|---|---|---|---|
| Vehicle | - | 1571 | 9/13 | 9/13 |
| Example 75 | 15 | 423 | 2/10 | 0/10 |
| | 30 | 306 | 0/10 | 0/10 |
| Example 202 | 15 | 568 | 1/10 | 1/10 |
| | 30 | 112 | 1/10 | 1/10 |

As is clear from Table 65, Example 75 and Example 202 exhibit effects of suppressing an increase in anti-double-stranded DNA antibody titer, decreasing the onset rate of proteinuria, and improving the survival rate, and thus have remarkable effects on systemic lupus erythematosus and lupus nephritis.

## Claims

1. A 1,3,4-oxadiazolone compound of the formula [1]: 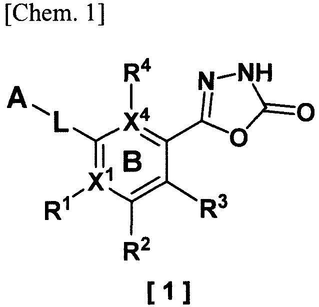 wherein
X¹ is a carbon atom or a nitrogen atom,
when X¹ is a carbon atom, R¹ is a hydrogen atom, a halogen atom, alkyl, alkenyl, a non-aromatic carbocyclic group, dihaloalkyl, trihaloalkyl, alkoxy, dihaloalkoxy, trihaloalkoxy, alkylsulfonyl, cyano, an aromatic carbocyclic group, or an aromatic heterocyclic group,
when X¹ is a nitrogen atom, R¹ does not exist,
R² is a hydrogen atom, a halogen atom, alkyl, a non-aromatic carbocyclic group, trihaloalkyl, pentafluorosulfanyl (SF₅), cyano, amino, or nitro,
R¹ and R² optionally combine with adjacent atoms to form an indazole ring,
R³ is a hydrogen atom, a halogen atom, or alkyl,
X⁴ is a carbon atom or a nitrogen atom,
when X⁴ is a carbon atom, R⁴ is a hydrogen atom, a halogen atom, or alkyl
when X⁴ is a nitrogen atom, R⁴ does not exist,
both X¹ and X⁴ are not nitrogen atoms at the same time,
L is a bond, an alkylene, an alkenylene, an alkynylene, or a group represented by L-1, L-2, L-3, or L-4: 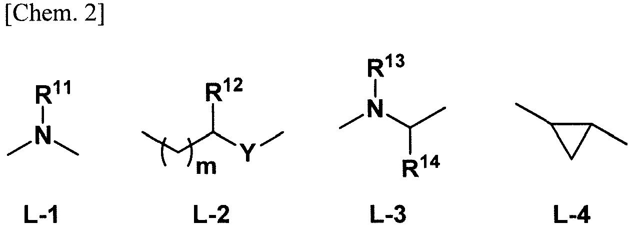 wherein the bond on the left side of each group is attached to A in the formula [1], the bond on the right side of each group is attached to a ring B in the formula [1], R¹¹, R¹³, and R¹⁴ are each a hydrogen atom or alkyl, R¹² is a hydrogen atom, alkyl, monohaloalkyl, dihaloalkyl, or trihaloalkyl, R¹³ is a hydrogen atom or alkyl, Y is O, S, or -NR¹⁵- (R¹⁵ is a hydrogen atom or alkyl), and m is 0, 1, or 2,
R¹ and R¹⁵ (if L is L-2 and Y is -NR¹⁵-) combine with adjacent atoms to form a group represented by z-1, z-2, or z-3: 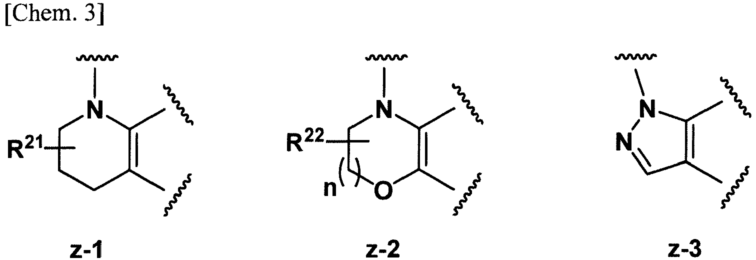
wherein R²¹ is a hydrogen atom, oxo (=O), or an alkoxyimino (=N-O-R²³), n is 1 or 2, and R²² is a hydrogen atom or alkyl,
A represents aminoalkylamino, a non-aromatic heterocyclic group, a non-aromatic carbocyclic group, an aromatic carbocyclic group, an aromatic heterocyclic group, or 1,3-dioxa-2-yl,
the non-aromatic heterocyclic group for A is optionally substituted with one or two groups selected from the group consisting of the following (1) to (7):
(1) amino (-NH₂),
(2) alkyl,
(3) aminoalkyl,
(4) alkyl substituted with amino and hydroxy,
(5) halogen,
(6) alkylcarbonyl, and
(7) alkoxycarbonyl,
the non-aromatic carbocyclic group for A is optionally substituted with 1 to 3 groups selected from the group consisting of the following (1) to (15):
(1) amino,
(2) alkyl,
(3) alkylamino substituted with a non-aromatic carbocyclic group,
(4) trihaloalkylamino,
(5) hydroxyalkyl,
(6) aminoalkyl,
(7) hydroxy,
(8) monoalkylamino,
(9) hydroxyalkylamino,
(10) alkoxycarbonyl,
(11) carboxyl,
(12) carbamoyl,
(13) acetamide (Me-C(=O)-NH-),
(14) piperazinyl, and
(15) alkylamino,
the aromatic carbocyclic group for A is optionally substituted with one group selected from the group consisting of the following (1) to (4):
(1) aminoalkyl,
(2) aminoalkoxy,
(3) alkoxy substituted with piperidinyl, and
(4) alkoxycarbonylaminoalkyl,
the aromatic heterocyclic group for A is optionally substituted with a piperazinyl group, and
A and L are selected from any of the following cases (a) to (h):
(a) when L is a bond,
A is aminoalkylamino, a non-aromatic heterocyclic group, an aromatic carbocyclic group, or an aromatic heterocyclic group,
(b) when L is an alkylene,
A is a non-aromatic heterocyclic group or a non-aromatic carbocyclic group,
(c) when L is an alkenylene,
A is a non-aromatic heterocyclic group,
(d) when L is an alkynylene,
A is a non-aromatic heterocyclic group,
(e) when L is L-1,
A is a non-aromatic heterocyclic group or a non-aromatic carbocyclic group,
(f) when L is L-2,
A is a non-aromatic heterocyclic group or a non-aromatic carbocyclic group,
(g) when L is L-3,
A is a non-aromatic heterocyclic group, and
(h) when L is L-4,
A is a non-aromatic heterocyclic group,
or a pharmaceutically acceptable salt thereof, or a solvate thereof.

2. The 1,3,4-oxadiazolone compound according to claim 1, or a pharmaceutically acceptable salt thereof, or a solvate thereof, wherein X¹ is a carbon atom, and X² is a carbon atom.

3. The 1,3,4-oxadiazolone compound according to claim 1 or 2, or a pharmaceutically acceptable salt thereof, or a solvate thereof, wherein L is a bond, an alkylene, an alkenylene, an alkynylene, L-1, or L-2.

4. The 1,3,4-oxadiazolone compound according to any one of claims 1 to 3, or a pharmaceutically acceptable salt thereof, or a solvate thereof, wherein A is aminoalkylamino, a non-aromatic heterocyclic group, a non-aromatic carbocyclic group, an aromatic carbocyclic group, or an aromatic heterocyclic group.

5. The 1,3,4-oxadiazolone compound according to any one of claims 1 to 4, or a pharmaceutically acceptable salt thereof, or a solvate thereof, wherein L is a bond, L-1, or L-2.

6. The 1,3,4-oxadiazolone compound according to any one of claims 1 to 5, or a pharmaceutically acceptable salt thereof, or a solvate thereof, wherein A and L are any of groups of the following (aa), (ee), and (ff):
(aa) when L is a bond, A is aminoalkylamino, a non-aromatic heterocyclic group, an aromatic carbocyclic group, or an aromatic heterocyclic group,
(ee) when L is L-1, A is a non-aromatic heterocyclic group or a non-aromatic carbocyclic group, or
(ff) when L is L-2, A is a non-aromatic heterocyclic group or a non-aromatic carbocyclic group.

7. The 1,3,4-oxadiazolone compound according to any one of claims 1 to 5, or a pharmaceutically acceptable salt thereof, or a solvate thereof, wherein L is L-2, and A is a non-aromatic heterocyclic group or a non-aromatic carbocyclic group.

8. The 1,3,4-oxadiazolone compound according to claim 7, or a pharmaceutically acceptable salt thereof, or a solvate thereof, wherein L is L-2, m is 0, Y is -NR¹⁵-, and A is a non-aromatic heterocyclic group or a non-aromatic carbocyclic group.

9. The 1,3,4-oxadiazolone compound according to any one of claims 1 to 8, or a pharmaceutically acceptable salt thereof, or a solvate thereof, wherein
the non-aromatic heterocyclic group for A is piperidinyl, piperazinyl, pyrrolidinyl, azepanyl, azocanyl, 1,3-dioxanyl, tetrahydrofuranyl, 6-azaspiro[2.5]octanyl, 3,9-diazaspiro[5.5]undecanyl, 2,7-diazaspiro[3.5]nonan-7-yl, 7-azaspiro[3.5]nonanyl, 3-azabicyclo[3.2.1]octanyl, or 2-azaspiro[3.3]heptan-6-yl,
the non-aromatic carbocyclic group for A is cyclohexanyl, cyclopentyl, cyclobutenyl, bicyclo[2.2.1]heptanyl, bicyclo[1.1.1]pentanyl, cuban-1-yl, or 2-azaspiro[3.3]heptanyl,
the aromatic carbocyclic group for A is phenyl, and
the aromatic heterocyclic group for A is pyridyl.

10. The 1,3,4-oxadiazolone compound according to claim 9, or a pharmaceutically acceptable salt thereof, or a solvate thereof, wherein
X¹ is a carbon atom,
R¹ is a halogen atom, dihaloalkyl, trihaloalkyl, dihaloalkyl, or trihaloalkoxy,
R² is a halogen atom or trihaloalkyl,
R³ is a hydrogen atom,
X⁴ is a carbon atom,
R⁴ is a hydrogen atom,
L is L-2,
m is 0,
Y is NR¹⁵,
R¹⁵ is a hydrogen atom,
R¹² is a hydrogen atom or alkyl, and
A is piperidinyl, piperazinyl, pyrrolidinyl, azepanyl, azocanyl, 1,3-dioxanyl, tetrahydrofuranyl, 6-azaspiro[2.5]octanyl, 3,9-diazaspiro[5.5]undecanyl, 2,7-diazaspiro[3.5]nonan-7-yl, 7-azaspiro[3.5]nonanyl, 3-azabicyclo[3.2.1]octanyl, or 2-azaspiro[3.3]heptan-6-yl.

11. The 1,3,4-oxadiazolone compound according to any one of claims 1 to 9, or a pharmaceutically acceptable salt thereof, or a solvate thereof, wherein the 1,3,4-oxadiazolone compound is any one of the following compounds (1) to (254):
(1) 5-{3-[(4-aminobutyl)amino]-4-(trifluoromethyl)phenyl}-1,3,4-oxadiazol-2(3H)-one,
(2) 5-{3-[(3-aminopropyl)amino]-4-(trifluoromethyl)phenyl}-1,3,4-oxadiazol-2(3H)-one,
(3) 5-{3-[(5-aminopentyl)amino]-4-(trifluoromethyl)phenyl}-1,3,4-oxadiazol-2(3H)-one,
(4) 5-{3-[(6-aminohexyl)amino]-4-(trifluoromethyl)phenyl}-1,3,4-oxadiazol-2(3H)-one,
(5) 5-{3-[(6-aminohexan-2-yl)amino]-4-(trifluoromethyl)phenyl}-1,3,4-oxadiazol-2(3H)-one,
(6) 5-(3-[4-(aminomethyl)piperidin-1-yl]-4-chlorophenyl}-1,3,4-oxadiazol-2(3H)-one,
(7) tert-butyl 4-[2-chloro-5-(5-oxo-4,5-dihydro-1,3,4-oxadiazol-2-yl)phenyl]piperazine-1-carboxylate,
(8) 5-[4-chloro-3-(piperazin-1-yl)phenyl]-1,3,4-oxadiazol-2(3H)-one,
(9) 5-[3-(4-aminopiperidin-1-yl)-4-(trifluoromethyl)phenyl]-1,3,4-oxadiazol-2(3H)-one,
(10) 5-{3-[4-(2-aminoethyl)piperidin-1-yl]-4-(trifluoromethyl)phenyl}-1,3,4-oxadiazol-2(3H)-one,
(11) 5-{3-[3-(2-aminoethyl)piperidin-1-yl]-4-(trifluoromethyl)phenyl}-1,3,4-oxadiazol-2(3H)-one,
(12) 5-{4-[4-(2-aminoethyl)piperidin-1-yl]-1H-indazol-6-yl}-1,3,4-oxadiazol-2(3H)-one,
(13) 5-{3-[4-(1-amino-2-methylpropan-2-yl)piperidin-1-yl]-4-(trifluoromethyl)phenyl}-1,3,4-oxadiazol-2(3H)-one,
(14) 5-{3-[4-(2-amino-1-hydroxyethyl)piperidin-1-yl]-4-(trifluoromethyl)phenyl}-1,3,4-oxadiazol-2(3H)-one,
(15) 5-[3-(3,9-diazaspiro[5.5]undecan-3-yl)-4-(trifluoromethyl)phenyl]-1,3,4-oxadiazol-2(3H)-one,
(16) 5-[3-(2,7-diazaspiro[3.5]nonan-7-yl)-4-(trifluoromethyl)phenyl]-1,3,4-oxadiazol-2(3H)-one,
(17) tert-butyl {[2'-chloro-5'-(5-oxo-4,5-dihydro-1,3,4-oxadiazol-2-yl)[1,1'-biphenyl]-3-yl]methyl}carbamate,
(18) 5-[3'-(aminomethyl)-6-chloro[1,1'-biphenyl]-3-yl]-1,3,4-oxadiazol-2(3H)-one,
(19) tert-butyl {[5'-(5-oxo-4,5-dihydro-1,3,4-oxadiazol-2-yl)-2'-(trifluoromethyl)[1,1'-biphenyl]-4-yl]methyl}carbamate,
(20) 5-[4'-(aminomethyl)-6-(trifluoromethyl)[1,1'-biphenyl]-3-yl]-1,3,4-oxadiazol-2(3H)-one,
(21) 5-[3'-(aminomethyl)-6-(trifluoromethyl)[1,1'-biphenyl]-3-yl]-1,3,4-oxadiazol-2(3H)-one,
(22) 5-[4'-(2-aminoethyl)-6-(trifluoromethyl)[1,1'-biphenyl]-3-yl]-1,3,4-oxadiazol-2(3H)-one,
(23) 5- {4'-[(piperidin-4-yl)methoxy]-6-(trifluoromethyl)[1,1'-biphenyl]-3-yl}-1,3,4-oxadiazol-2(3H)-one,
(24) 5-[4'-{[(2S)-1-aminopropan-2-yl]oxy}-6-(trifluoromethyl)[1,1-biphenyl]-3-yl]-1,3,4-oxadiazol-2(3H)-one,
(25) 5-{3-[5-(piperazin-1-yl)pyridin-3-yl]-4-(trifluoromethyl)phenyl}-1,3,4-oxadiazol-2(3H)-one,
(26) 5-{3-[2-(piperidin-4-yl)ethyl]-4-(trifluoromethyl)phenyl}-1,3,4-oxadiazol-2(3H)-one,
(27) 5-[3-{2-[(1r,4s)-4-aminocyclohexyl]ethyl}-4-(trifluoromethyl)phenyl]-1,3,4-oxadiazol-2(3H)-one,
(28) 5-[3-{2-[(2r,5r)-5-amino-1,3-dioxan-2-yl]ethyl}-4-(trifluoromethyl)phenyl]-1,3,4-oxadiazol-2(3H)-one,
(29) 5-{3-[(piperidin-4-yl)ethynyl]-4-(trifluoromethyl)phenyl}-1,3,4-oxadiazol-2(3H)-one,
(30) 5-{3-[(E)-2-(piperidin-4-yl)ethenyl]-4-(trifluoromethyl)phenyl}-1,3,4-oxadiazol-2(3H)-one,
(31) 5-{4-chloro-3-[(piperidin-4-yl)amino]phenyl}-1,3,4-oxadiazol-2(3H)-one,
(32) 5-(3-{[(1r,4r)-4-aminocyclohexyl]amino}-4-chlorophenyl)-1,3,4-oxadiazol-2(3H)-one,
(33) 5-(3-{[(1s,4s)-4-aminocyclohexyl]amino}-4-chlorophenyl)-1,3,4-oxadiazol-2(3H)-one,
(34) 5-[3-{[(1r,4r)-4-aminocyclohexyl]amino}-4-(trifluoromethyl)phenyl]-1,3,4-oxadiazol-2(3H)-one,
(35) 5-(3-{[(1r,4r)-4-aminocyclohexyl]amino}-4-bromophenyl)-1,3,4-oxadiazol-2(3H)-one,
(36) 5-[3-{[(1r,4r)-4-aminocyclohexyl]amino}-5-fluoro-4-(trifluoromethyl)phenyl]-1,3,4-oxadiazol-2(3H)-one ,
(37) 5-[3-{[(1r,4r)-4-(aminomethyl)cyclohexyl]amino}-4-(trifluoromethyl)phenyl]-1,3,4-oxadiazol-2(3H)-one,
(38) 5-[3-{[(1r,4r)-4-aminocyclohexyl]amino}-4-(trifluoromethoxy)phenyl]-1,3,4-oxadiazol-2(3H)-one,
(39) 5-[3-{[(1r,4r)-4-(1-aminoethyl)cyclohexyl]amino}-4-(trifluoromethyl)phenyl]-1,3,4-oxadiazol-2(3H)-one,
(40) 5-(3-{[(1r,4r)-4-aminocyclohexyl]amino}-4-chloro-5-fluorophenyl)-1,3,4-oxadiazol-2(3H)-one,
(41) 5-{3-[4-(aminomethyl)anilino]-4-(trifluoromethyl)phenyl}-1,3,4-oxadiazol-2(3H)-one,
(42) 5-{3-[(6-azaspiro[2.5]octan-1-yl)amino]-4-(trifluoromethyl)phenyl}-1,3,4-oxadiazol-2(3H)-one,
(43) 5-{3-[(6-aminospiro[3.3]heptan-2-yl)amino]-4-(trifluoromethyl)phenyl}-1,3,4-oxadiazol-2(3H)-one,
(44) 5-[3-{[(1r,4r)-4-(2-aminoethyl)cyclohexyl]amino}-4-(trifluoromethyl)phenyl]-1,3,4-oxadiazol-2(3H)-one,
(45) 5-[3-{[(1S)-7-azaspiro[3.5]nonan-1-yl]amino}-4-(trifluoromethyl)phenyl]-1,3,4-oxadiazol-2(3H)-one,
(46) 5-[3-{[(1R)-7-azaspiro[3.5]nonan-1-yl]ammo}-4-(trifluoromethyl)phenyl]-1,3,4-oxadiazol-2(3H)-one,
(47) 5-(4-chloro-3-{[(piperidin-4-yl)methyl]amino}phenyl)-1,3,4-oxadiazol-2(3H)-one,
(48) 5-[3-{[(piperidin-4-yl)methyl]amino}-4-(trifluoromethyl)phenyl]-1,3,4-oxadiazol-2(3H)-one,
(49) 5-[4-chloro-3-({[(3R)-pyrrolidin-3-yl]methyl}amino)phenyl]-1,3,4-oxadiazol-2(3H)-one,
(50) 5-(4-bromo-3-{[(piperidin-4-yl)methyl]amino}phenyl)-1,3,4-oxadiazol-2(3H)-one,
(51) 5-[3-{methyl[(piperidin-4-yl)methyl]amino}-4-(trifluoromethyl)phenyl]-1,3,4-oxadiazol-2(3H)-one,
(52) 5-[3-{[1-(piperidin-4-yl)ethyl]amino}-4-(trifluoromethyl)phenyl]-1,3,4-oxadiazol-2(3H)-one,
(53) 5-[3-({[(3S)-piperidin-3-yl]methyl}amino)-4-(trifluoromethyl)phenyl]-1,3,4-oxadiazol-2(3H)-one,
(54) 5-[3-({[(3R)-piperidin-3-yl]methyl}amino)-4-(trifluoromethyl)phenyl]-1,3,4-oxadiazol-2(3H)-one,
(55) 5-[3-({[(1r,4r)-4-aminocyclohexyl]methyl}amino)-4-(trifluoromethyl)phenyl] - 1,3,4-oxadiazol-2(3H)-one,
(56) 5-[3-{[1-(piperidin-4-yl)propyl]amino}-4-(trifluoromethyl)phenyl]-1,3,4-oxadiazol-2(3H)-one,
(57) 5-[3-{[(4-methylpiperidin-4-yl)methyl]amino}-4-(trifluoromethyl)phenyl]-1,3,4-oxadiazol-2(3H)-one,
(58) 5-[3-{[(1S)-1-(piperidin-4-yl)ethyl]amino}-4-(trifluoromethyl)phenyl]-1,3,4-oxadiazol-2(3H)-one,
(59) 5-[3-{[(1R)-1-(piperidin-4-yl)ethyl]amino}-4-(trifluoromethyl)phenyl]-1,3,4-oxadiazol-2(3H)-one,
(60) 5-[3-{[2-(piperidin-3-yl)ethyl]amino}-4-(trifluoromethyl)phenyl]-1,3,4-oxadiazol-2(3H)-one,
(61) 5-[3-({[(1r,4r)-4-aminocyclohexyl]methyl}amino)-4-chlorophenyl]-1,3,4-oxadiazol-2(3H)-one,
(62) 5-[3-({[(1r,4r)-4-aminocyclohexyl]methylamino)phenyl]-1,3,4-oxadiazol-2(3H)-one,
(63) 5-[3-({1-[(1r,4r)-4-aminocyclohexyl]ethyl}amino)-4-(trifluoromethyl)phenyl]-1,3,4-oxadiazol-2(3H)-one,
(64) 5-[3-{[2-(piperidin-4-yl)ethyl]amino}-4-(trifluoromethyl)phenyl]-1,3,4-oxadiazol-2(3H)-one,
(65) 5-[3-{[2-(piperazin-1-yl)ethyl]amino}-4-(trifluoromethyl)phenyl]-1,3,4-oxadiazol-2(3H)-one,
(66) 5-[3-({[(1r,4r)-4-aminocyclohexyl]methyl}amino)-4-(trifluoromethoxy)phenyl]-1,3,4-oxadiazol-2(3H)-one,
(67) 5-[3-({[(1r,4r)-4-aminocyclohexyl]methyl}amino)-4-methylphenyl]-1,3,4-oxadiazol-2(3H)-one,
(68) 5-[3-({[(1r,4r)-4-aminocyclohexyl]methyl}amino)-4-bromophenyl]-1,3,4-oxadiazol-2(3H)-one,
(69) 5-[3-({[(1r,4r)-4-aminocyclohexyl]methyl} amino)-5-fluoro-4-(trifluoromethyl)phenyl]-1,3,4-oxadiazol-2(3H)-one,
(70) 5-[3-{[2-(4-methylpiperidin-4-yl)ethyl]amino}-4-(trifluoromethyl)phenyl]-1,3,4-oxadiazol-2(3H)-one,
(71) 5-[2-({[(1r,4r)-4-aminocyclohexyl]methyl}amino)[1,1'-biphenyl]-4-yl]-1,3,4-oxadiazol-2(3H)-one,
(72) 5-[3-({2-[(3R)-3-aminopiperidin-1-yl]ethyl}amino)-4-(trifluoromethyl)phenyl]-1,3,4-oxadiazol-2(3H)-one,
(73) 5-[3-({2-[(3S)-3-aminopiperidin-1-yl]ethyl}amino)-4-(trifluoromethyl)phenyl]-1,3,4-oxadiazol-2(3H)-one,
(74) 5-[3-({[(1s,4s)-4-aminocyclohexyl]methyl}amino)-4-(trifluoromethyl)phenyl]-1,3,4-oxadiazol-2(3H)-one,
(75) 5-[3-fluoro-5-{[(1S)-1-(piperidin-4-yl)ethyl]amino}-4-(trifluoromethyl)phenyl]-1,3,4-oxadiazol-2(3H)-one,
(76) 5-(4-chloro-3-fluoro-5-{[(1S)-1-(piperidin-4-yl)ethyl]amino}phenyl)-1,3,4-oxadiazol-2(3H)-one,
(77) 5-(4-chloro-3-{[(1S)-1-(piperidin-4-yl)ethyl]amino}phenyl)-1,3,4-oxadiazol-2(3H)-one,
(78) 5-(4-bromo-3-{ [(1S)-1-(piperidin-4-yl)ethyl]amino}phenyl)-1,3,4-oxadiazol-2(3H)-one,
(79) 5-(3,4-dichloro-5-{[(1S)-1-(piperidin-4-yl)ethyl]amino}phenyl)-1,3,4-oxadiazol-2(3H)-one,
(80) 5-(4-fluoro-3-{[(1S)-1-(piperidin-4-yl)ethyl]amino}phenyl)-1,3,4-oxadiazol-2(3H)-one,
(81) 5-[3-{[1-(piperidin-4-yl)propan-2-yl]amino} -4-(trifluoromethyl)phenyl]-1,3,4-oxadiazol-2(3H)-one,
(82) 5-(4-methoxy-3-{[(1S)-1-(piperidin-4-yl)ethyl]amino}phenyl)-1,3,4-oxadiazol-2(3H)-one,
(83) 5-(4-bromo-3-fluoro-5-{[(1 S)-1-(piperidin-4-yl)ethyl]amino}phenyl)-1,3,4-oxadiazol-2(3 H)-one,
(84) 5-(4-chloro-3-methyl-5-{[(1S)-1-(piperidin-4-yl)ethyl]amino}phenyl)-1,3,4-oxadiazol-2(3H)-one,
(85) 4-(5-oxo-4,5-dihydro-1,3,4-oxadiazol-2-yl)-2-{[(1S)-1-(piperidin-4-yl)ethyl]amino}benzonitrile,
(86) 5-[3-({[(1r,4r)-4-aminocyclohexyl]methyl}amino)-4-chloro-5-fluorophenyl]-1,3,4-oxadiazol-2(3H)-one,
(87) 5-(4,5-dichloro-2-fluoro-3-{[(1S)-1-(piperidin-4-yl)ethyl]amino}phenyl)-1,3,4-oxadiazol-2(3H)-one,
(88) 5-(4-chloro-2,5-difluoro-3-{[(1S)-1-(piperidin-4-yl)ethyl]amino}phenyl)-1,3,4-oxadiazol-2(3 H)-one,
(89) 5-(3,4-difluoro-5-{[(1S)-1-(piperidin-4-yl)ethyl]amino}phenyl)-1,3,4-oxadiazol-2(3H)-one,
(90) 5-[4-(difluoromethyl)-3-{[(1S)-1-(piperidin-4-yl)ethyl]amino}phenyl]-1,3,4-oxadiazol-2(3H)-one,
(91) 5-(4-chloro-3-nitro-5-{[(1S)-1-(piperidin-4-yl)ethyl]amino}phenyl)-1,3,4-oxadiazol-2(3H)-one,
(92) 5-(3-amino-4-chloro-5-{[(1S)-1-(piperidin-4-yl)ethyl]amino}phenyl)-1,3,4-oxadiazol-2(3H)-one,
(93) 5-(4,5-dichloro-2-methyl-3-{[(1S)-1-(piperidin-4-yl)ethyl]amino}phenyl)-1,3,4-oxadiazol-2(3H)-one,
(94) 5-(4-chloro-2-methyl-5-{[(1S)-1-(piperidin-4-yl)ethyl]amino}phenyl)-1,3,4-oxadiazol-2(3H)-one,
(95) 5-(4-chloro-2-fluoro-5-{[(1S)-1-(piperidin-4-yl)ethyl]amino}phenyl)-1,3,4-oxadiazol-2(3H)-one,
(96) 5-(2,4-dichloro-5-{[(1S)-1-(piperidin-4-yl)ethyl]amino}phenyl)-1,3,4-oxadiazol-2(3H)-one,
(97) 5-(3-bromo-4-chloro-5-{[(1S)-1-(piperidin-4-yl)ethyl]amino}phenyl)-1,3,4-oxadiazol-2(3H)-one,
(98) 5-(3-chloro-4-methyl-5-{[(1S)-1-(piperidin-4-yl)ethyl]amino}phenyl)-l,3,4-oxadiazol-2(3H)-one,
(99) 5-(3-fluoro-4-methyl-5-{[(1S)-1-(piperidin-4-yl)ethyl]amino}phenyl)-1,3,4-oxadiazol-2(3H)-one,
(100) 5-(4-{[(1S)-1-(piperidin-4-yl)ethyl]amino}-1H-indazol-6-yl)-1,3,4-oxadiazol-2(3H)-one,
(101) 5-[4-chloro-3-{[(1S)-1-(piperidin-4-yl)ethyl]amino}-5-(trifluoromethyl)phenyl]-1,3,4-oxadiazol-2(3H)-one,
(102) 5-(4-chloro-3-cyclopropyl-5-{[(1S)-1-(piperidin-4-yl)ethyl]amino}phenyl)-1,3,4-oxadiazol-2(3H)-one,
(103) 5-(3-chloro-5-{[(1S)-1-(piperidin-4-yl)ethyl]amino}-4-[(propan-2-yl)oxy]phenyl)-1,3,4-oxadiazol-2(3H)-one,
(104) 5-(3-chloro-4-methoxy-5-{[(1S)-1-(piperidin-4-yl)ethyl]amino}phenyl)-1,3,4-oxadiazol-2(3H)-one,
(105) 5-(3-chloro-5-{[(1S)-1-(piperidin-4-yl)ethyl]amino}phenyl)-1,3,4-oxadiazol-2(3H)-one,
(106) 5-[3-({(1S)-1-[(1r,4S)-4-aminocyclohexyl]ethyl}amino)-4-(trifluoromethyl)phenyl]-1,3,4-oxadiazol-2(3H)-one,
(107) 5-[3-(pentafluoro-λ6-sulfanyl)-5-{[(1S)-1-(piperidin-4-yl)ethyl]amino}phenyl]-1,3,4-oxadiazol-2(3H)-one,
(108) 5-[3-{[(4-fluoropiperidin-4-yl)methyl]amino-4-(trifluoromethyl)phenyl]-1,3,4-oxadiazol-2(3H)-one,
(109) 5-[3-{[(3-fluoropiperidin-3-yl)methyl]amino}-4-(trifluoromethyl)phenyl]-1,3,4-oxadiazol-2(3H)-one,
(110) 2-chloro-5-(5-oxo-4,5-dihydro-1,3,4-oxadiazol-2-yl)-3-{[(1S)-1-(piperidin-4-yl)ethyl]amino}benzonitrile,
(111) 5-[3-({(1R)-1-[(1r,4R)-4-aminocyclohexyl]ethyl}amino)-4-(trifluoromethyl)phenyl]-1,3,4-oxadiazol-2(3H)-one,
(112) 5-(2-{[(1S)-1-(piperidin-4-yl)ethyl]amino}pyridin-4-yl)-1,3,4-oxadiazol-2(3H)-one,
(113) 5-[3-{[(1R)-2,2-difluoro-1-(piperidin-4-yl)ethyl]amino}-4-(trifluoromethyl)phenyl]-1,3,4-oxadiazol-2(3H)-one,
(114) 5-[6-{[(1S)-1-(piperidin-4-yl)ethyl)amino}-5-(trifluoromethyl)pyridin-2-yl)-1,3,4-oxadiazol-2(3H)-one,
(115) 5-[3-{[1-(4-methylpiperidin-4-yl)ethyl]amino}-4-(trifluoromethyl)phenyl]-1,3,4-oxadiazol-2(3H)-one,
(116) 5-(4-{[(4-fluoropiperidin-4-yl)methyl]amino}-1H-indazol-6-yl)-1,3,4-oxadiazol-2(3H)-one,
(117) 5-[3-({2-[(1r,4r)-4-aminocyclohexyl]ethyl}amino)-4-(trifluoromethyl)phenyl]-1,3,4-oxadiazol-2(3H)-one,
(118) tert-butyl (1r,4r)-4-{[5-(5-oxo-4,5-dihydro-1,3,4-oxadiazol-2-yl)-2-(trifluoromethyl)anilino]methyl}cyclohexane-1-carboxylate,
(119) (1r,4r)-4-{[5-(5-oxo-4,5-dihydro-1,3,4-oxadiazol-2-yl)-2-(trifluoromethyl)anilino]methyl}cyclohexane-1-carboxylic acid,
(120) 5-[3-({[(1r,4r)-4-(hydroxymethyl)cyclohexyl]methyl}amino)-4-(trifluoromethyl)phenyl]-1,3,4-oxadiazol-2(3H)-one,
(121) (1r,4r)-4-{[5-(5-oxo-4,5-dihydro-1,3,4-oxadiazol-2-yl)-2-(trifluoromethyl)anilino]methyl}cyclohexane-1-carboxamide,
(122) 5-[3-{[(4-ethylpiperidin-4-yl)methyl]amino}-4-(trifluoromethyl)phenyl]-1,3,4-oxadiazol-2(3H)-one,
(123) 5-(4-chloro-3-fluoro-5-{[(4-fluoropiperidin-4-yl)methyl]amino}phenyl)-1,3,4-oxadiazol-2(3 H)-one,
(124) 5-{4-bromo-1-[(piperidin-4-yl)methyl]-1H-indazol-6-yl}-1,3,4-oxadiazol-2(3H)-one,
(125) 5-[3-{[(1S)-1-(4-fluoropiperidin-4-yl)ethyl]amino}-4-(trifluoromethyl)phenyl]-1,3,4-oxadiazol-2(3H)-one,
(126) 5-[3-({[(3S,4R)-3-fluoropiperidin-4-yl]methyl}amino)-4-(trifluoromethyl)phenyl]-1,3,4-oxadiazol-2(3H)-one,
(127) 5-[3-({[(3S,4S)-3-fluoropiperidin-4-yl]methyl}amino)-4-(trifluoromethyl)phenyl]-1,3,4-oxadiazol-2(3H)-one,
(128) 5-[4-(methanesulfonyl)-3-{[(1S)-1-(piperidin-4-yl)ethyl]amino}phenyl]-1,3,4-oxadiazol-2(3H)-one,
(129) 5-[3-fluoro-5-{[(4-fluoropiperidin-4-yl)methyl]amino}-4-(trifluoromethyl)phenyl]-1,3,4-oxadiazol-2(3H)-one,
(130) 5-{3-[{[(1r,4r)-4-aminocyclohexyl]methyl}(methyl)amino]-4-(trifluoromethyl)phenyl}-1,3,4-oxadiazol-2(3H)-one,
(131) 5-[3-({[(1r,4r)-4-(methylamino)cyclohexyl]methyl}amino)-4-(trifluoromethyl)phenyl]-1,3,4-oxadiazol-2(3H)-one,
(132) 5-[3-(methyl{[(1r,4r)-4-(methylamino)cyclohexyl]methyl}amino)-4-(trifluoromethyl)phenyl]-1,3,4-oxadiazol-2(3H)-one,
(133) 5-[3-{[(3-azabicyclo[3.2.1]octan-8-yl)methyl]amino}-4-(trifluoromethyl)phenyl]-1,3,4-oxadiazol-2(3H)-one,
(134) 5-[3-({1-[(3S)-pyrrolidin-3-yl]propan-2-yl}amino)-4-(trifluoromethyl)phenyl]-1,3,4-oxadiazol-2(3H)-one,
(135) 5-[3-({[(1R,3s,5S)-8-azabicyclo[3.2.1]octan-3-yl]methyl}amino)-4-(trifluoromethyl)phenyl]-1,3,4-oxadiazol-2(3H)-one,
(136) 5-[3-({1-[(3R)-pyrrolidin-3-yl]propan-2-yl}amino)-4-(trifluoromethyl)phenyl]-1,3,4-oxadiazol-2(3H)-one,
(137) 5-[3-({(1S)-1-[(1R,3S)-3-amino-2,2-dimethylcyclobutyl]ethyl}amino)-4-(trifluoromethyl)phenyl]-1,3,4-oxadiazol-2(3H)-one,
(138) 5-[3-{[(1S)-1-(piperidin-4-yl)propyl]amino}-4-(trifluoromethyl)phenyl]-1,3,4-oxadiazol-2(3H)-one,
(139) 5-(4-chloro-3-fluoro-5-{[(1S)-1-(piperidin-4-yl)propyl]amino}phenyl)-1,3,4-oxadiazol-2(3 H)-one,
(140) 5-[3-{[(1R)-2,2-difluoro-1-(4-fluoropiperidin-4-yl)ethyl]amino}-4-(trifluoromethyl)phenyl]-1,3,4-oxadiazol-2(3H)-one,
(141) 5-[3-({[(3R,4R)-3-methylpiperidin-4-yl]methyl}amino)-4-(trifluoromethyl)phenyl]-1,3,4-oxadiazol-2(3H)-one,
(142) 5-[3-{[(1S)-1-(7-azaspiro[3.5]nonan-2-yl)ethyl]amino}-4-(trifluoromethyl)phenyl]-1,3,4-oxadiazol-2(3H)-one,
(143) 5-[3-{[(2-methylpiperidin-4-yl)methyl]amino}-4-(trifluoromethyl)phenyl]-1,3,4-oxadiazol-2(3H)-one,
(144) 5-[3-{[(1S)-1-(4-methylpiperidin-4-yl)ethyl]amino}-4-(trifluoromethyl)phenyl]-1,3,4-oxadiazol-2(3H)-one,
(145) 5-[3-({(1S)-1-[(1s,3R)-3-aminocyclobutyl]ethyl}amino)-4-(trifluoromethyl)phenyl]-1,3,4-oxadiazol-2(3H)-one,
(146) 5-[4-(trifluoromethyl)-3-{[(1R)-2,2,2-trifluoro-1-(piperidin-4-yl)ethyl]amino}phenyl]-1,3,4-oxadiazol-2(3H)-one,
(147) 5-[4-(trifluoromethyl)-3-{[(1S)-2,2,2-trifluoro-1-(piperidin-4-yl)ethyl]amino)phenyl]-1,3,4-oxadiazol-2(3H)-one,
(148) 5-[3-({(1S)-1-[(1r,4S)-4-aminocyclohexyl]ethyl}amino)-4-chlorophenyl]-1,3,4-oxadiazol-2(3H)-one,
(149) 5-[3-{[(1S)-1-(2-azaspiro[3.3]heptan-6-yl)ethyl]amino}-4-(trifluoromethyl)phenyl]-1,3,4-oxadiazol-2(3H)-one,
(150) 5-[3-({(1S)-1-[(1R,3S)-3-amino-2,2-dimethylcyclobutyl]ethyl}amino)-5-fluoro-4-(trifluoromethyl)phenyl]-1,3,4-oxadiazol-2(3H)-one,
(151) 5-[3-({(1S)-1-[(1r,4S)-4-aminocyclohexyl]ethyl}amino)-5-fluoro-4-(trifluoromethyl)phenyl]-1,3,4-oxadiazol-2(3H)-one,
(152) 5-[3-{[(1R)-2-fluoro-1-(piperidin-4-yl)ethyl]amino}-4-(trifluoromethyl)phenyl]-1,3,4-oxadiazol-2(3H)-one,
(153) 5-[3-({(1S)-1-[(1S,3R)-3-amino-2,2-dimethylcyclobutyl]ethyl}amino)-4-(trifluoromethyl)phenyl]-l,3,4-oxadiazol-2(3H)-one,
(154) 5-[3-({(1S)-1-[(1r,4S)-4-aminocyclohexyl]ethyl}amino)-4-bromophenyl]-1,3,4-oxadiazol-2(3H)-one,
(155) 5-[3-({(1S)-1-[(1R,5S,8r)-3-azabicyclo[3.2.1]octan-8-yl]ethyl}amino)-4-(trifluoromethyl)phenyl]-1,3,4-oxadiazol-2(3H)-one,
(156) 5-[3-({(1S)-1-[(1R,5S,8s)-3-azabicyclo[3.2.1]octan-8-yl]ethyl}amino)-4-(trifluoromethyl)phenyl]-1,3,4-oxadiazol-2(3H)-one,
(157) 5-[3-{[(1S)-1-(azepan-4-yl)ethyl]amino}-4-(trifluoromethyl)phenyl]-1,3,4-oxadiazol-2(3H)-one,
(158) 5-[3-({(1S)-1-[(1R,3S)-3-amino-2,2-dimethylcyclobutyl]ethyl}amino)-4-chlorophenyl]-1,3,4-oxadiazol-2(3H)-one,
(159) 5-[3-fluoro-5-{[(1S)-1-(4-fluoropiperidin-4-yl)ethyl]amino}-4-(trifluoromethyl)phenyl]-1,3,4-oxadiazol-2(3H)-one,
(160) 5-[3-fluoro-5-{[(1R)-2-fluoro-1-(piperidin-4-yl)ethyl]amino}-4-(trifluoromethyl)phenyl]-1,3,4-oxadiazol-2(3H)-one,
(161) 5-[3-{2-[(2r,5r)-5-amino-1,3-dioxan-2-yl]cyclopropyl}-4-(trifluoromethyl)phenyl]-1,3,4-oxadiazol-2(3H)-one,
(162) 5-[3-{[(1S)-1-(azocan-5-yl)ethyl]amino}-4-(trifluoromethyl)phenyl]-1,3,4-oxadiazol-2(3H)-one,
(163) 5-[3-{[(1R)-2,2-difluoro-1-(4-fluoropiperidin-4-yl)ethyl]amino}-5-fluoro-4-(trifluoromethyl)phenyl]-1,3,4-oxadiazol-2(3H)-one,
(164) 5-[3-({(1S)-1-[(1S,3R)-3-amino-2,2-dimethylcyclobutyl]ethyl}amino)-5-fluoro-4-(trifluoromethyl)phenyl]-1,3,4-oxadiazol-2(3H)-one,
(165) 5-[3-({(1S)-1-[(1s,3R)-3-aminocyclobutyl]ethyl}amino)-5-fluoro-4-(trifluoromethyl)phenyl]-1,3,4-oxadiazol-2(3H)-one,
(166) 5-[3-({[(1R,3S)-3-amino-2,2-dimethylcyclobutyl]methyl}amino)-4-(trifluoromethyl)phenyl]-1,3,4-oxadiazol-2(3H)-one,
(167) 5-[3-({(1S)-1-[(1r,4S)-4-aminocyclohexyl]ethyl}amino)-5-fluoro-4-methoxyphenyl]-1,3,4-oxadiazol-2(3H)-one,
(168) 5-[3-({(1S)-1-[(1S,3R)-3-amino-2,2-dimethylcyclobutyl]ethyl}amino)-4-chlorophenyl]-1,3,4-oxadiazol-2(3H)-one,
(169) 5-[3-({(1S)-1-[(1r,4S)-4-aminocyclohexyl]ethyl}amino)-4-chloro-2,5-difluorophenyl]-1,3,4-oxadiazol-2(3H)-one,
(170) 5-[3-({(1S)-1-[(1r,4S)-4-aminocyclohexyl]ethyl}amino)-5-fluoro-4-(trifluoromethoxy)phenyl]-1,3,4-oxadiazol-2(3H)-one,
(171) 5-[3-({(1S)-1-[(1r,4S)-4-aminocyclohexyl]ethyl}amino)-5-bromo-4-chlorophenyl]-1,3,4-oxadiazol-2(3H)-one,
(172) 5-[3-({(1S)-1-[(1r,4S)-4-aminocyclohexyl]ethyl}amino)-5-fluoro-4-methylphenyl]-1,3,4-oxadiazol-2(3H)-one,
(173) 5-[3-({(1S)-1-[(1r,4S)-4-aminocyclohexyl]ethyl}amino)-4-ethoxy-5-fluorophenyl]-1,3,4-oxadiazol-2(3H)-one,
(174) 5-[3-({(1S)-1-[(1r,4S)-4-aminocyclohexyl]ethyl}amino)-4,5-dichlorophenyl]-1,3,4-oxadiazol-2(3H)-one,
(175) 5-{3-({(1S)-1-[(1r,4S)-4-aminocyclohexyl]ethyl}amino)-5-fluoro-4-[(propan-2-yl)oxy]phenyl}-1,3,4-oxadiazol-2(3H)-one,
(176) 5-[3-({[(1S,3R)-3-amino-2,2-dimethylcyclobutyl]methyl}amino)-4-(trifluoromethyl)phenyl]-1,3,4-oxadiazol-2(3H)-one,
(177) 5-[3-({(1S)-1-[(1r,4S)-4-aminocyclohexyl]ethyl}amino)-4-chloro-5-fluorophenyl]-1,3,4-oxadiazol-2(3H)-one,
(178) 5-[3-({(1S)-1-[(1s,3R)-3-aminocyclobutyl]ethyl}amino)-4-chloro-5-fluorophenyl]-1,3,4-oxadiazol-2(3H)-one,
(179) 5-[3-({(1S)-1-[(1s,3R)-3-aminocyclobutyl]ethyl}amino)-4,5-dichlorophenyl]-1,3,4-oxadiazol-2(3H)-one,
(180) 5-[3-({(1S)-1-[(1s,3R)-3-aminocyclobutyl]ethyl}amino)-5-fluoro-4-(trifluoromethoxy)phenyl]-1,3,4-oxadiazol-2(3H)-one,
(181) 5-[3-({(1S)-1-[(1r,4S)-4-aminocyclohexyl]propyl}amino)-4-(trifluoromethyl)phenyl]-1,3,4-oxadiazol-2(3H)-one,
(182) 5-[3-({(1S)-1-[(1s,3R)-3-aminocyclobutyl]ethyl}amino)-5-bromo-4-chlorophenyl]-1,3,4-oxadiazol-2(3H)-one,
(183) 5-[3-({(1S)-1-[(1s,3R)-3-aminocyclobutyl]ethyl}amino)-4-chloro-5-(trifluoromethyl)phenyl]-1,3,4-oxadiazol-2(3H)-one,
(184) N-[(1S,3R)-2,2-dimethyl-3-1[5-(5-oxo-4,5-dihydro-1,3,4-oxadiazol-2-yl)-2-(trifluoromethyl)anilino]methyl}cyclobutyl]acetamide
(185) 5-[3-{[(1S)-1-(3-aminobicyclo[1.1.1]pentan-1-yl)ethyi]amino}-4-(trifluoromethyl)phenyl]-1,3,4-oxadiazol-2(3H)-one,
(186) 5-[3-({(1S)-1-[(2S,3R)-4-aminocuban-1-yl]ethyl}amino)-4-(trifluoromethyl)phenyl]-1,3,4-oxadiazol-2(3H)-one,
(187) 5-[3-{[(1S)-1-(4-aminobicyclo[2.2.1]heptan-1-yl)ethyl]amino}-4-(trifluoromethyl)phenyl]-1,3,4-oxadiazol-2(3H)-one,
(188) 5-[3-({(S)-1-[(2S,5R)-5-aminotetrahydro-2H-pyran-2-yl]ethyl}amino)-4-(trifluoromethyl)phenyl]-1,3,4-oxadiazol-2(3H)-one,
(189) 5-[3-({(1S)-1-[(1r,4S)-4-(methylamino)cyclohexyl]ethyl}amino)-4-(trifluoromethyl)phenyl]-1,3,4-oxadiazol-2(3H)-one,
(190) 5-[3-fluoro-5-({(1S)-1-[(1r,4S)-4-(methylamino)cyclohexyl]ethyl}amino)-4-(trifluoromethyl)phenyl]-1,3,4-oxadiazol-2(3H)-one,
(191) 5-[3-({(1S)-1-[(1R,3S)-3-aminocyclohexyl]ethyl}amino)-4-(trifluoromethyl)phenyl]-1,3,4-oxadiazol-2(3H)-one,
(192) 5-[3-({(1S)-1-[(1s,3R)-3-(methylamino)cyclobutyl]ethyl}amino)-4-(trifluoromethyl)phenyl]-1,3,4-oxadiazol-2(3H)-one,
(193) 5-[3-({(1S)-1-[(1r,4S)-4-(ethylamino)cyclohexyl]ethyl}amino)-4-(trifluoromethyl)phenyl]-1,3,4-oxadiazol-2(3H)-one,
(194) 5-[3-({(1S)-1-[(1S,3R)-3-aminocyclohexyl]ethyl}amino)-4-(trifluoromethyl)phenyl]-1,3,4-oxadiazol-2(3H)-one,
(195) 5-[3-({(1S)-1-[(1s,3R)-3-(ethylamino)cyclobutyl]ethyl}amino)-4-(trifluoromethyl)phenyl]-1,3,4-oxadiazol-2(3H)-one,
(196) 5-[3-{[(1S)-1-{(1s,3R)-3-[(cyclopropylmethyl)amino]cyclobutyl}ethyl]amino}-4-(trifluoromethyl)phenyl]-1,3,4-oxadiazol-2(3H)-one,
(197) 5-[3-{[(1S)-1-{(1r,4S)-4-[(2,2,2-trifluoroethyl)amino]cyclohexyl}ethyl]amino}-4-(trifluoromethyl)phenyl]-1,3,4-oxadiazol-2(3H)-one,
(198) 5-[3-({(1S)-1-[(1r,4S)-4-aminocyclohexyl]propyl}amino)-5-fluoro-4-(trifluoromethyl)phenyl]-1,3,4-oxadiazol-2(3H)-one,
(199) 5-[3-({(1S)-1-[(1S,3R)-3-aminocyclopentyl]ethyl}amino)-4-(trifluoromethyl)phenyl]-1,3,4-oxadiazol-2(3H)-one,
(200) 5-[3-({(1S)-1-[(1R,3S)-3-aminocyclopentyl]ethyl}amino)-4-(trifluoromethyl)phenyl]-1,3,4-oxadiazol-2(3H)-one,
(201) 5-[3-{[(1S)-1-{(1r,4S)-4-[(2-hydroxyethyl)amino]cyclohexyl}ethyl]amino}-4-(trifluoromethyl)phenyl]-1,3,4-oxadiazol-2(3H)-one,
(202) 5-[3-fluoro-5-{[(1S)-1-(piperidin-4-yl)ethyl]amino}-4-(trifluoromethoxy)phenyl]-1,3,4-oxadiazol-2(3H)-one,
(203) 5-[3-fluoro-5-({(1S)-1-[(1s,3R)-3-(methylamino)cyclobutyl]ethyl}amino)-4-(trifluoromethyl)phenyl]-1,3,4-oxadiazol-2(3H)-one,
(204) 5-[3-fluoro-5-({(1S)-1-[(1r,4S)-4-(methylamino)cyclohexyl]ethyl}amino)-4-(trifluoromethoxy)phenyl]-1,3,4-oxadiazol-2(3H)-one,
(205) 5-(4-chloro-3-{[methyl(piperidin-4-yl)amino]methyl}phenyl)-1,3,4-oxadiazol-2(3H)-one,
(206) 5-[3-fluoro-5-{[(1S)-1-(4-methylpiperidin-4-yl)ethyl]amino}-4-(trifluoromethyl)phenyl]-1,3,4-oxadiazol-2(3H)-one,
(207) 5-[3-fluoro-5-{[(1S)-1-(4-methylpiperidin-4-yl)ethyl]amino}-4-(trifluoromethoxy)phenyl]-1,3,4-oxadiazol-2(3H)-one,
(208) 5-[3-fluoro-5-({(1S)-1-[(1s,3R)-3-(methylamino)cyclobutyl]ethyl}amino)-4-(trifluoromethoxy)phenyl]-1,3,4-oxadiazol-2(3H)-one,
(209) 5-[3-{[(1S)-2-fluoro-1-(4-fluoropiperidin-4-yl)ethyl]amino}-4-(trifluoromethyl)phenyl]-1,3,4-oxadiazol-2(3H)-one,
(210) 5-[3-fluoro-5-({(1S)-1-[(3S,4S)-3-methylpiperidin-4-yl]ethyl}amino)-4-(trifluoromethyl)phenyl]-1,3,4-oxadiazol-2(3H)-one,
(211) 5-[3-fluoro-5-({(1S)-1-[(3R,4R)-3-methylpiperidin-4-yl]ethyl}amino)-4-(trifluoromethyl)phenyl]-1,3,4-oxadiazol-2(3H)-one,
(212) 5-[4-(difluoromethoxy)-3-fluoro-5-{[(1S)-1-(piperidin-4-yl)ethyl]amino}phenyl]-1,3,4-oxadiazol-2(3H)-one,
(213) 5-[4-(difluoromethoxy)-3-fluoro-5-{[(1S)-1-(4-methylpiperidin-4-yl)ethyl]amino}phenyl]-1,3,4-oxadiazol-2(3H)-one,
(214) 5-[4-(difluoromethoxy)-3-fluoro-5-({(1S)-1-[(3S,4S)-3-methylpiperidm-4-yl]ethyl}amino)phenyl]-1,3,4-oxadiazol-2(3H)-one,
(215)5-(3-{[(1-acetylpiperidin-4-yl)(methyl)amino]methyl}-4-chlorophenyl)-1,3,4-oxadiazol-2(3H)-one,
(216) 5-[4-(difluoromethoxy)-3-fluoro-5-({(1S)-1-[(2R,4R)-2-methylpiperidin-4-yl]ethyl}amino)phenyl]-1,3,4-oxadiazol-2(3H)-one,
(217) 5-[3-({(1S)-1-[(3S,4S)-3-methylpiperidin-4-yl]ethyl}amino)-4-(trifluoromethyl)phenyl]-1,3,4-oxadiazol-2(3H)-one,
(218) 5-[3-(((1S)-1-[(3S,4S)-3-methylpiperidin-4-yl]ethyl}amino)-4-(trifluoromethoxy)phenyl]-1,3,4-oxadiazol-2(3H)-one,
(219) 5-[3-fluoro-5-({(1S)-1-[(3S,4S)-3-methylpiperidin-4-yl]ethyl}amino)-4-(trifluoromethoxy)phenyl]-1,3,4-oxadiazol-2(3H)-one,
(220) 5-[4-(difluoromethoxy)-3-({(1S)-1-[(3S,4S)-3-methylpiperidin-4-yl]ethyl}amino)phenyl]-1,3,4-oxadiazol-2(3H)-one,
(221) 5-[4-chloro-3-({(1S)-1-[(3S,4S)-3-methylpiperidin-4-yl]ethyl}amino)phenyl]-1,3,4-oxadiazol-2(3H)-one,
(222) 5-[4-chloro-3-fluoro-5-({(1S)-1-[(3S,4S)-3-methylpiperidin-4-yl]ethyl)amino)phenyl]-1,3,4-oxadiazol-2(3H)-one,
(223) 5-[3-fluoro-5-({(1S)-1-[(3S,4R)-3-fluoropiperidin-4-yl]ethyl}amino)-4-(trifluoromethyl)phenyl]-1,3,4-oxadiazol-2(3H)-one,
(224) 5-[3-fluoro-5-({(1S)-1-[(3S,4R)-3-fluoropiperidin-4-yl]ethyl}amino)-4-(trifluoromethoxy)phenyl]-1,3,4-oxadiazol-2(3H)-one,
(225) 5-[3-({(1S)-1-[(3S,4S)-3-ethylpiperidin-4-yl]ethyl}amino)-5-fluoro-4-(trifluoromethyl)phenyl]-1,3,4-oxadiazol-2(3H)-one,
(226) 5-[3-({(1S)-1-[(3R,4R)-3-ethylpiperidin-4-yl]ethyl}amino)-5-fluoro-4-(trifluoromethyl)phenyl]-1,3,4-oxadiazol-2(3H)-one,
(227) 5-[3-fluoro-5-({(1S)-1-[(3S,4R)-3-fluoropiperidin-4-yl]ethyl}amino)-4-methylphenyl]-1,3,4-oxadiazol-2(3H)-one,
(228) 5-[3-fluoro-4-methyl-5-({(1S)-1-[(3S,4S)-3-methylpiperidin-4-yl]ethyl}amino)phenyl]-1,3,4-oxadiazol-2(3H)-one,
(229) 5-[3-({(1S)-1-[(3S,4S)-3-ethylpiperidin-4-yl]ethyl}amino)-5-fluoro-4-methylphenyl]-1,3,4-oxadiazol-2(3H)-one,
(230) 5-[3-({(1S)-1-[(1r,4S)-4-aminocyclohexyl]ethyl}amino)-4-cyclopropyl-5-fluorophenyl]-1,3,4-oxadiazol-2(3H)-one,
(231) 5-[3-({(1S)-1- [(1r,4S)-4-aminocyclohexyl]ethyl}amino)-4-ethyl-5-fluorophenyl]-1,3,4-oxadiazol-2(3H)-one,
(232) 5-[3-({(1S)-1-[(1r,4S)-4-aminocyclohexyl]ethyl}amino)-5-fluoro-4-(prop-1-en-2-yl)phenyl]-1,3,4-oxadiazol-2(3H)-one,
(233) 5-[3-({(1S)-1-[(1r,4S)-4-aminocyclohexyl]ethyl}amino)-5-fluoro-4-(propan-2-yl)phenyl]-1,3,4-oxadiazol-2(3H)-one,
(234) 5-[3-({[(1r,4r)-4-aminocyclohexyl]methyl}sulfanyl)-4-(trifluoromethyl)phenyl]-1,3,4-oxadiazol-2(3H)-one,
(235) 5-{4-bromo-3-[(piperidin-4-yl)methoxy]phenyl}-1,3,4-oxadiazol-2(3H)-one,
(236) 5-{4-bromo-3-[1-(piperidin-4-yl)ethoxy]phenyl}-1,3,4-oxadiazol-2(3H)-one,
(237) 5-(3-{1-[(1r,4r)-4-aminocyclohexyl]ethoxy}-4-bromophenyl)-1,3,4-oxadiazol-2(3H)-one,
(238) 5-(3-{[(1r,4r)-4-aminocyclohexyl]methoxy}-4-bromophenyl)-1,3,4-oxadiazol-2(3H)-one,
(239) 5-{3-[1-(piperidin-4-yl)ethoxy]-4-(trifluoromethyl)phenyl}-1,3,4-oxadiazol-2(3H)-one,
(240) 5-[3-{[(1r,4r)-4-aminocyclohexyl]methoxy}-4-(trifluoromethyl)phenyl]-1,3,4-oxadiazol-2(3H)-one,
(241) 5-(3-{[(1r,4r)-4-aminocyclohexyl]methoxy}-4-chlorophenyl)-1,3,4-oxadiazol-2(3H)-one,
(242) 5-(4-chloro-3-{[(1s,3s)-3-(piperazin-1-yl)cyclobutyl]methoxy}phenyl)-1,3,4-oxadiazol-2(3H)-one,
(243) 5-(3 - {[(1s,4s)-4-aminocyclohexyl]methoxy} -4-chlorophenyl)-1,3 ,4-oxadiazol-2(3H)-one,
(244) 5-(1-{[(1r,4r)-4-aminocyclohexyl]methyl}-1,2,3,4-tetrahydroquinolin-7-yl)-1,3,4-oxadiazol-2(3H)-one,
(245) 5-fluoro-1-{(1S)-1-[(3S,4S)-3-methylpiperidin-4-yl]ethyl}-7-(5-oxo-4,5-dihydro-1,3 ,4-oxadiazol-2-yl)-2,3-dihydroquinolin-4(1H)-one,
(246) 5-[(4E)-5-fluoro-4-(methoxyimino)-1-{(1S)-1-[(3S,4S)-3-methylpiperidin-4-yl] ethyl}-1,2,3,4-tetrahydroquinolin-7-yl]-1,3,4-oxadiazol-2(3H)-one,
(247) 5-(4-{(1S)-1-[(1r,4S)-4-aminocyclohexyl]ethyl}-3,4-dihydro-2H-1,4-benzoxazin-6-yl)-1,3,4-oxadiazol-2(3H)-one,
(248) 5-{8-fluoro-4-[(1S)-1-(piperidin-4-yl)ethyl]-3,4-dihydro-2H-1,4-benzoxazin-6-yl}-1,3,4-oxadiazol-2(3H)-one,
(249) 5-(4-{(1S)-1-[(1r,4S)-4-aminocyclohexyl]ethyl}-8-fluoro-3,4-dihydro-2H-1,4-benzoxazin-6-yl)-1,3,4-oxadiazol-2(3H)-one,
(250) 5-{(2R)-8-fluoro-2-methyl-4-[(1S)-1-(piperidin-4-yl)ethyl]-3,4-dihydro-2H-1,4-benzoxazin-6-yl}-1,3,4-oxadiazol-2(3H)-one,
(251) 5-{(2S)-8-fluoro-2-methyl-4-[(1S)-1-(piperidin-4-yl)ethyl]-3,4-dihydro-2H-1,4-benzoxazin-6-yl}-1,3,4-oxadiazol-2(3H)-one,
(252) 5-[(2S)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one,
(253) 5-[(2R)-4-{(1S)-1-[(1r,4S)-4-aminocyclohexyl]ethyl}-8-fluoro-2-methyl-3,4-dihydro-2H-1,4-benzoxazin-6-yl]-1,3,4-oxadiazol-2(3H)-one,
(254) 5-{9-fluoro-5-[(1S)-1-(piperidin-4-yl)ethyl]-2,3,4,5-tetrahydro-1,5-benzoxazepin-7-yl}-1,3,4-oxadiazol-2(3H)-one.

12. A pharmaceutical composition comprising the 1,3,4-oxadiazolone compound according to any one of claims 1 to 11, or a pharmaceutically acceptable salt thereof, or a solvate thereof, as an active ingredient.

13. A PIM kinase inhibitor comprising the 1,3,4-oxadiazolone compound according to any one of claims 1 to 11, or a pharmaceutically acceptable salt thereof, or a solvate thereof, as an active ingredient.

14. A therapeutic agent for multiple sclerosis, rheumatoid arthritis, food allergy, asthma, systemic lupus erythematosus, lupus nephritis, inflammatory bowel disease, ulcerative colitis, atopic dermatitis, autoimmune lymphoproliferative syndrome, chronic obstructive pulmonary disease, allergic airway disease, eosinophilic polyangiitis granulomatosis, hypereosinophilic syndrome, chorioamnionitis, ankylosing spondylitis, myasthenia gravis, psoriasis, prostate cancer, colon cancer, esophageal cancer, ovarian cancer, uterine cancer, renal cancer, liver cancer, pancreatic cancer, gastric cancer, breast cancer, lung cancer, head and neck cancer, glioma, osteosarcoma, bladder cancer, acute lymphocytic leukemia, acute myeloid leukemia, chronic lymphocytic leukemia, chronic myeloid leukemia, B-cell lymphoma, multiple myeloma, T-cell lymphoma, skin cancer, Kaposi's sarcoma, Hodgkin's lymphoma, myeloproliferative tumor, adenoid cystic carcinoma, Ewing's sarcoma, adult T-cell leukemia, mesothelioma, acute promyelocytic leukemia, choriocarcinoma, liposarcoma, neuroblastoma, seminoma, or lymphoblastic lymphoma, Epstein-Barr virus infection, hemophagocytic syndrome in which Epstein-Barr virus is known to be involved, influenza, hepatitis C, salmonellosis, herpesvirus infection, vaginal trichomonas infection, human granulocytic ehrlichiosis, aplastic anemia, atherosclerosis, pulmonary hypertension, diabetes, enlarged prostate, or Alzheimer's disease in all of which PIM kinase is involved, the therapeutic agent comprising the 1,3,4-oxadiazolone compound according to any one of claims 1 to 13, or a pharmaceutically acceptable salt thereof, or a solvate thereof, as an active ingredient.
